# FASCICULE DE BREVET EUROPEEN

(11) **EP 1 467 974 B1**
(45) Date de publication et mention de la délivrance du brevet: **15.08.2007**
(21) Numéro de dépôt: 02805404.7
(22) Date de dépôt: 20.12.2002
(51) Int. Cl.: C07D 235/28, C07D 235/30, C07D 401/12, C07D 409/14, C07D 401/14, C07D 405/12, C07D 401/06, C07D 409/12, C07D 405/14, C07D 403/12, C07D 413/12, C07D 401/04, C07D 403/14, C07D 471/04, C07D 417/06

(54) **DERIVES DE BENZIMIDAZOLE ET LEUR UTILISATION COMME ANTAGONISTES DE GNRH**
BENZIMIDAZOLDERIVATE UND DEREN VERWENDUNG ALS GNRH-ANTAGONISTEN
BENZIMIDAZOLE DERIVATIVES AND THEIR USE AS GNRH ANTAGONISTS

(30) Priorité: 21.12.2001 FR 0116647
(43) Date de publication de la demande: 20.10.2004
(73) Titulaire: SOCIETE DE CONSEILS DE RECHERCHES ET D'APPLICATIONS SCIENTIFIQUES S.A., F-75016 Paris (FR)
(72) Inventeur: POITOUT, Lydie, F-94270 Le Kremlin Bicêtre (FR); BRAULT, Valérie, F-91190 Gif-sur-Yvette (FR); FERRANDIS, Eric, F-78470 Saint-Rémy-les-Chevreuse (FR); THURIEAU, Christophe, F-75116 Paris (FR)
(74) Mandataire: Bourgouin, André
(86) Numéro de dépôt international: PCT/FR2002/004477
(87) Numéro de publication internationale: WO 2003/053939

(56) Documents cités:
- WO-A-97/21435
- DATABASE WPI Section Ch, Week 200030 Derwent Publications Ltd., London, GB; Class B02, AN 2000-342515 XP002210484 & JP 2000 095767 A (TAKEDA CHEM IND LTD), 4 avril 2000 (2000-04-04)

## Description

La présente demande a pour objet de nouveaux dérivés de benzimidazoles (amino- et thio- benzimidazoles). Ces produits possèdent une activité antagoniste de la GnRH (Gonadotropin-Releasing Hormone). L'invention concerne également des compositions pharmaceutiques contenant lesdits produits et leur utilisation pour la préparation d'un médicament.

GnRH (Gonadotropin-Releasing Hormone), appelé également LHRH (Luteinizing-Hormone-Releasing Hormone) est un décapeptide hypothalamique (pGlu-His-Trp-Ser-Tyr-Gly-Leu-Arg-Pro-Gly-NH₂) qui régule le système de reproduction chez les vertébrés. Il est déversé dans les capillaires du système porte hypothalamo-hypophysaire de l'éminence médiane et de la tige pituitaire. Par ce réseau il gagne l'hypophyse antérieure et atteint, par un deuxième réseau capillaire, la cellule cible gonadotrope. GnRH agit au niveau de la membrane des cellules cibles, par l'intermédiaire de récepteurs à sept segments transmembranaires couplés à la phospholipase C par l'intermédiaire de protéines G conduisant à une élévation du flux calcique intracellulaire. Son action induit la biosynthèse et la libération des hormones gonadotropes FSH (follicle-stimulating hormone) et LH (luteinizing hormone). Les agonistes et antagonistes de GnRH se sont révélés efficaces chez la femme dans le traitement de l'endométriose, le fibrome, le syndrome des ovaires polykystiques, le cancer du sein, de l'ovaire et de l'endomètre, la désensibilisation hypophysaire gonadotrope lors des protocoles de procréation médicalement assistée ; chez l'homme, dans le traitement de l'hyperplasie bénigne de la prostate et le cancer de la prostate ; et dans le traitement de la puberté précoce masculine ou féminine.

Les antagonistes de GnRH actuellement utilisés sont des composés peptidiques qui doivent être généralement administrés par voie intraveineuse ou sous-cutanée en raison de leur faible bio-disponibilité orale. Les antagonistes non peptidiques de GnRH, qui présentent quant à eux l'avantage de pouvoir être administrés par voie orale, font l'objet de nombreux efforts de recherche. Par exemple, des composés non-peptidiques antagonistes de GnRH ont été décrits dans J. Med. Chem, 41, 4190-4195 (1998), Bioorg. Med. Chem. Lett, 11, 2597-2602 (2001) et la demande WO 97/21435 ; cette dernière décrit des dérivés de l'indole.

La présente invention concerne une nouvelle famille de composés non peptidiques antagonistes de GnRH.

L'invention a donc pour objet un composé de formule générale **(I)** 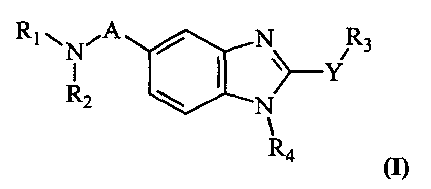 sous forme racémique, d'énantiomère ou toutes combinaisons de ces formes et dans laquelle :
A représente -CH₂- ou -C(O)- ;
Y représente -S- ou -NH- ;
R₁ et R₂ représentent, indépendamment, l'atome d'hydrogène, un radical (C₁-C₈)alkyle, un (C₅-C₉)bicyaoalkyle éventuellement substitué par un ou plusieurs radicaux (C₁-C₆)alkyle identiques ou différents, ou un radical de formule -(CH₂)ₙ-X dans laquelle
   X représente, amino, (C₁-C₆)alkylamino, di((C₁-C₆)alkylamino, (C₃-C₇)cycloalkyle, adamantyle, hétérocycloalkyle, aryle, aryl-carbonyle ou hétéroaryle, ou bien un radical de formule 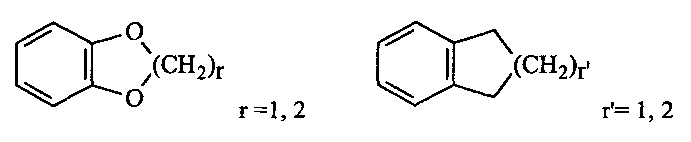 les radicaux (C₃-C₇)cycloalkyle, hétérocycloalkyle, aryle et hétéroaryle étant éventuellement substitués par un ou plusieurs substituants identiques ou différents choisis parmi : - (CH₂)_{n'}-X'-Y', halo, oxo, nitro, cyano, amino, (C₁-C₆)alkylamino et di((C₁-C₈)alkyl)amino, hydroxy, N₃ ;
   X' représente -O-, -S-, -C(O)-, -C(O)-O-, -NH-C(O)-, -NH-SO₂- ou une liaison covalente ;
   Y' représente un radical (C₁-C₆)alkyle éventuellement substitué par un ou plusieurs radicaux halo identiques ou différents ; hétéroaryle ou aryle ou hétérocycloalkyle éventuellement substitué par un ou plusieurs substituants identiques ou différents choisis parmi : (C₁-C₆)alkyle, (C₁-C₆)alkoxy, halo, nitro, cyano, amino, CF₃, OCF₃, hydroxy, N₃, (C₁-C₆)alkylamino et di((C₁-C₈)alkyl)amino ;
   n représente un entier de 0 à 6 et n' un entier de 0 à 2 ;
   ou bien R₁ et R₂ forment ensemble, avec l'atome d'azote auquel ils sont rattachés, un hétérocycloalkyle, un hétérobicycloalkyle ou un radical de formule : 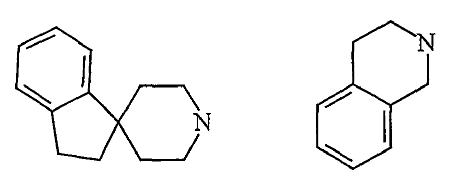 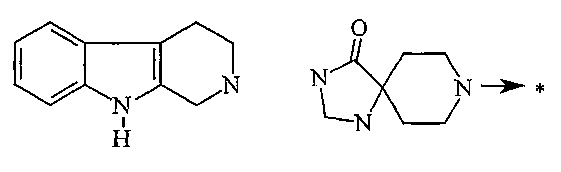 le radical que forment ensemble R₁ et R₂ étant éventuellement substitué par un ou plusieurs substituants identiques ou différents choisis parmi :
   -(CH₂)_{n"}-X"-Y" oxo , hydroxy, halo, nitro, cyano;
   X" représente -O-, -C(O)-, -C(O)-O- ou une liaison covalente ;
   Y" représente un radical (C₁-C₆)alkyle, amino, (C₁-C₆)alkylamino, di((C₁-C₆)alkyl)amino, (C₃-C₇)cycloalkyle, hétérocycloalkyle, arylalkyle, ou aryle ou hétéroaryle éventuellement substitué par un ou plusieurs substituants identiques ou différents choisis parmi : (C₁-C₆)alkyle, (C₁-C₆)alkoxy, (C₁-C₆)alkyl-carbonyle, halo, hydroxy, nitro, cyano, CF₃, OCF₃, amino, (C₁-C₆)alkylamino et di((C₁-C₆)alkyl)amino) ; ou bien un radical de formule 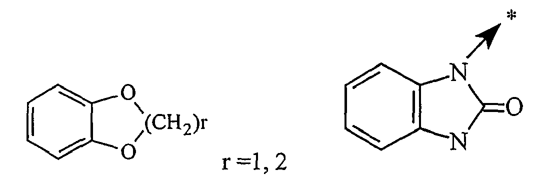
   n" représente un entier de 0 à 4 ;

- R₃: représente -(CH₂)ₚ-W₃-(CH₂)_{p'}-Z₃
W₃ représente une liaison covalente, -CH(OH)- ou -C(O)- ;
Z₃ représente un radical (C₁-C₆)alkyle, adamantyle, aryle, un hétéroaryle, ou bien un radical de formule 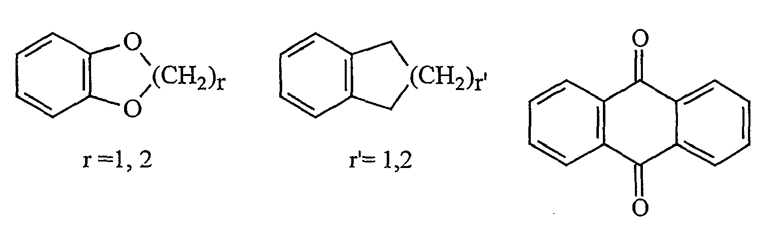 le radical aryle étant éventuellement substitué par un ou plusieurs substituants identiques ou différents choisis parmi : -(CH₂)_{p"}-V₃-Y₃, halo, nitro, cyano, N₃, hydroxy ;
V₃ représente -O-, -S-, -C(O)-, -C(O)-O-, -SO₂- ou une liaison covalente ;
Y₃ représente un radical (C₁-C₆)alkyle éventuellement substitué par un ou plusieurs radicaux halo identiques ou différents, amino, (C₁-C₆)alkylamino, di((C₁-C₆)alkyl)amino, phénylcarbonylméthyle, hétérocycloalkyle ou aryle ;
p, p' et p" représentent, indépendamment, un entier de 0 à 4 ;
- R₄: représente un radical de formule-(CH₂)ₛ-R"₄
R"₄ représente un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle ; ou un radical de formule -NW₄W'₄
W₄ représente l'atome d'hydrogène, (C₁-C₈)alkyle ou (C₃-C₇)cycloalkyle ;
W'₄ représente un radical de formule -(CH₂)_{s'}-Q₄-Z₄ ;
Q₄ représente une liaison covalente, -CH₂-CH(OH)-[CH₂]ₜ-[O]_{t'}-[CH₂]_{t"}-ou -C(O)-O-;
t, t' et t" représentent, indépendamment, 0 ou 1 ;
Z₄ représente l'atome d'hydrogène, (C₁-C₈)alkyle éventuellement substitué par un ou plusieurs substituents identiques ou différents choisis parmi : (C₁-C₆)alkoxy, (C₁-C₆)alkylthio, (C₁-C₆)alkyldithio et hydroxy ; (C₂-C₆)alkenyle ; (C₂-C₆)alkynyle ; (C₃-C₇)cycloalkyle éventuellement substitué par un ou plusieurs substituents identiques ou différents choisis parmi : (C₁-C₆)alkyle, (C₁-C₆)alkoxycarbonyle et (C₁C₆)hydroxyalkyle; cyclohexène ; adamantyle ; hétéroaryle ; aryle éventuellement substitué par un ou plusieurs radicaux identiques ou différents choisis de formule -(CH₂)_{q"}-V₄-Y₄, hydroxy, halo, nitro, cyano ;
V₄ représente -O-, -S-, -NH-C(O)- ou une liaison covalente ;
Y₄ représente un radical (C₁-C₆)alkyle éventuellement substitué par di((C₁-C₆)alkyl)amino ou un ou plusieurs radicaux halo identiques ou différents ; amino ; (C₁-C₆)alkylamino ; di((C₁-C₆)alkyl)amino; aralkyle ; hétérocycloalkyle;
q" représente un entier de 0 à 4 ;
ou bien Z₄ représente un radical de formule 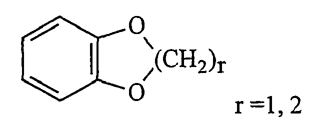 s et s' représentent, indépendamment, un entier de 0 à 6 ;
ou un sel pharmaceutiquement acceptable de ce dernier.

Dans les définitions indiquées ci-dessus, l'expression halo représente le radical fluoro, chloro, bromo ou iodo, de préférence chloro, fluoro ou bromo. L'expression alkyle (lorsqu'il n'est pas donné plus de précision), représente de préférence un radical alkyle ayant de 1 à 6 atomes de carbone, linéaire ou ramifié, tels que les radicaux méthyle, éthyle, propyle, isopropyle, butyle, isobutyle, *sec*-butyle et *tert*-butyle, pentyle ou amyle, isopentyle, neopentyle, 2,2-diméthyl-propyle, hexyle, isohexyle ou 1,2,2-triméthylpropyle. Le terme (C₁-C₈)alkyle désigne un radical alkyle ayant de 1 à 8 atomes de carbone, linéaire ou ramifié, tels les radicaux contenant de 1 à 6 atomes de carbone tels que définis ci-dessus mais également heptyle, octyle, 1,1,2,2-tétraméthyl-propyle, 1,1,3,3-tétraméthyl-butyle. Le terme alkyl-carbonyle désigne de préférence les radicaux dans lesquels le radical alkyle est tel que défini ci-dessus comme par exemple méthylcarbonyle, éthylcarbonyle. Le terme hydroxyalkyle désigne les radicaux dans lesquels le radical alkyle est tel que défini ci-dessus comme par exemple hydroxyméthyle, hydroxyéthyle.

Par alkényle, lorsqu'il n'est pas donné plus de précision, on entend un radical alkyle linéaire ou ramifié comptant de 1 à 6 atomes de carbone et présentant au moins une insaturation (double liaison), comme par exemple vinyle, allyle, propényle, butènyle ou pentènyle. Par alkynyle, lorsqu'il n'est pas donné plus de précision, on entend un radical alkyle linéaire ou ramifié comptant de 1 à 6 atomes de carbone et présentant au moins une double insaturation (triple liaison) comme par exemple un radical éthynyle, propargyle, butynyle ou pentynyle.

Le terme alkoxy désignent les radicaux dans lesquels le radical alkyle est tel que défini ci-dessus comme par exemple les radicaux méthoxy, éthoxy, propyloxy ou isopropyloxy mais également butoxy linéaire, secondaire ou tertiaire, pentyloxy. Le terme alkoxycarbonyle désigne de préférence les radicaux dans lesquels le radical alkoxy est tel que défini ci-dessus comme par exemple méthoxycarbonyle, éthoxycarbonyle. Le terme alkylthio désigne les radicaux dans lesquels le radical alkyle est tel que défini ci-dessus comme par exemple méthylthio, éthylthio. Le terme alkyldithio désigne de préférence les radicaux dans lesquels le radical alkyle est tel que défini ci-dessus comme par exemple méthyldithio (CH₃-S-S-), éthyldithio ou propyldithio.

Le terme (C₃-C₇)cycloalkyle désigne un système monocyclique carboné saturé comprenant de 3 à 7 atomes de carbone, et de préférence les cycles cyclopropyle, cyclobutyle, cyclopentyle, cyclohexyle ou cycloheptyle. L'expression hétérocycloalkyle désigne un système saturé monocyclique ou bicyclique condensé contenant de 2 à 7 atomes de carbone et au moins un hétéroatome. Ce radical peut contenir plusieurs hétéroatomes identiques ou différents. De préférence, les hétéroatomes sont choisis parmi l'oxygène, le soufre ou l'azote. Comme exemple d'hétérocycloalkyle, on peut citer les cycles contenant au moins un atome d'azote tels que pyrrolidine, imidazolidine, pyrrazolidine, isothiazolidine, thiazolidine, isoxazolidine, oxazolidine, pipéridine, pipérazine, azépane, diazépane, morpholine, décahydroisoquinoline mais également les cycles ne contenant aucun atome d'azote tels tétrahydrofurane ou tétrahydrothiophène.

Le terme (C₅-C₉)bicycloalkyle désigne un système bicyclique hydrocarboné saturé non condensé contenant de 5 à 9 atomes de carbone, tel que bicyclo-heptane comme par exemple bicylo[2,2,1]heptane, ou bicyclo-octane comme par exemple bicyclo[2,2,2]octane ou bicylo[3,2,1]octane. Le terme hétérobicycloalkyle désigne un système bicyclique hydrocarboné saturé non condensé contenant de 5 à 8 atomes de carbone et au moins un hétéroatome choisi parmi l'azote, l'oxygène et le soufre. Comme exemple d'hétérobicycloalkyle, on peut citer les aza-bicycloheptane et aza-bicyclooctane tels que 7-aza-bicyclo[2,2,1]heptane, 2-aza-bicyclo [2,2,2] octane ou 6-aza-bicyclo[3,2,1]octane.

L'expression aryle représente un radical aromatique, constitué d'un cycle ou de cycles condensés, comme par exemple le radical phényle, naphtyle ou fluorényle. L'expression hétéroaryle désigne un radical aromatique, constitué d'un cycle ou de cycles condensés, avec au moins un cycle contenant un ou plusieurs hétéroatomes identiques ou différents choisis parmi le soufre, l'azote ou l'oxygène. Comme exemple de radical hétéroaryle, on peut citer les radicaux contenant au moins un atome d'azote tels que pyrrolyle, imidazolyle, pyrazolyle, isothiazolyle, thiazolyle, isoxazolyle, oxazolyle, triazolyle, thiadiazolyle, pyridyle, pyrazinyle, pyrimidyle, quinolyle, isoquinolyle, quinoxalinyle, indolyle, benzoxadiazoyle, carbazolyle mais également les radicaux ne contenant pas d'atome d'azote tels que thiényle, benzothiényle, furyle, benzofuryle ou pyranyle.

Le terme aralkyle (arylalkyle) désigne de préférence les radicaux dans lesquels le radical aryle et alkyle sont tels que définis ci-dessus ; comme exemple de arylalkyle, on peut citer benzyle, phenéthyle, phénylpropyle et phénylbutyle. Le terme aryl-carbonyle désigne de préférence les radicaux dans lesquels le radical aryle est tel que défini ci-dessus, comme par exemple phénylcarbonyle.

Les termes alkylamino et dialkylamino désignent de préférence les radicaux dans lesquels les radicaux alkyle sont tels que définis ci-dessus, comme par exemple méthylamino, éthylamino, diméthylamino, diéthylamino ou (méthyl)(éthyl)amino.

Dans la présente demande également, le radical (CH₂)ᵢ (i entier pouvant représenter n, n', n", p, p', p", s, s', s" et q"" tels que définis ci-dessus), représente une chaîne hydrocarbonée, linéaire ou ramifiée, de i atomes de carbone.

L'invention a pour objet également un composé de formule générale **(I')** 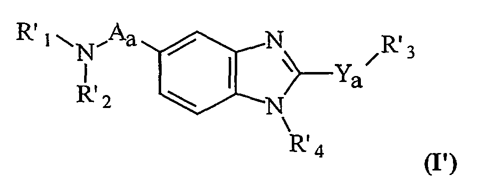 sous forme racémique, d'énantiomère ou toutes combinaisons de ces formes et dans laquelle :
Aₐ représente -CH₂- ou -C(O)-;
Yₐ représente -S- ou -NH- ;
R'₁ et R'₂ représentent, indépendamment, l'atome d'hydrogène, un radical (C₁-C₈)alkyle, un (C₅-C₉)bicycloalkyle éventuellement substitué par un ou plusieurs radicaux (C₁-C₆)alkyle identiques ou différents, ou un radical de formule - (CH₂)ₙ-X dans laquelle
   X représente, amino, (C₁-C₆)alkylamino, di((C₁-C₆)alkyl)amino,
   (C₃-C₇)cycloalkyle, adamantyle, hétérocycloalkyle, aryle, aryl-carbonyle ou hétéroaryle, ou bien un radical de formule 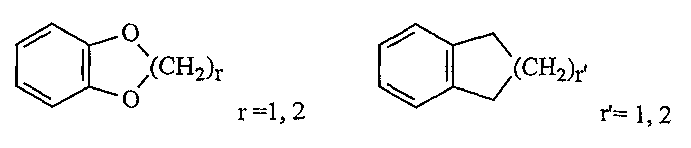 les radicaux (C₃-C₇)cycloalkyle, hétérocycloalkyle, aryle et hétéroaryle étant éventuellement substitués par un ou plusieurs substituants identiques ou différents choisis parmi : - (CH₂)_{n'}-X'-Y', halo, oxo, nitro, cyano, amino, (C₁-C₆)alkylamino et di((C₁-C₈)alkyl)amino, hydroxy, N₃ ;
   X' représente -O-, -S-, -C(O)-, -C(O)-O-, -NH-C(O)-, -NH-SO₂- ou une liaison covalente ;
   Y' représente un radical (C₁-C₆)alkyle éventuellement substitué par un ou plusieurs radicaux halo identiques ou différents ; hétéroaryle ou aryle ou hétérocycloalkyle éventuellement substitué par un ou plusieurs substituants identiques ou différents choisis parmi : (C₁-C₆)alkyle, (C₁-C₆)alkoxy, halo, nitro, cyano, amino, CF₃, OCF₃, hydroxy, N₃, (C₁-C₆)alkylamino et di((C₁-C₈)alkyl)amino ;
   n représente un entier de 0 à 6 et n' un entier de 0 à 2 ;
   ou bien R'₁ et R'₂ forment ensemble, avec l'atome d'azote auquel ils sont rattachés, un hétérocycloalkyle, un hétérobicycloalkyle ou un radical de formule : 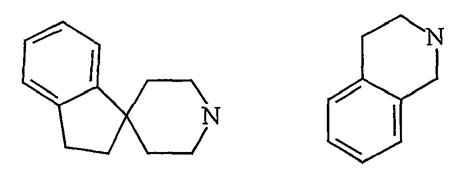 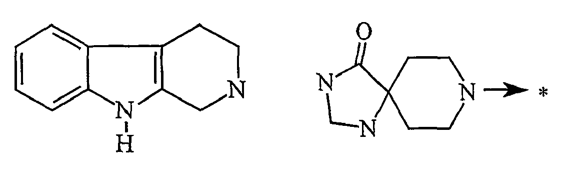 le radical que forment ensemble R'₁ et R'₂ étant éventuellement substitué par un ou plusieurs substituants identiques ou différents choisis parmi :
   -(CH₂)_{n"}-X"-Y", oxo , hydroxy, halo, nitro, cyano;
   X" représente -O-, -C(O)-, -C(O)-O- ou une liaison covalente ;
   Y" représente un radical (C₁-C₆)alkyle, amino, (C₁-C₆)alkylamino, di((C₁-C₆)alkyl)amino, (C₃-C₇)cycloalkyle, hétérocycloalkyle, arylalkyle, ou aryle ou hétéroaryle éventuellement substitué par un ou plusieurs substituants identiques ou différents choisis parmi : (C₁-C₆)alkyle, (C₁-C₆)alkoxy, (C₁-C₆)alkyl-carbonyle, halo, hydroxy, nitro, cyano, CF₃, OCF₃, amino, (C₁-C₆)alkylamino et di((C₁-C₆)alkyl)amino) ; ou bien un radical de formule 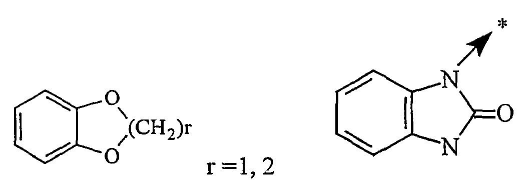
   n" représente un entier de 0 à 4 ;

- R'₃: représente -(CH₂)ₚ-W₃-(CH₂)_{p'}-Z₃
W₃ représente une liaison covalente, -CH(OH)- ou -C(O)- ;
Z₃ représente un radical (C₁-C₆)alkyle, adamantyle, aryle, un hétéroaryle, ou bien un radical de formule 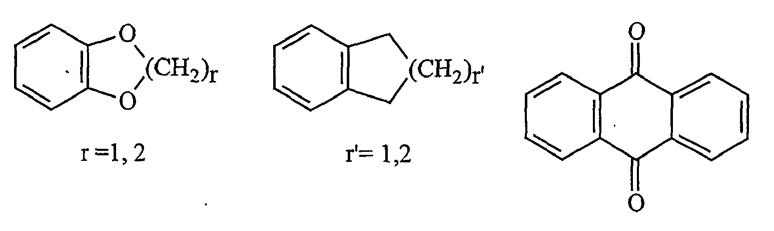 le radical aryle étant éventuellement susbtitué par un ou plusieurs substituants identiques ou différents choisis parmi : -(CH₂)_{p"}-V₃-Y₃, halo, nitro, cyano, N₃, hydroxy ;
V₃ représente -O-, -S-, -C(O)-, -C(O)-O-, -SO₂- ou une liaison covalente ;
Y₃ représente un radical (C₁-C₆)alkyle éventuellement substitué par un ou plusieurs radicaux halo identiques ou différents, amino, (C₁-C₆)alkylamino, di((C₁-C₆)alkyl)amino, phénylcarbonylméthyle, hétérocycloalkyle ou aryle ;
p, p' et p" représentent, indépendamment, un entier de 0 à 4 ;
- R'₄: représente un radical de formule-(CH₂)ₛ-R"₄
R"₄ représente un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle ; ou un radical de formule -NW₄W'₄
W₄ représente l'atome d'hydrogène, (C₁-C₈)alkyle ou (C₃-C₇)cycloall<yle ;
W'₄ représente un radical de formule -(CH₂)_{s'}-Q₄-Z₄ ;
Q₄ représente une liaison covalente, -CH₂-CH(OH)-[CH₂]ₜ-[O]_{t'}-[CH₂]_{t"}-ou -C(O)-O-;
t, t' et t" représentent, indépendamment, 0 ou 1 ;
Z₄ représente l'atome d'hydrogène, (C₁-C₈)alkyle, (C₃-C₇)cycloalkyle ; hétéroaryle ; aryle éventuellement substitué par un ou plusieurs radicaux identiques ou différents choisis de formule -(CH₂)_{q"}-V₄-Y₄, hydroxy, halo, nitro, cyano ;
V₄ représente -O-, -S-, -NH-C(O)- ou une liaison covalente ;
Y₄ représente un radical (C₁-C₆)alkyle éventuellement substitué par di((C₁-C₆)alkyl)amino ou un ou plusieurs radicaux halo identiques ou différents ; amino ; (C₁-C₆)alkylamino ; di((C₁-C₆)alkyl)amino; aralkyle ; hétérocycloalkyle ;
q" représente un entier de 0 à 4 ;
ou bien Z₄ représente un radical de formule
s et s' représentent, indépendamment, un entier de 0 à 6 ;
ou un sel pharmaceutiquement acceptable de ce dernier.

La présente invention a plus particulièrement pour objet un composé de formule I ou I' telle que définie ci-dessus ou un sel pharmaceutiquement acceptable de ce dernier, et dans laquelle A représente -C(O)-.

La présente invention a plus particulièrement pour objet un composé de formule I telle que définie ci-dessus ou un sel pharmaceutiquement acceptable de ce dernier, et dans laquelle
le cycloalkyle que représente X est le cyclohexyle ou le cycloheptyle,
l'hétérocycloalkyle que représente X est choisi parmi : pipéridine, pyrrolidine, thiazolidine, morpholine et tétrahydrothiophène ;
l'aryle que représente X est le radical phényle, naphtyle ou fluorènyle ;
l'aryle du radical aryl-carbonyle que représente X, est le radical phényle ;
l'hétéroaryle que représente X est choisi parmi : pyridine, imidazole, thiophène, indole, carbazole et isoquinoléine ;
l'hétéroaryle que représente Y' est choisi parmi oxazole et imidazole ;
l'aryle que représente Y' est le radical phényle ;
l'hétérocycloalkyle que représente Y' est la pipérazine ;
l'hétérocycloalkyle que forment ensemble R₁ et R₂ avec l'atome d'azote auquel ils sont rattachés, est choisi parmi : pipéridine, pipérazine, diazépane, thiazolidine et morpholine ;
le cycloalkyle que représente Y" est le cyclopentyle ou le cyclohexyle ;
l'hétérocycloalkyle que représente Y" est choisi parmi : pipéridine, pyrrolidine et morpholine ;
l'arylalkyle et l'aryle que représente Y" sont respectivement le radical benzyle et le radical phényle ;
l'hétéroaryle que représente Y" est choisi parmi : pyridine, pyrazine, furane et thiophène.

La présente invention a plus particulièrement pour objet également un composé de formule I telle que définie ci-dessus ou un sel pharmaceutiquement acceptable de ce dernier, et dans laquelle
l'aryle que représente Z₃ est le radical phényle ou naphtyle ;
l'hétéroaryle que représente Z₃ est choisi parmi benzo[b]thiophène et benzo[b]furanne ;
l'hétérocycloalkyle et l'aryle que représente Y₃ sont respectivement les radicaux pyrrolidine et phényle.

La présente invention a plus particulièrement pour objet également un composé de formule I telle que définie ci-dessus ou un sel pharmaceutiquement acceptable de ce dernier, et dans laquelle
l'hétérocycloalkyle que représente R"₄ est choisis parmi : pipérazine, pipéridine, morpholine et pyrrolidine ;
l'aralkyle qui substitue éventuellement l'hétérocycloalkyle que représente R"₄ est le radical benzyle ;
l'hétéroaryle que représente R"₄ est l'imidazole ;
le (C₃-C₇)cycloalkyle que représente Z₄ est le cyclopropyle, cyclobutyle, cyclopentyle, cyclohexyle ou cycloheptyle;
l'hétéroaryle que représente Z₄ est choisi parmi : pyridine, thiophène, indole et furane ;
l'aryle que représente Z₄ et est le phényle ou naphtyle ;
l'aralkyle que représente Y₄ est benzyle ;
l'hétérocycloalkyle que représente Y₄ est la pyrrolidine ;
l'aralkyle qui est substitué sur l'hétérocycloalkyle que forment ensemble W₄ et W'₄ est le radical benzyle.

De manière préférentielle, l'invention a pour objet un composé de formule I telle que définie ci-dessus ou un sel pharmaceutiquement acceptable de ce dernier et dans laquelle A représente -C(O)- et R₁ et R₂ représentent, indépendamment, l'atome d'hydrogène, un radical ((C₁-C₈)alkyle ou un radical de formule -(CH₂)ₙ-X dans laquelle
X représente, amino, di(alkyl)amino, adamentyle, cyclohexyle, cycloheptyle, pipéridine, morpholine, pyrrolidine, phényle, pyridine, imidazole, thiophène, indole, carbazole étant éventuellement substitué (C₁-C₆)alkyle, ou bien un radical de formule 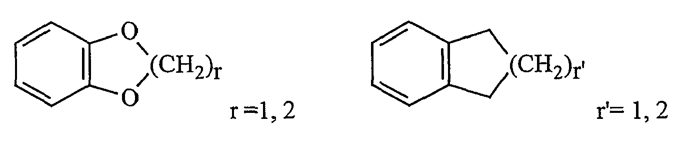 les radicaux pipéridine, pyrrolidine et phényle étant éventuellement substitués par un ou plusieurs substituants identiques ou différents choisis parmi :-(CH₂)_{n'}-X'-Y', halo, oxo, amino et di((C₁-C₈)alkyl)amino ;
X' représente -O-, -S-, -C(O)-O-, -NH-C(O)-, -NH-SO₂- ou une liaison covalente ;
Y' représente un radical (C₁-C₆)alkyle, oxazole, phényle éventuellement substitué par (C₁-C₄)alkyle ou pipérazine éventuellement substitué par (C₁-C₄)alkyle ;
ou bien R₁ et R₂ forment ensemble, avec l'atome d'azote auquel ils sont rattachés, pipéridine, pipérazine et diazépane, thiazolidine, morpholine, ou un radical cyclique de formule : 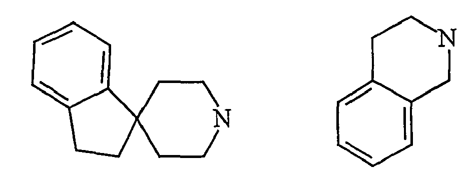 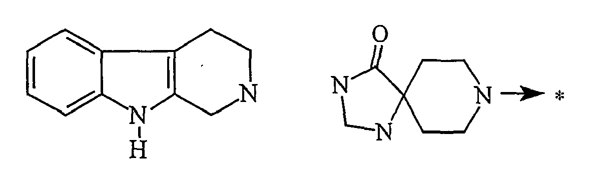 le radical que forment ensemble R₁ et R₂ étant éventuellement substitué par un ou plusieurs substituants identiques ou différents choisis parmi :
-(CH2)ₙX"-Y" ;
X" représente -C(O)-, -C(O)-O- ou une liaison covalente ;
Y" représente un radical (G₁-C₆)alkyle ; di(alkcyl)amino, cyclopentyle, cyclohexyle, pipéridine, pyrrolidine, morpholine, benzyle, pyridine, pyrazine, furane, thiophène, ou phényle éventuellement substitué par un ou plusieurs substituants identiques ou différents choisis parmi (C₁-C₆)alkyle, (C₁-C₆)alkoxy, (C₁-C₆)alkyl-carbonyle et halo ; ou bien Y" représente un radical de formule

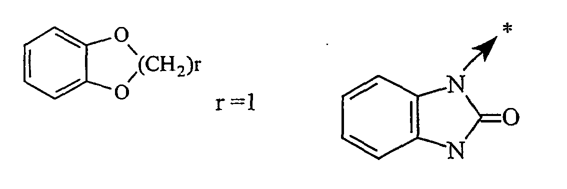

De manière préférentielle, l'invention a pour objet un composé de formule I telle que définie ci-dessus ou un sel pharmaceutiquement acceptable de ce dernier et dans laquelle A représente -C(O)- et R₃ représente -(CH₂)ₚ-W₃-(CH₂)_{p'}-Z₃
W₃ représente une liaison covalente, -CH(OH)- ou -C(O)- ;
Z₃ représente un radical (C₁-C₆)alkyle, phényle, naphtyle, benzo[b]thiophène, benzo[b]furannyle, ou bien un radical de formule 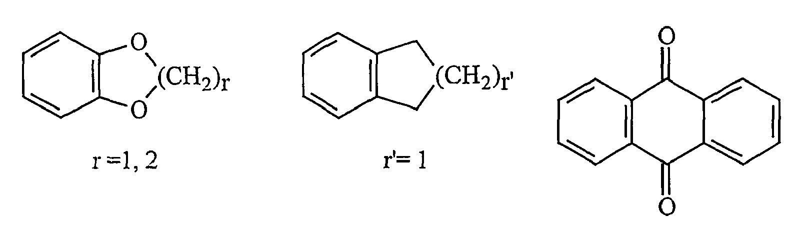 le radical phényle étant éventuellement substitué par un ou plusieurs substituants identiques ou différents choisis parmi : -(CH₂)_{p"}-V₃-Y₃, halo, nitro, cyano ;
V₃ représente -O-, -S-, -C(O)-, -C(O)-O-, -SO₂- ou une liaison covalente ;
Y₃ représente un radical (C₁-C₆)alkyle éventuellement substitué par un ou plusieurs radicaux halo identiques ou différents ; amino ; di((C₁-C₆)alkyl)amino ; phénylcarbonylméthyle ; pyrrolidine ou phényle ;
p, p' et p" représentent, indépendamment, un entier de 0 à 2.

De manière préférentielle, l'invention a pour objet un composé de formule I' telle que définie ci-dessus ou un sel pharmaceutiquement acceptable de ce dernier et dans laquelle Aₐ représente -C(O)- et les radicaux R'₁, R'₂, R'₃ et R'₄ ont respectivement les définitions des radicaux R₁, R₂, R₃ et R₄ tels que définis ci-dessus.

De manière préférentielle, l'invention a pour objet un composé de formule I telle que définie ci-dessus ou un sel pharmaceutiquement acceptable de ce dernier et dans laquelle A représente -C(O)- et R₄ représente un radical de formule-(CH₂)ₛ-R"₄
R"₄ représente le cycle pipéridine éventuellement substituée par benzyle, pipérazine éventuellement substituée par benzyle, ou un radical de formule -NW₄W'₄
W₄ représente l'atome d'hydrogène ou (C₁-C₈)alkyle ;
W'₄ représente un radical de formule -(CH₂)_{s'}-Q₄-Z₄ ;
Q₄ représente une liaison covalente, -CH₂-CH(OH)-, -CH₂-CH(OH)-CH₂-O-, -CH₂-CH(OH)-CH₂-, -CH₂-CH(OH)-CH₂-O-CH₂-ou -C(O)-O- ;
Z₄ représente l'atome d'hydrogène, (C₁-C₈)alkyle éventuellement substitué par (C₁-C₆)alkoxy, (C₁-C₆)alkylthio, (C₁-C₆)alkyldithio ou un ou deux radicaux hydroxy ; (C₂-C₆)alkenyle ; (C₂-C₆)alkynyle ; cyclopropyle éventuellement substitué par alkoxycarbonyle ; cyclobutyle, cyclopentyle éventuellement substitué par hydroxyalkyle; cyclohexyle éventuellement substitué par un ou plusieurs radicaux alkyle ; cycloheptyle, cyclohexène, adamantyle, pyridine, thiophène, indole, furane, naphtyle ; phényle éventuellement substitué par un ou plusieurs radicaux identiques ou différents choisis parmi : -(CH₂)_{q"}-X₄-Y₄, hydroxy, halo et cyano ;
X₄ représente -O- ou une liaison covalente ;
Y₄ représente un radical (C₁-C₆)alkyle, di((C₁-C₆)alkyl)amino ou pyrrolidine.

De manière très préférentielle également, l'invention a pour objet un composé de formule I telle que définie ci-dessus dans laquelle A représente -C(O)-, Y représente - NH- et
- R₁ R₂: et représentent, indépendamment, un radical (C₁-C₈)alkyle ;
- R₃: représente -(CH₂)ₚ-W₃-(CH₂)_{p'}-Z₃
W₃ représente une liaison covalente ; Z₃ représente le radical phényle susbtitué par un ou plusieurs substituants identiques ou différents choisis parmi : -(CH₂)_{p"}-V₃-Y₃ et halo ; V₃ représente -O- ou -S- ; et Y₃ représente un radical (C₁-C₆)alkyle ; p, p' et p" représentent 0 ;
- R₄: représente un radical de formule-(CH₂)ₛ-R"₄
R"₄ représente un radical de formule -NW₄W'₄
W₄ représente l'atome d'hydrogène ou (C₁-C₈)alkyle ;
W'₄ représente un radical de formule -(CH₂)_{s'}-Q₄-Z₄ ;
Q₄ représente une liaison covalente ;
Z₄ représente l'atome d'hydrogène, (C₁-C₈)alkyle éventuellement substitué par hydroxy, (C₃-C₇)cycloalkyle, hétéroaryle, aryle éventuellement substitué par un ou plusieurs radicaux identiques ou différents choisis de formule -(CH₂)_{q"}-V₄-Y₄ ;
V₄ représente -O- ou une liaison covalente ;
Y₄ représente un radical (C₁-C₆)alkyle ou di((C₁-C₆)alkyl)amino ;
q" représente 0 ; s représente un entier de 2 à 4, et s' un entier de 1 à 2.
et de manière très préférentielle le (C₃-C₇)cycloalkyle est choisi parmi cyclopentyle et cyclohexyle, l'hétéroaryle représente la pyridine et l'aryle le phényle ; ou un sel pharmaceutiquement acceptable de ces derniers.

De manière préférentielle, l'invention a pour objet un composé de formule I' telle que définie ci-dessus ou un sel pharmaceutiquement acceptable de ce dernier et dans laquelle Aₐ représente -C(O)-, Yₐ -NH-, les radicaux R'₁, R'₂ et R'₃ ont respectivement les définitions des radicaux R₁, R₂ et R₃ tels que définis ci-dessus, et R'₄ représente un radical de formule-(CH₂)ₛ-R"₄
R"₄ représente un radical de formule -NW₄W'₄
W₄ représente l'atome d'hydrogène ou (C₁-C₈)alkyle ;
W'₄ représente un radical de formule -(CH₂)_{s'}-Q₄-Z₄ ;
Q₄ représente une liaison covalente ;
Z₄ représente l'atome d'hydrogène, (C₁-C₈)alkyle, (C₃-C₇)cycloalkyle, hétéroaryle, aryle éventuellement substitué par un ou plusieurs radicaux identiques ou différents choisis de formule -(CH₂)_{q"}-V₄-Y₄ ;
V₄ représente -O- ou une liaison covalente ;
Y₄ représente un radical (C₁-C₆)alkyle ou di((C₁-C₆)alkyl)amino ;
q" représente 0 ; s représente un entier de 2 à 4, et s' un entier de 1 à 2.
et de manière très préférentielle le (C₃-C₇)cycloalkyle est choisi parmi cyclopentyle et cyclohexyle, l'hétéroaryle représente la pyridine et l'aryle le phényle ; ou un sel pharmaceutiquement acceptable de ces derniers.

De manière très préférentielle également, l'invention a pour objet un composé de formule I telle que définie ci-dessus dans laquelle A représente -C(O)-, Y représente l'atome de soufre et
R₁ et R₂ représentent, indépendamment, un radical (C₁-C₈)alkyle ;
R₃ représente -(CH₂)ₚ-W₃-(CH₂)_{p'}-Z₃
W₃ représente une liaison covalente ou -C(O)- ; Z₃ représente le radical phényle susbtitué par un ou plusieurs substituants identiques ou différents choisis parmi : -(CH₂)_{p"}-V₃-Y₃ et halo ; V₃ représente -O- ou une liaison covalente ; et Y₃ représente un radical (C₁-C₆)alkyle ou di((C₁-C₆)alkyl)amino ; p représente 1, et p' et p" représentent 0 ;
R₄ représente un radical de formule-(CH₂)ₛ-R"₄
R"₄ représente un radical de formule -NW₄W'₄
W₄ représente l'atome d'hydrogène ou (C₁-C₈)alkyle ;
W'₄ représente un radical de formule -(CH₂)_{s'}-Q₄-Z₄ ;
Q₄ représente une liaison covalente ;
Z₄ représente l'atome d'hydrogène, (C₁-C₈)alkyle, hétéroaryle, aryle s représente un entier de 2 à 4, et s' un entier de 1 à 2,
et de manière très préférentielle l'hétéroaryle représente la pyridine et l'aryle le phényle ;
ou un sel pharmaceutiquement acceptable de ce dernier.

De manière préférentielle, l'invention a pour objet un composé de formule I' telle que définie ci-dessus ou un sel pharmaceutiquement acceptable de ce dernier et dans laquelle Aₐ représente -C(O)-, Yₐ un atome de soufre et les radicaux R'₁, R'₂, R'₃ et R'₄ ont respectivement les définitions des radicaux R₁, R₂ , R₃ et R₄ tels que définis ci-dessus lorsque A représente -C(O)- et Y un atome de soufre.

De manière préférentielle également, l'invention a pour objet un composé de formule I telle que définie ci-dessus dans laquelle A représente -CH₂-, Y -NH- et R₁ et R₂ représentent, indépendamment, un radical ((C₁-C₆)alkyle ; R₃ représente un phényle substitué par un ou plusieurs substituants (C₁-C₆)alkoxy identiques ou différents ; R₄ représente un radical de formule-(CH₂)ₛ-R"₄ ; R"₄ représente un radical de formule -NW₄W'₄ ; W₄ représente (C₁-C₈)alkyle ; W'₄ représente un radical de formule -(CH₂)_{s'}-Q₄-Z₄ ; Q₄ représente une liaison covalente et Z₄ représente la pyridine ; ou un sel pharmaceutiquement acceptable de ce dernier.

De manière préférentielle, l'invention a pour objet un composé de formule I' telle que définie ci-dessus ou un sel pharmaceutiquement acceptable de ce dernier et dans laquelle Aₐ représente -CH₂-, Yₐ -NH- et les radicaux R'₁, R'₂, R'₃ et R'₄ ont respectivement les définitions des radicaux R₁, R₂ , R₃ et R₄ tels que définis ci-dessus lorsque A représente -CH₂- et Y -NH-.

Dans la présente demande, le symbole -> correspond au point de rattachement du radical. Lorsque le site de rattachement n'est pas précisé sur le radical, cela signifie que le rattachement s'effectue sur un des sites disponibles de ce radical pour un tel rattachement.

Suivant les définitions des groupes variables A, Y, R₁, R₂, R₃ et R₄, les composés selon l'invention peuvent être préparés en phase liquide selon les différentes procédures A à H décrites ci-dessous.

### A. Préparation selon le schéma réactionnel A :

Les composés de formule I selon l'invention dans laquelle Y représente -NH- et A -C(O)-, peuvent être préparés selon le schéma A suivant :

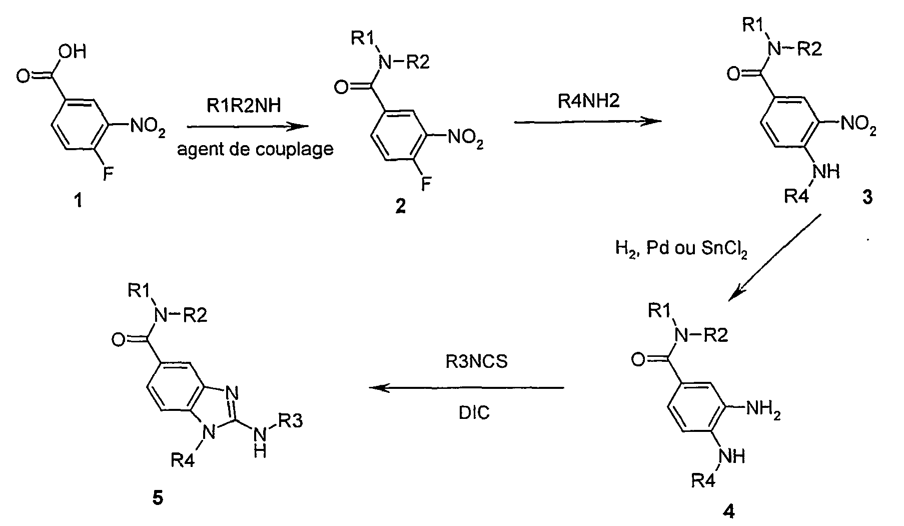

Comme décrit dans le schéma A, l'acide 4-fluoro-3-nitrobenzoïque (1) peut être couplé avec une amine primaire ou secondaire en présence d'un agent de couplage tel que le diisopropylcarbodiimide, le dicyclohexylcarbodiimide, avec ou sans du 1-hydroxybenzotriazole (HOBt) dans un solvant organique inerte tel que du chlorure de méthylène, tétrahydrofuranne ou diméthylformamide à température ambiante pendant 3 à 24 heures pour conduire à l'amide correspondante (2). Le traitement du dérivé fluoré (2) avec une amine primaire en présence d'une base inorganique telle que du carbonate de césium ou de potassium dans un solvant organique inerte tel que le diméthylformamide ou l'acétonitrile à une température de 20-70° C pendant 2 à 16 heures conduit au dérivé (3). La fonction nitro du composé (3) est réduite par traitement avec du chlorure d'étain dihydrate dans un solvant inerte tel que l'acétate d'éthyle ou le diméthylformamide à une température de 60-80° C pendant 3 à 15 heures, ou par hydrogénation catalytique en présence de palladium sur charbon 10 % dans un solvant inerte tel que le méthanol, éthanol, acétate d'éthyle ou un mélange de ces solvants, à une température de 18-25° C, pendant 2 à 8 heures pour conduire à la dianiline (4). Le dérivé (4) est ensuite traité par un isothiocyanate en présence d'un agent de couplage supporté sur une résine ou non tel que le diisopropylcarbodiimide ou dicyclohexylcarbodiimide ou la résine *N*-méthylcyclohexylcarbodiimide *N*-méthyl polystyrène dans un solvant inerte tel que le tétrahydrofuranne, chlorure de méthylène, ou chloroforme à une température de 20-70° C pendant 2 à 72 heures pour conduire au dérivé (5). Alternativement, le dérivé (4) peut être traité par un isothiocyanate dans un solvant inerte tel que le tétrahydrofuranne, chlorure de méthylène ou chloroforme puis la thiourée résultante peut être traitée par de l'iodure de méthyle dans un solvant polaire tel que l'éthanol pendant 3 à 24 heures à une température de 20-70° C pour conduire à (5).

### Exemple A1 : chlorhydrate de N,N-diisobutyl-1-{3-[méthyl(2-pyridin-2-yléthyl) amino]propyl}-2-[(3,4,5-triméthoxyphényl)amino]-1H-benzimidazole-5-carboxamide

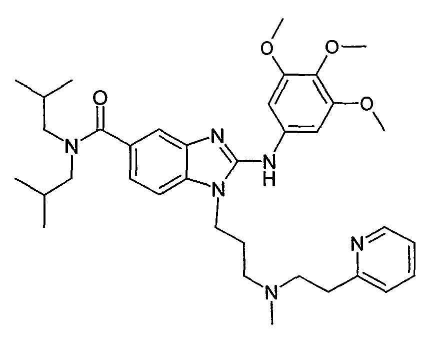

### Etape 1 : 4-fluoro-N,N-diisobutyl-3-nitrobenzamide

A l'acide 4-fluoro-3-nitrobenzoïque (15 g, 1 éq) en solution dans le THF (150 ml) est additionné le diisopropylcarbodiimide (13,8 ml, 1.2 éq). Le mélange est agité 3 heures à une température d'environ 20° C puis la diisobutylamine (12,9 ml, 1 éq) est additionnée. Après 15 heures d'agitation à environ 20° C, le mélange réactionnel est évaporé sous pression réduite à 40° C. Le résidu est repris par du dichlorométhane (200 ml) et de l'eau (70 ml). Après décantation et extraction, les phases organiques combinées sont lavées avec de la saumure, séchées sur Na₂SO₄ puis évaporées sous pression réduite à 40° C. La purification du composé par chromatographie éclair sur gel de silice (éluant : heptane / acétate d'éthyle 8 :2) donne le composé attendu sous forme d'un solide jaune (13,8 g ; 63 % rendement).
SM/CL : MM calculée = 296,3 ; m/z = 297,2 (MH+) - Point de fusion = 47° C

### Etape 2 : N,N-diisobutyl-4-({3-[méthyl(2-pyridin-2-yléthyl)amino]propyl}amino)-3-nitrobenzamide

Un mélange de 4-fluoro-*N*,*N*-diisobutyl-3-nitrobenzamide (2,07 g, 1 éq), de *N*-(2-pyridin-2-yléthyl)propane-1,3-diamine (1,6 g, 1.2 éq) et de carbonate de césium (4,5 g, 2 éq) dans l'acétonitrile (70 ml) est chauffé au reflux pendant 3 heures puis concentré sous pression réduite à 40° C. Le résidu est repris par du dichlorométhane (100 ml) et de l'eau (40 ml). Après décantation et extraction, les phases organiques combinées sont lavées avec de la saumure, séchées sur Na₂SO₄ puis évaporées sous pression réduite à 40° C. La purification du résidu par chromatographie éclair sur gel de silice (éluant : dichlorométhane 100 à dichlorométhane/ méthanol 8:2) donne le composé attendu sous forme d'une huile jaune (3,1 g ; 92 % rendement).
SM/CL : MM calculée = 469,6 ; m/z = 470,3 (MH+)
RMN (¹H, 400 MHz, DMSO-*d₆*): *δ*0,79 (m, 12H), 1,75 (m, 2H), 1,90 (m, 2H), 2,23 (s, 3H), 2,48 (t, 3H,³*J* = 6 Hz), 2,71 (t, 2H, ³*J* = 7 Hz), 2,87 (t, 2H, *³J* = 7 Hz), 3,19 (d,4H, ³*J* = 7 Hz), 3,33 (m, 2H), 7,01 (d, 1H), 7,10 (m, 1H), 7,23 (d, 1H), 7,50 (m, 1H), 7,60 (m, 1H), 7,99 (s, 1H), 8,41 (m, 1H), 8,59 (t, 1H, ³*J* = 5 Hz).

### Etape 3 : 3-amino-N,N-diisobutyl-4-({3-[méthyl(2-pyridin-2-yléthyl)amino]propyl} amino)benzamide

Dans un autoclave sont additionnés le *N,N-*diisobutyl-4-({3-[méthyl(2-pyridin-2-yléthyl)amino]propyl}amino)-3-nitrobenzamide (2,9 g) en solution dans un mélange d'acétate d'éthyle/ éthanol (100 ml), et le palladium sur charbon 10 % (290 mg). Après 7 heures d'agitation sous atmosphère d'hydrogène (3 bars), le catalyseur est éliminé par filtration sur célite et le filtrat est concentré sous pression réduite à 40° C pour donner le composé attendu sous forme d'une huile (2,5 g, 92 % rendement).
SM/CL : MM calculée = 439,6 ; m/z = 440,3 (MH+)
RMN (¹H, 400 MHz, DMSO-d*₆*) : *δ* 0,77 (m, 12H), 1,71 (m, 2H), 1,90 (m, 2H), 2,22 (s, 3H), 2,47 (m, 3H), 2,70 (t, 2H, *³J* = 7 Hz), 2,87 (t, 2H, ³*J* = 7 Hz), 3,0 (m, 2H), 3,17 (d, 4H, *³J* = 7,5 Hz), 4,62 (s, 2H), 4,71 (s, 1H), 6,33 (d, 1H), 6,50 (d, 1H), 6,57 (s, 1H), 7,15 (m, 1H), 7,25 (d, 1H), 7,63 (m, 1H), 8,45 (m, 1H).

### Etape 4 : chlorhydrate de N,N-diisobutyl-1-{3-[méthyl(2-pyridin-2-yléthyl)amino] propyl}-2-[(3,4,5-triméthoxyphényl)amino]-1H-benzimidazole-5-carboxamide

A une solution de 3-amino-*N*,*N*-diisobutyl-4-({3-[méthyl(2-pyridin-2-yléthyl)amino] propyl}amino) benzamide (48 mg, 1 éq) dans le tétrahydrofuranne (2 ml) sont successivement additionnés le 3,4,5 triméthoxyphénylisothiocyanate (27 mg, 1,2 éq) et la résine *N*-méthylcyclohexylcarbodiimide-*N*-méthyl-polystyrène (acquise auprès de Novabiochem ; charge 1,69 mmol/g, 236 mg, 4 éq). Le mélange est chauffé à reflux pendant 18 heures puis refroidi à température ambiante et additionné de résine aminométhylpolystyrène (acquise auprès de Novabiochem, 2 éq). Après 4 h d'agitation à température ambiante, le mélange est filtré sur fritté et le filtrat est concentré sous pression réduite à 40° C. Le résidu obtenu est dissout dans de l'éther éthylique et une solution de HCl 1N dans l'éther éthylique est additionnée goutte à goutte pour donner le composé attendu sous forme de sel de chlorhydrate (80 mg, 89 % rendement).
SM/CL : MM calculée = 630,8 ; m/z = 631,4 (MH+)
RMN (¹H, 400 MHz, DMSO-*d₆*): *δ* 0,66 (m, 6H), 0,91 (m, 6H), 1,71-2,03 (m, 2H), 2,49 (m, 2H), 2,86 (s, 3H), 3,01-3,72 (m, 10H), 3,81 (s, 3H), 3,88 (s, 6H), 4,58 (t, 2H, ³*J* = 7 Hz ), 6,93 (s, 2H), 7,30 (m, 2H), 7,60 (m, 1H), 7,70 (m, 1H), 7,82 (d, 1H), 8,12 (m, 1H), 8,67 (d, 1H), 11,2 (s, 1H), 11,7 (s, 1H), 13,0 (s, 1H).

### Exemple A2 : chlorhydrate de 1-{3-[benzyl(méthyl)amino]propyl}-2-[(3,5-diméthoxyphényl)amino]-N,N-diisobutyl-1H-benzimidazole-5-carboxamide

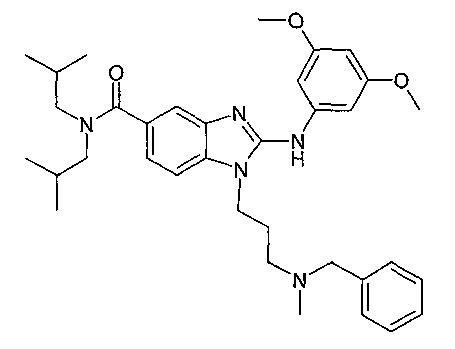

### Etape 1 : 3-amino-4-({3-[benzyl(méthyl)amino]propyl}amino)-N,N-diisobutyl benzamide

A une solution de 4-({3-[benzyl(méthyl)amino]propyl}amino)-*N*,*N*-diisobutyl-3-nitrobenzamide (1,44 g, préparé selon la procédure décrite pour l'exemple A1), dans l'acétate d'éthyle (40 ml) est additionné le chlorure d'étain dihydrate (3,58 g, 5éq). Le mélange est chauffé à reflux pendant 7 h puis refroidi à une température d'environ 20° C. et versé dans une solution saturée en NaHCO₃. Après décantation et extraction à l'acétate d'éthyle, les phases organiques sont combinées, lavées avec de la saumure, séchées sur sulfate de sodium et concentrées sous pression réduite à 40° C. La purification par chromatographie éclair sur gel de silice (éluant dichlorométhane /méthanol 95 : 5) donne le composé sous forme d'une mousse (1,06 g, 78 % rendement).
SM/CL : MM calculée = 424,3 ; m/z = 425,3 (MH+)
RMN (¹H, 400 MHz, DMSO-*d₆*) : *δ* 0,77 (m, 12H), 1,78 (m, 2H), 1,90 (m, 2H), 2,12 (s, 3H), 2,49 (m, 3H), 3,06 (t, 2H, ³*J* = 7 Hz), 3,17 (d, 4H, ³*J* = 7,5 Hz), 3,48 (s, 2H), 4,61 (s, 2H), 4,72 (s, 1H), 6,38 (d, 1H), 6,51 (m, 1H), 6,59 (s, 1H), 7,19-7,30 (m, 5H).

### Etape 2 : chlorhydrate de 1-{3-[benzyl(méthyl)amino]propyl}-2-[(3,5-diméthoxy phényl) amino]-N,N-diisobutyl-1H-benzimidazole-5-carboxamide

A une solution de 3-amino-4-({3-[benzyl(méthyl)amino]propyl}amino)-*N,N-*diisobutylbenzamide (65 mg, 1 éq) dans le tétrahydrofuranne (2 ml) sont successivement additionnés le 3,4 diméthoxyphénylisothiocyanate (35 mg, 1,2 éq) et la résine *N*-méthylcyclohexylcarbodiimide-*N*-méthyl-polystyrène (acquise auprès de Novabiochem ; charge 1,69 mmol/g, 355 mg, 4 éq). Le mélange est chauffé à reflux pendant 18 heures puis refroidi à température ambiante et additionné de résine aminométhylpolystyrène (acquise auprès de Novabiochem, 2 éq). Après 4 h d'agitation à température ambiante, le mélange est filtré sur fritté et le filtrat est concentré sous pression réduite à 40° C. Le résidu obtenu est dissout dans de l'éther éthylique et une solution de HCl 1N dans l'éther éthylique est additionnée goutte à goutte pour donner le composé attendu sous forme de sel de chlorhydrate (81 mg, 92 % rendement).
SM/CL : MM calculée = 585,3 ; m/z = 586,5 (MH+)
RMN (¹H, 400 MHz, DMSO-*d₆*) : *δ* 0,85 (m, 6H), 0,92 (m, 6H), 1,85 (m, 1H), 2,05 (m, 1H), 2,28 (m, 2H), 2,86 (s, 3H), 3,08-3,3 (m, 6H), 3,78 (s, 6H), 4,20-4,40 (m, 2H), 4,50 (m, 2H), 6,42 (s, 1H), 6,90 (m, 2H), 7,22 (m, 1H), 7,22-7,64 (m, 8H), 10,98 (m, 1H).

Les composés suivants ont été préparés selon le schéma réactionnel A et de façon analogue à la procédure décrite pour la synthèse du *N,N*-diisobutyl-1-{3-[méthyl(2-pyridin-2-yléthyl)amino]propyl}-2-[(3,4,5-triméthoxyphényl)amino]-1H-benzimidazole -5-carboxamide ou du 1-{3-[benzyl(méthyl)amino]propyl}-2-[(3,5-diméthoxyphényl) amino]-*N,N*-diisobutyl-1H-benzimidazole-5-carboxamide :

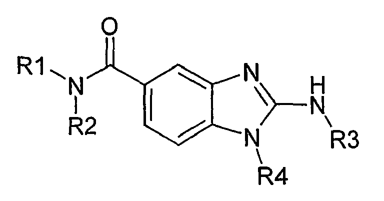

Dans la formule ci-dessus, R₁R₂N représente l'un des radicaux ci-après : 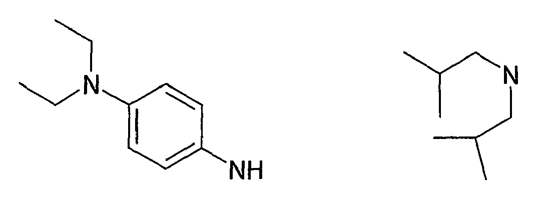 R₃ représente l'un des radicaux ci-après 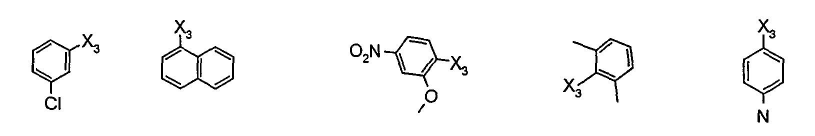 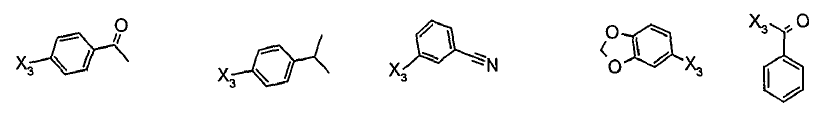 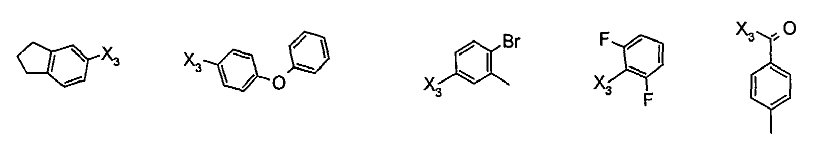 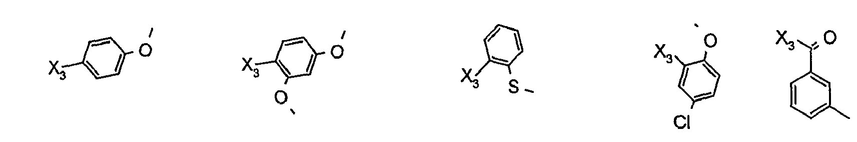 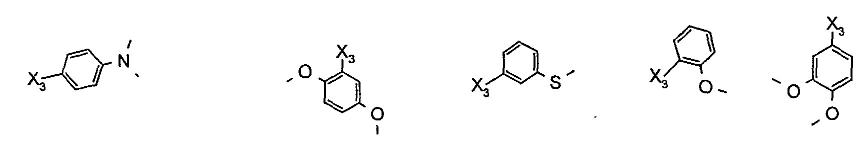 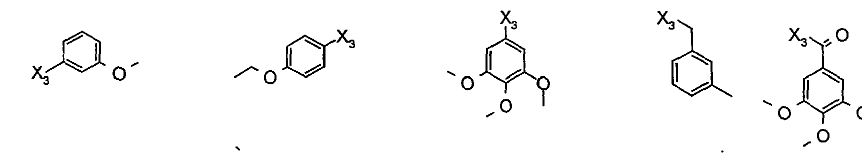 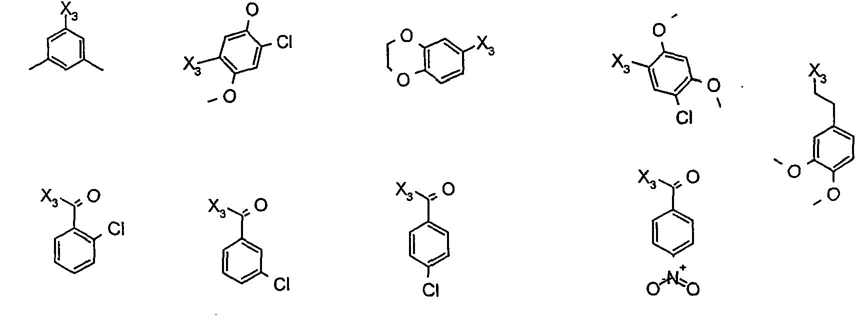 et R₄ représente l'un des radicaux ci-après (lorsque R₄ représente un radical comportant une terminaison amine secondaire, par exemple propylaminométhyl, les composés ont été obtenus par hydrogénation catalytique en présence de palladium sur charbon des dérivés *N*-benzyl correspondants ; et lorsque R₄ représente un radical comportant une terminaison amine primaire, par exemple éthylamino, les composés ont été obtenus par traitement acide des dérivés correspondants protégés par un groupement tertbutoxycarbonyl).

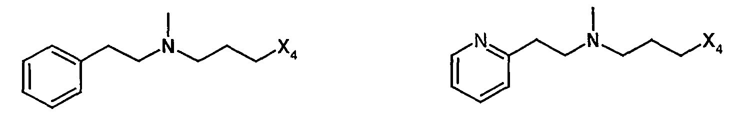

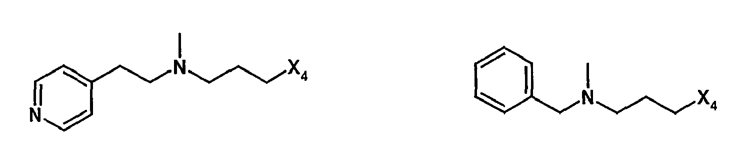

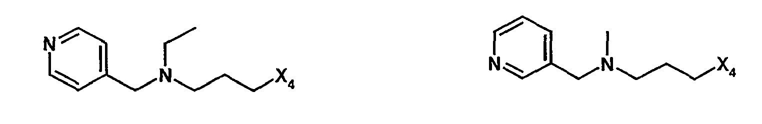

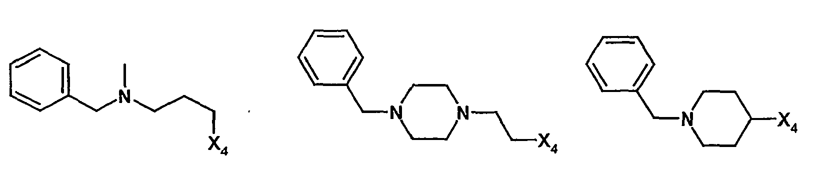

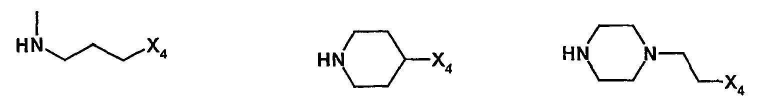

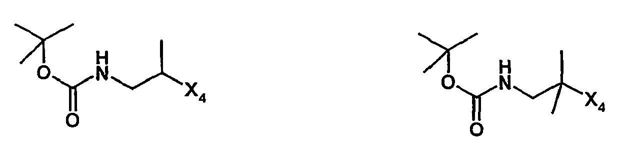

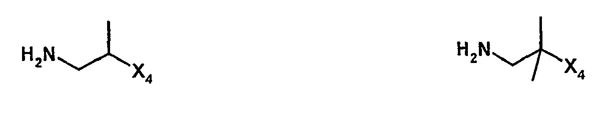

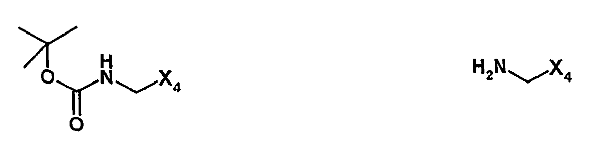

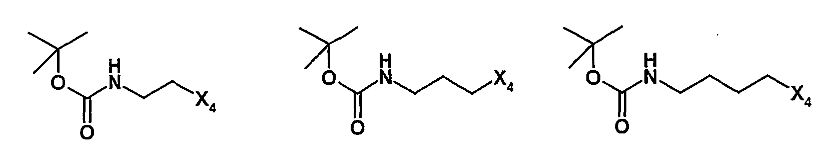

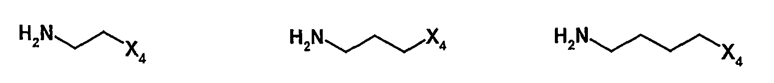

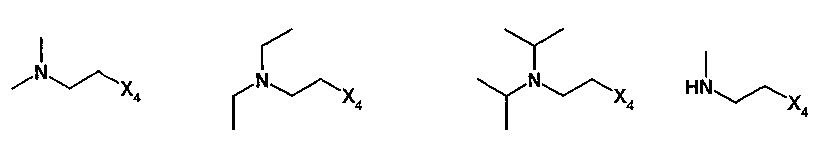

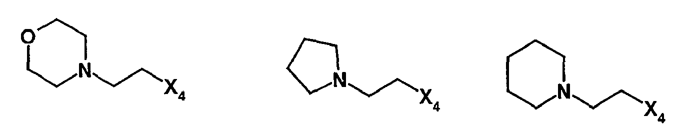

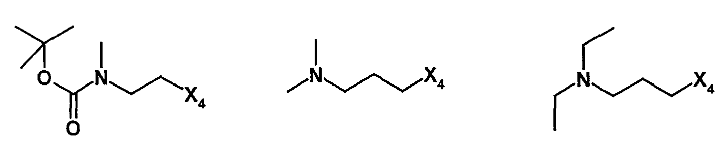

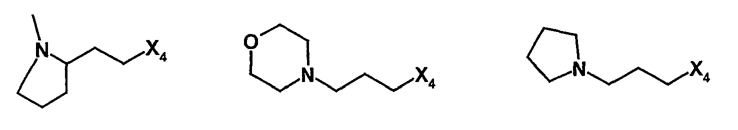

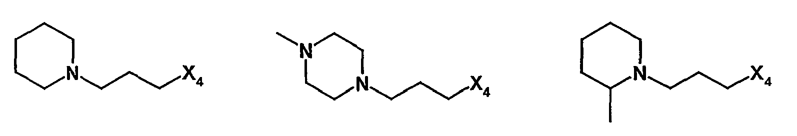

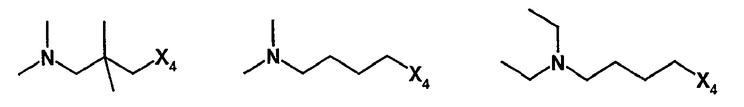

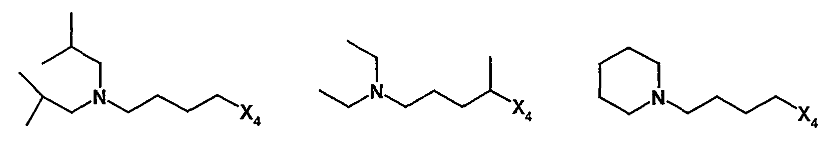

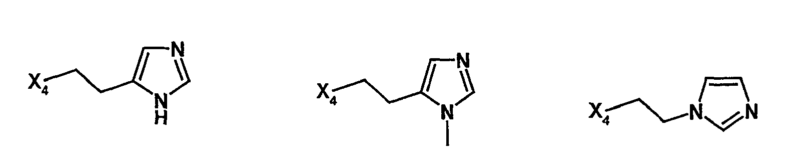

### B. Préparation selon le schéma réactionnel B :

Les composés de formule I selon l'invention dans laquelle Y représente -S- et A -C(O)-, peuvent être préparés selon le schéma B suivant :

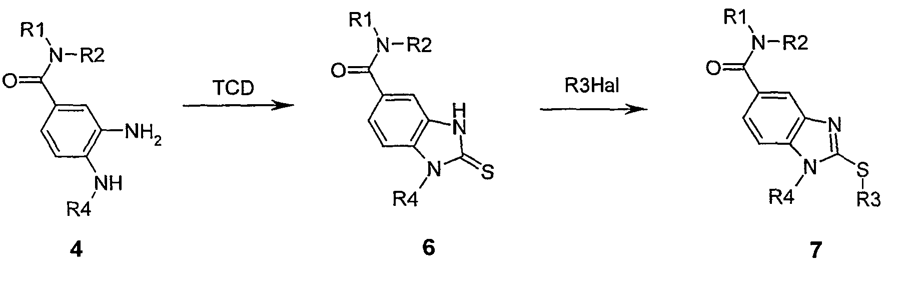

Comme décrit dans le schéma B, la dianiline (4) peut être traitée avec du thiocarbonyldiimidazole (TCD) ou du thiophosgène dans un solvant organique inerte tel que le tétrahydrofuranne, le chlorure de méthylène ou le chloroforme à température ambiante pendant 2 à 17 heures pour conduire au dérivé (6). Le composé (6) est ensuite alkylé par réaction avec un dérivé halogéné tel qu'un iodure, bromure ou chlorure d'alkyle ou de benzyle ou une bromocétone, en présence d'une base tertiaire telle que la triéthylamine ou la diisopropyléthylamine, ou en présence d'une base tertiaire supportée sur une résine telle que la résine morpholinométhyl-polystyrène, dans un solvant organique inerte tel que le tetrahydrofuranne, le choroforme ou le chlorure de méthylène, à une température de 20-70° C pendant 3 à 24 heures. Le dérivé thiobenzimidazole (7) résultant peut être isolé, soit par chromatographie éclair sur gel de silice, soit par addition au mélange réactionnel d'un réactif nucléophile supporté sur un polymère comme par exemple une résine aminométhylpolystyrène, et d'un réactif électrophile supporté sur un polymère comme par exemple la résine 4-bromométhylphénoxyméthylpolystyrène, suivie d'une filtration et de l'évaporation du filtrat.

### Exemple B1 : N,N-diisobutyl-1-{3-[méthyl(2-pyridin-2-yléthyl)amino]propyl}-2- {[2-oxo-2-(3,4,5-triméthoxyphényl)éthyl]thio}-1H-benzimidazole-5-carboxamide

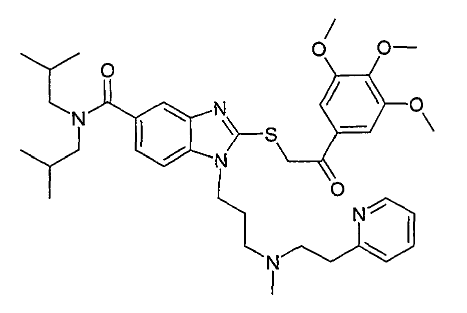

### Etape 1 : N,N-diisobutyl-1-{3-[méthyl(2-pyridin-2-yléthyl)amino]propyl}-2-thioxo-2,3-dihydro-1H-benzimidazole-5-carboxamide

Un mélange de 3-amino-*N*,*N*-diisobutyl-4-({3-[méthyl(2-pyridin-2-yléthyl)amino] propyl} amino) benzamide (1,52 g, 1 éq) et de thiocarbonyldiimidazole (0,74 g, 1.2 éq) dans le THF (30 ml) est agité à environ 20° C pendant 15 heures. Après concentration sous pression réduite à 40° C, le résidu obtenu est repris dans du dichlorométhane (80 ml) et de l'eau (30 ml). Après décantation et extraction, les phases organiques combinées sont lavées avec de la saumure, séchées sur Na₂SO₄ puis évaporées sous pression réduite à 40° C. La purification du résidu par chromatographie éclair sur gel de silice (éluant : dichlorométhane 100 % à dichlorométhane/ méthanol 8:2) donne le composé attendu sous forme d'une mousse beige claire (1,2 g ; 72 % rendement).
SM/CL: MM calculée = 481,7 ; m/z = 482,3 (MH+)
RMN (¹H, 400 MHz, DMSO-*d₆*): *δ* 0,64 (m, 6H), 0,91 (m, 6H), 1,79-2,03 (m, 4H), 2,18 (s, 3H), 2,37 (t, 3H, ³*J* = 6,5 Hz), 2,66 (t, 2H, ³*J* = 7 Hz), 2,83 (t, 2H, ³*J* = 7 Hz), 3,19 (m, 2H), 3,24 (m, 2H), 4,16 (t, 2H, ³*J* = 7 Hz), 7,05-7,65 (m, 6H), 8,43 (d, 1H), 12,79 (s, 1H).

### Etape 2 : N,N-diisobutyl-1-{3-[méthyl(2-pyridin-2-yléthyl)amino]propyl}-2-{[2-oxo-2-(3,4,5-triméthoxyphényl)éthyl]thio}-1H-benzimidazole-5-carboxamide

A une solution de *N,N*-diisobutyl-1-{3-[méthyl(2-pyridin-2-yléthyl)amino]propyl}-2-thioxo-2,3-dihydro-1H-benzimidazole-5-carboxamide dans le tétrahydrofuranne sont additionnées successivement la résine morpholinométhylpolystyrène (acquise auprès de Novabiochem, 2 éq) et la 2-bromo-1-(3,4,5-triméthoxyphényl)éthanone. Le mélange est agité 15 heures à environ 20° C puis additionné de tétrahydrofuranne, de résine aminométhylpoystyrène (2 éq, acquise auprès de Novabiochem) et de résine 4-bromométhylphénoxyméthyl-polystyrène (3 éq, acquise auprès de Novabiochem). Après 6h d'agitation, le mélange est filtré sur fritté. Le filtrat est ensuite concentré à sec sous pression réduite à 40° C pour donner le composé attendu.
SM/CL : MM calculée = 689,9 ; m/z = 690,5 (MH+)
RMN (¹H, 400 MHz, DMSO-*d₆*): *δ* 0,61 (m, 6H), 0,91 (m, 6H), 1,71-2,03 (m, 4H), 2,19 (s, 3H), 2,35 (t, 3H,³*J* = 6,5 Hz), 2,67 (t, 2H, ³*J* = 7 Hz), 2,85 (t, 2H, ³*J* = 7 Hz), 3,08-3,30 (m, 4H), 3,75 (s, 3H), 3,84 (s, 6H), 4,15 (t, 2H, ³*J* = 7 Hz), 5,09 (s, 2H), 7,11-7,67 (m, 8H), 8,45 (d, 1H).

Les composés suivants ont été préparés selon le schéma réactionnel B et de façon analogue à la procédure décrite pour la synthèse du *N,N*-diisobutyl-1-{3-[méthyl(2-pyridin-2-yléthyl)amino]propyl}-2-{[2-oxo-2-(3,4,5-triméthoxyphényl)éthyl]thio}-1H-benzimidazole-5-carboxamide

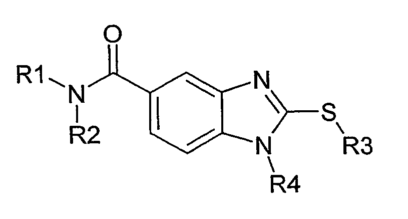

Dans la formule ci-dessus, R₁R₂N représente l'un des radicaux ci-après : 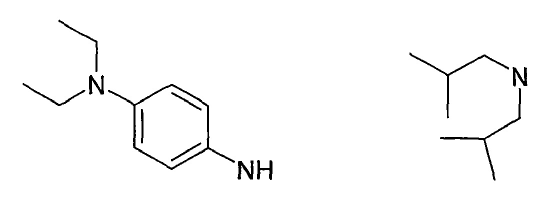 R₃ représente l'un des radicaux ci-après 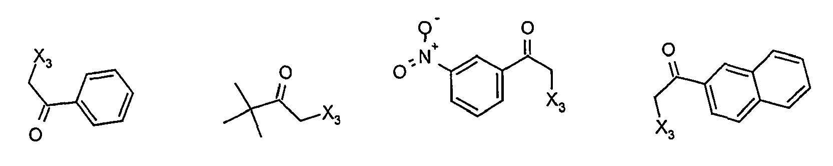 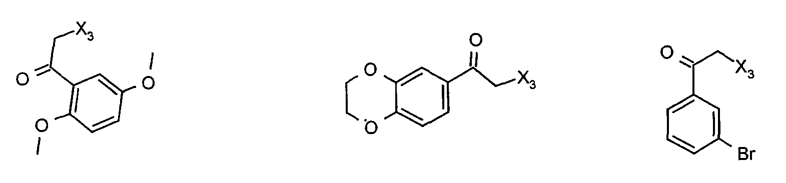 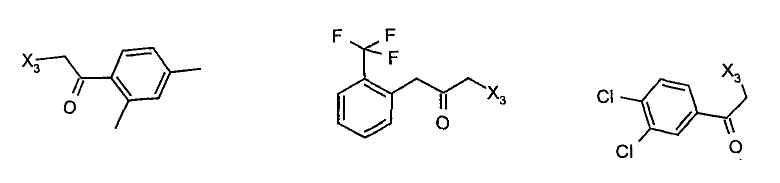 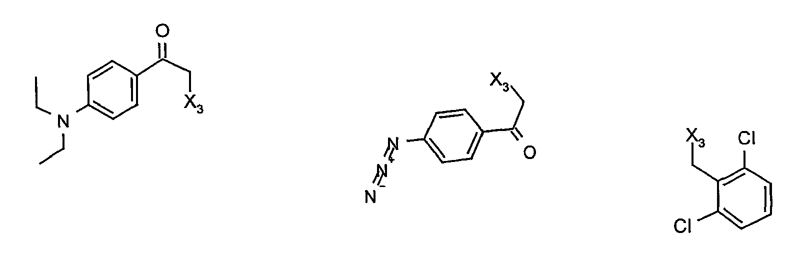 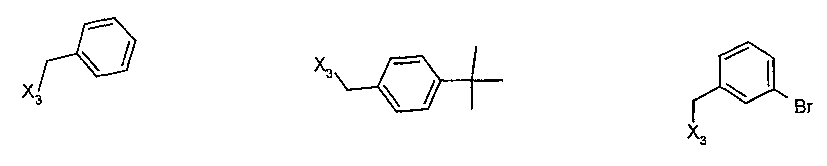 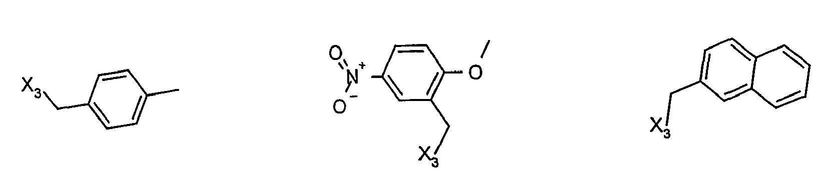 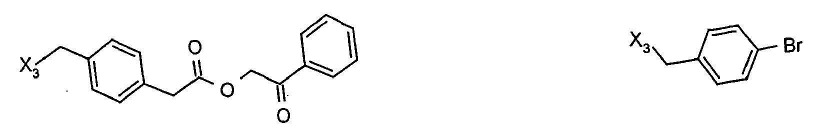 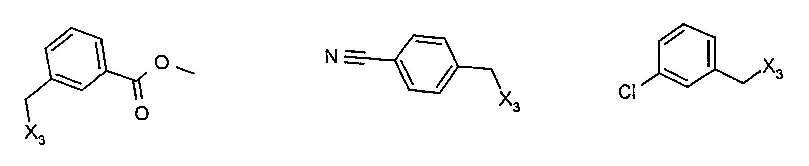 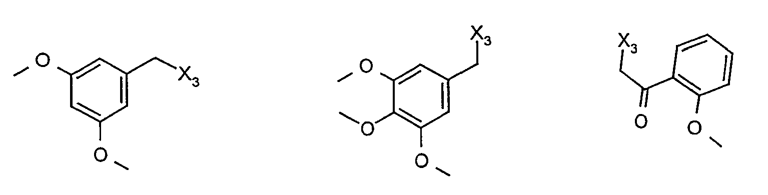 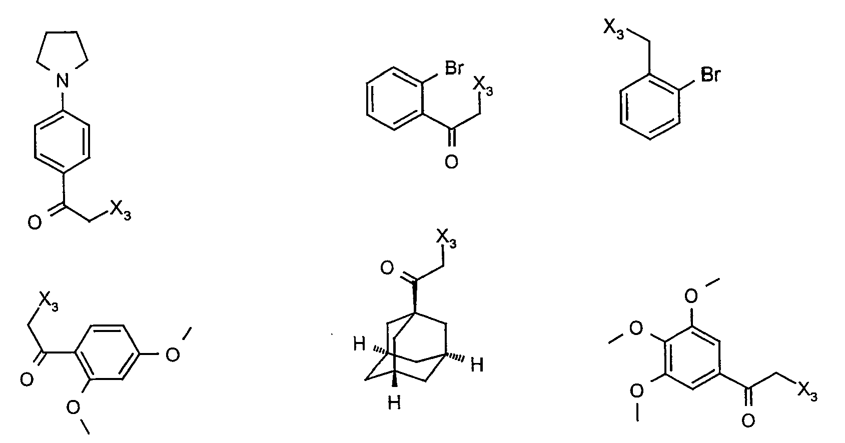 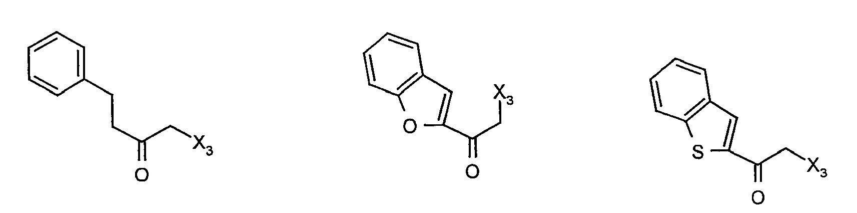 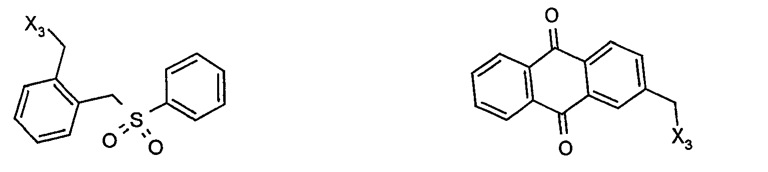 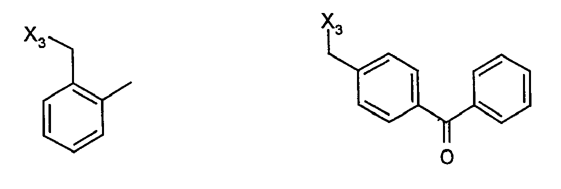 et R₄ représente l'un des radicaux ci-après

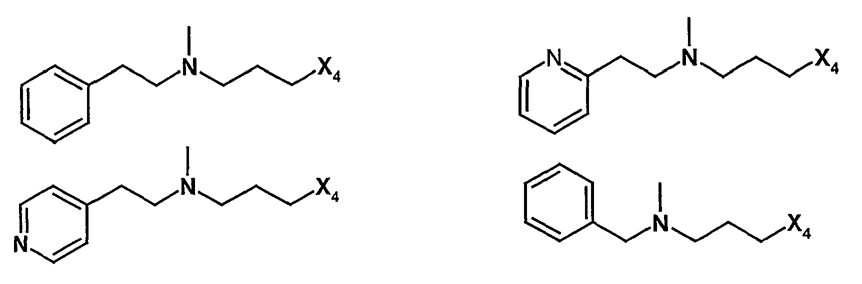

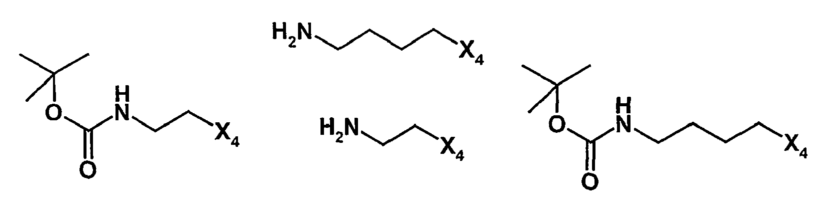

### C. Préparation selon le schéma réactionnel C :

Les composés de formule I selon l'invention dans laquelle Y représente -NH- et A -C(O)-, peuvent être préparés selon le schéma C suivant :

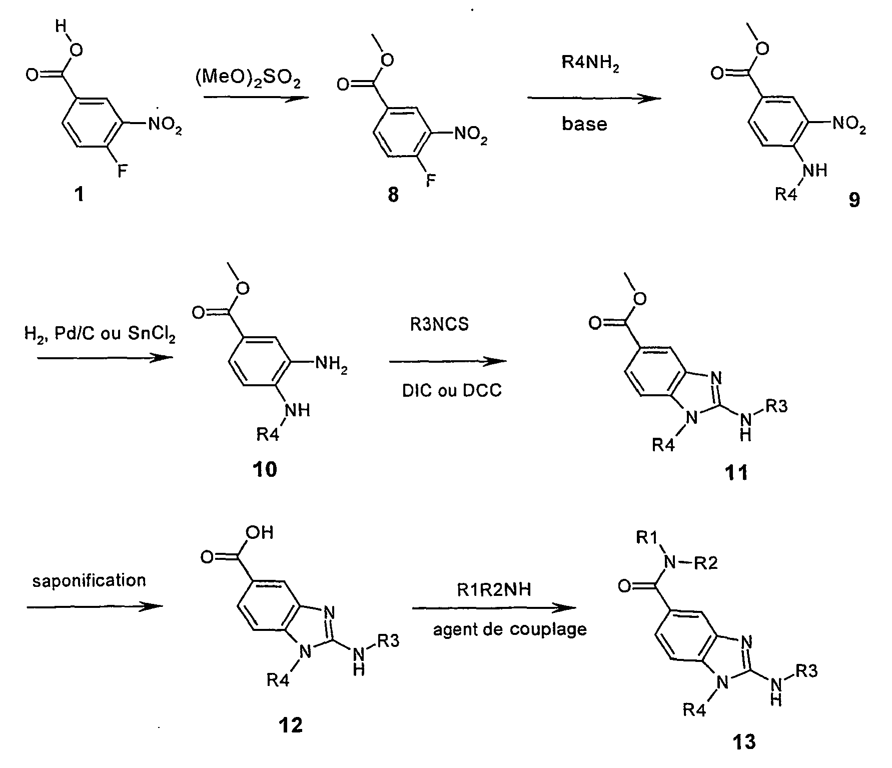

Comme décrit dans le schéma C, l'acide 4-fluoro-3-nitrobenzoïque peut être converti en ester méthylique (8) par formation d'un sel de carboxylate à l'aide d'une base inorganique telle que l'hydroxyde de lithium dihydrate ou le carbonate de césium, à température ambiante pendant 30 min à 2 heures, dans un solvant organique inerte tel que le tétrahydrofuranne, suivi de l'addition de diméthylsulfate à température ambiante et agitation à reflux pendant 5 à 15 heures. Le dérivé fluoré (8) peut-être traité avec une amine primaire en présence d'une base inorganique telle que le carbonate de césium ou de potassium dans un solvant organique inerte tel que le diméthylformamide ou l'acétonitrile à une température de 20-70° C pendant 2 à 16 heures pour conduire au dérivé (9). La fonction nitro du composé (9) est réduite par traitement avec du chlorure d'étain dihydrate dans un solvant inerte telle que l'acétate d'éthyle ou le diméthylformamide, à une température de 60-80° C pendant 3 à 15 heures, ou par hydrogénation catalytique en présence de palladium sur charbon 10 % dans un solvant inerte tel que le méthanol, l'éthanol, l'acétate d'éthyle ou un mélange de ces solvants, à une température de 18-25° C, pendant 2 à 8 heures, pour conduire à la dianiline (10). Le dérivé (10) est ensuite traité par un isothiocyanate en présence d'un agent de couplage tel que la diisopropylcarbodiimide ou la dicyclohexylcarbodiimide dans un solvant inerte tel que le tétrahydrofuranne, le chlorure de méthylène ou le chloroforme à une température de 20-70° C pendant 2 à 72 heures pour conduire au dérivé (11). Alternativement, le dérivé (10) peut être traité par un isothiocyanate dans un solvant inerte tel que le tétrahydrofuranne, chlorure de méthylène ou chloroforme puis la thiourée résultante peut être traitée par de l'iodure de méthyle dans un solvant polaire tel que l'éthanol pendant 3 à 24 heures à une température de 20-70° C pour conduire à (11). L'ester méthylique (11) peut ensuite être saponifié en présence d'une base inorganique telle que l'hydroxyde de lithium dihydrate dans un mélange de solvants polaires tels que l'eau et le tétrahydrofuranne à une température de 20 à 70° C pendant 3 à 17 heures. L'acide résultant (12) peut être couplé avec une amine primaire ou secondaire en présence d'un agent de couplage tel que le diisopropylcarbodiimide, le dicyclohexylcarbodiimide ou du carbonyldiimidazole, avec ou sans du 1-hydroxybenzotriazole (HOBt) dans un solvant organique inerte tel que du chlorure de méthylène, tétrahydrofuranne ou diméthylformamide à température ambiante pendant 3 à 24 heures. L'amide correspondante (13) peut être isolée, soit par chromatographie éclair sur gel de silice, soit par addition au mélange réactionnel d'un réactif nucléophile supporté sur un polymère comme par exemple une résine aminométhylpolystyrène et d'un réactif électrophile supporté sur un polymère comme par exemple la résine méthylisothiocyanate-polystyrène, suivie d'une filtration et de l'évaporation du filtrat.

### Exemple C1 : 1-(2-[(3,5-diméthoxyphényl)amino]-1-{3-[méthyl(2-pyridin-2-yléthyl) amino]propyl}-1H-benzimidazol-5-yl)-3-thien-2-yl propan-1-one

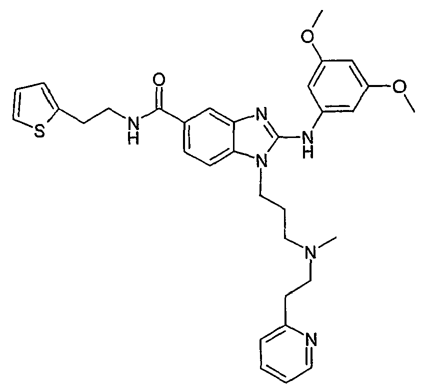

### Etape 1 : méthyl 4-fluoro-3-nitrobenzoate

A une solution de l'acide 4-fluoro-3-nitrobenzoïque (20 g, 1éq) dans le tétrahydrofuranne (100 ml) est additionné par petites portions l'hydroxyde de lithium monohydrate (4,5 g, 1 éq). Après 1h d'agitation à environ 20° C, le diméthylsulfate (10,2 ml) est additionné goutte à goutte au précipité jaune. Le mélange réactionnel est ensuite chauffé au reflux pendant 8 heures puis concentré sous pression réduite à 40° C. Le résidu est dilué dans le dichlorométhane et additionné d'eau saturé en Na₂CO₃. Après décantation et extraction, les phases organiques combinées sont lavées avec de la saumure, séchées sur sulfate de sodium et concentrées sous pression réduite à 40° C. Le solide jaune obtenu est recristallisé dans un mélange diéthylether / éther de pétrole pour conduire au composé attendu sous forme d'une poudre jaune clair (16,7 g, 78 %, de rendement). Point de fusion = 59°C.
RMN (¹H, 400 MHz, DMSO-*d₆*) : *δ* 3,99 (s, 3H), 7,39 (m, 1H), 8,33 (s, 1H), 8,74 (s, 1H).

### Etape 2 : méthyl 4-({3-[méthyl(2-pyridin-2-yléthyl)amino]propyl}amino)-3-nitro benzoate

Un mélange de méthyl 4-fluoro-3-nitrobenzoate (5,08 g, 1éq), de *N*-(2-pyridin-2-yléthyl)propane-1,3-diamine (5,4 g, 1.2 éq) et de carbonate de potassium (7,0 g, 2 éq) dans l'acétonitrile (180 ml) est chauffé au reflux pendant 3 heures puis concentré sous pression réduite à 40° C. Le résidu est repris par du dichlorométhane (150 ml) et de l'eau (60 ml). Après décantation et extraction, les phases organiques combinées sont lavées avec de la saumure, séchées sur Na₂SO₄ puis évaporées sous pression réduite à 40° C. La purification du composé par chromatographie éclair sur gel de silice (éluant : dichlorométhane à dichlorométhane/ méthanol 9 :1) donne le composé attendu sous forme d'une huile orangée (9,2 g ; 97 % rendement).
SM/CL : MM calculée = 372,4; m/z = 373,3 (MH+)
RMN (¹H, 400 MHz, DMSO-*d₆*): *δ* 1,75 (m, 2H), 2,23 (s, 3H), 2,48 (t, 3H, ³*J* = 6 Hz), 2,71 (t, 2H,³*J* = 7,8 Hz), 2,86 (t, 2H, ³*J* = 7,8 Hz), 3,35 (m, 2H), 3,81 (s, 3H), 7,05 (d, 1H), 7,10 (m, 1H), 7,23 (d, 1H), 7,59 (m, 1H), 7,93 (m,1H), 8,40 (d, 1H), 8,59 (s, 1H), 8,87 (t, 1H, ³*J* = 5 Hz).

### Etape 3 : méthyl 3-amino-4-({3-[méthyl(2-pyridin-2-yléthyl)amino]propyl}amino) benzoate

Dans un autoclave sont additionnés le méthyl 4-({3-[méthyl(2-pyridin-2-yléthyl)amino]propyl}amino)-3-nitrobenzoate (9,1 g) en solution dans un mélange d'acétate d'éthyle/ méthanol et le palladium sur charbon 10 % (910 mg). Après 4 heures d'agitation sous atmosphère d'hydrogène (3 bars), le catalyseur est éliminé par filtration sur célite et le filtrat est concentré sous pression réduite à 40° C pour donner le composé attendu sous forme d'une huile (8,2 g, 98 % rendement).
SM/CL : MM calculée = 342,4; m/z = 343,3 (MH+)
RMN (¹H, 400 MHz, DMSO-*d₆*): *δ* 1,71 (m, 2H), 2,21 (s, 3H), 2,46 (t, 3H, ³*J* = 6,8 Hz), 2,68 (t, 2H, ³*J* = 7 Hz), 2,86 (t, 2H, ³*J* = 7 Hz), 3,05 (m, 2H), 3,71 (s, 3H), 4,70 (s, 2H), 5,23 (t, 1H, ³*J* = 7 Hz), 6,37 (d, 1H), 7,14-7,26 (m, 4H), 7,64 (m, 1H), 8,45 (m, 1H).

### Etape 4 : méthyl-2-[(3,5-diméthoxyphényl)amino]-1-{3-[méthyl(2-pyridin-2-yléthyl) amino]propyl}-1H-benzimidazole-5-carboxylate

A une solution de méthyl 3-amino-4-({3-[méthyl(2-pyridin-2-yléthyl)amino] propyl}amino)benzoate (1,0 g, 1 éq) dans le tétrahydrofuranne (10 ml) sont successivement additionnés le 3,5 diméthoxyphénylisothiocyanate (571 mg, 1 éq) et le diisopropylcarbodiimide (1,35 ml, 4 éq). Le mélange est chauffé à reflux pendant 18 heures puis refroidi à température ambiante et concentré sous pression réduite à 40° C. Le résidu est repris par de l'acétate d'éthyle (100 ml) et de l'eau (40 ml). Après décantation et extraction, les phases organiques combinées sont lavées avec de la saumure, séchées sur Na₂SO₄ puis évaporées sous pression réduite à 40° C. La purification du résidu par chromatographie éclair sur gel de silice (éluant: dichlorométhane/ méthanol 99 :1 à 98 :2) donne le composé attendu sous forme d'une mousse beige (1,12 g ; 76 % rendement).
SM/CL : MM calculée = 503,6 m/z = 504,3 (MH+)
RMN (¹H, 400 MHz, CDCl₃) : δ 2,08 (m, 2H), 2,40 (t, 2H, ³J = 7 Hz), 2,45 (s, 3H), 2,99 (t, 2H, ³J = 7 Hz), 3,09 (t, 2H, ³J = 7 Hz), 3,82 (s, 6H), 3,93 (s, 3H), 4,01 (t, 2H, ³J = 6 Hz), 6,15 (m, 1H), 6,92-7,54 (m, 6H), 7,87 (m, 1H), 8,25 (s, 1H), 8,51 (m, 1H), 9.37 (s, 1H).

### Etape 5 : 2-[(3,5-diméthoxyphényl)amino]-1-{3-[méthyl(2-pyridin-2-yléthyl)amino] propyl}-1H-benzimidazole-5-carboxylic acide

A une solution de méthyl-2-[(3,5-diméthoxyphényl)amino]-1-{3-[méthyl(2-pyridin-2-yléthyl)amino] propyl}-1H-benzimidazole-5-carboxylate (1,05 g, 1 éq) dans un mélange de tétrahydrofuranne (10 ml) et d'eau (5 ml) est additionné l'hydroxyde de lithium (0,350 g, 4 éq). Le mélange est agité à 65° C pendant 18 heures puis refroidi à température ambiante et concentré sous pression réduite à 40° C. Le résidu est additionné d'acétate d'éthyle et d'eau. Le mélange est acidifié par addition d'acide acétique jusqu'à pH 5. Après décantation et extraction, les phases organiques combinées sont séchées sur sulfate de sodium et concentrées sous pression réduite. La purification par chromatographie éclair sur gel de silice (éluant : dichlorométhane/éthanol 95/5 à 70/30) donne le composé attendu sous forme d'une mousse blanche (0,93 g, 91 % rendement).
SM/CL: MM calculée = 489,6 ; m/z = 490,1 (MH+)
RMN (¹H, 400 MHz, DMSO-*d₆*): *δ* 1,88 (m, 2H), 2,23 (s, 3H), 2,31 (t, 2H, ³*J* = 6,5 Hz), 2,74 (t, 2H, ³*J* = 7 Hz), 2,91 (t, 2H, ³*J* = 7 Hz), 3,72 (s, 6H), 4,14 (t, 2H, ³*J* = 6,5 Hz), 6,14 (m, 1H), 7,09-7,72 (m, 8H), 7,93 (s, 1H), 8,44 (m, 1H), 9.21 (s, 1H).

### Etape 6 : 1-(2-[(3,5-diméthoxyphényl)amino]-1-{3-[méthyl(2-pyridin-2-yléthyl) amino]propyl}-1H-benzimidazol-5-yl)-3-thien-2-ylpropan-1-one

A une solution d'acide 2-[(3,5-diméthoxyphényl)amino]-1-{3-[méthyl(2-pyridin-2-yléthyl)amino]propyl}-1H-benzimidazole-5-carboxylique (24 mg, 1 éq) dans un mélange de diméthylformamide (0,2 ml) et tétrahydrofuranne (0,4 ml) est additionné le carbonyldiimidazole (10,5 mg, 1,3 éq) en solution dans le chloroforme (0,2 ml). Le mélange est agité pendant 15 heures à environ 20° C puis la thiophène-2-éthylamine (13 mg, 2 éq) en solution dans le tétrahydrofuranne (0,1 ml) est additionnée. Après 15 heures d'agitation à environ 20° C, au mélange dilué dans du dichlorométhane sont additionnées la résine aminométhylpolystyrène (2 éq), la résine TBD-méthyl polystyrène (2 éq) et la résine méthylisothiocyanate-polystyrène (4 éq). Après 6 heures d'agitation à environ 20° C, le mélange est filtré et le filtrat est concentré sous pression réduite à 40° C pour donner le composé attendu sous forme d'une huile (27 mg, 90 % rendement).
SM/CL : MM calculée = 598,8 ; m/z = 599,2 (MH+)
RMN (¹H, 400 MHz, DMSO-*d₆*) : *δ* 1,87 (m, 2H), 2,26 (s, 3H), 2,48 (t, 2H, ³*J* = 6,5 Hz), 2,78 (m, 2H), 2,93 (t, 2H, ³*J* = 7 Hz), 3,08 (t, 2H, ³*J* = 7 Hz), 3,50 (m, 2H), 3,72 (s, 6H), 4,14 (t, 2H, ³*J* = 6,5 Hz), 6,14 (m, 1H), 6,92-7,93 (m, 12H), 8,45 (m, 1H), 9,16 (s, 1H).

Les composés suivants ont été préparés selon le schéma réactionnel C et de façon analogue à la procédure décrite pour la synthèse du 1-(2-[(3,5-diméthoxyphényl)amino]-1-{3-[méthyl(2-pyridin-2-yléthyl)amino]propyl}-1H-benzimidazol-5-yl)-3-thien-2-ylpropan-1-one :

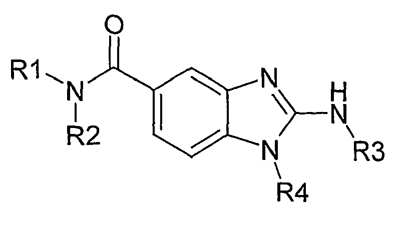

Dans la formule ci-dessus, R₁R₂N représente l'un des radicaux ci-après : 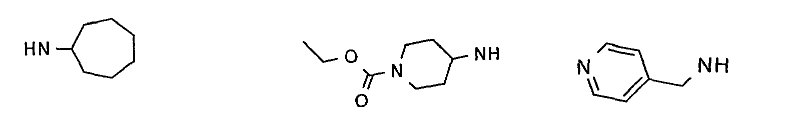 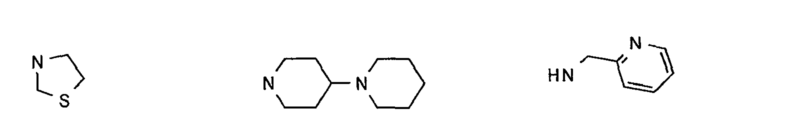 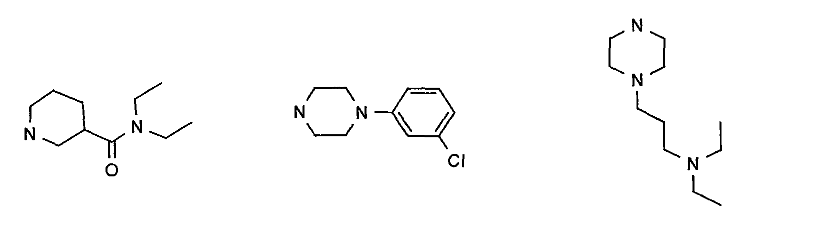 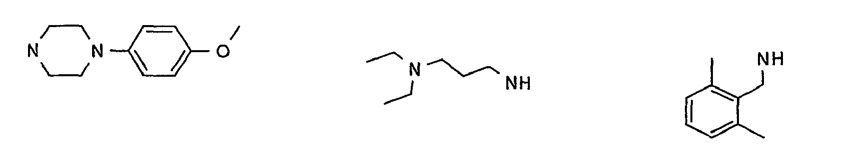 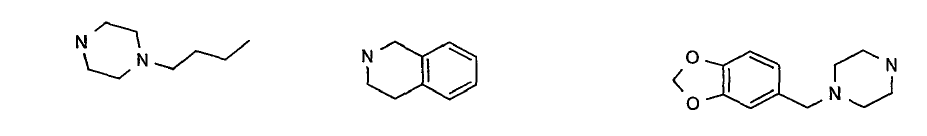 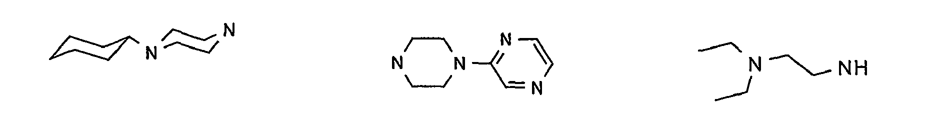 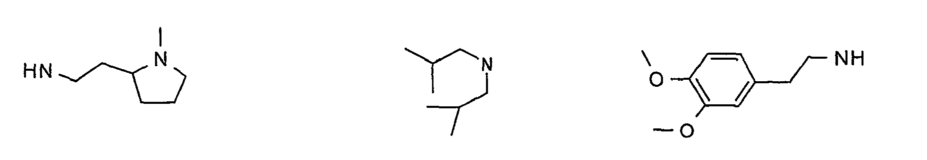 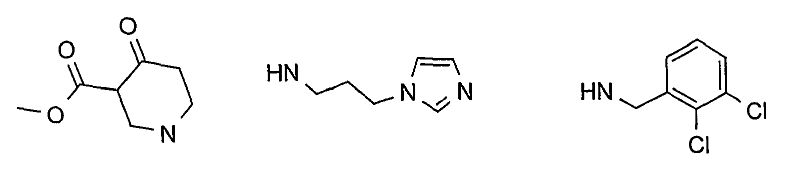 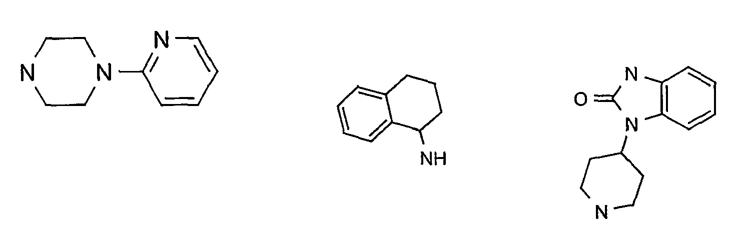 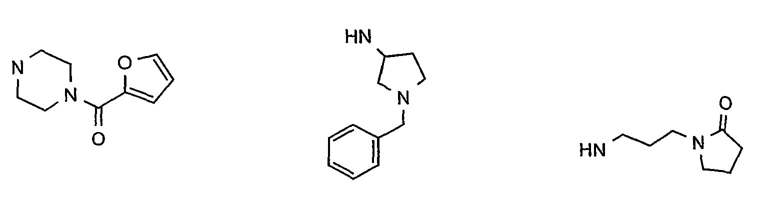 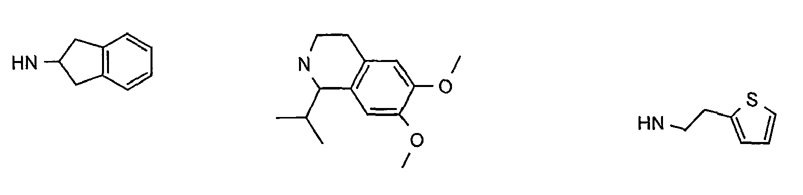 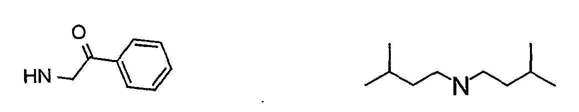 R₃ représente l'un des radicaux ci-après : 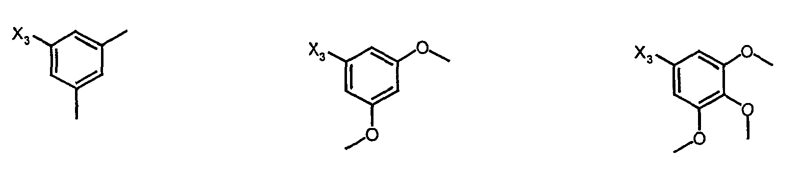 et R₄ représente l'un des radicaux ci-après :

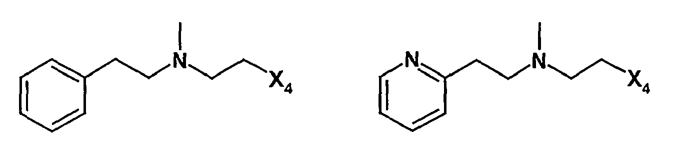

### D. Préparation selon le schéma réactionnel D :

Les composés de formule I selon l'invention dans laquelle Y représente -S- et A -C(O)-, peuvent être préparés selon le schéma D suivant :

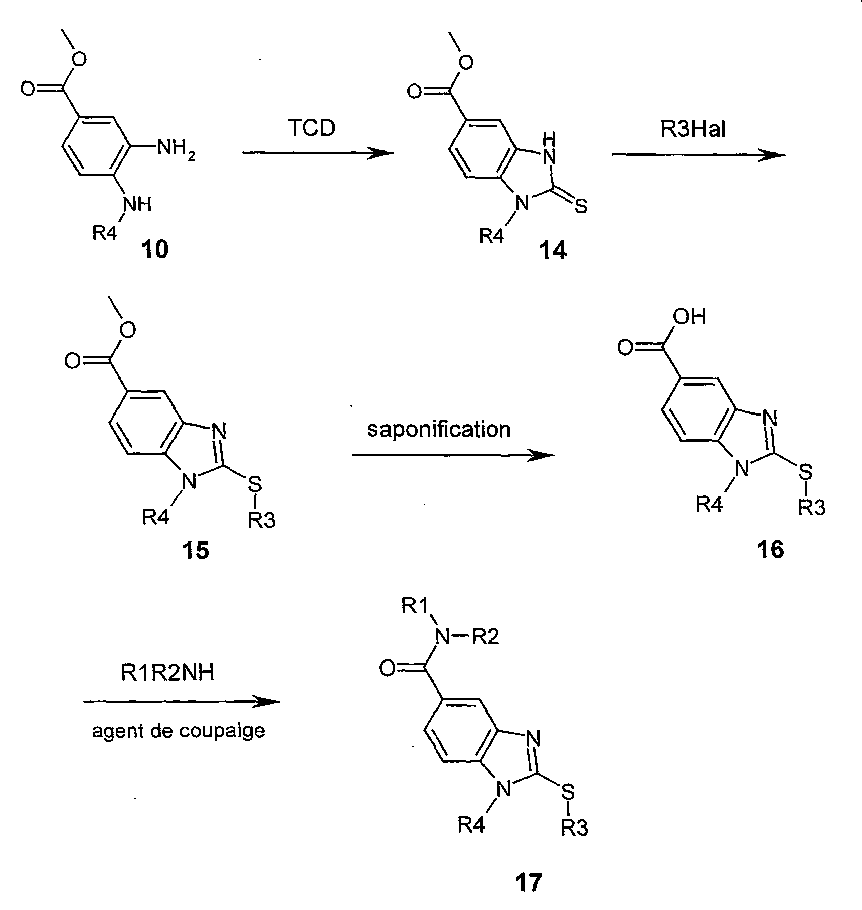

Comme décrit dans le schéma D, la dianiline (10) peut être traitée avec du thiocarbonyldiimidazole (TCD) ou du thiophosgène dans un solvant inorganique inerte tel que le tétrahydrofuranne, à température ambiante pendant 2 à 17 heures pour conduire au dérivé (14). Le composé (14) est ensuite alkylé par réaction avec un dérivé halogéné tel qu'un iodure, bromure ou chlorure d'alkyle ou de benzyle ou une bromocétone, en présence d'une base tertiaire telle que la triéthylamine ou la diisopropyléthylamine, dans un solvant organique inerte tel que le tétrahydrofuranne, le choroforme ou le chlorure de méthylène, à une température de 20-70° C pendant 3 à 24 heures pour conduire au dérivé thiobenzimidazole (15). L'ester méthylique (15) peut ensuite être saponifié en présence d'une base inorganique telle que l'hydroxyde de lithium monohydrate dans un mélange de solvants polaires tels que l'eau et le tétrahydrofuranne à une température de 20 à 70° C pendant 3 à 17 heures. L'acide résultant (16) peut être couplé avec une amine primaire ou secondaire en présence d'un agent de couplage tel que le diisopropylcarbodiimide, le dicyclohexylcarbodiimide ou le carbonyldiimidazole, avec ou sans du 1-hydroxybenzotriazole (HOBt) dans un solvant organique inerte tel que du chlorure de méthylène, tétrahydrofuranne ou diméthylformamide à température ambiante pendant 3 à 24 heures. L'amide correspondante (17) peut être isolée, soit par chromatographie éclair sur gel de silice, soit par addition au mélange réactionnel d'un réactif nucléophile supporté sur un polymère comme par exemple une résine aminométhylpolystyrène et d'un réactif électrophile supporté sur un polymère comme par exemple la résine méthylisothiocyanate-polystyrène, suivie d'une filtration et de l'évaporation du filtrat.

### Exemple D1 : 3-(2-[(3-bromobenzyl)sulfanyl]-5-{[4-(1-pyrrolidinyl)-1-pipéridinyl] carbonyl}-1H-benzimidazol-1-yl)-N-méthyl-N-[2-(2-pyridinyl)éthyl]-1-propanamine

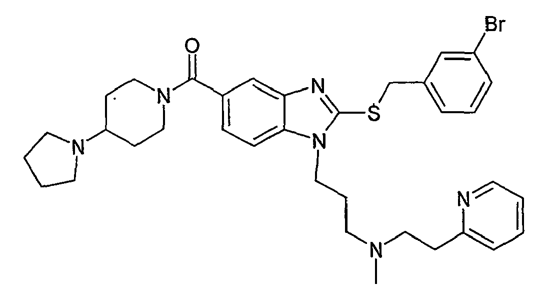

### Etape 1 : méthyl 1-{3-[méthyl(2-pyridin-2-yléthyl)amino]propyl}-2-thioxo-2,3-dihydro-1H-benzimidazole-5-carboxylate

Un mélange de méthyl 3-amino-4-({3-[méthyl(2-pyridin-2-yléthyl)amino] propyl}amino)benzoate (4,09 g, 1éq) et de thiocarbonyldiimidazole (2,77 g, 1,3 éq) dans le tétrahydrofuranne (100 ml) est agité à environ 20° C pendant 15 heures. Après concentration sous pression réduite à 40° C, le résidu obtenu est repris dans du dichlorométhane (150 ml) et de l'eau (50 ml). Après décantation et extraction, les phases organiques combinées sont lavées avec de la saumure, séchées sur Na₂SO₄ puis évaporées sous pression réduite à 40° C. La purification par chromatographie éclair sur gel de silice (éluant : dichlorométhane 100 % à dichlorométhane/ méthanol 9:1) donne le composé attendu sous forme d'une mousse (3,94 g ; 85 % rendement).
SM/CL : MM calculée = 384,5; m/z = 385,2 (MH+)
RMN (¹H, 400 MHz, DMSO-*d₆*) : *δ* 1,86 (m, 2H), 2,18 (s, 3H), 2,37 (t, 3H, ³*J* = 6,8 Hz), 2,65 (t, 2H, ³*J* = 7 Hz), 2,84 (t, 2H, ³*J* = 7 Hz), 3,85 (s, 3H), 4,16 (t, 2H, ³*J* = 7 Hz), 7,16-7,81 (m, 6H), 8,44 (m, 1H), 12,95 (s, 1H).

### Etape 2 : méthyl 2-[(3-bromobenzyl)thio]-1-{3-[méthyl(2-pyridin-2-yléthyl)amino] propyl}-1H-benzimidazole-5-carboxylate

A une solution de méthyl 1-{3-[méthyl(2-pyridin-2-yléthyl)amino]propyl}-2-thioxo-2,3-dihydro-1H-benzimidazole-5-carboxylate (1,5 g) dans le tétrahydrofuranne (30 ml) sont additionnées successivement la triéthylamine (0,82 ml, 1,6 éq) et la 3-bromobenzylbromide (0,97 g, 1 éq). Le mélange est agité 15 heures à environ 20° C puis concentré sous pression réduite à 40° C. Le résidu obtenu est dilué dans l'acétate d'éthyle et l'eau. Après décantation et extraction, les phases organiques sont lavées avec de la saumure, séchées sur sulfate de sodium et concentrées sous pression réduite à 40° C. La purification par chromatographie éclair sur gel de silice (éluant : dichlorométhane/ méthanol 95/5 à 90/10) donne le composé attendu sous forme d'une huile incolore (1,5 g ; 70 % rendement).
SM/CL : MM calculée = 553,5; m/z = 553,3 (MH+)
RMN (¹H, 400 MHz, DMSO-*d₆*): *δ* 1,76 (m, 2H), 2,14 (s, 3H), 2,27 (t, 3H, ³*J* = 6,5 Hz), 2,62 (t, 2H, ³*J* = 7 Hz), 2,81 (t, 2H, ³*J* = 7 Hz), 3,86 (s, 3H), 4,06 (t, 2H, ³*J* = 7 Hz), 4,61 (s, 2H), 7,15-7,82 (m, 9H), 8,13 (s, 1H), 8.43 (d, 1H).

### Etape 3 : acide 2-[(3-bromobenzyl)thio]-1-{3-[méthyl(2-pyridin-2-yléthyl)amino] propyl}-1H-benzimidazole-5-carboxylique

A une solution de méthyl 2-[(3-bromobenzyl)thio]-1-{3-[méthyl(2-pyridin-2-yléthyl)amino]propyl}-1H-benzimidazole-5-carboxylate (1,03 g, 1 éq) dans un mélange de tétrahydrofuranne (10 ml) et d'eau (5 ml) est additionné l'hydroxyde de lithium (0,315 g, 3 éq). Le mélange est chauffé à reflux pendant 18 heures puis refroidi à température ambiante et concentré sous pression réduite à 40° C. Le résidu est additionné d'acétate d'éthyle et d'eau. Le mélange est acidifié par addition d'acide acétique jusqu'à pH 5. Après décantation et extraction, les phases organiques combinées sont séchées sur sulfate de sodium et concentrées sous pression réduite. La purification par chromatographie éclair sur gel de silice (éluant : dichlorométhane/méthanol 95/5 à 80/20) donne le composé attendu sous forme d'une mousse (0,85 g, 85 % rendement).
SM/CL : MM calculée = 539,5 ; m/z = 539,2 (MH+)
RMN (¹H, 400 MHz, DMSO-*d₆*) : *δ* 1,76 (m, 2H), 2,14 (s, 3H), 2,29 (t, 3H, ³*J* = 6,5 Hz), 2,62 (t, 2H, ³*J* = 7 Hz), 2,82 (t, 2H, ³*J* = 7 Hz), 4,04 (t, 2H, ³*J* = 7 Hz), 4,61 (s, 2H), 7,15-7,82 (m, 9H), 8,10 (s, 1H), 8,43 (d, 1H).

### Etape 4 : 3-(2-[(3-bromobenzyl)sulfanyl]-5-{[4-(1-pyrrolidinyl)-1-piperidinyl] carbonyl}-1H-benzimidazol-1-yl)-N-méthyl-N-[2-(2-pyridinyl)éthyl]-1-propanamine

A une solution d'acide 2-[(3-bromobenzyl)thio]-1-{3-[méthyl(2-pyridin-2-yléthyl)amino]propyl}-1H-benzimidazole-5-carboxylique (27 mg, 1 éq) dans un mélange de diméthylformamide (0,2 ml) et tétrahydrofuranne (0,4 ml) est additionné le carbonyldiimidazole (10,5 mg, 1,3 éq) en solution dans le chloroforme (0,2 ml). Le mélange est agité pendant 15 heures à environ 20° C puis la 4-(1-pyrrolidinyl)piperidine (15 mg, 2 éq) est additionnée. Après 15 heures d'agitation à environ 20° C, au mélange dilué dans du dichlorométhane sont additionnées la résine aminométhylpolystyrène (2 éq, acquise auprès de Novabiochem), la résine TBD -méthyl polystyrène (2 éq, acquise auprès de Novabiochem) et la résine méthylisothiocyanate-polystyrène (4 éq, acquise auprès de Novabiochem). Après 6 heures d'agitation à environ 20° C, le mélange est filtré et le filtrat est concentré sous pression réduite à 40° C pour donner le composé attendu sous forme d'une huile (28 mg, 84 % rendement).
SM/CL : MM calculée = 675,7 ; m/z = 674,2 (MH+)
RMN (¹H, 400 MHz, CDCl₃) : *δ* 1,4-1,98 (m, 10H), 2,26 (s, 3H), 2,32 (m, 5H), 2,60-3,15 (m, 8H), 3,81 (m, 1H), 4,01 (t, 2H, ³J= 7 Hz), 4,50 (m, 1H), 4,57 (s, 2H), 7,08-7,72 (m, 10H), 8,51 (d, 1H).

Les composés suivants ont été préparés selon le schéma réactionnel D et de façon analogue à la procédure décrite pour la synthèse du 3-(2-[(3-bromobenzyl)sulfanyl]-5-{[4-(1-pyrrolidinyl)-1-piperidinyl]carbonyl)-1H-benzimidazol-1-yl)-*N*-méthyl-*N-*[2-(2-pyridinyl)éthyl]-1-propanamine :

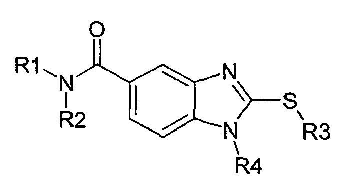

Dans la formule ci-dessus, R₁R₂N représente l'un des radicaux ci-après : 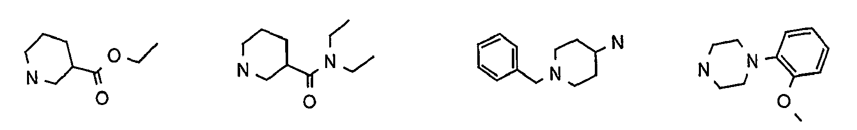 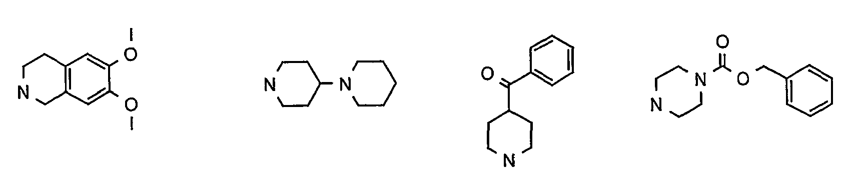 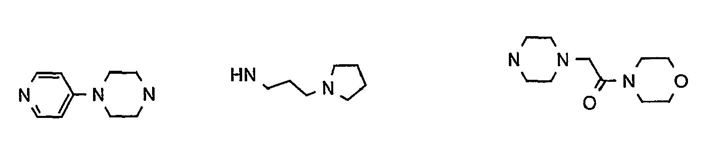 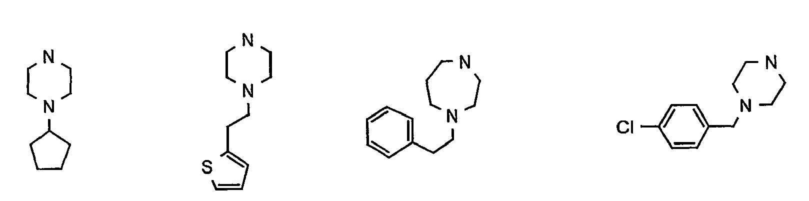 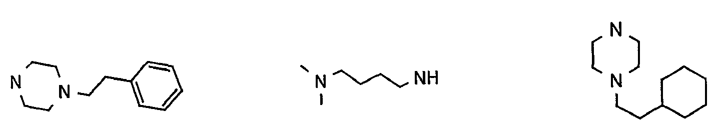 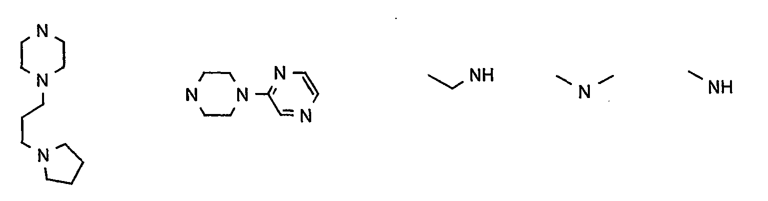 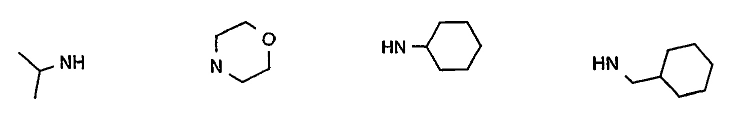 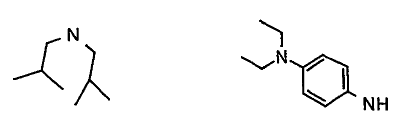 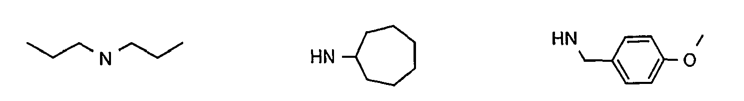 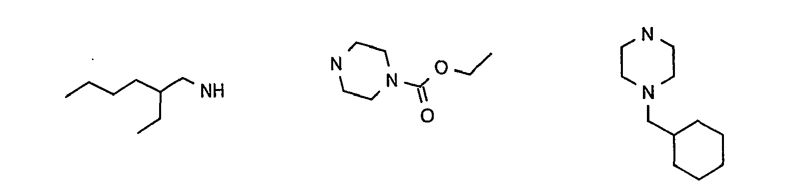 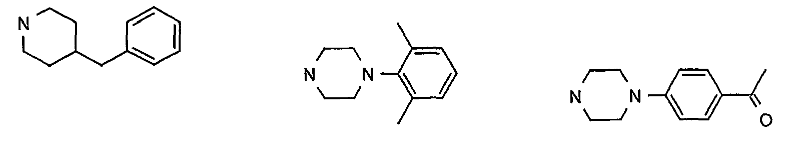 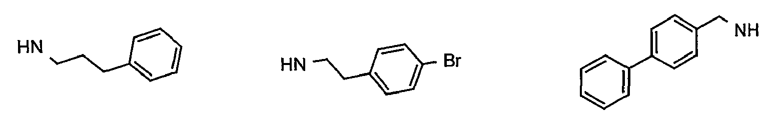 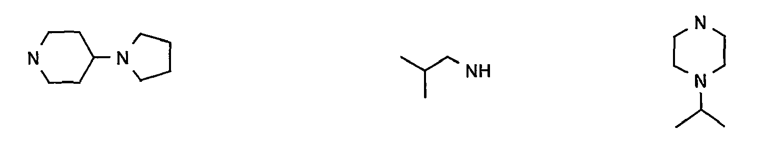 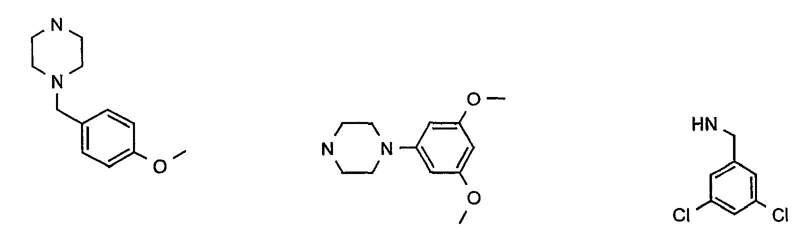 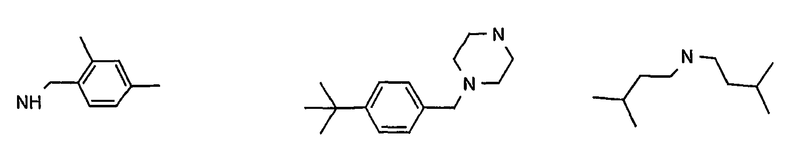 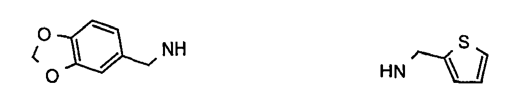 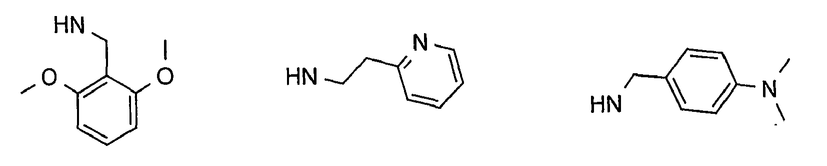 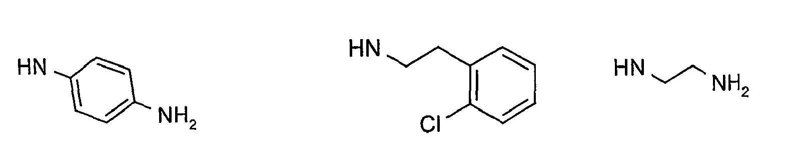 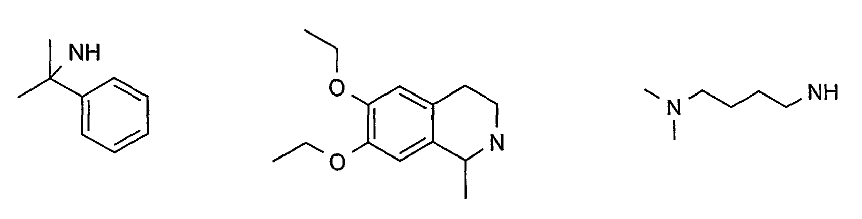 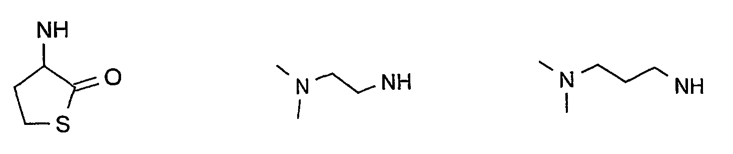 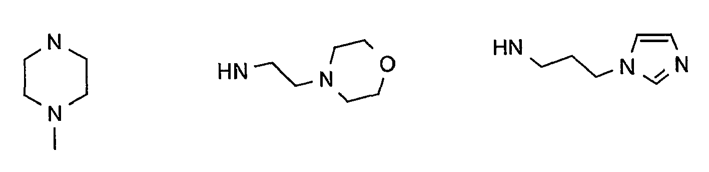 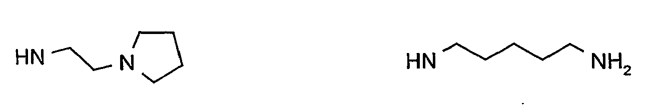 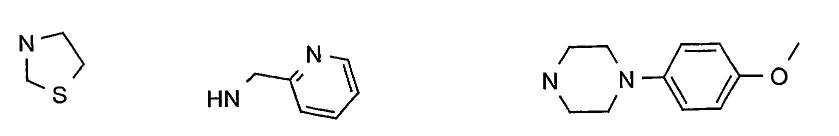 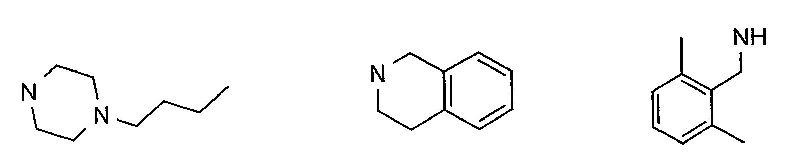 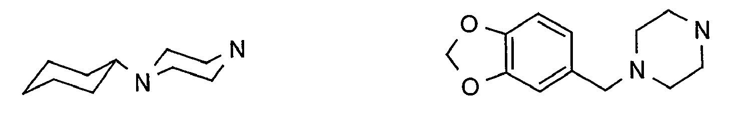 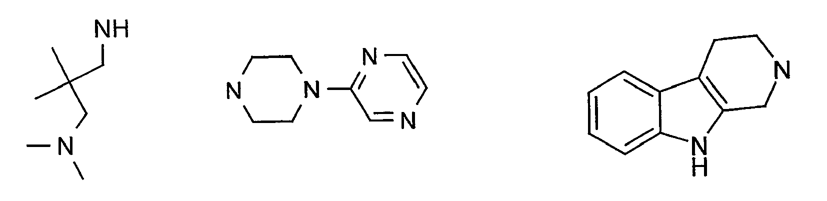 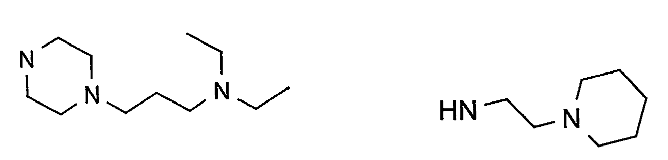 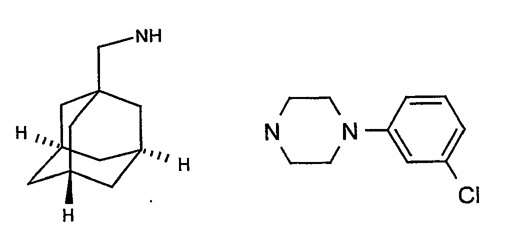 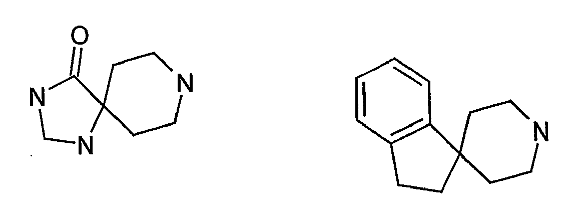 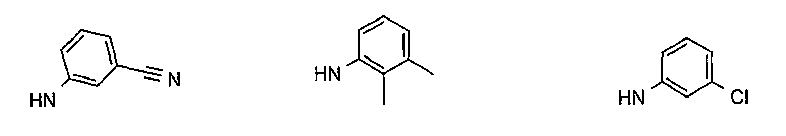 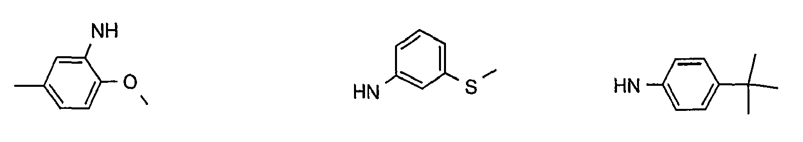 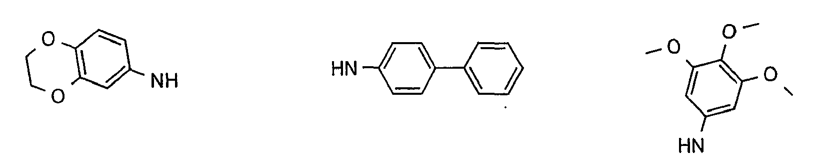 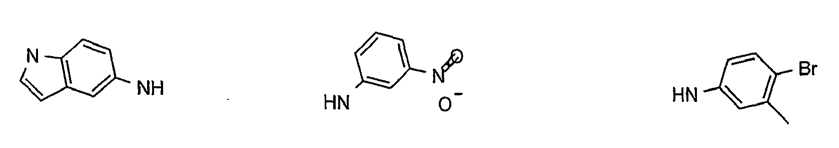 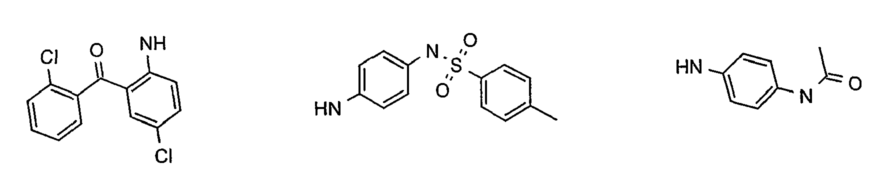 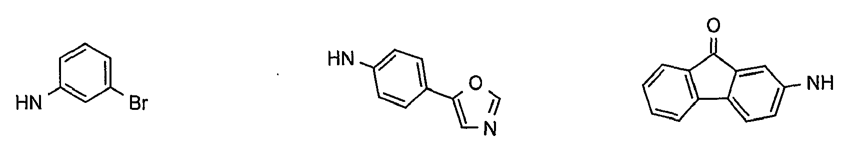 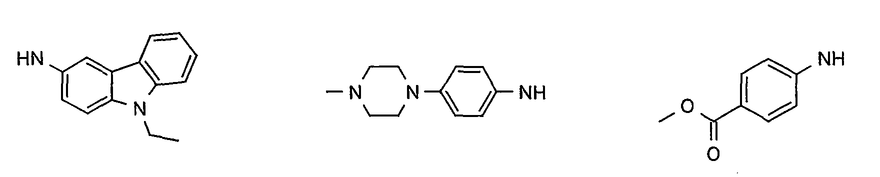 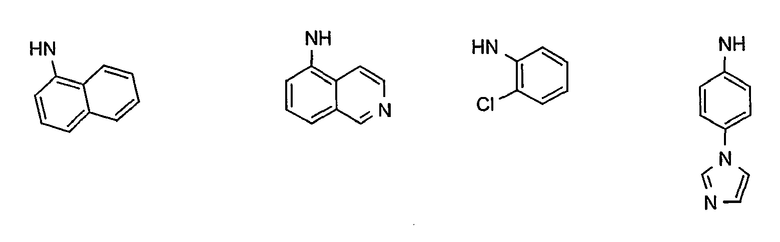 R₃ représente l'un des radicaux ci-après : 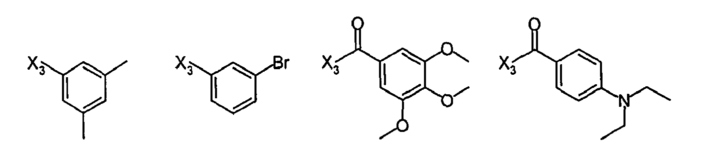 et R₄ représente l'un des radicaux ci-après :

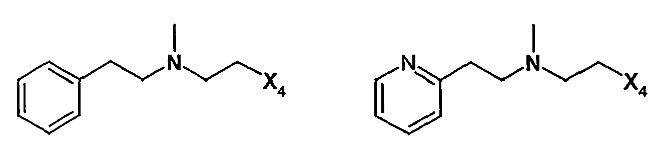

### E. Préparation selon le schéma réactionnel E :

Les composés de formule I selon l'invention dans laquelle A représente -(CH₂)- peuvent être préparés à partir des composés dans laquelle A représente -C(O)-, selon le schéma E suivant :

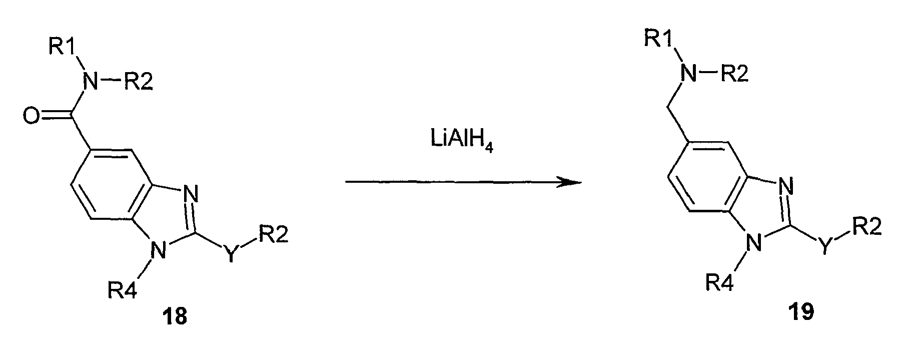

Comme décrit dans le schéma E, l'amide (18) préparée selon les schémas réactionnels A ou B, peut être réduite en amine correspondante (19) à l'aide de borane ou d'hydrure de lithium aluminium dans un solvant aprotique tel que le tétrahydrofuranne ou l'éther diéthylique à une température de 0 à 70° C, pendant 1 à 6 heures.

### Exemple E1 : 5-[(diisobutylamino)méthyl]-1-(3-{méthyl[2-(2-pyridinyl)éthyl] amino}propyl)-N-(3,4,5-triméthoxyphényl)-1H-benzimidazol-2-amine

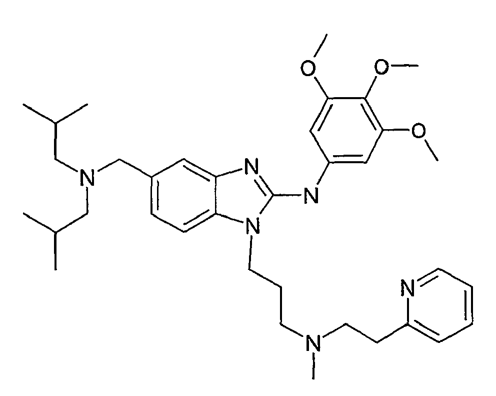

A une solution refroidie à 0° C de *N,N*-diisobutyl-1-{3-[méthyl(2-pyridin-2-yléthyl) amino]propyl}-2-[(3,4,5-triméthoxyphényl)amino]-1H-benzimidazole-5-carboxamide (105 mg, 1 éq. préparé selon l'exemple A1) dans le tétrahydrofuranne (3 ml) est additionnée goutte à goutte une solution molaire d'hydrure de lithium aluminium dans le tétrahydrofuranne (0,83 ml, 5 éq). Après 15 minutes d'agitation à 0° C, le mélange est chauffé à 60° C pendant 3 heures puis refroidi à 0° C et hydrolysé. Après addition d'acétate d'éthyle, décantation et extraction, les phases organiques réunies sont lavées avec de la saumure, séchées sur sulfate de sodium et concentrées sous pression réduite. La purification par chromatographie éclair sur gel de silice (éluant : dichlorométhane 100 % à dichlorométhane/méthanol 9 :1) donne le composé attendu sous forme d'une mousse (63 mg, 62 % rendement).
SM/CL : MM calculée = 616,8; m/z = 617,4 (MH+)
RMN (¹H, 400 MHz, DMSO-d₆) : *δ* 0,81 (d, 12H), 1,77 (m, 2H), 1,86 (m, 2H), 2,06 (d, 4H), 2,24 (s, 3H), 2,49 (t, 2H, ³J = 6 Hz), 2,74 (t, 2H, ³J = 7 Hz), 2,91 (t, 2H, ³J = 7 Hz), 3,48 (s, 2H), 3,62 (s, 3H), 3,78 (s, 6H), 4,05 (m, 2H), 6,97 (d, 1H), 7,13-7,24 (m, 5H), 7,63 (m, 1H), 8,43 (d, 1H), 8,94 (s, 1H).

### F. Préparation selon le schéma réactionnel F :

Les composés de formule I selon l'invention dans laquelle Y représente -S- et NH- et A -CH₂-, peuvent être préparés selon le schéma F suivant :

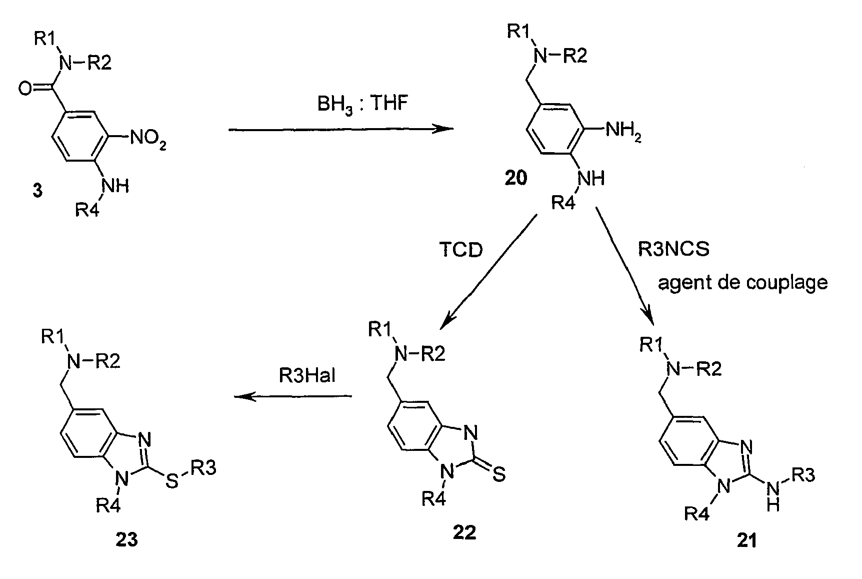

Comme décrit dans le schéma F, le dérivé (3) peut être réduit en composé (20) à l'aide de borane dans un solvant aprotique tel que le tétrahydrofuranne ou l'éther diéthylique à une température de 0 à 70° C, pendant 18 à 24 heures. La dianiline (20) peut être ensuite traitée par un isothiocyanate en présence d'un agent de couplage supporté sur une résine ou non tel que le diisopropylcarbodiimide ou dicyclohexylcarbodiimide ou la résine *N*-méthylcyclohexylcarbodiimide *N*-méthyl polystyrène dans un solvant inerte tel que le tétrahydrofuranne, chlorure de méthylène, ou chloroforme à une température de 20-70° C pendant 2 à 72 heures pour conduire au dérivé (21). Alternativement, le dérivé (20) peut être traité par un isothiocyanate dans un solvant inerte tel que le tétrahydrofuranne, chlorure de méthylène ou chloroforme puis la thiourée résultante peut être traitée par de l'iodure de méthyle dans un solvant polaire tel que l'éthanol pendant 3 à 24 heures à une température de 20-70° C pour conduire à (21).

Comme décrit également dans le schéma réactionnel B et l'exemple B1, la dianiline (20) peut être traitée avec du thiocarbonyldiimidazole (TCD) ou du thiophosgène dans un solvant organique inerte tel que le tétrahydrofuranne, le chlorure de méthylène ou le chloroforme à température ambiante pendant 2 à 17 heures pour conduire au dérivé (22). Le composé (22) est ensuite alkylé par réaction avec un dérivé halogéné tel qu'un iodure, bromure ou chlorure d'alkyle ou de benzyle ou une bromocétone, en présence d'une base tertiaire telle que la triéthylamine ou la diisopropyléthylamine, ou en présence d'une base tertiaire supportée sur une résine telle que la résine morpholinométhyl-polystyrène, dans un solvant organique inerte tel que le tetrahydrofuranne, le choroforme ou le chlorure de méthylène, à une température de 20-70° C pendant 3 à 24 heures. Le dérivé thiobenzimidazole (23) résultant peut être isolé, soit par chromatographie éclair sur gel de silice, soit par addition au mélange réactionnel d'un réactif nucléophile supporté sur un polymère comme par exemple une résine aminométhylpolystyrène, et d'un réactif électrophile supporté sur un polymère comme par exemple la résine 4-bromométhylphénoxyméthylpolystyrène, suivie d'une filtration et de l'évaporation du filtrat.

### Exemple F1 : 5-[(diisobutylamino)méthyl]-1-(3-{méthyl[2-(2-pyridinyl)éthyl amino}propyl)-N-(3,4,5-triméthoxyphéhyl)-1H-benzimidazol-2-amine

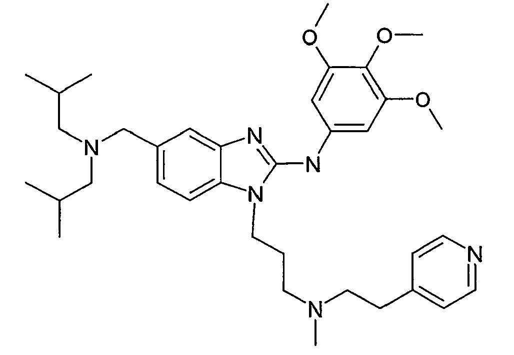

### Etape 1 : 4-[(diisobutylamino)méthyl]-N-(3-{méthyl[2-(4-pyridinyl)éthyl]amino} propyl)-1,2-benzènediamine

A une solution de *N,N*-diisobutyl-4-({3-[méthyl(2-pyridin-4-yléthyl)amino]propyl} amino)-3-nitrobenzamide (200 mg, 1 éq) dans le tétrahydrofuranne (3 ml) refroidie à 0° C, est additionnée goutte à goutte une solution molaire de complexe borane-tétrahydrofuranne (6,25 ml, 15 éq). Le mélange est chauffé au reflux pendant 20 heures puis refroidi à 0° C et hydrolysé par une solution aqueuse d'acide chlorhydrique 6N (12 ml). Après 1 h 30 de reflux, le mélange est refroidi à 0° C et amené à pH basique par une solution aqueuse de soude 6N. Après addition d'acétate d'éthyle, décantation et extraction, les phases organiques sont réunies, lavées par de la saumure, séchées sur sulfate de sodium et évaporées sous pression réduite. La purification par chromatographie éclair sur gel de silice (éluant: dichlorométhane 100 % à dichlorométhane / méthanol 8:2) donne le composé attendu sous forme d'une huile (92 mg, 51 % rendement).
SM/CL : MM calculée = 425,6 ; m/z = 426,4 (MH+)
RMN (¹H, 400 MHz, DMSO-d₆) : *δ* 0,83 (d, 12H), 1,72 (m, 4H), 2,03 (d, 4H, ³J = 7 Hz), 2,23 (s, 3H), 2,48 (t, 2H, ³J = 7 Hz), 2,60 (t, 2H, ³J = 7 Hz), 2,75 (t, 2H, ³J = 7 Hz), 2,96 (m, 2H), 3,38 (s, 2H), 4,30 (m, 3H), 6,30 (d, 1H), 6,42 (d, 1H), 6,51 (s, 1H), 7,25 (d, 1H), 7,45 (m, 1H), 8,41 (m, 2H).

### Etape 2 : 5-[(diisobutylamino)méthyl]-1-(3-{méthyl[2-(2-pyridinyl)éthyl]amino} propyl)-N-(3,4,5-triméthoxyphényl)-1H-benzimidazol-2-amine

A une solution de 4-[(diisobutylamino)méthyl]-*N*-(3-{méthyl[2-(4-pyridinyl)éthyl] amino}propyl)-1,2-benzenediamine (90 mg, 1 éq) dans le tétrahydrofuranne (2 ml) sont successivement additionnés le 3,4,5 triméthoxyphénylisothiocyanate (57 mg, 1,2 éq) et la résine *N*-méthylcyclohexylcarbodiimide-*N*-méthyl-polystyrène (acquise auprès de Novabiochem ; charge 1,69 mmol/g, 501 mg, 4 éq). Le mélange est chauffé à reflux pendant 18 heures puis refroidi à température ambiante et additionné de résine aminométhylpolystyrène (acquise auprès de Novabiochem, 2 éq). Après 4 h d'agitation à température ambiante, le mélange est filtré sur fritté et le filtrat est concentré sous pression réduite à 40° C. La purification par chromatographie éclair sur gel de silice (éluant : dichlorométhane 100 % à dichlorométhane/méthanol 9:1) donne le composé attendu sous forme d'une mousse beige (92 mg, 83 % rendement).
SM/CL : MM calculée = 616,8; m/z = 617,4 (MH+)
RMN (¹H, 400 MHz, DMSO-d₆) : *δ* 0,81 (d, 12H), 1,77 (m, 2H), 1,86 (m, 2H), 2,06 (d, 4H), 2,22 (s, 3H), 2,31 (t, 2H, ³J = 6 Hz), 2,55 (t, 2H, ³J = 7 Hz), 2,71 (t, 2H, ³J = 7 Hz), 3,49 (s, 2H), 3,68 (s, 3H), 3,77 (s, 6H), 4,11 (m, 2H), 6,99 (d, 1H), 7,13-7,25 (m, 6H), 8,39 (d, 2H), 8,90 (s, 1H).

### G. Préparation selon le schéma réactionnel G :

Les composés de formule I selon l'invention dans laquelle A représente -C(O)- et R₄ représente -NW₄W'₄, peuvent être préparés selon le schéma G suivant :

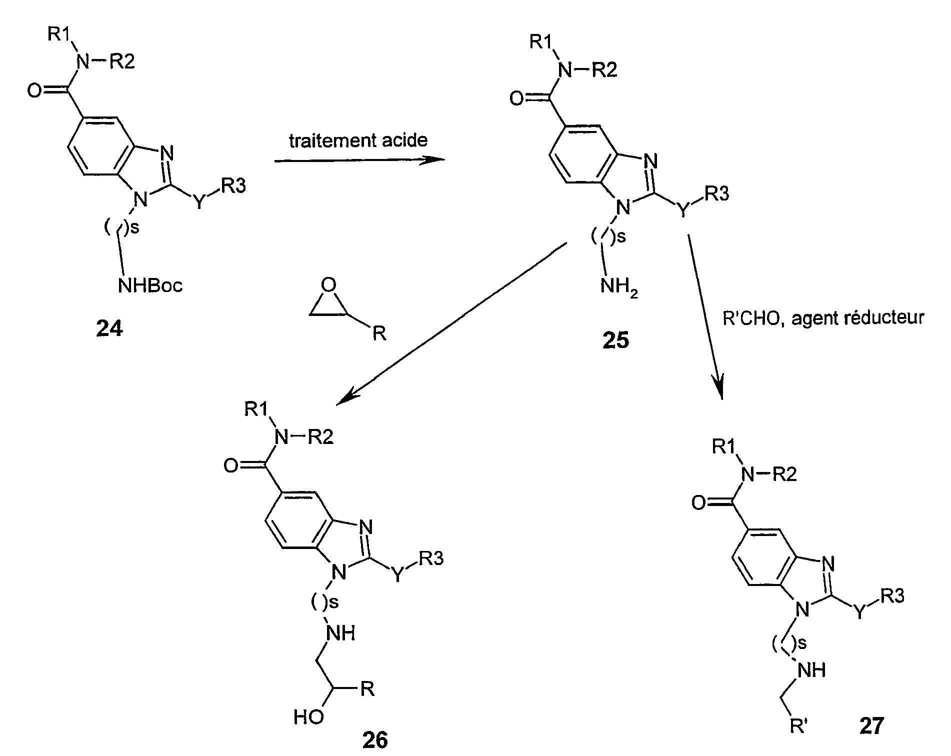

Comme décrit dans le schéma G, le dérivé benzimidazole (24), préparé selon les schémas réactionnels A, B, C ou D peut être traité avec un acide organique ou inorganique comme l'acide trifluoroacétique ou le chlorure d'hydrogène (aqueux ou sous forme gazeuse) dans un solvant aprotique tel que le dichlorométhane ou l'acétate d'éthyle à une température de 0-20° C pendant 0,5 à 5 heures, pour conduire à l'amine (25). L'amine (25) peut ensuite être traitée par un époxyde dans un solvant polaire protique ou aprotique tel que le méthanol, l'éthanol ou l'acétonitrile, en présence ou non de perchlorate de lithium ou de triflate d'ytterbium, à une température de 20-80° C pendant 4 à 48 heures pour conduire au composé (26). L'amine (25) peut également réagir avec un aldéhyde dans un solvant protique ou aprotique, telle que le dichlorométhane, tétrahydrofuranne ou le méthanol, pendant 1 à 15 heures à une température de 0-50° C. L'imine résultante est ensuite réduite *in situ* par un agent réducteur supporté sur une résine ou non, de préférence le triacétoxyborohydrure de sodium, le cyanoborohydrure de sodium ou borohydrure supporté sur une résine, avec ou sans la présence d'un acide tel que l'acide acétique, à une température de 20 à 50° C pendant une durée de 0,2 à 5 heures, pour conduire au composé (27).

Les composés **27** pour lesquels s = 3 peuvent également être préparés selon le schéma G' suivant :

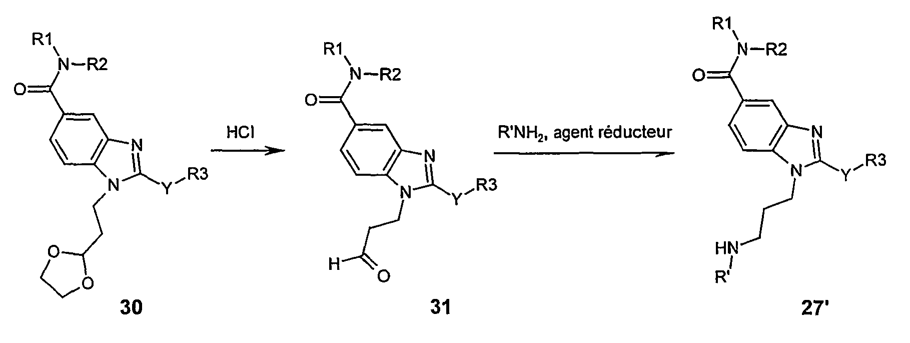

Comme décrit dans le schéma G', le dérivé (30) préparé selon les schémas réactionnels A, B, C ou D peut être traité soit par un acide organique tel que le tosylate de pyridinium ou l'acide paratoluènesulfonique dans un solvant aprotique tel que l'acétone en présence d'eau, à une température de 20-70° C pendant 2 à 12 heures, soit par un acide inorganique tel que le chlorure d'hydrogène aqueux dans un solvant aprotique tel que le tétrahydrofuranne à une température de 0-20° C pendant 6 à 18 heures pour conduire au composé (31). L'aldéhyde (31) peut ensuite être traité par une amine dans un solvant protique ou aprotique tel que le dichlorométhane, le tétrahydrofuranne ou le méthanol pendant 1 à 18 heures à une température de 20° C. L'imine résultante est ensuite réduite *in situ* par un agent réducteur, de préférence le triacétoxyborohydrure de sodium ou le cyanoborohydrure de sodium, en présence ou non d'un acide tel que l'acide acétique, à une température de 20-50° C pendant une durée de 0,2 à 6 heures, pour conduire au composé (27').

### Exemple G1 : 1-{2-[(cyclohéxylméthyl)amino]éthyl}-N,N-diisobutyl-2-[(3,4,5-triméthoxyphényl)amino]-1H-benzimidazole-5-carboxamide

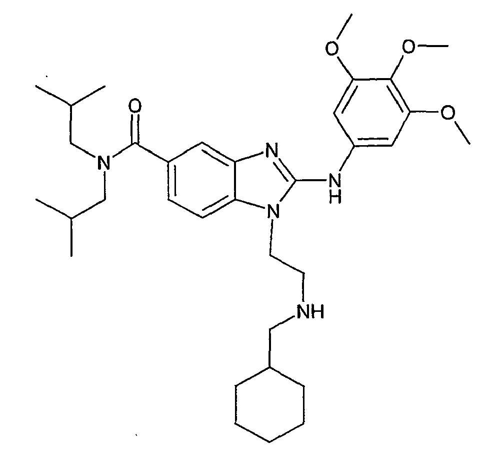

### Etape 1 : chlorhydrate de 1-(2-aminoéthyl)-N,N-diisobutyl-2-[(3,4,5-triméthoxy phényl)amino]-1H-benzimidazole-5-carboxamide

Une solution de tert-butyl 2-{5-[(diisobutylamino)carbonyl]-2-[(3,4,5-triméthoxy phényl)amino]-1H-benzimidazol-1-yl}éthylcarbamate (2,56 g, préparé selon la procédure décrite dans l'exemple A1, schéma réactionnel A) dans de l'acétate d'éthyle (100 ml), refroidie à 0° C est traversée par un flux de HCl sec jusqu'à ce que la CCM (acétate d'éthyle 100 %) montre une disparition complète du produit de départ. Le mélange résultant est alors évaporé sous pression réduite. Le solide obtenu est trituré dans du diéthyléther et filtré pour donner le composé attendu sous forme de cristaux blancs (2,25 g, 97 % rendement).
SM/CL : MM calculée = 497,6 ; m/z = 498,3 (MH+)
RMN (¹H, 400 MHz, DMSO-*d₆*) : *δ* 0,67 (m, 6H), 0,92 (m, 6H), 1,84-2,03 (m, 2H), 3,10-3,17 (m, 4H), 3,38 (m, 2H), 3,71 (s, 3H), 3,81 (s, 6H), 4,76 (m, 2H), 6,93 (s, 2H), 7,30 (m, 2H), 7,81 (d, 1H), 8,56 (m, 3H).

### Etape 2 : 1-{2-[(cyclohéxylméthyl)amino]éthyl}-N,N-diisobutyl-2-[(3,4,5-triméthoxy phényl) amino]-1H-benzimidazole-5-carboxamide

Une solution de 1-(2-aminoéthyl)-*N*,*N*-diisobutyl-2-[(3,4,5-triméthoxyphényl)amino]-1H-benzimidazole-5-carboxamide (30 mg, 1 éq) et de cyclohexanecarboxaldéhyde (5 mg, 0,8 éq) dans le méthanol (0,7 ml) est agitée à une température d'environ 20° C pendant 4 heures. La résine borohydrure (48 mg, 2,5 mmol/g, Amberlite ®, IRA-400) est additionnée et le mélange est agité pendant 18 heures puis additionné de dichlorométhane (0,5 ml) et de résine Wang benzyloxybenzaldéhyde (37 mg, 3,22 mmol/g, Novabiochem). Après une nuit d'agitation, le mélange est filtré et le filtrat est évaporé sous pression réduite pour donner le composé attendu sous forme d'une mousse beige (18 mg, 65 %).
SM/CL : MM calculée = 593,8 ; m/z = 594,4 (MH+)
RMN (¹H, 400 MHz, CDCl₃) : δ 0,65-1,80 (m, 23H), 2,60 (d, 2H), 3,13 (m, 2H), 3,82 (s, 3H), 3,90 (s, 6H), 4,10 (m, 2H), 6,91 (s, 2H), 7,07; 7,16 (AB, 2H), 7,53 (s, 1H), 10,1 (s, 1H).

Les composés suivants ont été préparés selon le schéma réactionnel G et de façon analogue à la procédure décrite pour la synthèse du 1-{2-[(cyclohéxylméthyl)amino]éthyl}-*N,N*-diisobutyl-2-[(3,4,5-triméthoxyphényl)amino]-1H-benzimidazole-5-carboxamide (une purification finale par chromatographie éclair sur gel de silice peut également être réalisée) :

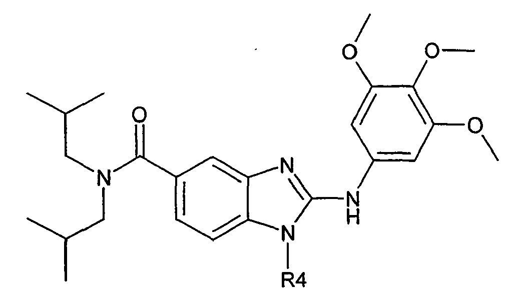

Dans la formule ci-dessus, R₄ représente l'un des radicaux ci-après :

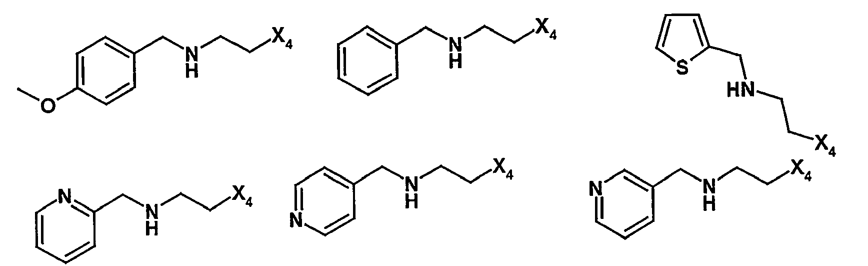

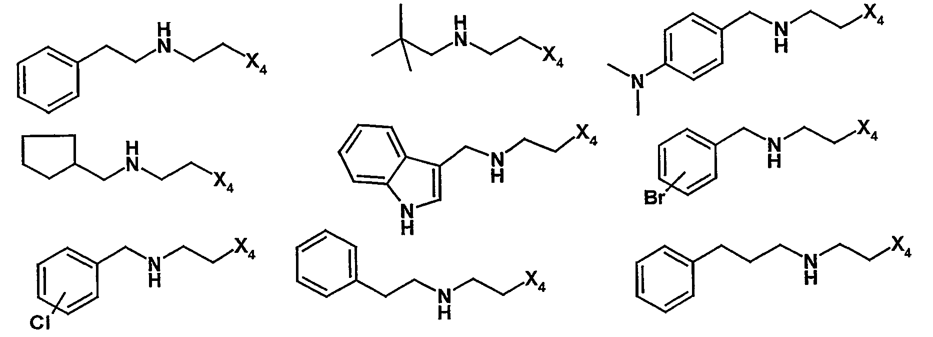

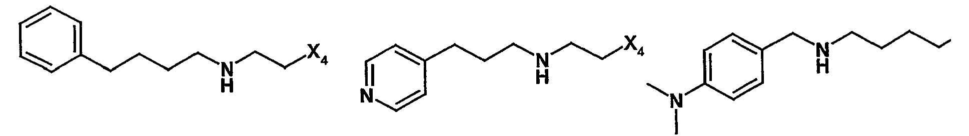

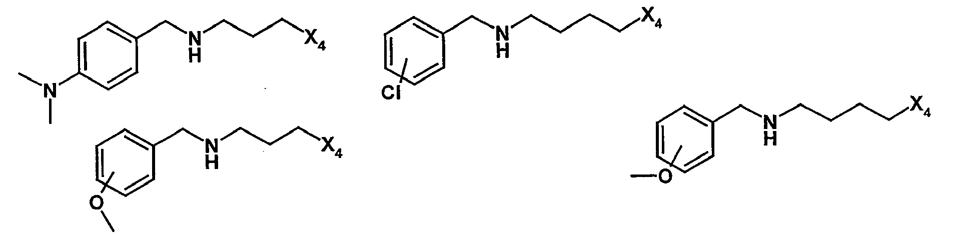

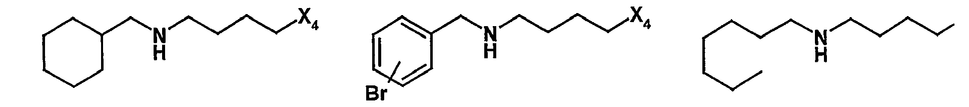

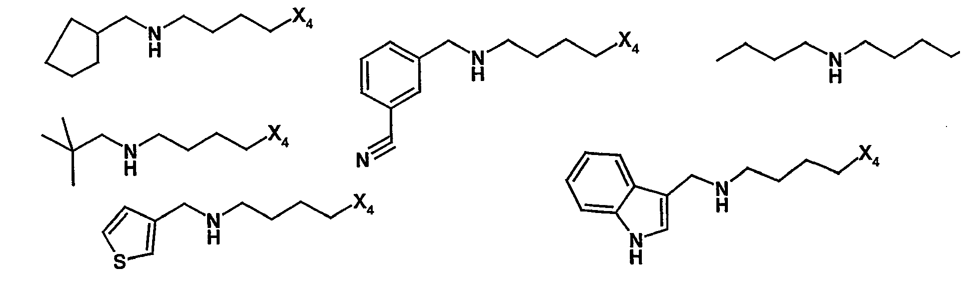

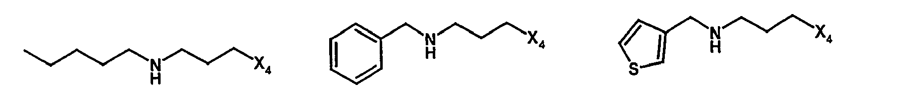

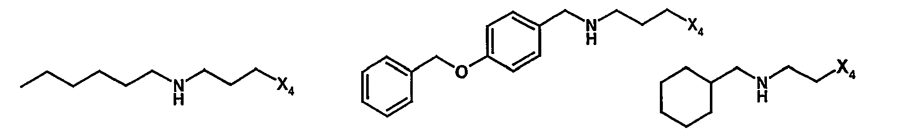

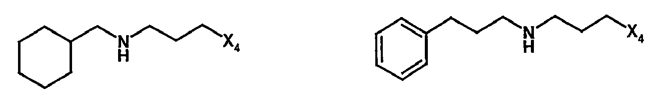

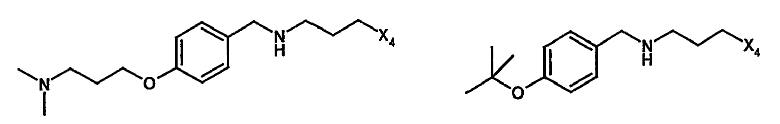

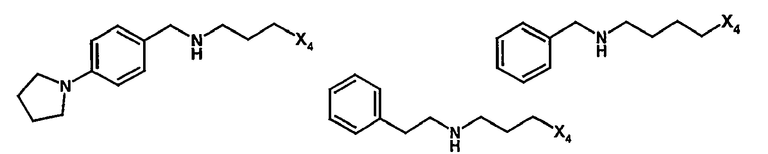

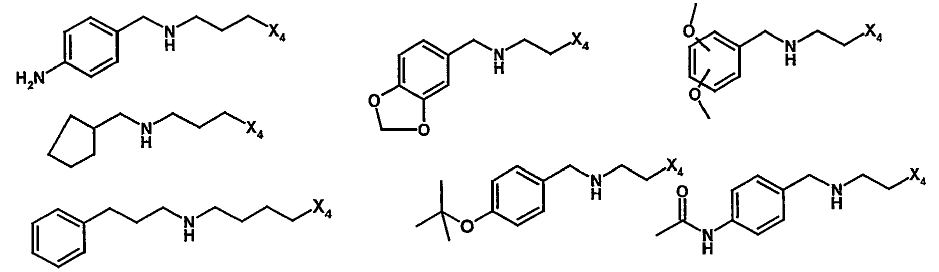

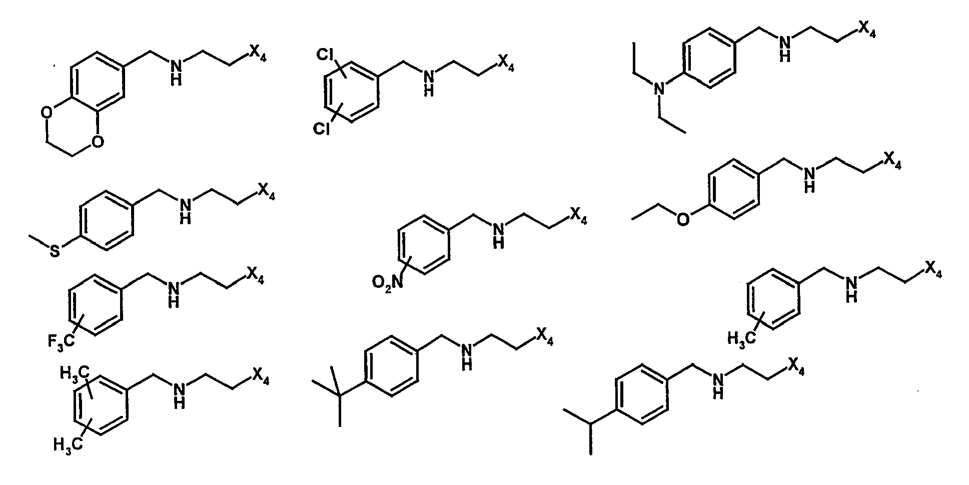

### Exemple G2 : 1-{2-[(1-hydroxy-2-phényléthyl)amino]éthyl}-N,N-diisobutyl-2-[(3,4,5-triméthoxyphényl)amino]-1H-benzimidazole-5-carboxamide

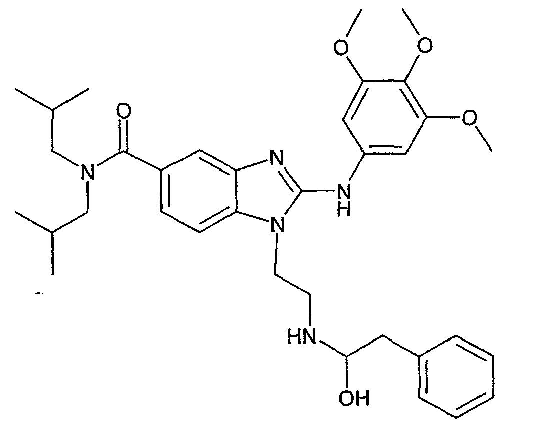

A une solution de 2,3- époxypropylbenzène (7 mg, 1éq) dans l'acétonitrile (0,5 ml) sont additionnés, à une température d'environ 20° C, le perchlorate de lithium (16 mg, 3 éq) puis après 5 min le 1-(2-aminoéthyl)-*N*,*N*-diisobutyl-2-[(3,4,5-triméthoxy phényl) amino]-1H-benzimidazole-5-carboxamide (25 mg, 1 éq). Le mélange est chauffé à reflux pendant 24 heures puis refroidi à température ambiante et additionné d'eau saturée en hydrogénocarbonate et de dichlorométhane. Après décantation et extraction, les phases organiques sont réunies et lavées avec de la saumure, séchées sur sulfate de sodium et évaporées sous pression réduite à 40° C. La purification de l'huile obtenue par chromatographie éclair sur gel de silice (dichlorométhane 100 % à dichlorométhane/méthanol 80:20) donne le composé attendu sous forme d'une huile (31 mg, 55 % rendement)
SM/CL : MM calculée = 631.8 ; m/z = 632.4 (MH+)
RMN (¹H, 400 MHz, DMSO-*d₆*): *δ* 0,83 (m, 6H), 0,91 (m, 6H), 1,81-2,10 (m, 2H), 2,57-2,65 (m, 3H), 2,91 (m, 2H), 3,21 (m, 4H), 3,62 (s, 3H), 3,75 (m, 7H), 4,22 (m, 2H), 4,74 (d, 1H), 6,97-7,33 (m, 10H).

Les composés suivants ont été préparés selon le schéma réactionnel G et de façon analogue à la procédure décrite pour la synthèse du 1-{2-[(1-hydroxy-2-phényléthyl)amino]éthyl}-*N*,*N*-diisobutyl-2-[(3,4,5-triméthoxyphényl)amino]-1H-benzimidazole-5-carboxamide :

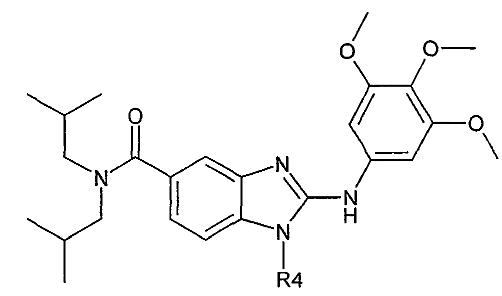

Dans la formule ci-dessus, R₄ représente l'un des radicaux ci-après :

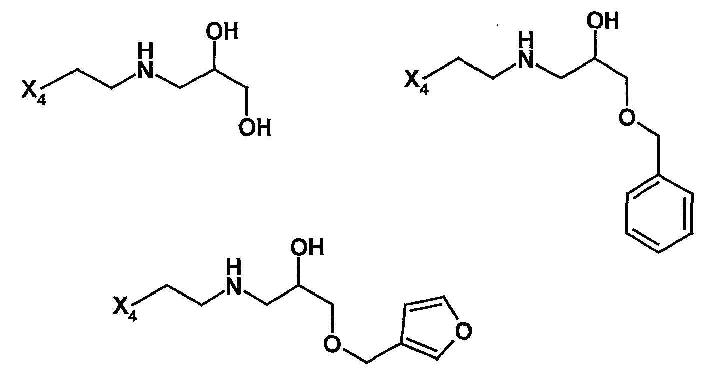

### H. Préparation selon le schéma réactionnel H :

Les composés de formule I selon l'invention dans laquelle A représente -C(O)-, Y -S- et R₃ -(CH2)ₚ-CH(OH)-(CH₂)_{p'}-Z₃, peuvent être préparés selon le schéma H suivant :

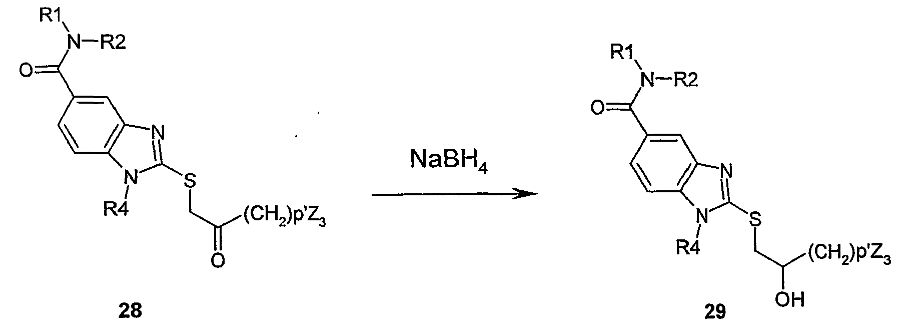

Comme décrit dans le schéma H, le dérivé thiobenzimidazole (28), préparé selon les schémas réactionnels B ou D, peut être traité par un agent réducteur tel le borohydrure de sodium dans un solvant protique tel que le méthanol à une température de 0-20°C pendant 0,2 à 1 heure, pour conduire à l'alcool correspondant (29).

### Exemple H1 : 2-([2-hydroxy-2-(3,4,5-triméthoxyphenyl)éthyl]thio)-N,N-diisobutyl-1-{3-[méthyl(2-pyridin-2-yléthyl)amino]propyl}-1H-benzimidazole-5-carboxamide

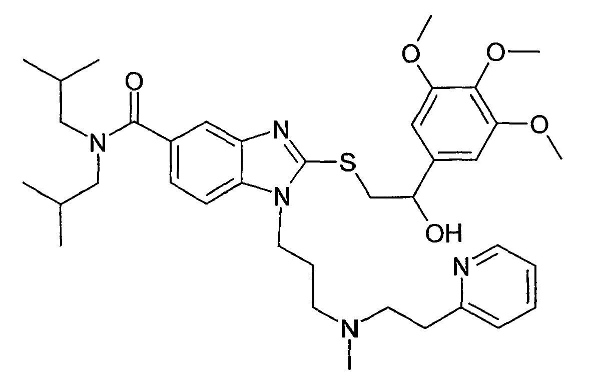

A une solution de *N,N*-diisobutyl-1-{3-[méthyl(2-pyridin-2-yléthyl)amino]propyl}-2-{[2-oxo-2-(3,4,5-triméthoxyphényl)éthyl]thio}-1H-benzimidazole-5-carboxamide (69 mg, 1 éq) dans le méthanol (2 ml) est additionné à 0° C, le borohydrure de sodium (8 mg, 2 éq). Après 10 minutes d'agitation à 0° C, le mélange est ramené à une température d'environ 20° C et agité à cette température pendant 30 minutes. Le mélange est ensuite concentré sous pression réduite à 40° C puis additionné d'eau saturée en chlorure d'ammonium et de dichlorométhane. Après décantation et extraction, les phases organiques sont réunies et lavées avec de la saumure, séchées sur sulfate de sodium et évaporées sous pression réduite à 40° C. La purification de l'huile obtenue par chromatographie éclair sur gel de silice (dichlorométhane 100 % à dichlorométhane/méthanol 80:20) donne le composé attendu sous forme d'une huile (61 mg, 88 % rendement).
SM/CL : MM calculée = 691,9 ; m/z = 692,4 (MH+)
RMN (¹H, 400 MHz, DMSO-*d₆*) : *δ* 0,61 (m, 6H), 0,91 (m, 6H), 1,71-2,03 (m, 4H), 2,17 (s, 3H), 2,31 (t, 3H,³*J* = 6,5 Hz), 2,65 (t, 2H, ³*J* = 7 Hz), 2,85 (t, 2H, ³*J* = 7 Hz), 3,08-3,30 (m, 4H), 3,56 (m, 1H), 3,60 (s, 3H), 3,71 (m, 1H), 3,75 (s, 6H), 4,05 (t, 2H, ³*J*= 7 Hz), 4,86 (m, 1H), 5,87 (d, 1H), 6,75 (s, 2H), 7,11- 7,65 (m, 6H), 8,43 (d, 1H).

### Préparation des réactifs de synthèse

### N-(2-pyridin-2-yl éthyl)propane-1,3-diamine

A une solution refroidie à environ 4° C de 2-[2-(méthylamino)éthyl]pyridine (19,5 ml, 1 éq) dans le méthanol (200 ml) est additionné lentement l'acrylonitrile (10,1 ml, 1,1 éq). Le milieu réactionnel est ensuite agité pendant 3 heures à environ 20° C puis concentré sous pression réduite à 40° C pour donner le 3-[(2-pyridin-2-yléthyl)amino]propanenitrile sous forme d'une huile jaune (25,6 g, 96 % de rendement).

Une solution de cette huile (15,3 g) dans le méthanol saturé en ammoniac (250 ml) est hydrogénée en présence de nickel de Raney (1,5 g) à environ 20° C pendant 15 heures. Le mélange réactionnel est ensuite filtré sur célite. Le filtrat est concentré sous pression réduite à environ 40° C pour donner le composé attendu sous forme d'une huile verdâtre (15,5 g, 97 % rendement).

Les composés suivants ont été préparés de façon analogue à la procédure décrite pour la synthèse du *N*-(2-pyridin-2-yléthyl)propane-1,3-diamine :

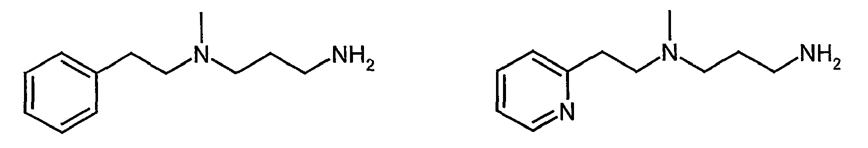

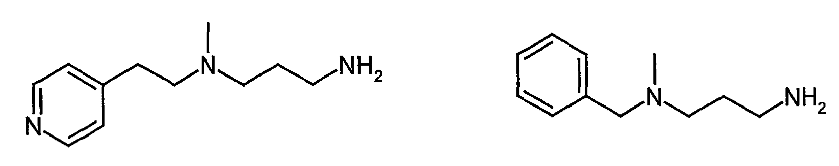

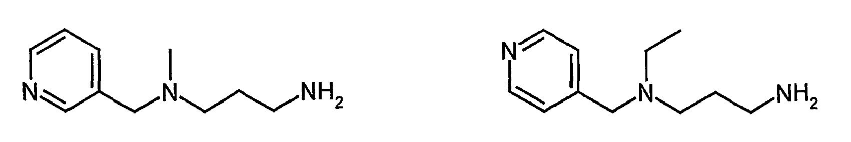

### 2-bromo-1-(3,4,5-triméthoxyphényl)éthanone

A une solution de 3,4,5-triméthoxy-acétophénone (10 g, 1 éq) dans le méthanol (150ml) est additionnée la pyridinehydrobromure perbromure supportée sur résine (23 g, 1 éq). Après 3 h d'agitation à environ 20° C, le mélange est filtré et le filtrat est concentré sous pression réduite. La purification du résidu obtenu par chromatographie éclair sur gel de silice (éluant : heptane /acétate d'éthyle 8 / 2 puis 7 / 3) donne le composé attendu sous forme d'une poudre blanche (8,2 g, 60 % rendement). Point de fusion = 66° C.

### 3,4,5-triméthoxybenzoyl isothiocyanate

A une solution de 3,4,5-triméthoxybenzoylchloride (2,3 g) dans l'acétonitrile (40 ml) est additionné le thiocyanate de potassium. Après 15 minutes d'agitation à environ 20° C, le mélange est filtré et le filtrat est concentré sous pression réduite pour donner le composé attendu sous forme d'une poudre beige (2,4 g, 96 % rendement). Point de fusion = 101 °C.

Les composés I (ou I') de la présente invention possèdent d'intéressantes propriétés pharmacologiques. C'est ainsi que l'on a découvert que les composés I (ou I') de la présente invention possèdent une activité antagoniste de la GnRH (Gonadotropin-Releasing hormone.

Les composés de la présente invention peuvent ainsi être utilisés dans différentes applications thérapeutiques. Ils peuvent avantageusement être utilisés chez la femme dans le traitement de l'endométriose, le fibrome, le syndrome des ovaires polykystiques, le cancer du sein, de l'ovaire et de l'endomètre, la désensibilisation hypophysaire gonadotrope lors des protocoles de procréation médicalement assistée ; chez l'homme, dans le traitement de l'hyperplasie bénigne de la prostate et le cancer de la prostate ; et dans le traitement de la puberté précoce masculine ou féminine. On trouvera ci-après, dans la partie expérimentale, une illustration des propriétés pharmacologiques des composés de l'invention.

La présente demande a également pour objet, à titre de médicaments, les produits de formule I (ou I') telle que définie ci-dessus, ainsi que les sels d'addition avec les acides minéraux ou organiques pharmaceutiquement acceptables desdits produits de formule I (ou I'), ainsi que les compositions pharmaceutiques contenant, à titre de principe actif, un au moins des médicaments tels que définis ci-dessus, en association avec un support pharmaceutiquement acceptable.

La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par intraveineuse.

Tous les termes techniques et scientifiques utilisés dans le présent texte ont la signification connue de l'homme de l'art. Par ailleurs, tous les brevets (ou demandes de brevet) ainsi que les autres références bibliographiques sont incorporés par référence.

### Partie expérimentale :

Les composés selon l'invention obtenus selon les procédures des exemples A, B, C, D, E, F, G et H précédemment décrites, sont rassemblés dans le tableau ci-dessous.

Les composés sont caractérisés par leur temps de rétention (tr) et leur pic moléculaire déterminé par spectrométrie de masse (MH+).

Pour la spectrométrie de masse, un spectromètre de masse simple quadripôle (Micromass, modèle Platform) équipé d'une source *electrospray* est utilisé avec une résolution de 0,8 Da à 50 % de vallée. Un calibrage est effectué mensuellement entre les masses 80 et 1000 Da à l'aide d'un mélange calibrant d'iodure de sodium et d'iodure de rubidium en solution dans un mélange isopropanol/eau (1/1 Vol.)

Pour la chromatographie liquide, un système Waters incluant un dégazeur en ligne, une pompe quaternaire Waters 600, un injecteur plaque Gilson 233 et un détecteur UV Waters PAD 996, est utilisé.

Les conditions d'élution employées sont les suivantes :

| | | |
|---|---|---|
| Eluant | **A** | eau + 0,04 % acide trifluoroacétique |
| | **B** | acétonitrile |

| **T (min)** | **A%** | **B%** |
|---|---|---|
| 1 | 95 | 5 |
| 8.5 | 5 | 95 |
| 10.5 | 5 | 95 |
| 10.6 | 95 | 5 |
| 14.9 | 95 | 5 |
| 15.0 | 95. | 5 |

Débit : 1 ml/min
Injection : 10 µL
Colonne : Uptisphere ODS 3 µm 75*4,6 mm i.d

Ces exemples sont présentés pour illustrer les procédures ci-dessus et ne doivent en aucun cas être considérés comme une limite à la portée de l'invention.

Dans chaque illustration des radicaux R₁, R₂, R₃ et R₄, les radicaux X₁, X₂, X₃ et X₄ représentent, respectivement, la partie restante du composé de formule générale (I).

Les exemples 1 à 253, 254 à 255 et 256 à 538 illustrent respectivement les composés I dans laquelle A représente -C(O)- et Y -S-, A représente -CH₂- et Y -NH- et A représente -C(O)- et Y -NH-.

| Exemples | R1 | R2 | R3 | R4 | [M+H]+ | tr (min) |
|---|---|---|---|---|---|---|
| 1 | 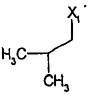 | 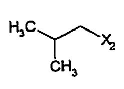 | 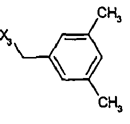 | 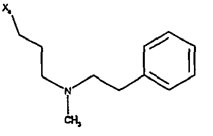 | 599,4 | 9,7 |
| 2 | 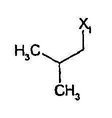 | 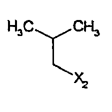 | 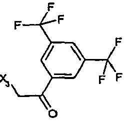 | 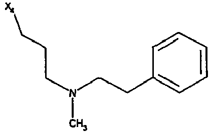 | 735,3 | 10,7 |
| 3 | 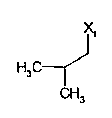 | 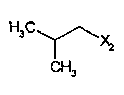 | 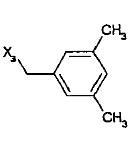 | 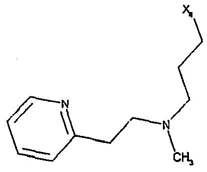 | 600,4 | 9,3 |
| 4 | 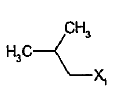 | 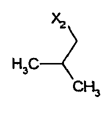 | 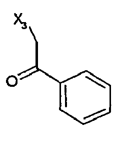 | 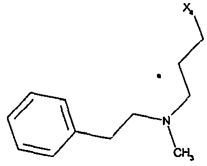 | 599,5 | 9,1 |
| 5 | 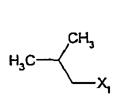 | 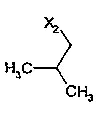 | 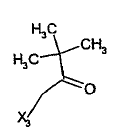 | 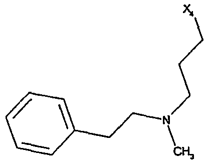 | 579,5 | 9,2 |
| 6 | 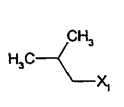 | 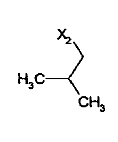 | 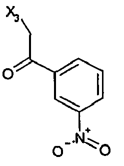 | 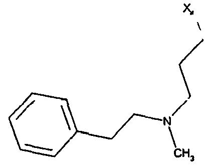 | 644,5 | 3,1 |
| 7 | 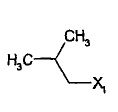 | 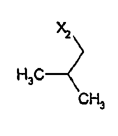 | 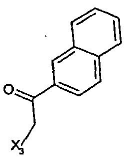 | 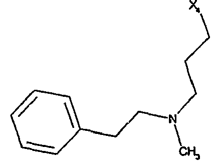 | 649,5 | 9,7 |
| 8 | 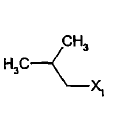 | 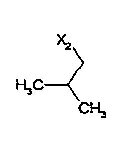 | 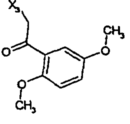 | 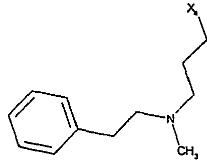 | 659,5 | 9,2 |
| 9 | 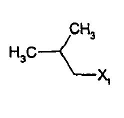 | 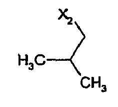 | 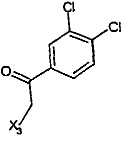 | 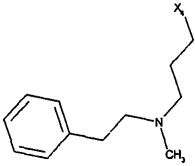 | 667,4 | 9,8 |
| 10 | 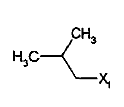 | 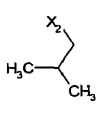 | 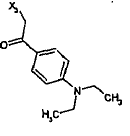 | 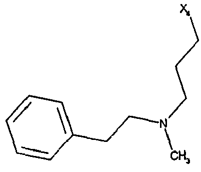 | 670,5 | 9,7 |
| 11 | 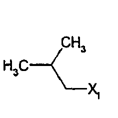 | 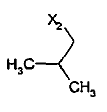 | 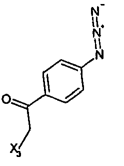 | 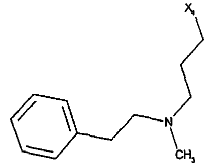 | 640,5 | 9,4 |
| 12 | 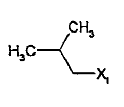 | 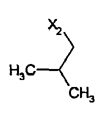 | 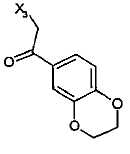 | 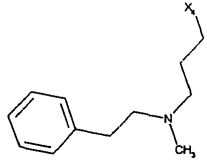 | 657,5 | 8,9 |
| 13 | 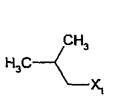 | 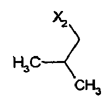 | 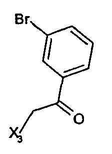 | 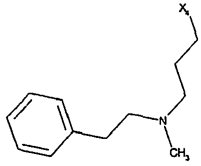 | 677,4 | 9,4 |
| 14 | 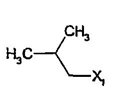 | 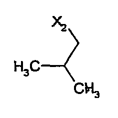 | 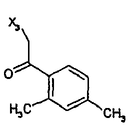 | 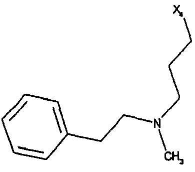 | 627,5 | 9,6 |
| 15 | 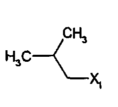 | 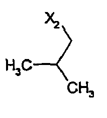 | 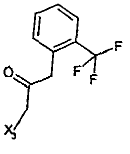 | 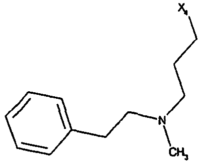 | 681,5 | 9,5 |
| 16 | H | 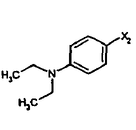 | 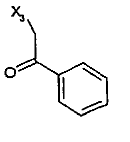 | 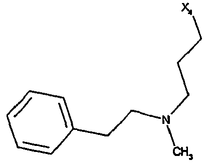 | 634,5 | 7,2 |
| 17 | H | 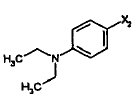 | 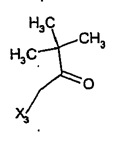 | 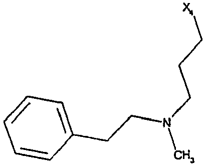 | 614,5 | 7,2 |
| 18 | H | 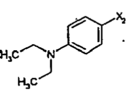 | 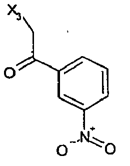 | 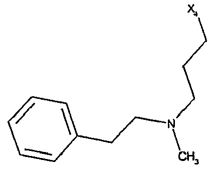 | 679,5 | 7,3 |
| 19 | H | 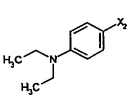 | 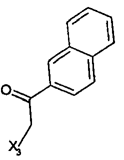 | 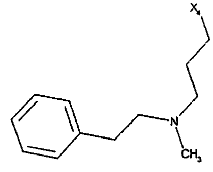 | 684,5 | 7,7 |
| 20 | H | 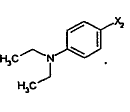 | 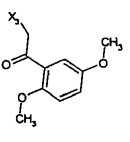 | 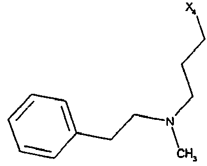 | 694,5 | 7,3 |
| 21 | H | 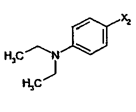 | 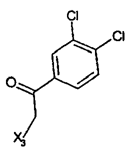 | 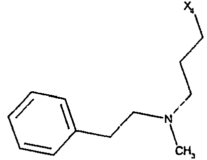 | 702,4 | 7,7 |
| 22 | H | 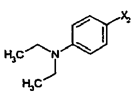 | 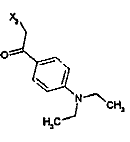 | 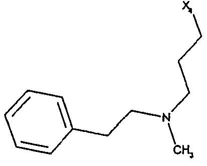 | 705,5 | 7,6 |
| 23 | H | 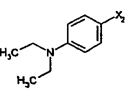 | 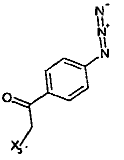 | 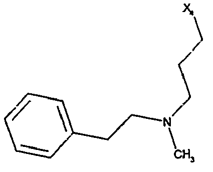 | 673,5 | 7,3 |
| 24 | H | 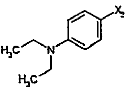 | 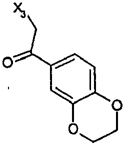 | 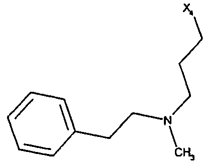 | 692,5 | 7,3 |
| 25 | H | 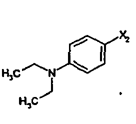 | 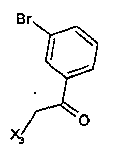 | 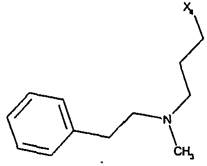 | 712,4 | 7,5 |
| 26 | H | 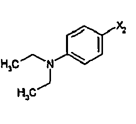 | 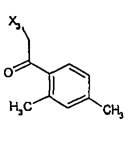 | 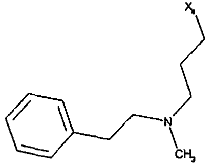 | 662,5 | 7,6 |
| 27 | H | 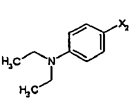 | 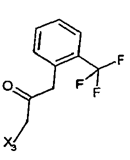 | 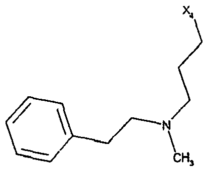 | 716,2 | 7,4 |
| 28 | 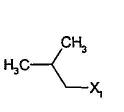 | 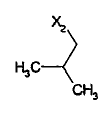 | 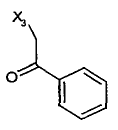 | 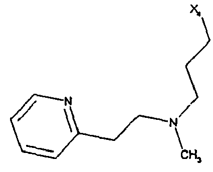 | 600,5 | 8,5 |
| 29 | 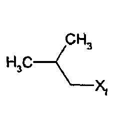 | 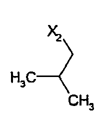 | 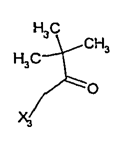 | 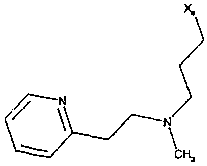 | 580,5 | 8,5 |
| 30 | 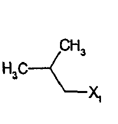 | 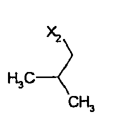 | 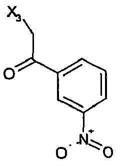 | 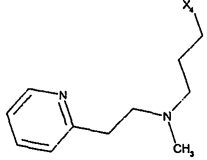 | 645,5 | 8,5 |
| 31 | 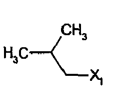 | 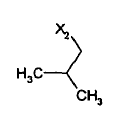 | 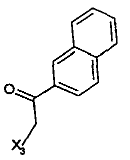 | 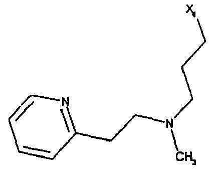 | 650,5 | 9,1 |
| 32 | 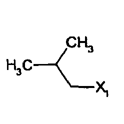 | 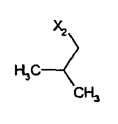 | 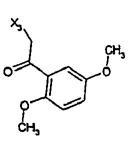 | 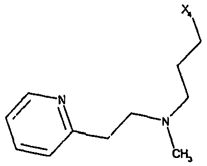 | 660,6 | 8,5 |
| 33 | 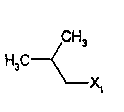 | 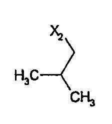 | 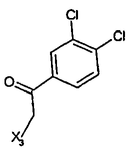 | 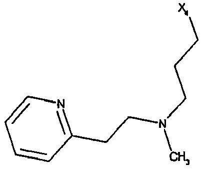 | 668,5 | 9,2 |
| 34 | 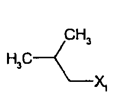 | 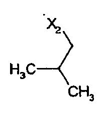 | 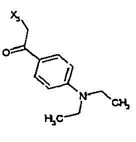 | 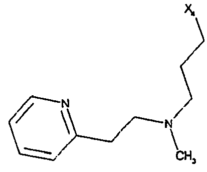 | 671,2 | 9,0 |
| 35 | 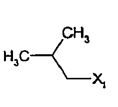 | 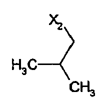 | 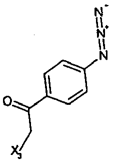 | 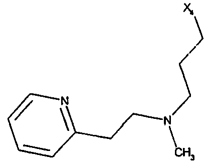 | 641,3 | 8,7 |
| 36 | 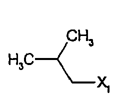 | 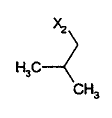 | 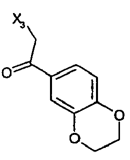 | 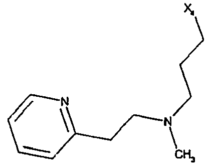 | 658,5 | 8,4 |
| 37 | 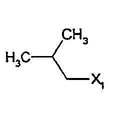 | 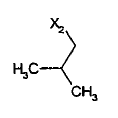 | 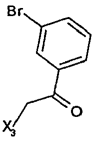 | 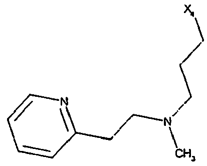 | 678,4 | 8,9 |
| 38 | 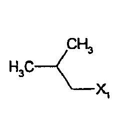 | 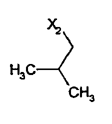 | 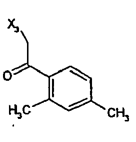 | 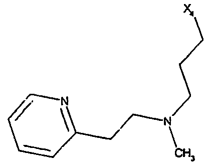 | 628,5 | 8,9 |
| 39 | 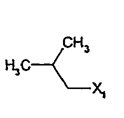 | 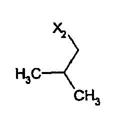 | 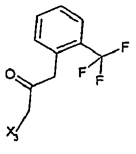 | 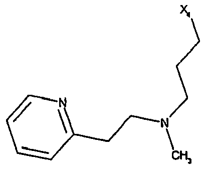 | 682,5 | 8,8 |
| 40 | 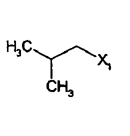 | 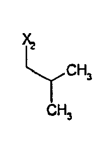 | 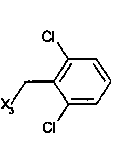 | 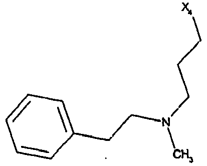 | 639,4 | 9,6 |
| 41 | 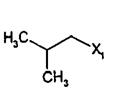 | 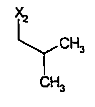 | 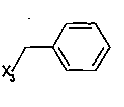 | 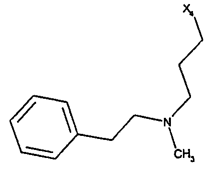 | 571,5 | 9,1 |
| 42 | 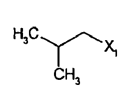 | 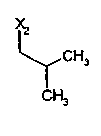 | 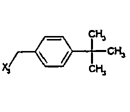 | 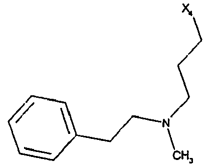 | 627,5 | 10,2 |
| 43 | 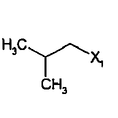 | 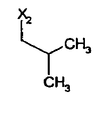 | 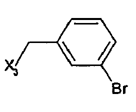 | 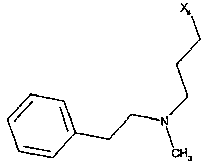 | 649,4 | 9,6 |
| 44 | 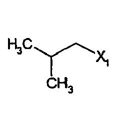 | 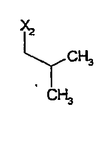 | 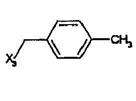 | 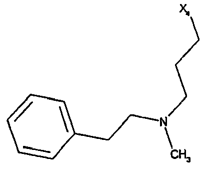 | 607,5 | 9,5 |
| 45 | 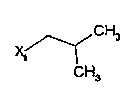 | 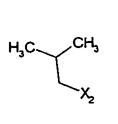 | 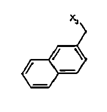 | 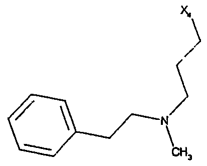 | 621,5 | 9,6 |
| 46 | 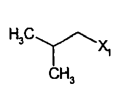 | 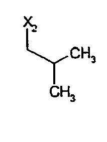 | 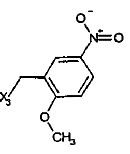 | 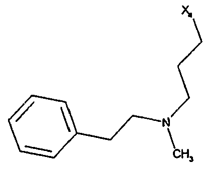 | 646,5 | 9,2 |
| 47 | 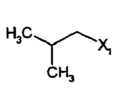 | 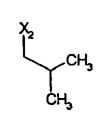 | 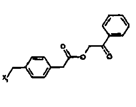 | 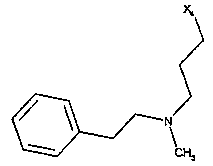 | 747,5 | 9,5 |
| 48 | 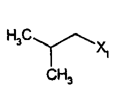 | 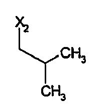 | 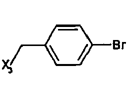 | 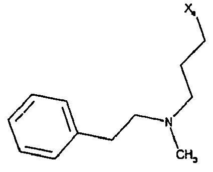 | 649,4 | 9,6 |
| 49 | 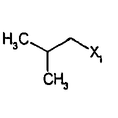 | 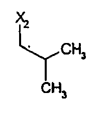 | 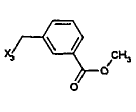 | 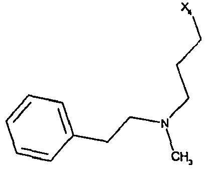 | 629,5 | 9,1 |
| 50 | 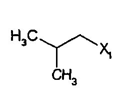 | 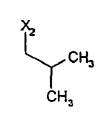 | 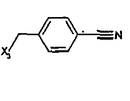 | 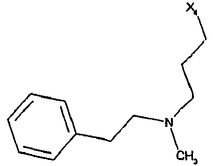 | 596,4 | 8,9 |
| 51 | 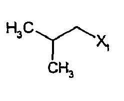 | 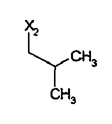 | 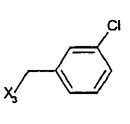 | 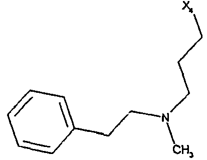 | 605,4 | 9,5 |
| 52 | 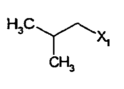 | 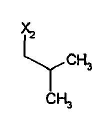 | 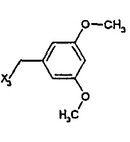 | 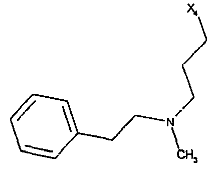 | 631,5 | 9,2 |
| 53 | H | 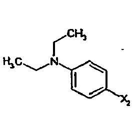 | 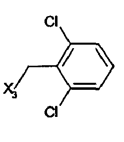 | 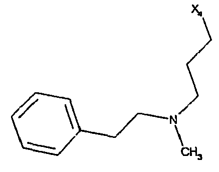 | 674,3 | 7,5 |
| 54 | H | 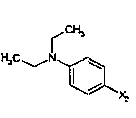 | 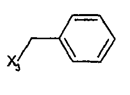 | 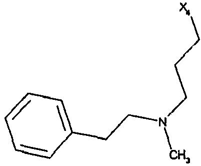 | 606,4 | 7,3 |
| 55 | H | 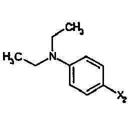 | 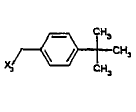 | 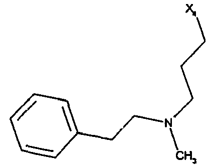 | 662,5 | 8,0 |
| 56 | H | 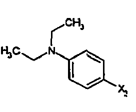 | 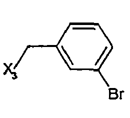 | 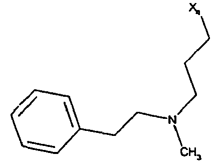 | 684,3 | 7,5 |
| 57 | H | 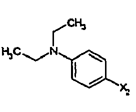 | 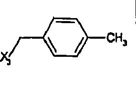 | 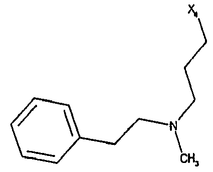 | 620,4 | 7,4 |
| 58 | H | 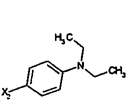 | 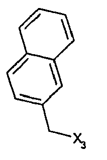 | 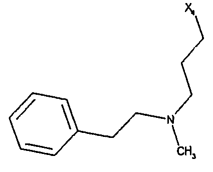 | 656,4 | 7,6 |
| 59 | H | 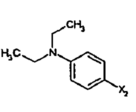 | 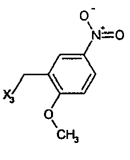 | 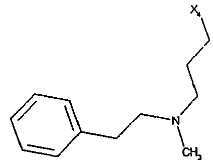 | 681,4 | 7,4 |
| 60 | H | 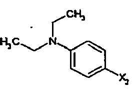 | 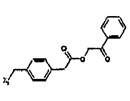 | 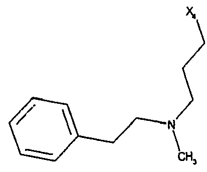 | 781,3 | 7,7 |
| 61 | H | 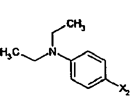 | 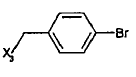 | 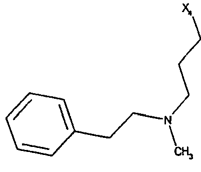 | 684,3 | 7,5 |
| 62 | H | 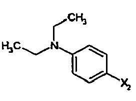 | 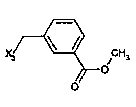 | 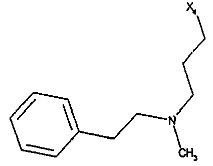 | 664,4 | 7,3 |
| 63 | H | 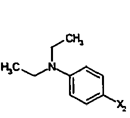 | 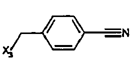 | 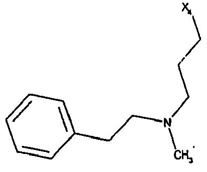 | 631,4 | 7,2 |
| 64 | H | 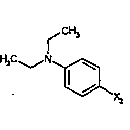 | 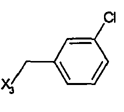 | 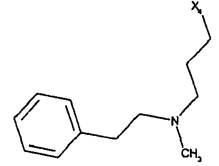 | 640,4 | 7,5 |
| 65 | H | 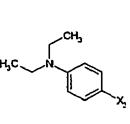 | 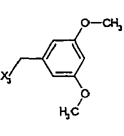 | 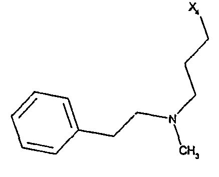 | 666,4 | 7,3 |
| 66 | 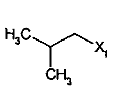 | 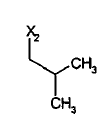 | 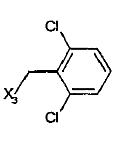 | 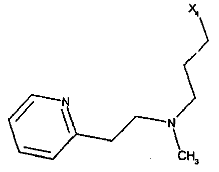 | 640,4 | 9,1 |
| 67 | 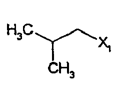 | 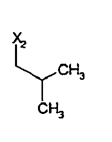 | 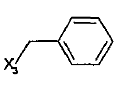 | 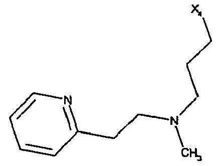 | 572,4 | 8,6 |
| 68 | 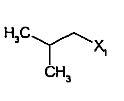 | 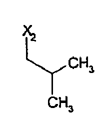 | 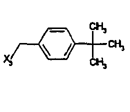 | 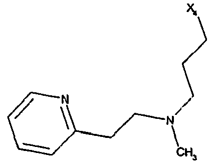 | 628,5 | 9,7 |
| 69 | 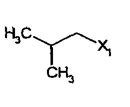 | 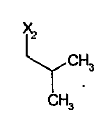 | 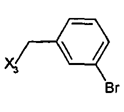 | 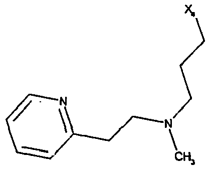 | 650,4 | 9,0 |
| 70 | 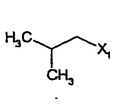 | 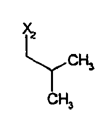 | 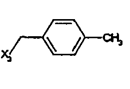 | 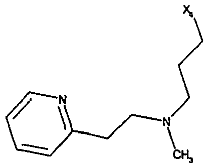 | 586,4 | 8,9 |
| 71 | 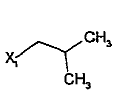 | 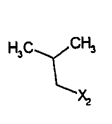 | 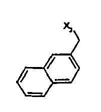 | 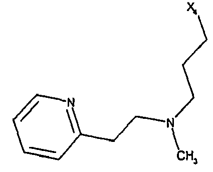 | 622,5 | 9,1 |
| 72 | 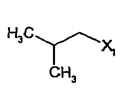 | 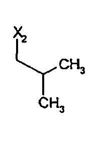 | 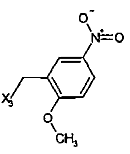 | 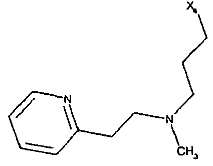 | 647,5 | 8,7 |
| 73 | 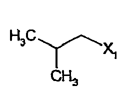 | 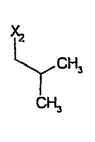 | 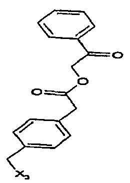 | 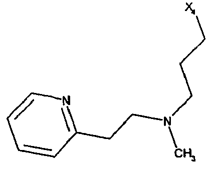 | 748,5 | 9,1 |
| 74 | 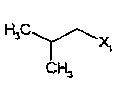 | 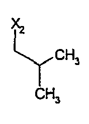 | 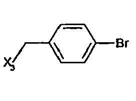 | 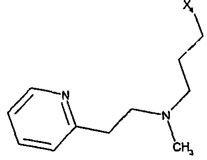 | 650,4 | 9,0 |
| 75 | 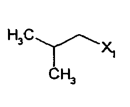 | 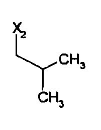 | 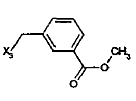 | 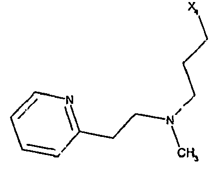 | 630,4 | 8,6 |
| 76 | 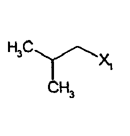 | 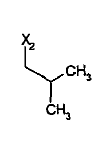 | 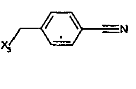 | 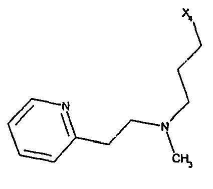 | 597,4 | 8,4 |
| 77 | 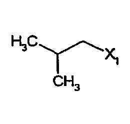 | 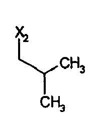 | 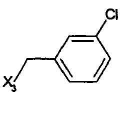 | 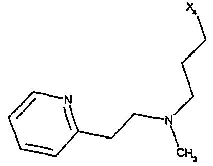 | 606,4 | 8,9 |
| 78 | 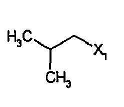 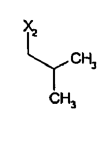 | | 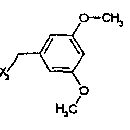 | 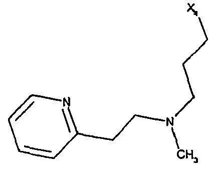 | 632,5 | 8,6 |
| 79 | 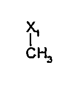 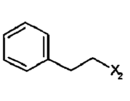 | | 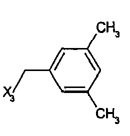 | 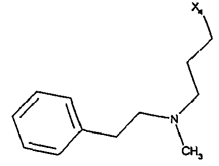 | 605,5 | 9,0 |
| 80 | 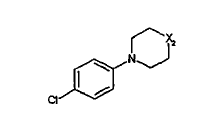 | | 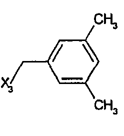 | 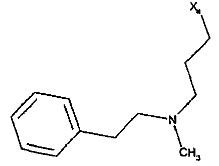 | 666,5 | 9,5 |
| 81 | H | 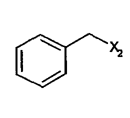 | 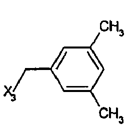 | 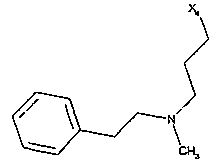 | 577,3 | 8,7 |
| 82 | H | 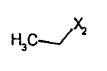 | 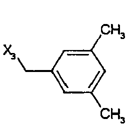 | 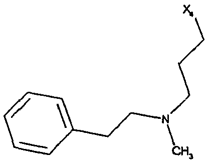 | 515,4 | 8,1 |
| 83 | 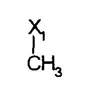 | 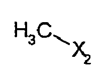 | 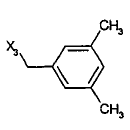 | 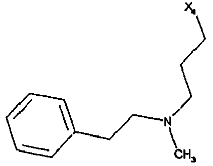 | 515,4 | 8,1 |
| 84 | H | 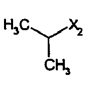 | 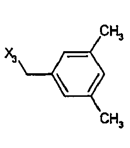 | 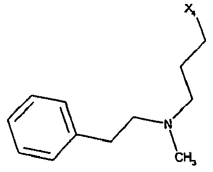 | 529,5 | 8,4 |
| 85 | 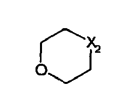 | | 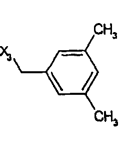 | 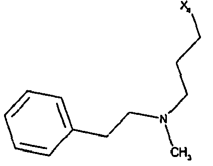 | 557,4 | 8,0 |
| 86 | H | 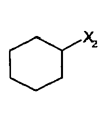 | 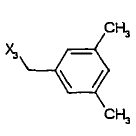 | 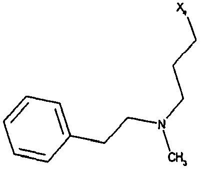 | 569,5 | 8,9 |
| 87 | H | 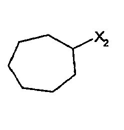 | 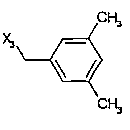 | 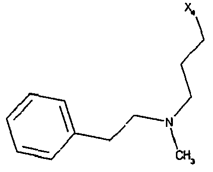 | 583,5 | 9,2 |
| 88 | H | 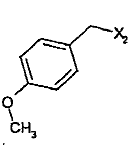 | 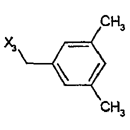 | 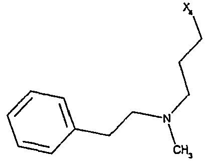 | 607,5 | 8,7 |
| 89 | H | 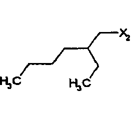 | 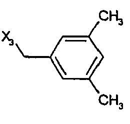 | 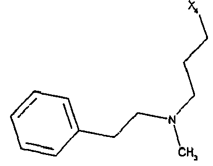 | 599,5 | 9,8 |
| 90 | 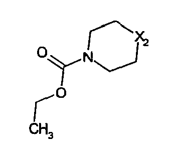 | | 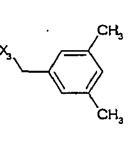 | 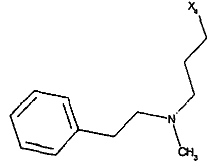 | 628,5 | 8,4 |
| 91 | 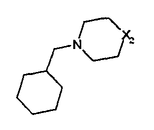 | | 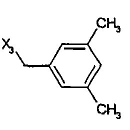 | 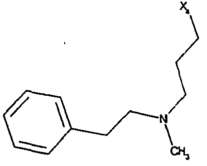 | 652,5 | 7,6 |
| 92 | 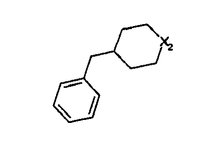 | | 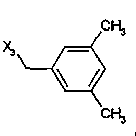 | 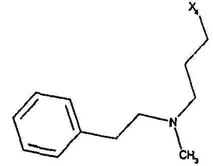 | 645,5 | 9,7 |
| 93 | 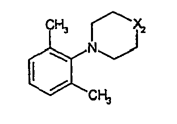 | | 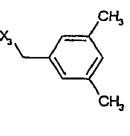 | 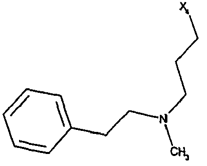 | 660,6 | 9,9 |
| 94 | 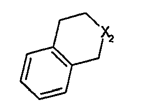 | | 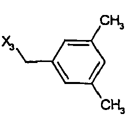 | 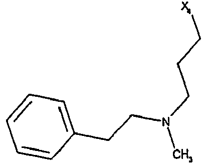 | 603,4 | 9,1 |
| 95 | H | 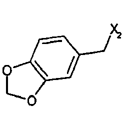 | 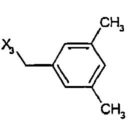 | 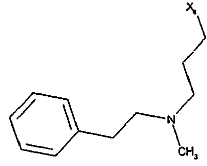 | 621,4 | 8,7 |
| 96 | H | 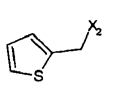 | 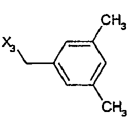 | 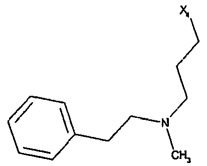 | 583,4 | 8,7 |
| 97 | 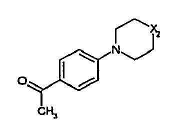 | | 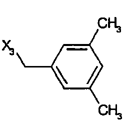 | 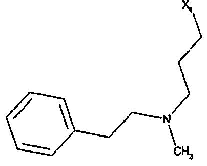 | 674,5 | 8,7 |
| 98 | H | 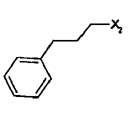 | 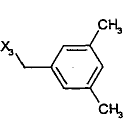 | 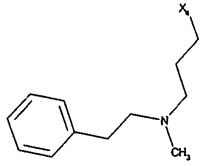 | 605,5 | 9,1 |
| 99 | H | 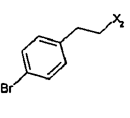 | 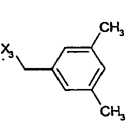 | 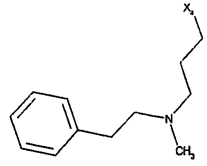 | 669,4 | 9,3 |
| 100 | 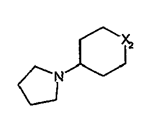 | | 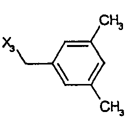 | 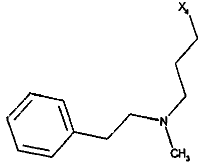 | 624,5 | 7,2 |
| 101 | H | 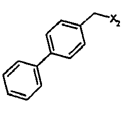 | 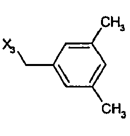 | 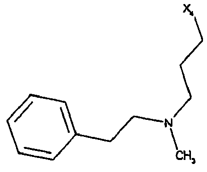 | 653,5 | 9,5 |
| 102 | H | 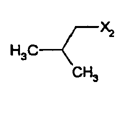 | 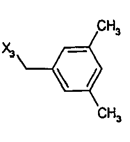 | 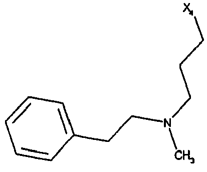 | 543,5 | 8,6 |
| 103 | H | 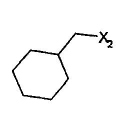 | 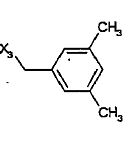 | 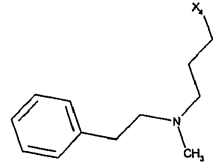 | 583,5 | 9,2 |
| 104 | 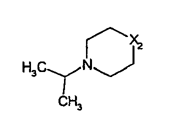 | | 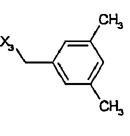 | 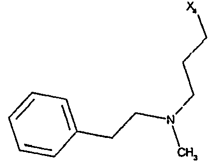 | 598,5 | 7,2 |
| 105 | 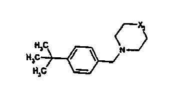 | | 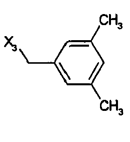 | 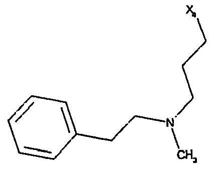 | 702,5 | 8,0 |
| 106 | 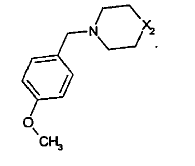 | | 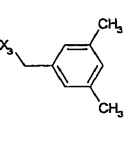 | 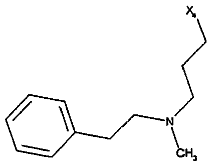 | 676,5 | 7,5 |
| 107 | 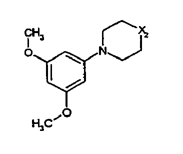 | | 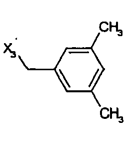 | 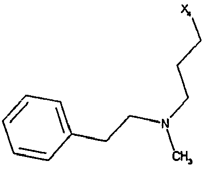 | 692,5 | 9,0 |
| 108 | H | 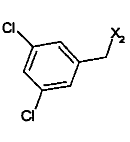 | 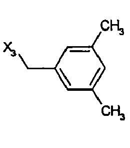 | 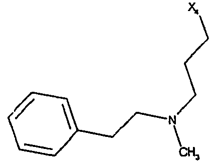 | 645,4 | 9,5 |
| 109 | H | 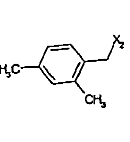 | 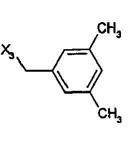 | 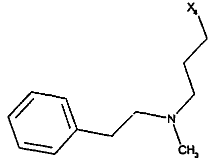 | 605,5 | 9,2 |
| 110 | 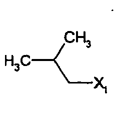 | 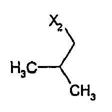 | 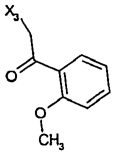 | 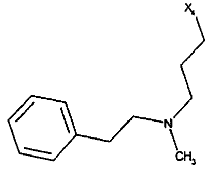 | 629,0 | 9,4 |
| 111 | 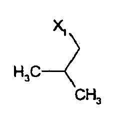 | 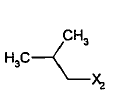 | 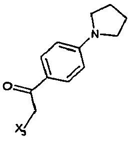 | 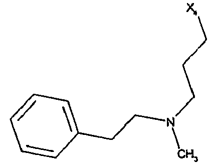 | 668,0 | 9,8 |
| 112 | 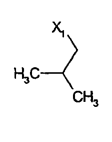 | 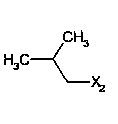 | 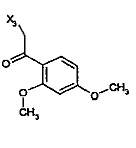 | 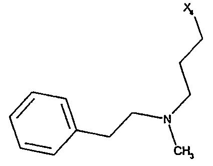 | 659,0 | 9,4 |
| 113 | 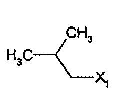 | 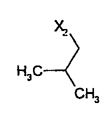 | 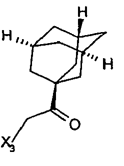 | 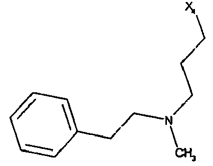 | 657,0 | 10,4 |
| 114 | 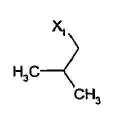 | 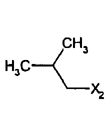 | 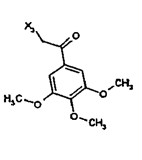 | 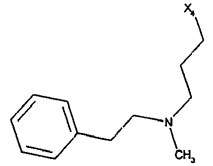 | 689,0 | 9,3 |
| 115 | 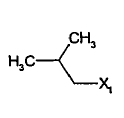 | 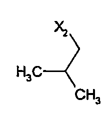 | 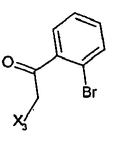 | 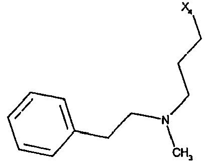 | 676,8 | 9,5 |
| 116 | 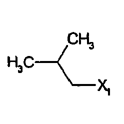 | 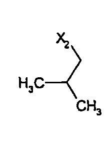 | 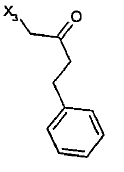 | 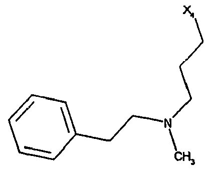 | 626,9 | 9,7 |
| 117 | 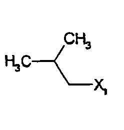 | 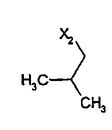 | 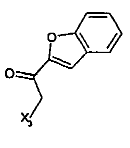 | 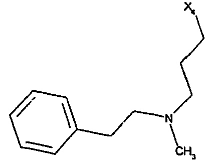 | 638,9 | 9,5 |
| 118 | 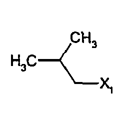 | 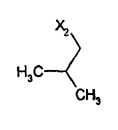 | 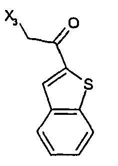 | 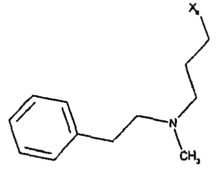 | 654,9 | 9,7 |
| 119 | H | 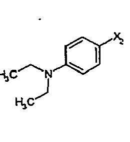 | 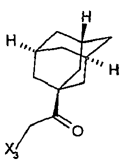 | 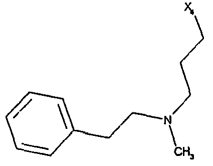 | 692,9 | 8,3 |
| 120 | H | 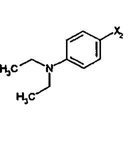 | 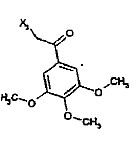 | 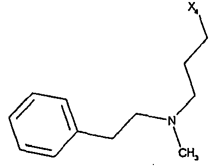 | 723,8 | 7,7 |
| 121 | H | 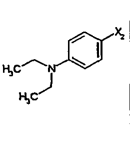 | 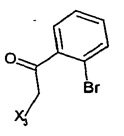 | 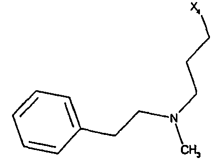 | 711,7 | 7,8 |
| 122 | H | 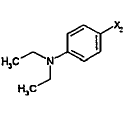 | 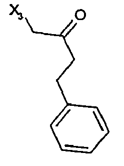 | 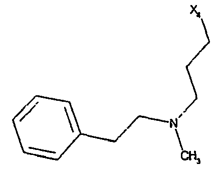 | 661,9 | 7,9 |
| 123 | H | 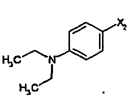 | 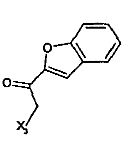 | 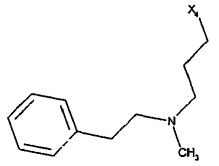 | 673,9 | 7,8 |
| 124 | H | 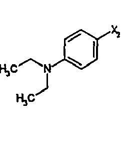 | 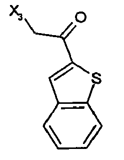 | 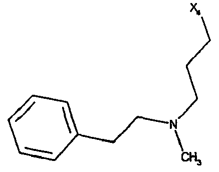 | 689,9 | 8,0 |
| 125 | 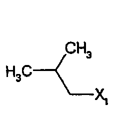 | 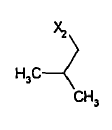 | 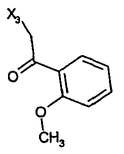 | 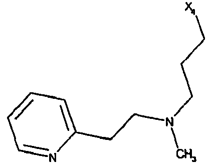 | 629,9 | 8,9 |
| 126 | 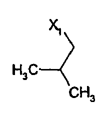 | 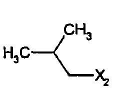 | 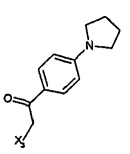 | 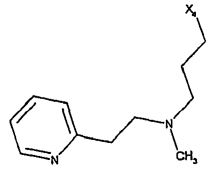 | 669,0 | 9,3 |
| 127 | 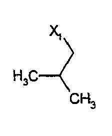 | 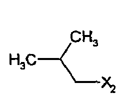 | 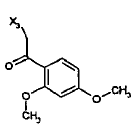 | 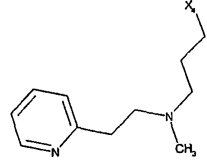 | 659,9 | 8,9 |
| 128 | 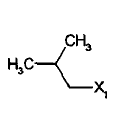 | 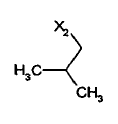 | 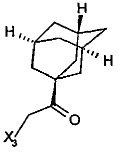 | 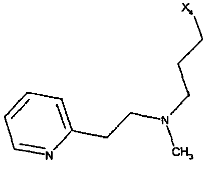 | 658,0 | 9,9 |
| 129 | 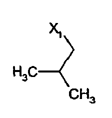 | 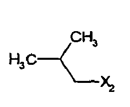 | 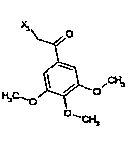 | 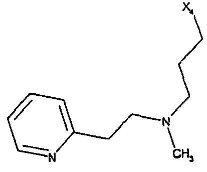 | 690,0 | 8,8 |
| 130 | 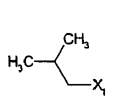 | 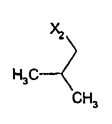 | 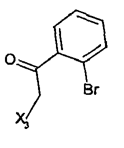 | 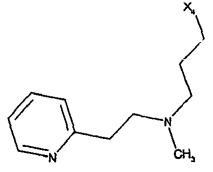 | 677,8 | 9,1 |
| 131 | 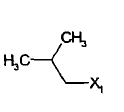 | 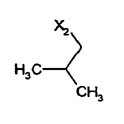 | 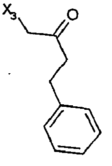 | 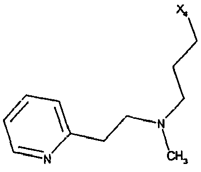 | 628,0 | 9,1 |
| 132 | 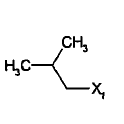 | 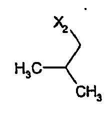 | 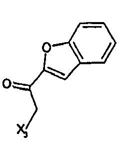 | 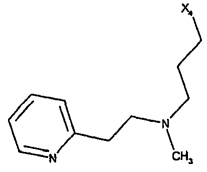 | 640,0 | 9,1 |
| 133 | 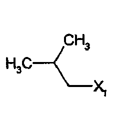 | 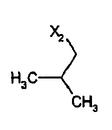 | 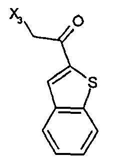 | 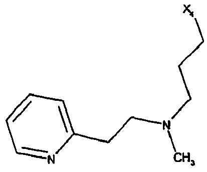 | 655,9 | 9,3 |
| 134 | H | 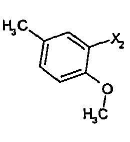 | 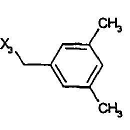 | 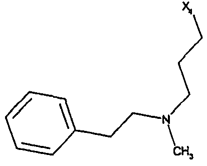 | 607,0 | 9,8 |
| 135 | H | 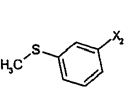 | 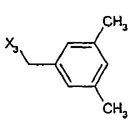 | 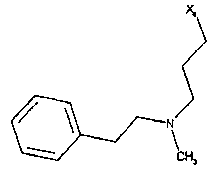 | 609,0 | 9,6 |
| 136 | H | 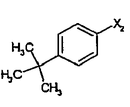 | 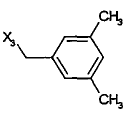 | 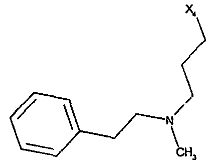 | 619,0 | 10,1 |
| 137 | H | 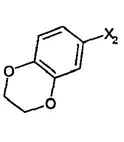 | 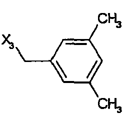 | 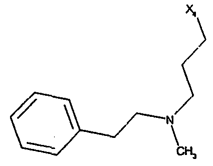 | 621,0 | 9,1 |
| 138 | H | 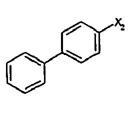 | 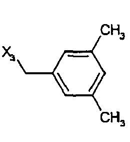 | 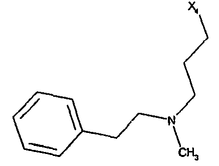 | 639,0 | 10,0 |
| 139 | H | 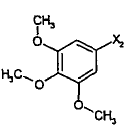 | 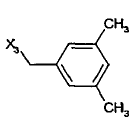 | 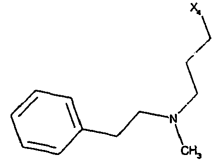 | 653,0 | 9,1 |
| 140 | H | 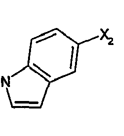 | 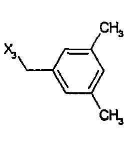 | 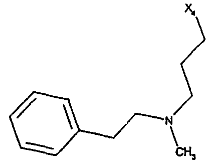 | 602,0 | 9,0 |
| 141 | H | 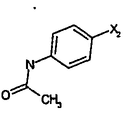 | 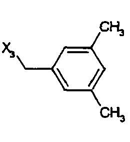 | 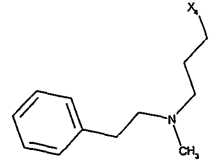 | 620,0 | 8,6 |
| 142 | H | 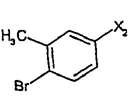 | 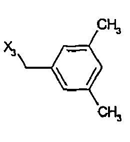 | 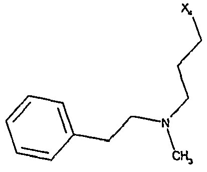 | 654,9 | 10,0 |
| 143 | H | 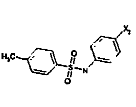 | 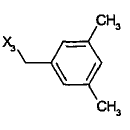 | 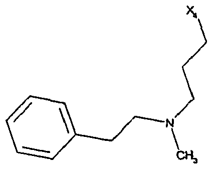 | 731,9 | 9,4 |
| 144 | H | 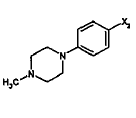 | 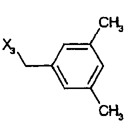 | 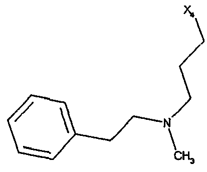 | 661,1 | 7,9 |
| 145 | H | 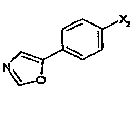 | 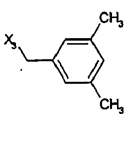 | 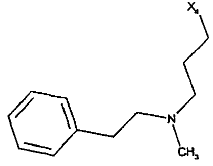 | 630,0 | 9,1 |
| 146 | H | 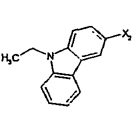 | 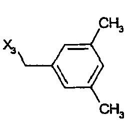 | 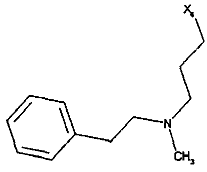 | 680,0 | 10,1 |
| 147 | 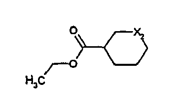 | | 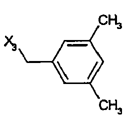 | 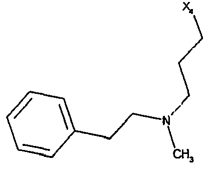 | 626,9 | 9,2 |
| 148 | 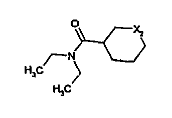 | | 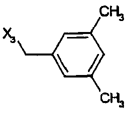 | 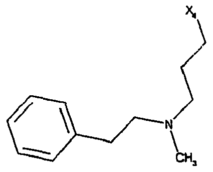 | 653,9 | 8,8 |
| 149 | H | 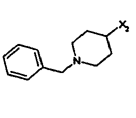 | 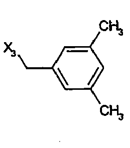 | 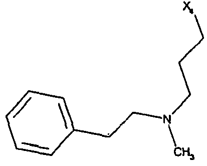 | 660,0 | 8,0 |
| 150 | 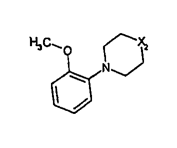 | | 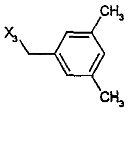 | 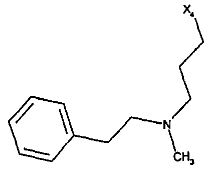 | 662,0 | 9,2 |
| 151 | 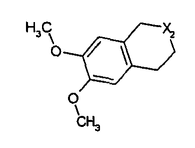 | | 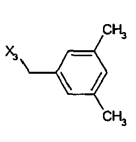 | 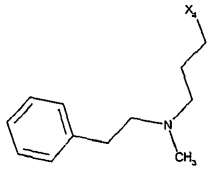 | 662,9 | 9,1 |
| 152 | 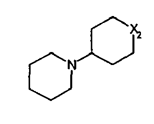 | | 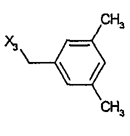 | 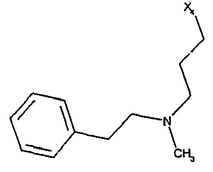 | 638,0 | 7,8 |
| 153 | 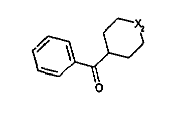 | | 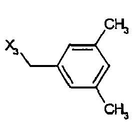 | 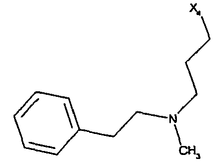 | 658,9 | 9,3 |
| 154 | 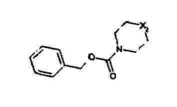 | | 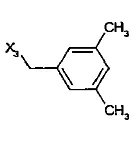 | 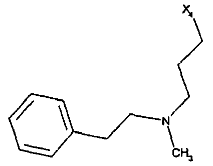 | 689,9 | 9,3 |
| 155 | 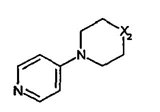 | | 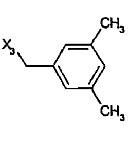 | 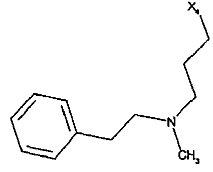 | 633,0 | 7,8 |
| 156 | H | 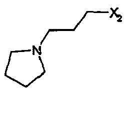 | 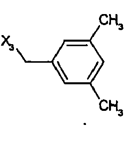 | 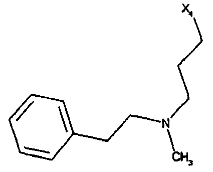 | 598,0 | 7,8 |
| 157 | 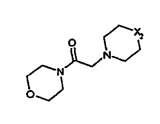 | | 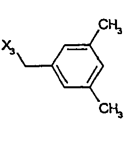 | 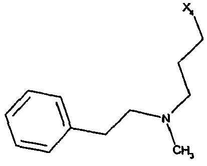 | 683,0 | 7,8 |
| 158 | 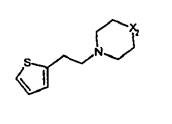 | | 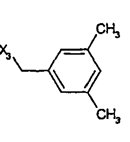 | 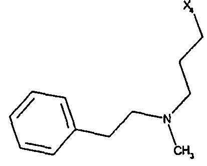 | 666,0 | 8,1 |
| 159 | 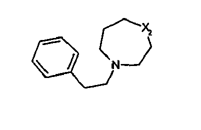 | | 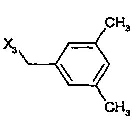 | 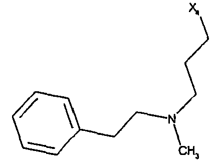 | 674,0 | 8,2 |
| 160 | 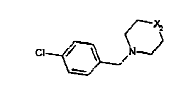 | | 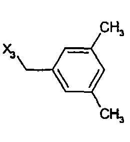 | 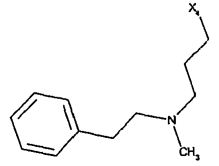 | 679,9 | 8,2 |
| 161 | 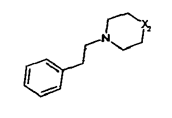 | | 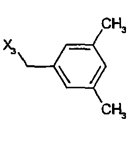 | 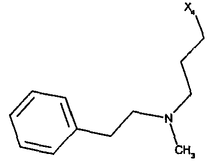 | 660,0 | 8,2 |
| 162 | H | 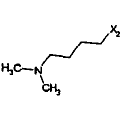 | 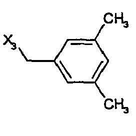 | 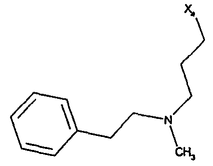 | 586,0 | 7,8 |
| 163 | 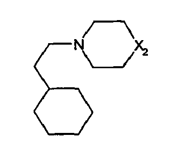 | | 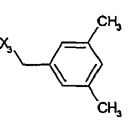 | 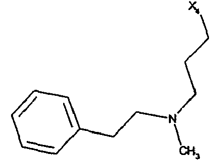 | 666,0 | 8,4 |
| 164 | 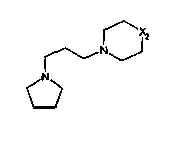 | | 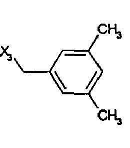 | 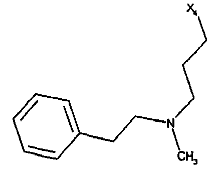 | 667,0 | 7,5 |
| 165 | 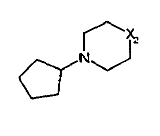 | | 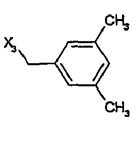 | 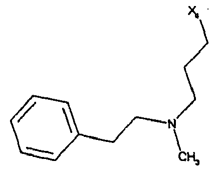 | 624,0 | 7,9 |
| 166 | 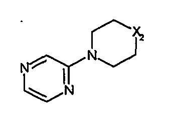 | | 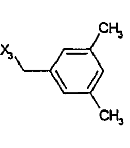 | 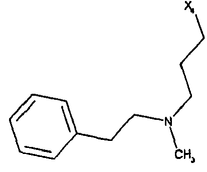 | 633,9 | 8,7 |
| 167 | 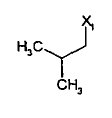 | 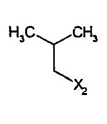 | 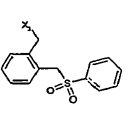 | 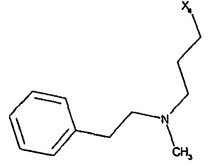 | 725,0 | 9,5 |
| 168 | 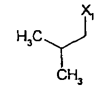 | 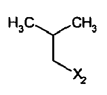 | 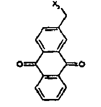 | 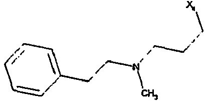 | 700,9 | 9,8 |
| 169 | 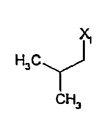 | 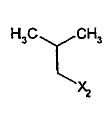 | 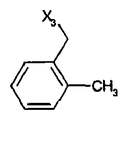 | 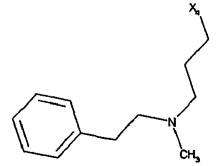 | 585,0 | 9,5 |
| 170 | 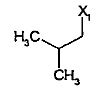 | 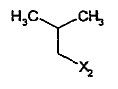 | 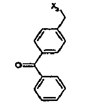 | 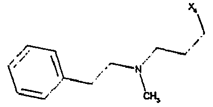 | 674,9 | 9,7 |
| 171 | 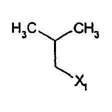 | 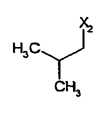 | 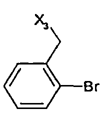 | 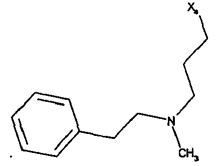 | 648,8 | 9,7 |
| 172 | 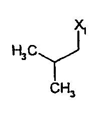 | 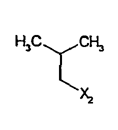 | 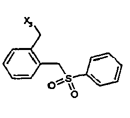 | 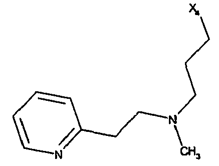 | 726,0 | 9,1 |
| 173 | 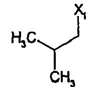 | 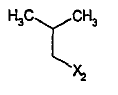 | 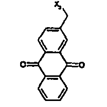 | 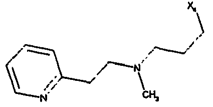 | 701,9 | 9,4 |
| 174 | 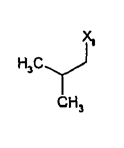 | 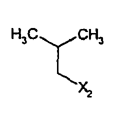 | 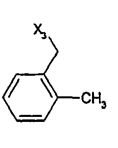 | 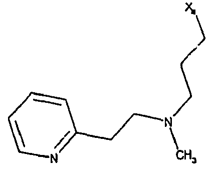 | 585,9 | 9,1 |
| 175 | 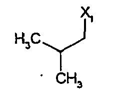 | 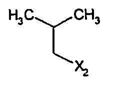 | 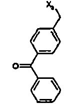 | 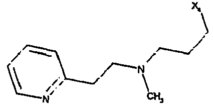 | 675,9 | 9,3 |
| 176 | 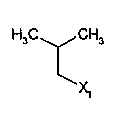 | 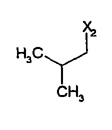 | 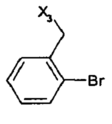 | 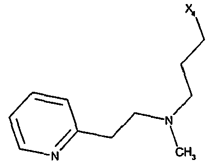 | 649,8 | 9,2 |
| 177 | 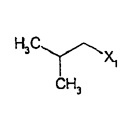 | 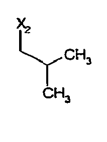 | 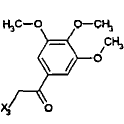 | 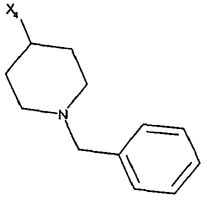 | 687,5 | 9,3 |
| 178 | 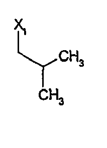 | 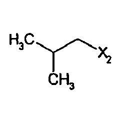 | 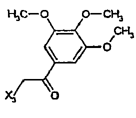 | 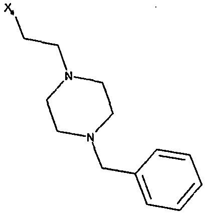 | 716,5 | 9,3 |
| 179 | 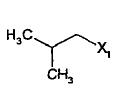 | 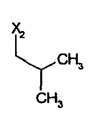 | 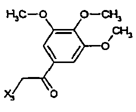 | 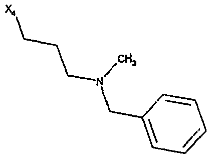 | 675,5 | 9,3 |
| 180 | 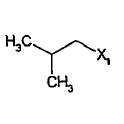 | 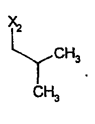 | 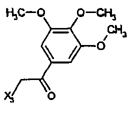 | 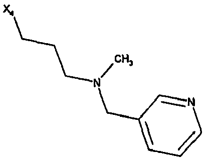 | 676,5 | 8,8 |
| 181 | 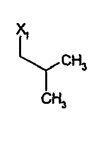 | 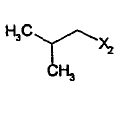 | 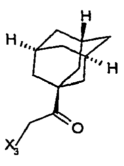 | 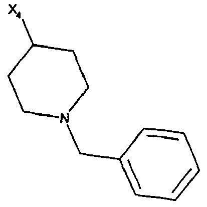 | 655,5 | 10,4 |
| 182 | 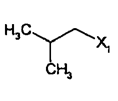 | | 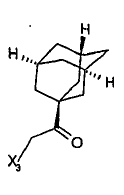 | 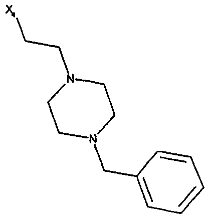 | 684,5 | 10,4 |
| 183 | 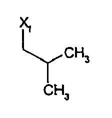 | | 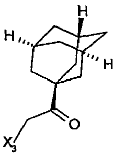 | 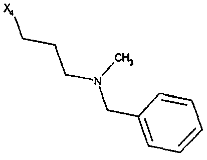 | 643,5 | 10,4 |
| 184 | 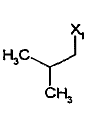 | | 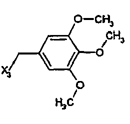 | 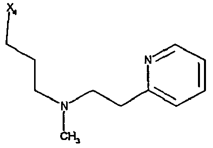 | 662,5 | 8,9 |
| 185 | 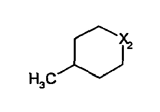 | | 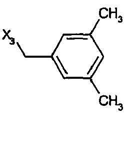 | 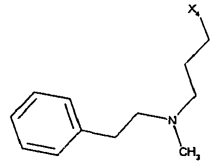 | 569,5 | 9,1 |
| 186 | H | 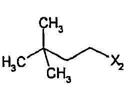 | 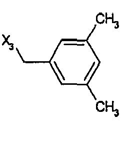 | 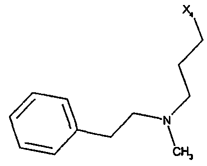 | 571,5 | 9,3 |
| 187 | 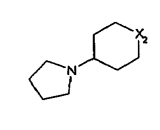 | | 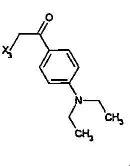 | 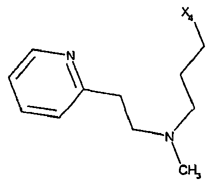 | 696,3 | 7,8 |
| 188 | 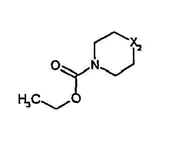 | | 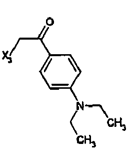 | 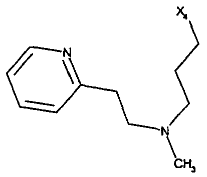 | 700,3 | 8,7 |
| 189 | 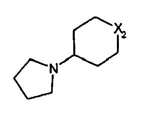 | | 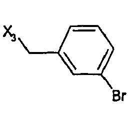 | 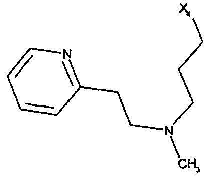 | 674,2 | 7,6 |
| 190 | 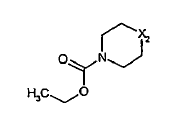 | | 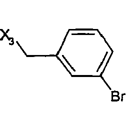 | 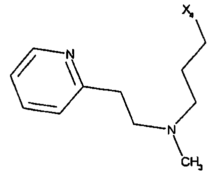 | 679,1 | 8,4 |
| 191 | H | 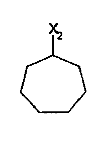 | 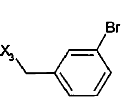 | 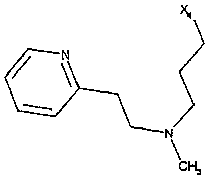 | 634,2 | 9,5 |
| 192 | 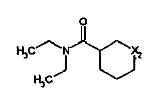 | | 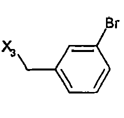 | 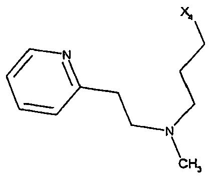 | 705,2 | 8,6 |
| 193 | 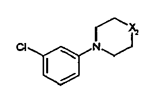 | | 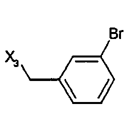 | 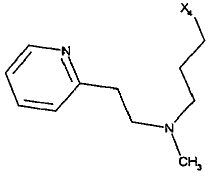 | 717,1 | 9,9 |
| 194 | H | 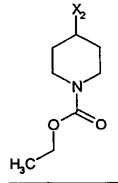 | 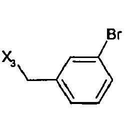 | 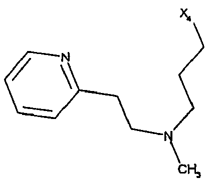 | 693,2 | 8,8 |
| 195 | 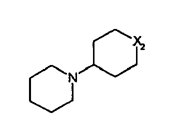 | | 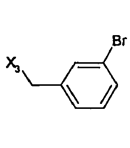 | 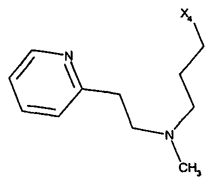 | 689,2 | 7,8 |
| 196 | 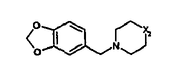 | | 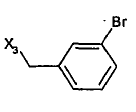 | 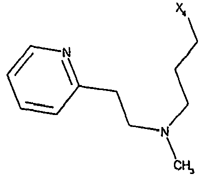 | 741,2 | 8,0 |
| 197 | 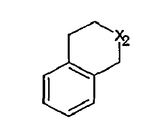 | | 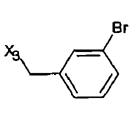 | 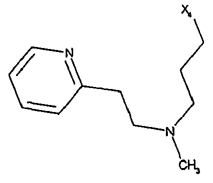 | 654,2 | 9,4 |
| 198 | 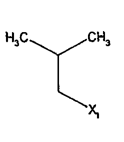 | 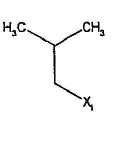 | 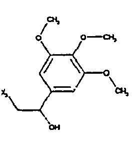 | 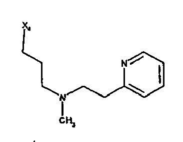 | 692,4 | 8,4 |
| 199 | 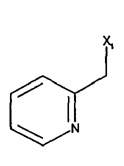 | H | 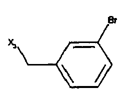 | 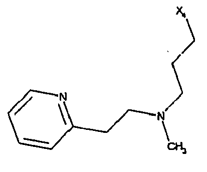 | 629,1 | 7,9 |
| 200 | 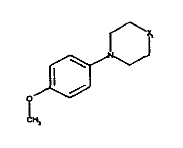 | | 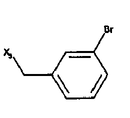 | 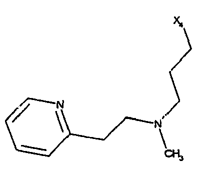 | 713,2 | 9,0 |
| 201 | 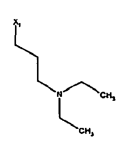 H | | 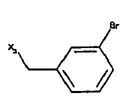 | 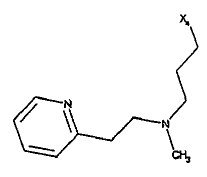 | 651,2 | 7,8 |
| 202 | 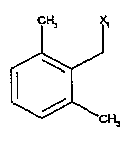 | | 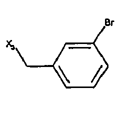 | 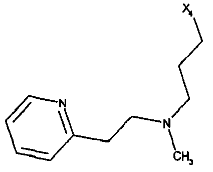 | 656,2 | 9,5 |
| 203 | 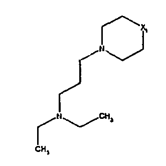 | | 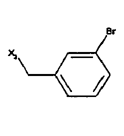 | 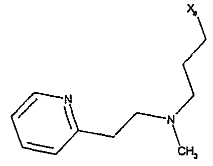 | 720,3 | 7,5 |
| 204 | 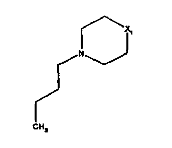 | | 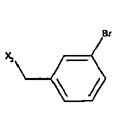 | 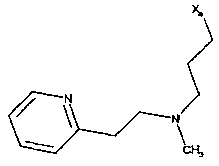 | 663,2 | 7,9 |
| 205 | 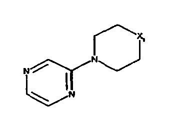 | | 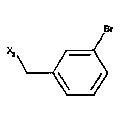 | 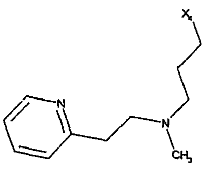 | 685,2 | 8,5 |
| 206 | 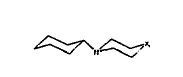 | | 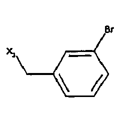 | 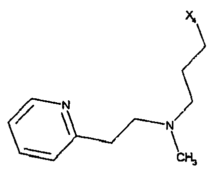 | 689,3 | 8,0 |
| 207 | 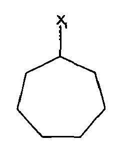 H | | 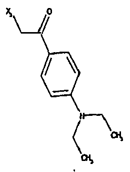 | 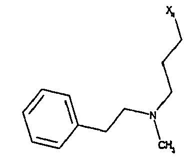 | 654,3 | 10,1 |
| 208 | 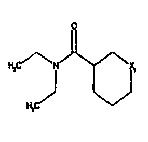 | | 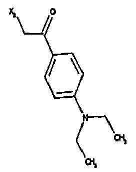 | 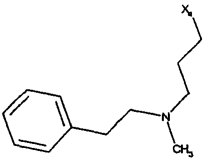 | 725,3 | 9,3 |
| 209 | 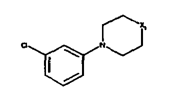 | | 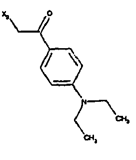 | 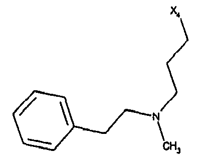 | 737,3 | 10,4 |
| 210 | 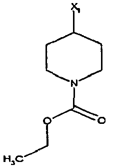 | | 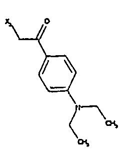 | 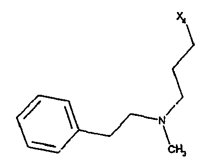 | 713,4 | 9,4 |
| 211 | 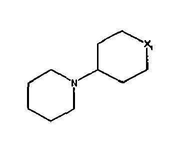 | | 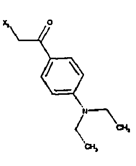 | 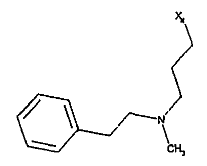 | 709,4 | 8,3 |
| 212 | 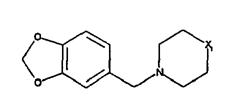 | | 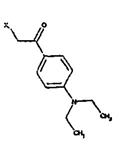 | 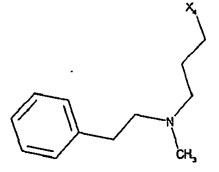 | 761,3 | 8,5 |
| 213 | 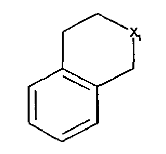 | | 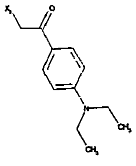 | 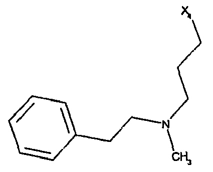 | 674,3 | 9,9 |
| 214 | 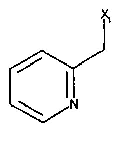 H | | 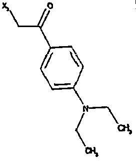 | 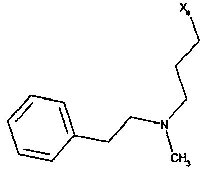 | 649,3 | 8,5 |
| 215 | 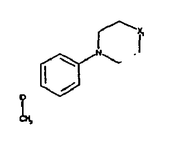 | | 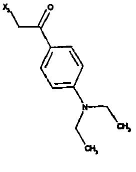 | 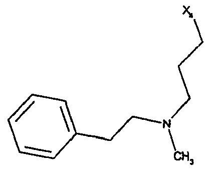 | 733,3 | 9,5 |
| 216 | 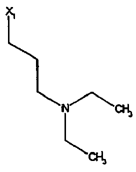 | H | 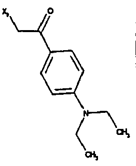 | 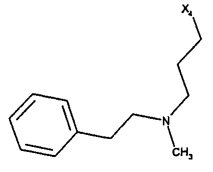 | 671,3 | 8,3 |
| 217 | 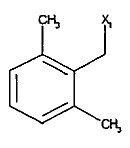 | H | 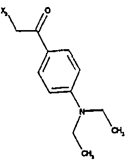 | 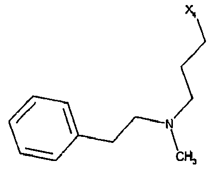 | 676,3 | 10,1 |
| 218 | 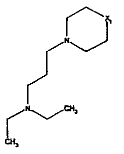 | | 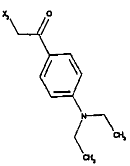 | 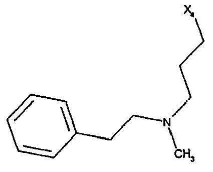 | 740,4 | 7,9 |
| 219 | 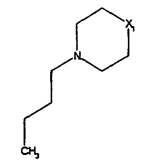 | | 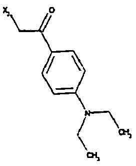 | 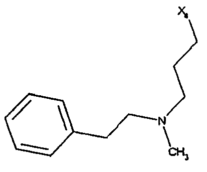 | 683,3 | 8,4 |
| 220 | 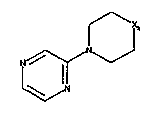 | | 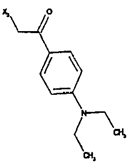 | 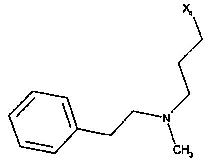 | 705,3 | 9,2 |
| 221 | 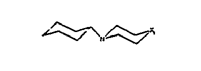 | | 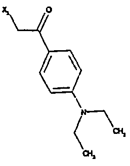 | 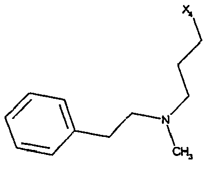 | 709,4 | 8,5 |
| 222 | 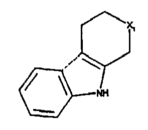 | | 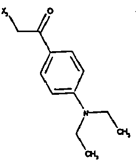 | 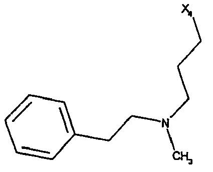 | 713,3 | 10,0 |
| 223 | 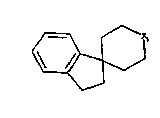 | | 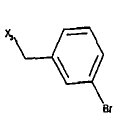 | 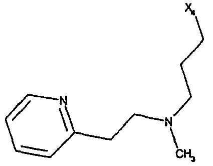 | 708,2 | 10,0 |
| 224 | 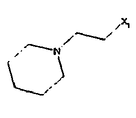 | H | 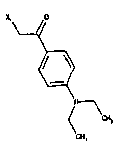 | 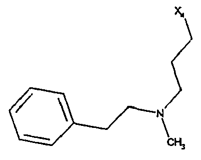 | 669,4 | 8,2 |
| 225 | 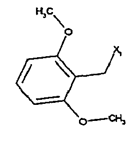 | H | 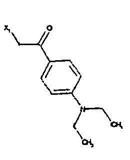 | 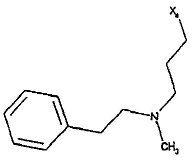 | 708,3 | 9,6 |
| 226 | 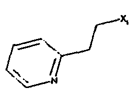 | H | 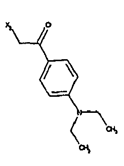 | 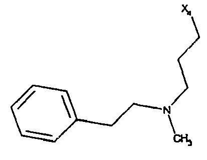 | 663,3 | 8,1 |
| 227 | 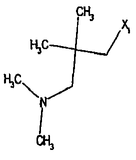 | H | 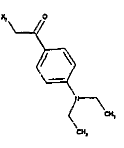 | 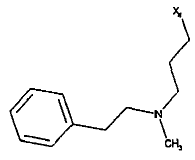 | 671,4 | 8,2 |
| 228 | 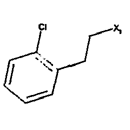 | H | 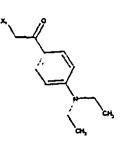 | 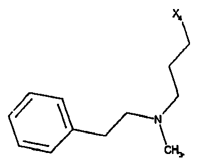 | 696,3 | 9,8 |
| 229 | 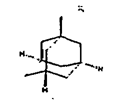 | H | 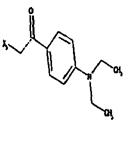 | 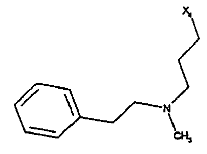 | 706,4 | 10,3 |
| 230 | 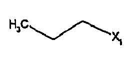 | 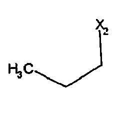 | 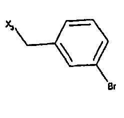 | 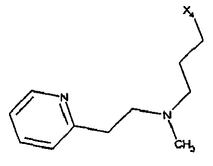 | 622,2 | 9,3 |
| 231 | 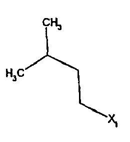 | 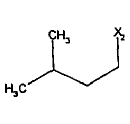 | 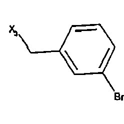 | 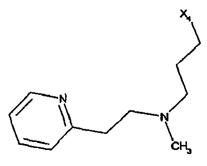 | 678,2 | 10,4 |
| 232 | 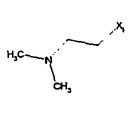 | H | 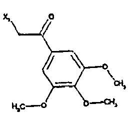 | 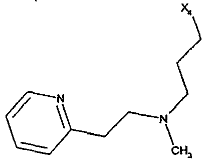 | 649,3 | 7,3 |
| 233 | 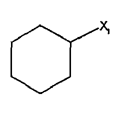 | H | 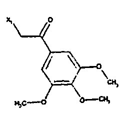 | 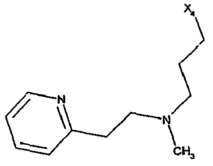 | 660,3 | 8,5 |
| 234 | 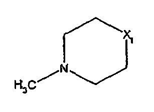 | | 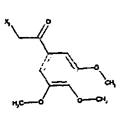 | 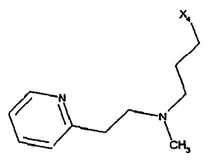 | 660,4 | 7,3 |
| 235 | 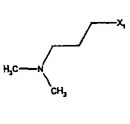 H | | 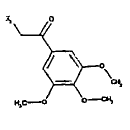 | 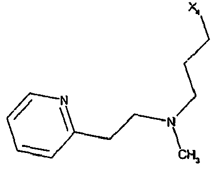 | 663,4 | 7,3 |
| 236 | 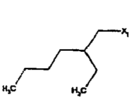 H | | 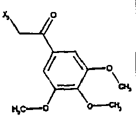 | 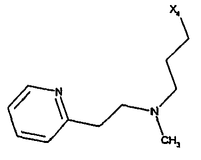 | 690,5 | 9,2 |
| 237 | 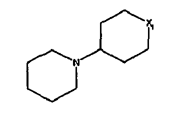 | | 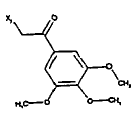 | 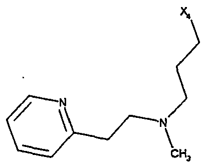 | 729,5 | 7,4 |
| 238 | 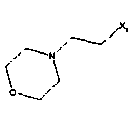 | H | 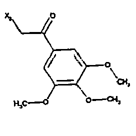 | 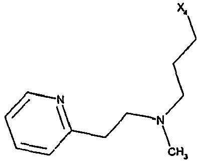 | 691,4 | 7,4 |
| 239 | 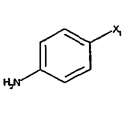 | H | 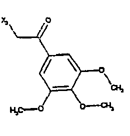 | 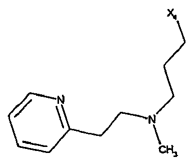 | 669,4 | 7,5 |
| 240 | 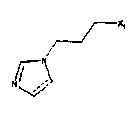 | H | 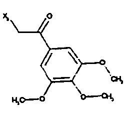 | 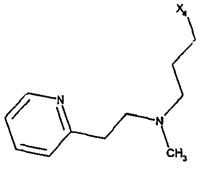 | 686,4 | 7,4 |
| 241 | 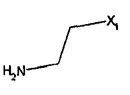 | H | 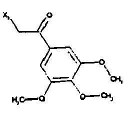 | 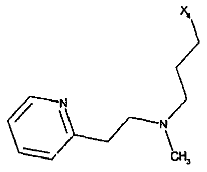 | 621,3 | 7,3 |
| 242 | 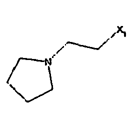 | H | 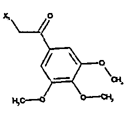 | 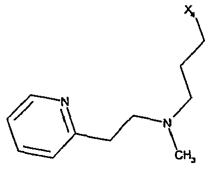 | 675,4 | 7,4 |
| 243 | 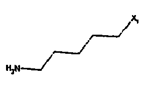 | H | 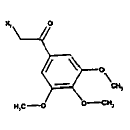 | 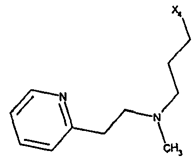 | 663,4 | 7,4 |
| 244 | 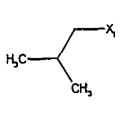 | H | 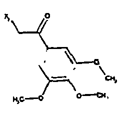 | 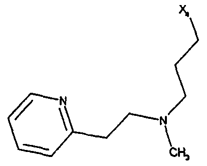 | 634,4 | 8,2 |
| 245 | 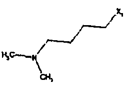 | H | 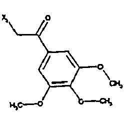 | 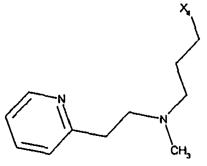 | 677,4 | 7,4 |
| 246 | 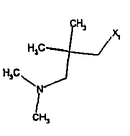 | H | 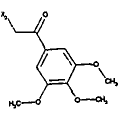 | 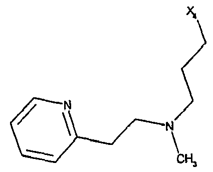 | 691,4 | 7,4 |
| 247 | 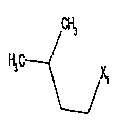 | 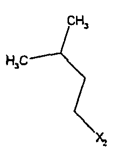 | 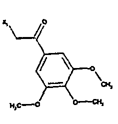 | 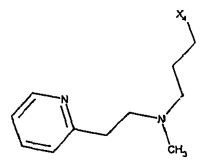 | 718,6 | 9,5 |
| 248 | 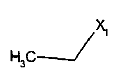 | 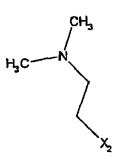 | 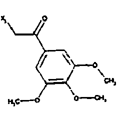 | 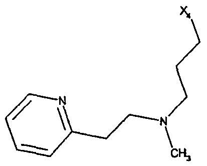 | 677,5 | 7,4 |
| 249 | 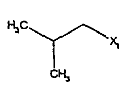 | 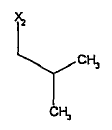 | 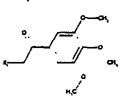 | 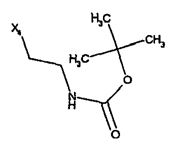 | 657,4 | 11,4 |
| 250 | 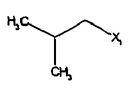 | 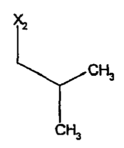 | 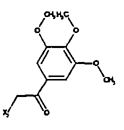 | 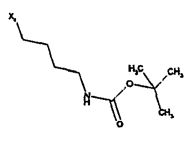 | 685,4 | 11,7 |
| 251 | 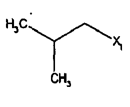 | 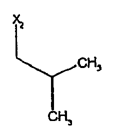 | 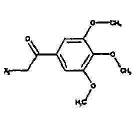 | 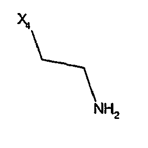 | 557,3 | 8,6 |
| 252 | 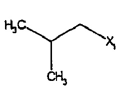 | 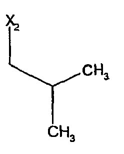 | 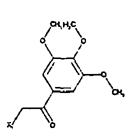 | 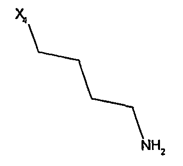 | 585,3 | 8,8 |
| 253 | 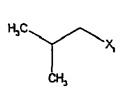 | 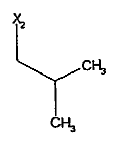 | 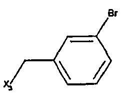 | 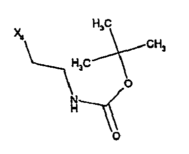 | 617,2 | 12,4 |
| 254 | 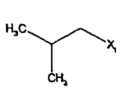 | 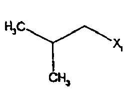 | 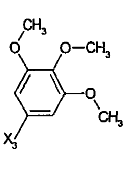 | 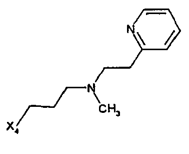 | 617,4 | 7,2 |
| 255 | 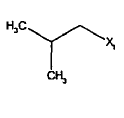 | 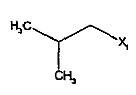 | 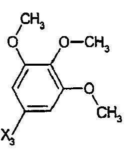 | 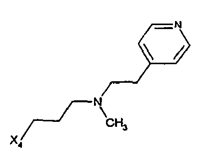 | 617,4 | 7,1 |
| 256 | 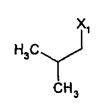 | 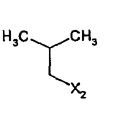 | 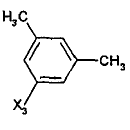 | 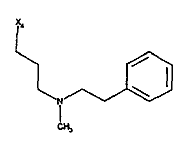 | 568,4 | 8,4 |
| 257 | 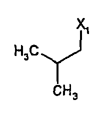 | 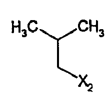 | 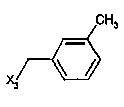 | 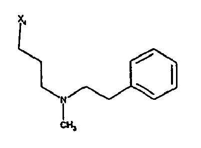 | 568,3 | 7,8 |
| 258 | 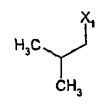 | 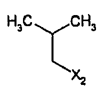 | 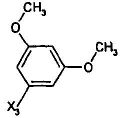 | 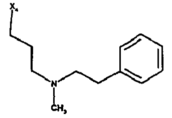 | 600,4 | 7,8 |
| 259 | H | 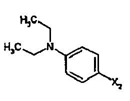 | 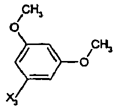 | 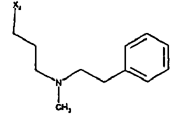 | 747,5 | 6,7 |
| 260 | 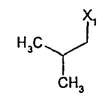 | 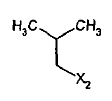 | 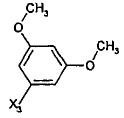 | 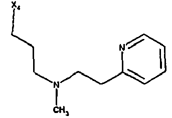 | 601,4 | 7,4 |
| 261 | 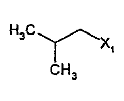 | 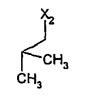 | 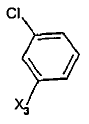 | 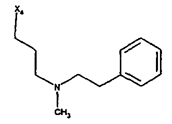 | 574,4 | 8,3 |
| 262 | 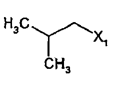 | 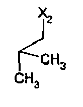 | 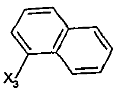 | 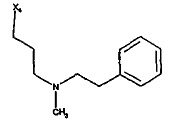 | 590,4 | 8,2 |
| 263 | 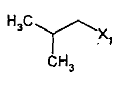 | 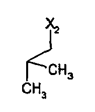 | 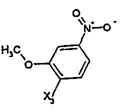 | 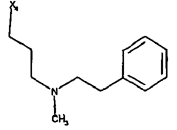 | 615,4 | 8,6 |
| 264 | 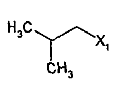 | 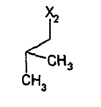 | 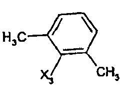 | 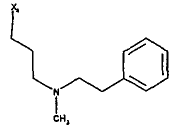 | 568,5 | 8,1 |
| 265 | 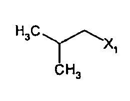 | 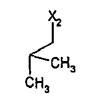 | 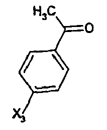 | 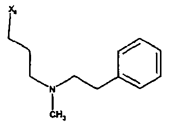 | 582,4 | 8,0 |
| 266 | 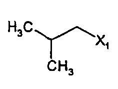 | 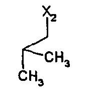 | 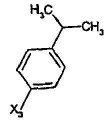 | 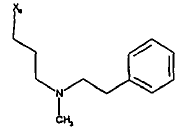 | 582,5 | 8,5 |
| 267 | 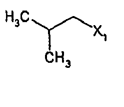 | 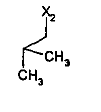 | 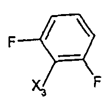 | 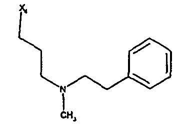 | 576,4 | 8,1 |
| 268 | 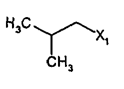 | 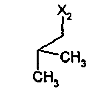 | 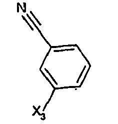 | 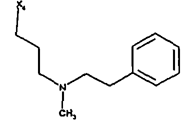 | 565,4 | 8,1 |
| 269 | 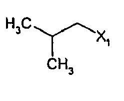 | 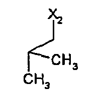 | 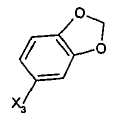 | 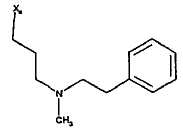 | 584,4 | 8,0 |
| 270 | 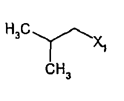 | 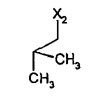 | 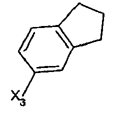 | 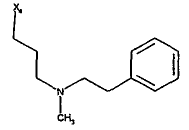 | 580,5 | 8,3 |
| 271 | 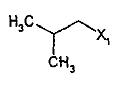 | 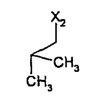 | 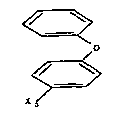 | 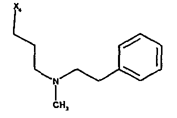 | 632,4 | 8,6 |
| 272 | 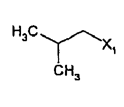 | 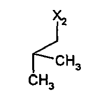 | 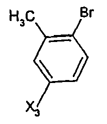 | 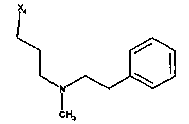 | 632,4 | 8,5 |
| 273 | H | 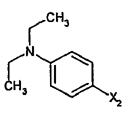 | 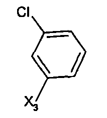 | 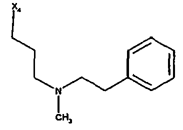 | 609,3 | 7,0 |
| 274 | H | 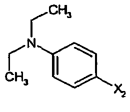 | 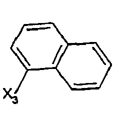 | 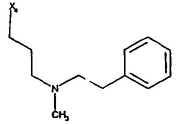 | 625,4 | 7,0 |
| 275 | H | 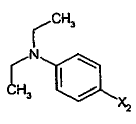 | 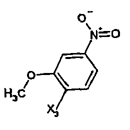 | 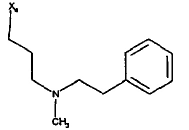 | 650,4 | 7,2 |
| 276 | H | 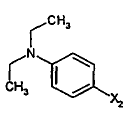 | 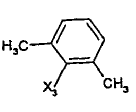 | 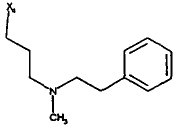 | 603,4 | 6,9 |
| 277 | H | 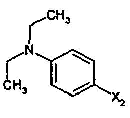 | 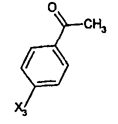 | 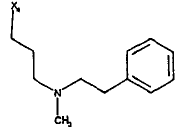 | 617,4 | 6,8 |
| 278 | H | 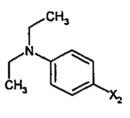 | 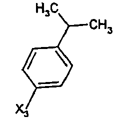 | 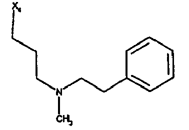 | 617,4 | 7,2 |
| 279 | H | 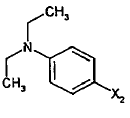 | 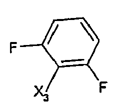 | 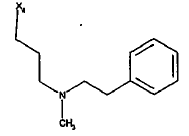 | 611,4 | 6,8 |
| 280 | H | 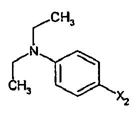 | 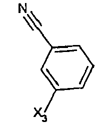 | 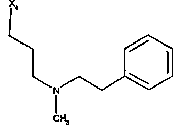 | 600,4 | 6,9 |
| 281 | H | 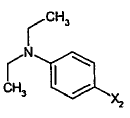 | 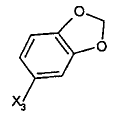 | 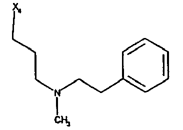 | 619,3 | 6,7 |
| 282 | H | 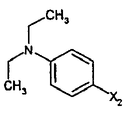 | 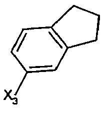 | 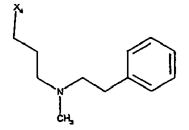 | 615,4 | 7,0 |
| 283 | H | 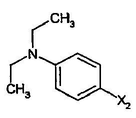 | 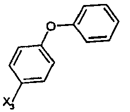 | 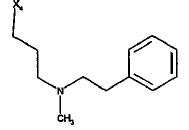 | 667,4 | 7,3 |
| 284 | H | 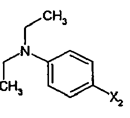 | 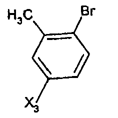 | 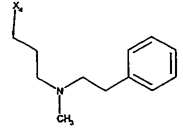 | 667,3 | 7,2 |
| 285 | 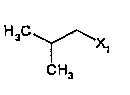 | 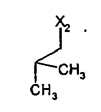 | 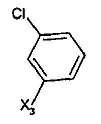 | 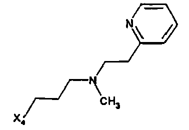 | 575,4 | 7,6 |
| 286 | 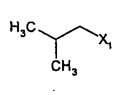 | 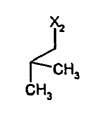 | 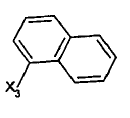 | 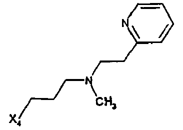 | 591,4 | 7,5 |
| 287 | 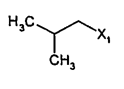 | 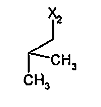 | 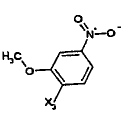 | 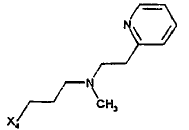 | 616,4 | 7,9 |
| 288 | 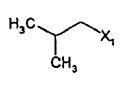 | 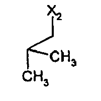 | 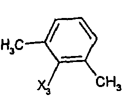 | 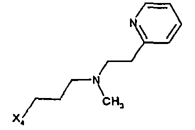 | 569,4 | 7,4 |
| 289 | 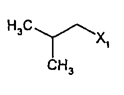 | 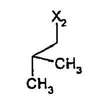 | 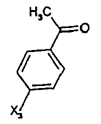 | 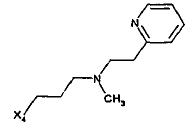 | 583,4 | 7,4 |
| 290 | 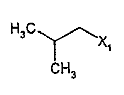 | 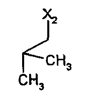 | 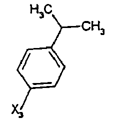 | 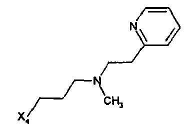 | 583,4 | 7,8 |
| 291 | 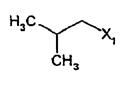 | 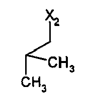 | 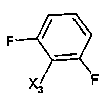 | 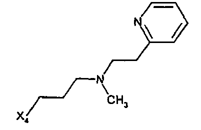 | 577,4 | 7,4 |
| 292 | 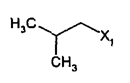 | 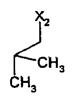 | 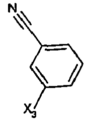 | 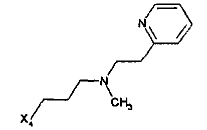 | 566,4 | 7,5 |
| 293 | 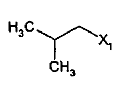 | 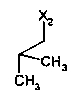 | 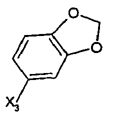 | 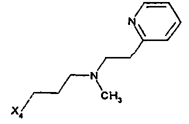 | 583,3 | 7,3 |
| 294 | 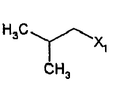 | 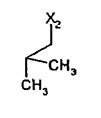 | 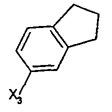 | 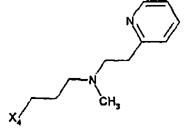 | 581,4 | 7,6 |
| 295 | 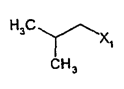 | 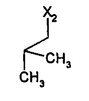 | 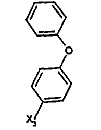 | 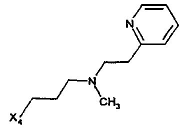 | 633,3 | 7,9 |
| 296 | 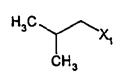 | 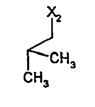 | 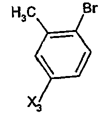 | 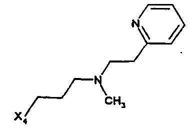 | 633,2 | 7,8 |
| 297 | 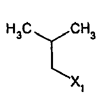 | 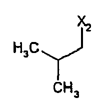 | 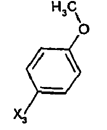 | 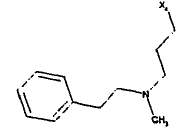 | 570,1 | 7,9 |
| 298 | 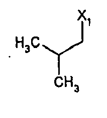 | 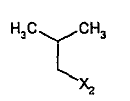 | 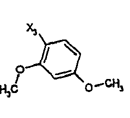 | 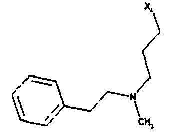 | 600,1 | 7,9 |
| 299 | 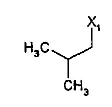 | 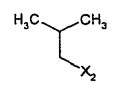 | 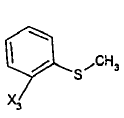 | 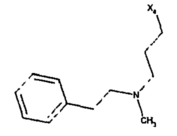 | 586,1 | 8,0 |
| 300 | 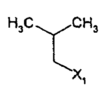 | 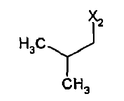 | 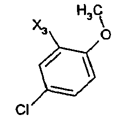 | 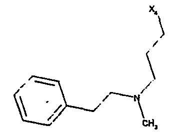 | 604,1 | 8,4 |
| 301 | 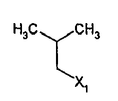 | 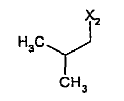 | 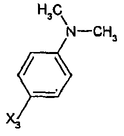 | 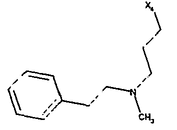 | 583,2 | 7,9 |
| 302 | 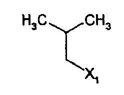 | 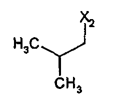 | 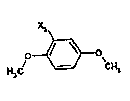 | 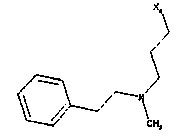 | 600,1 | 8,0 |
| 303 | 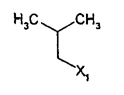 | 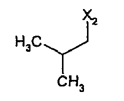 | 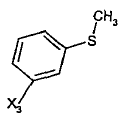 | 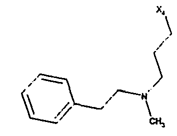 | 586,1 | 8,3 |
| 304 | 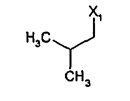 | 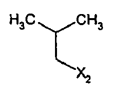 | 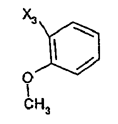 | 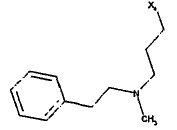 | 570,1 | 7,9 |
| 305 | 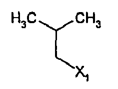 | 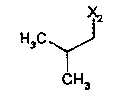 | 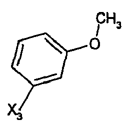 | 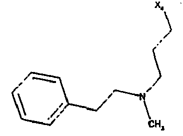 | 570,1 | 8,1 |
| 306 | 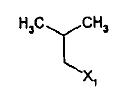 | 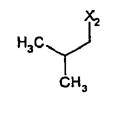 | 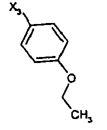 | 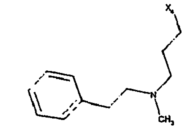 | 584,1 | 8,0 |
| 307 | 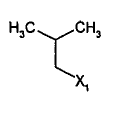 | 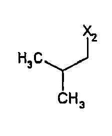 | 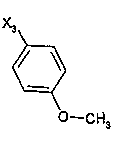 | 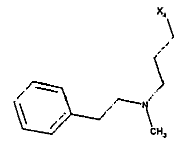 | 571,1 | 7,6 |
| 308 | 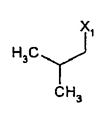 | 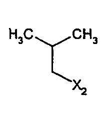 | 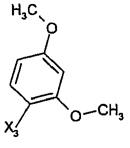 | 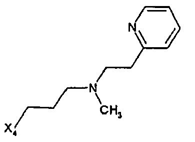 | 601,1 | 7,6 |
| 309 | 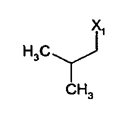 | 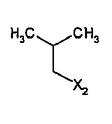 | 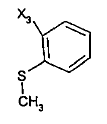 | 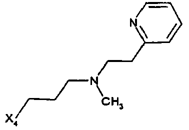 | 586,9 | 7,6 |
| 310 | 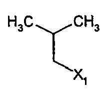 | 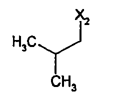 | 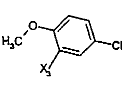 | 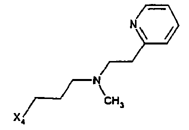 | 605,0 | 8,1 |
| 311 | 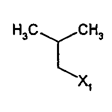 | 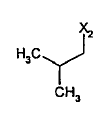 | 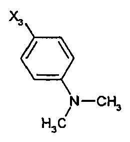 | 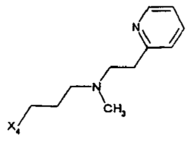 | 584,1 | 7,6 |
| 312 | 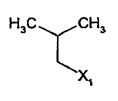 | 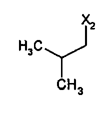 | 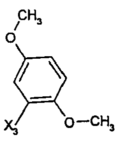 | 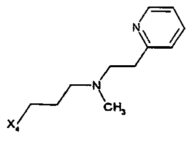 | 601,1 | 7,7 |
| 313 | 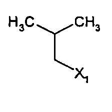 | 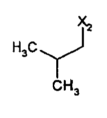 | 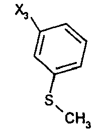 | 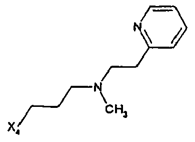 | 587,0 | 7,9 |
| 314 | 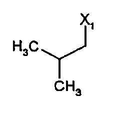 | 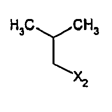 | 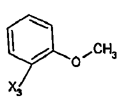 | 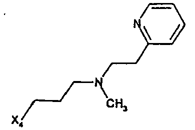 | 571,1 | 7,6 |
| 315 | 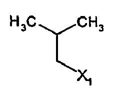 | 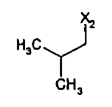 | 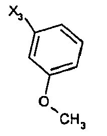 | 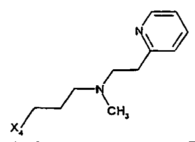 | 571,1 | 7,7 |
| 316 | 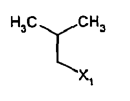 | 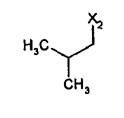 | 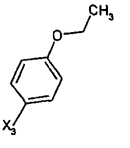 | 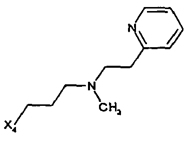 | 585,1 | 7,7 |
| 317 | H | 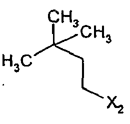 | 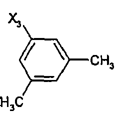 | 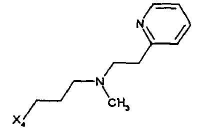 | 540,4 | 8,1 |
| 318 | 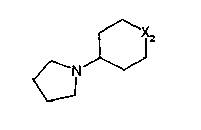 | | 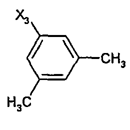 | 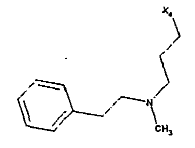 | 593,2 | 7,4 |
| 319 | 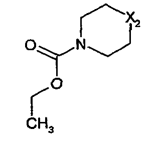 | | 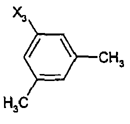 | 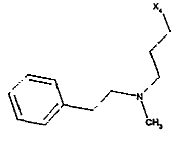 | 597,2 | 7,9 |
| 320 | 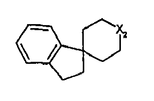 | | 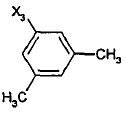 | 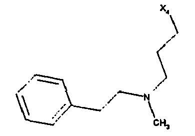 | 626,2 | 8,7 |
| 321 | 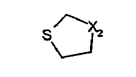 | | 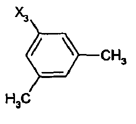 | 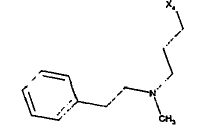 | 528,2 | 8,0 |
| 322 | H | 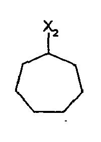 | 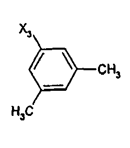 | 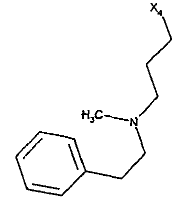 | 552,2 | 8,6 |
| 323 | 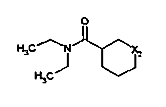 | | 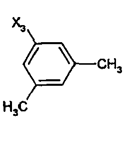 | 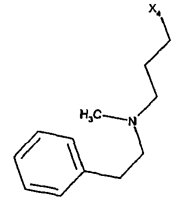 | 623,3 | 8,1 |
| 324 | 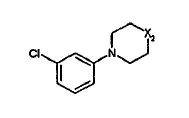 | | 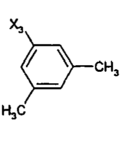 | 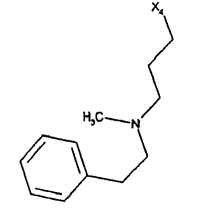 | 635,2 | 8,8 |
| 325 | H | 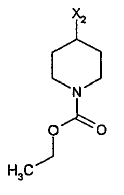 | 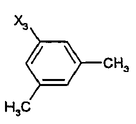 | 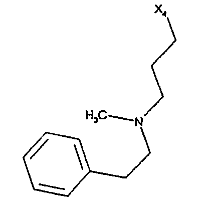 | 611,2 | 8,2 |
| 326 | 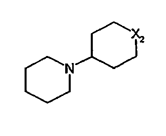 | | 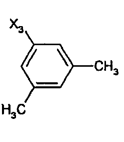 | 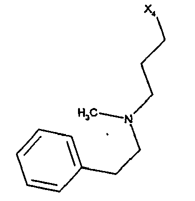 | 607,0 | 7,6 |
| 327 | 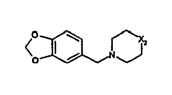 | | 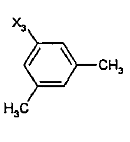 | 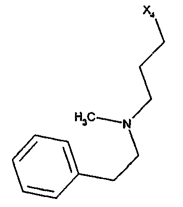 | 659,2 | 7,8 |
| 328 | 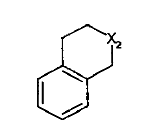 | | 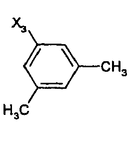 | 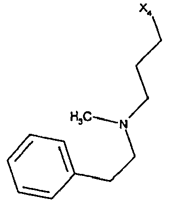 | 572,2 | 8,5 |
| 329 | H | 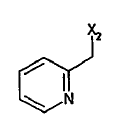 | 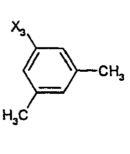 | 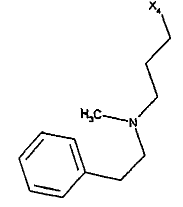 | 547,2 | 7,7 |
| 330 | 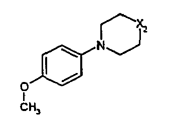 | | 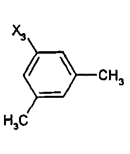 | 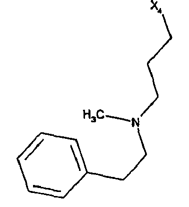 | 631,2 | 8,4 |
| 331 | H | 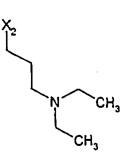 | 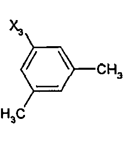 | 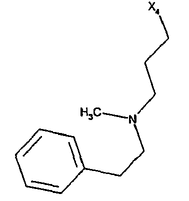 | 569,2 | 7,6 |
| 332 | H | 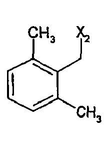 | 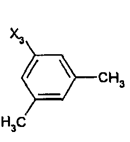 | 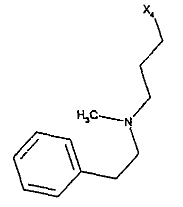 | 574,2 | 8,6 |
| 333 | 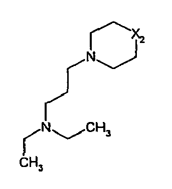 | | 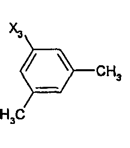 | 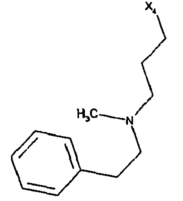 | 638,3 | 7,4 |
| 334 | 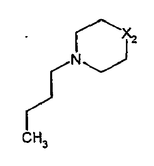 | | 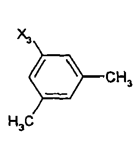 | 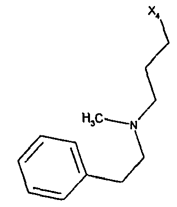 | 581,3 | 7,7 |
| 335 | 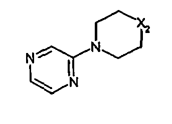 | | 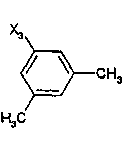 | 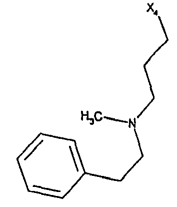 | 603,2 | 8,0 |
| 336 | 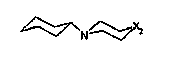 | | 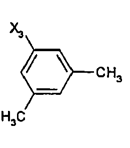 | 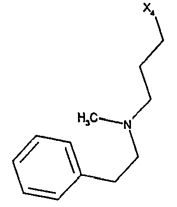 | 607,3 | 7,7 |
| 337 | H | 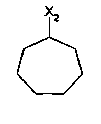 | 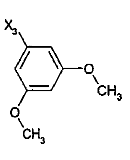 | 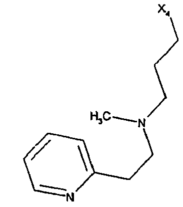 | 585,2 | 8,2 |
| 338 | 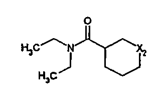 | | 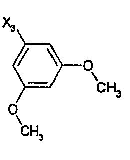 | 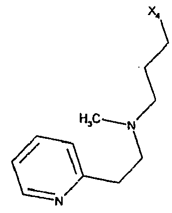 | 656,3 | 7,2 |
| 339 | 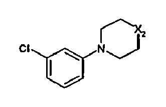 | | 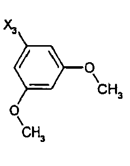 | 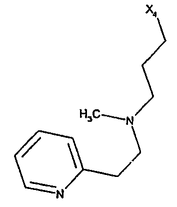 | 668,2 | 8,4 |
| 340 | H | 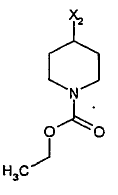 | 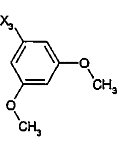 | 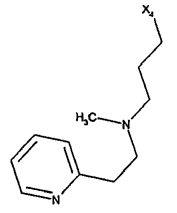 | 644,2 | 7,8 |
| 341 | 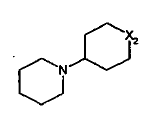 | | 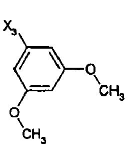 | 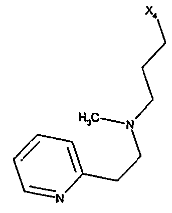 | 640,3 | 7,2 |
| 342 | 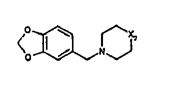 | | 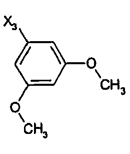 | 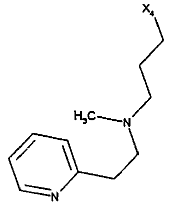 | 692,2 | 7,5 |
| 343 | 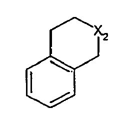 | | 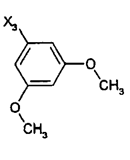 | 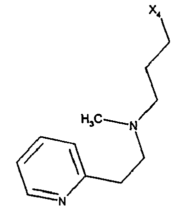 | 605,2 | 8,1 |
| 344 | H | 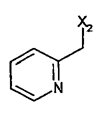 | 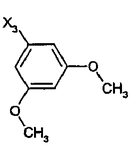 | 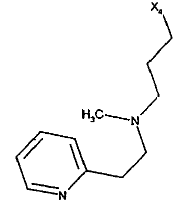 | 580,2 | 7,3 |
| 345 | 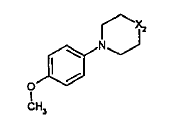 | | 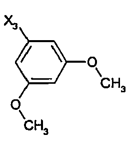 | 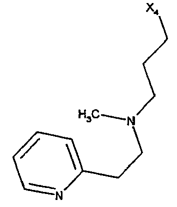 | 664,2 | 8,0 |
| 346 | H | 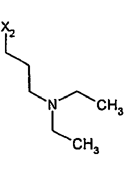 | 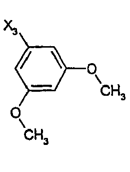 | 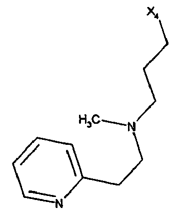 | 602,3 | 7,2 |
| 347 | H | 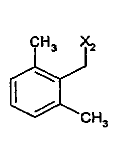 | 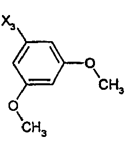 | 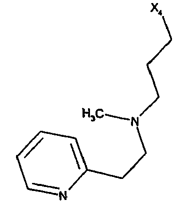 | 607,2 | 8,2 |
| 348 | 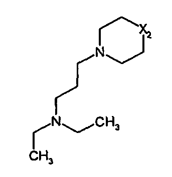 | | 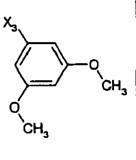 | 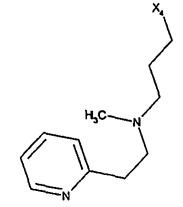 | 671,3 | 7,1 |
| 349 | 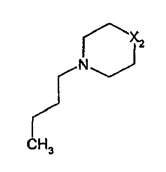 | | 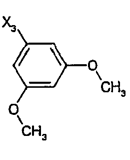 | 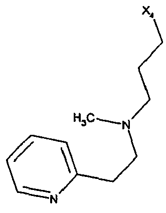 | 614,3 | 7,3 |
| 350 | 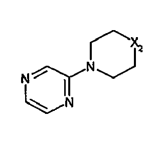 | | 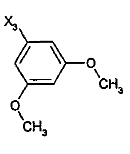 | 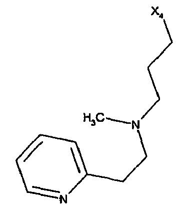 | 636,2 | 7,6 |
| 351 | 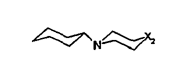 | | 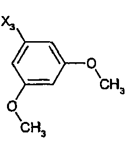 | 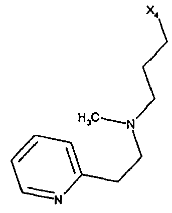 | 640,3 | 7,4 |
| 352 | 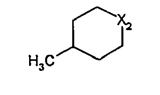 | | 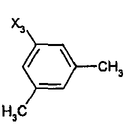 | 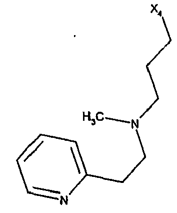 | 538,2 | 8,2 |
| 353 | H | 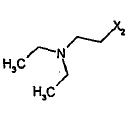 | 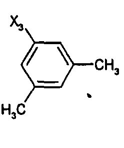 | 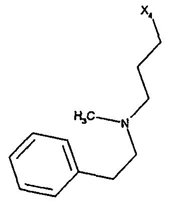 | 555,3 | 7,5 |
| 354 | H | 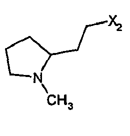 | 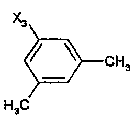 | 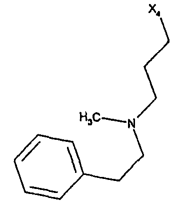 | 567,3 | 7,4 |
| 355 | H | 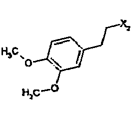 | 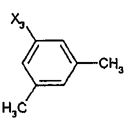 | 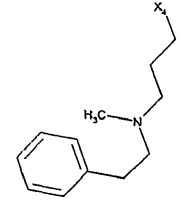 | 620,3 | 8,1 |
| 356 | H | 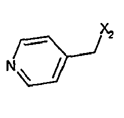 | 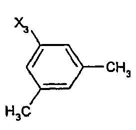 | 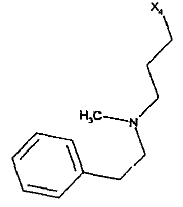 | 547,3 | 7,4 |
| 357 | 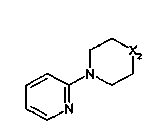 | | 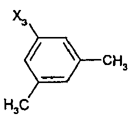 | 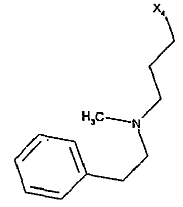 | 602,3 | 7,5 |
| 358 | H | 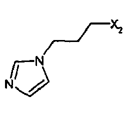 | 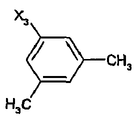 | 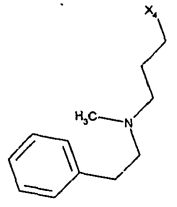 | 564,3 | 7,4 |
| 359 | H | 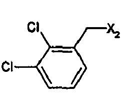 | 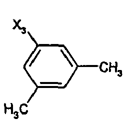 | 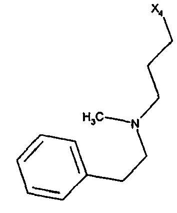 | 614,2 | 8,5 |
| 360 | H | 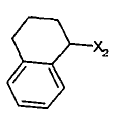 | 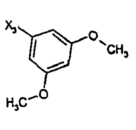 | 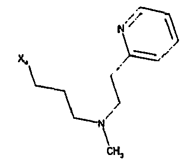 | 619,3 | 8,1 |
| 361 | 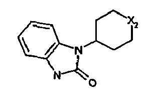 | | 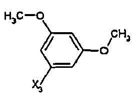 | 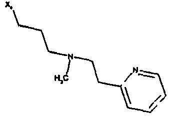 | 689,3 | 7,6 |
| 362 | 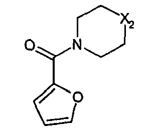 | | 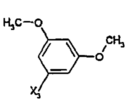 | 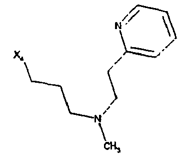 | 652,3 | 7,5 |
| 363 | H | 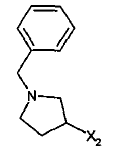 | 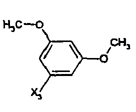 | 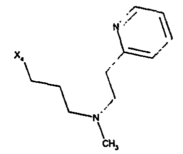 | 648,3 | 7,3 |
| 364 | H | 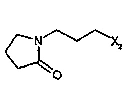 | 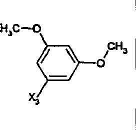 | 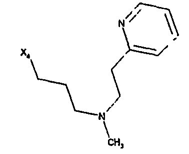 | 614,3 | 7,3 |
| 365 | H | 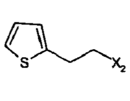 | 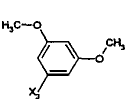 | 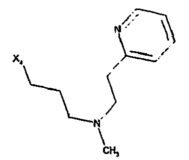 | 599,2 | 7,8 |
| 366 | H | 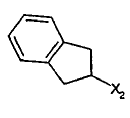 | 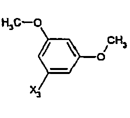 | 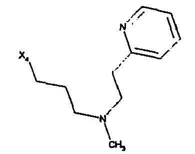 | 605,3 | 8,0 |
| 367 | 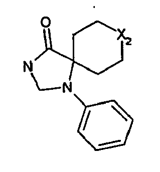 | | 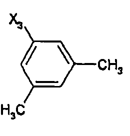 | 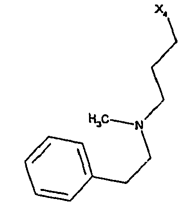 | 670,4 | 8,1 |
| 368 | 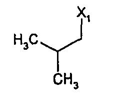 | 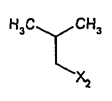 | 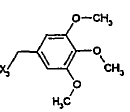 | 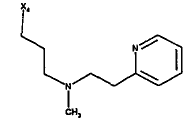 | 645,5 | 8,0 |
| 369 | 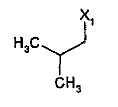 | 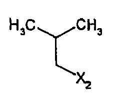 | 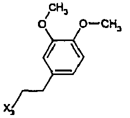 | 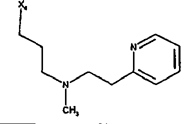 | 629,5 | 8,0 |
| 370 | 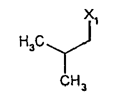 | 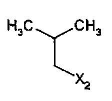 | 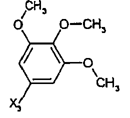 | 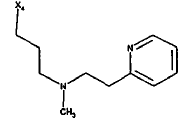 | 631,3 | 7,9 |
| 371 | 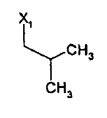 | 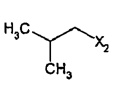 | 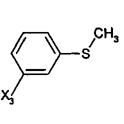 | 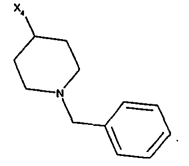 | 584,5 | 8,5 |
| 372 | 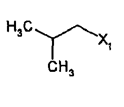 | 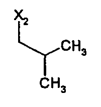 | 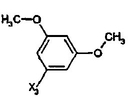 | 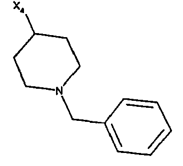 | 598,5 | 8,4 |
| 373 | 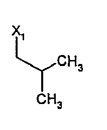 | 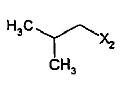 | 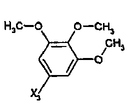 | 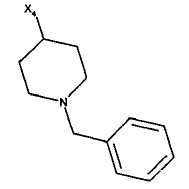 | 628,5 | 8,2 |
| 374 | 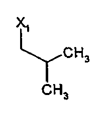 | 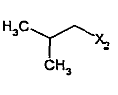 | 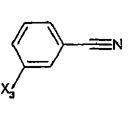 | 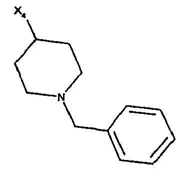 | 563,5 | 8,7 |
| 375 | 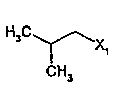 | 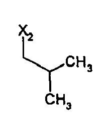 | 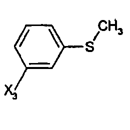 | 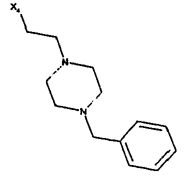 | 613,5 | 8,3 |
| 376 | 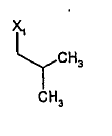 | 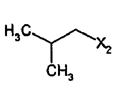 | 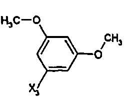 | 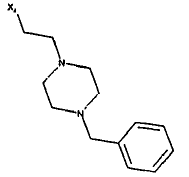 | 627,6 | 8,3 |
| 377 | 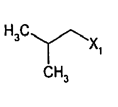 | 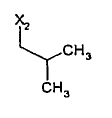 | 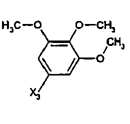 | 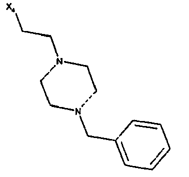 | 657,6 | 8,1 |
| 378 | 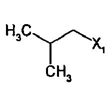 | 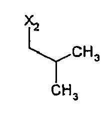 | 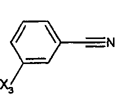 | 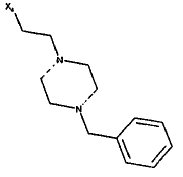 | 592,5 | 8,4 |
| 379 | 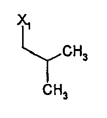 | 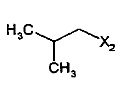 | 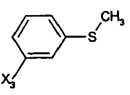 | 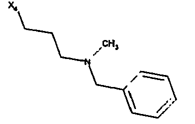 | 572,5 | 8,4 |
| 380 | 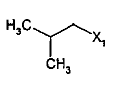 | 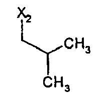 | 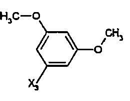 | 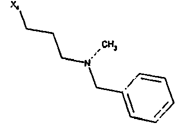 | 586,5 | 8,3 |
| 381 | 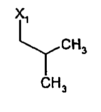 | 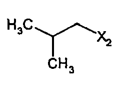 | 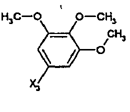 | 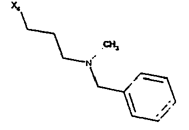 | 616 | 8,1 |
| 382 | 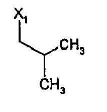 | 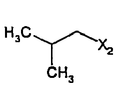 | 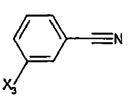 | 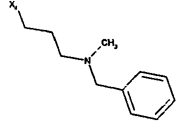 | 551,5 | 8,6 |
| 383 | 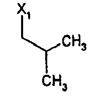 | 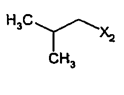 | 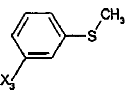 | 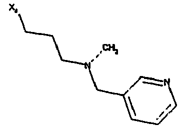 | 573,4 | 8,0 |
| 384 | 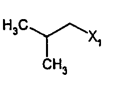 | 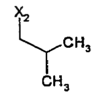 | 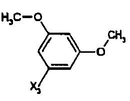 | 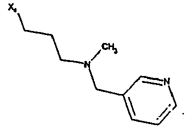 | 587,5 | 8,0 |
| 385 | 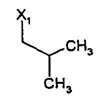 | 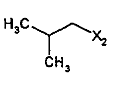 | 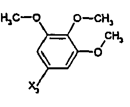 | 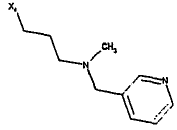 | 617,5 | 7,8 |
| 386 | 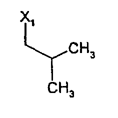 | 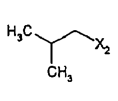 | 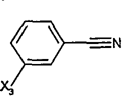 | 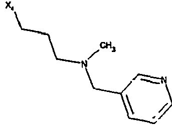 | 552,5 | 8,1 |
| 387 | 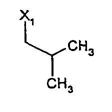 | 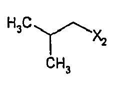 | 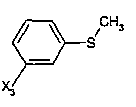 | 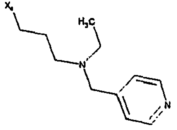 | 587,5 | 8,1 |
| 388 | 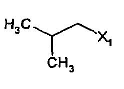 | 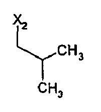 | 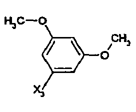 | 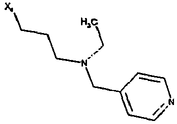 | 601,5 | 8,0 |
| 389 | 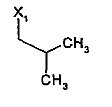 | 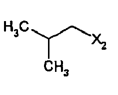 | 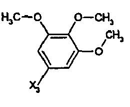 | 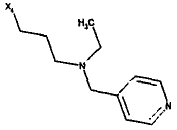 | 631,5 | 7,9 |
| 390 | 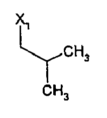 | 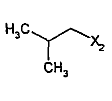 | 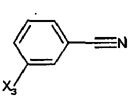 | 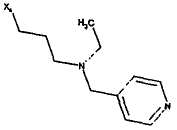 | 566,5 | 8,2 |
| 391 | 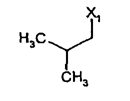 | 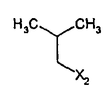 | 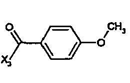 | 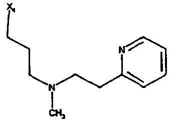 | 599,4 | 8,9 |
| 392 | 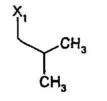 | 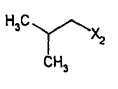 | 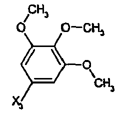 | 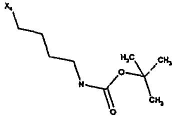 | 626,4 | 9,3 |
| 393 | 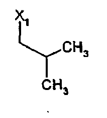 | 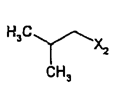 | 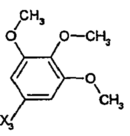 | 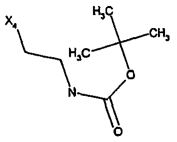 | 598,3 | 9,0 |
| 394 | 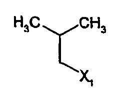 | 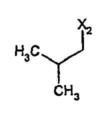 | 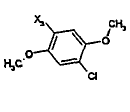 | 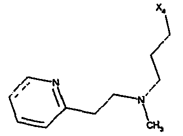 | 635,5 | 8,1 |
| 395 | 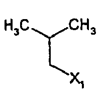 | 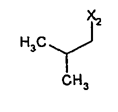 | 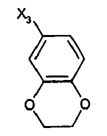 | 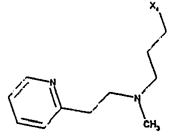 | 599,4 | 7,9 |
| 396 | 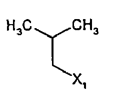 | 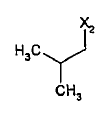 | 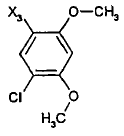 | 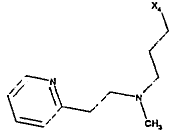 | 635,4 | 8,0 |
| 397 | 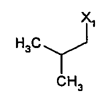 | 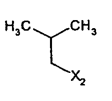 | 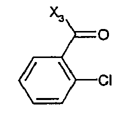 | 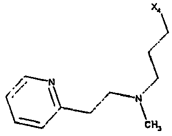 | 603,4 | 8,9 |
| 398 | 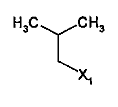 | 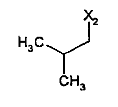 | 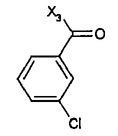 | 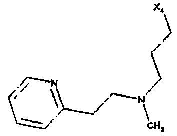 | 603,4 | 9,3 |
| 399 | 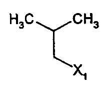 | 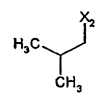 | 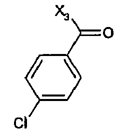 | 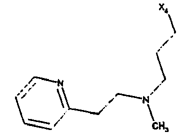 | 603,4 | 9,3 |
| 400 | 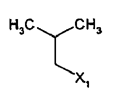 | 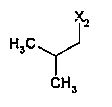 | 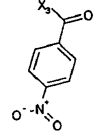 | 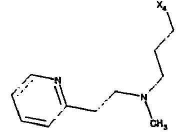 | 614,4 | 9,1 |
| 401 | 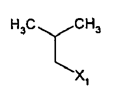 | 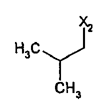 | 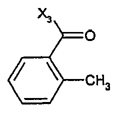 | 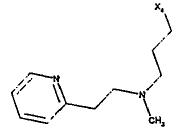 | 583,5 | 8,9 |
| 402 | 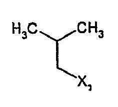 | 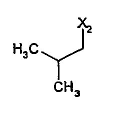 | 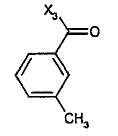 | 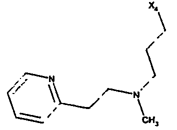 | 583,5 | 9,1 |
| 403 | 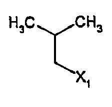 | 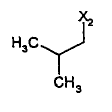 | 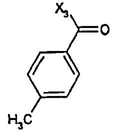 | 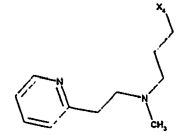 | 583,5 | 9,1 |
| 404 | 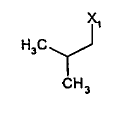 | 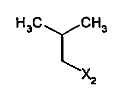 | 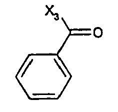 | 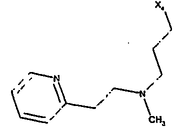 | 569,4 | 8,9 |
| 405 | 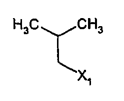 | 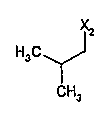 | 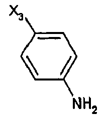 | 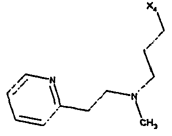 | 556,4 | 7,6 |
| 406 | 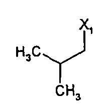 | 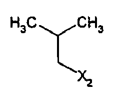 | 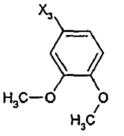 | 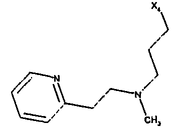 | 601,4 | 7,8 |
| 407 | 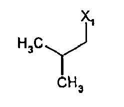 | 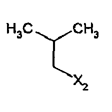 | 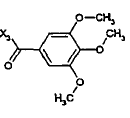 | 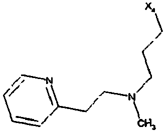 | 659,5 | 8,8 |
| 408 | 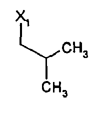 | 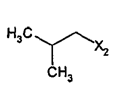 | 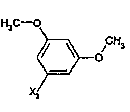 | 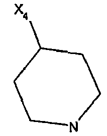 | 508,4 | 7,9 |
| 409 | 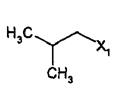 | 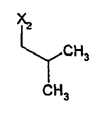 | 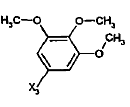 | 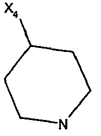 | 538,4 | 7,8 |
| 410 | 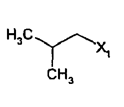 | 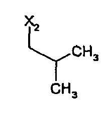 | 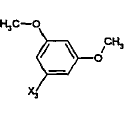 | 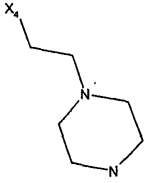 | 537,4 | 7,9 |
| 411 | 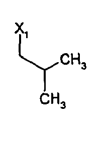 | 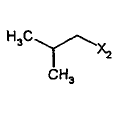 | 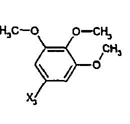 | 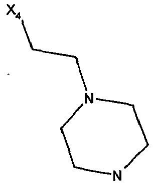 | 567,4 | 7,8 |
| 412 | 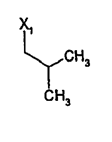 | 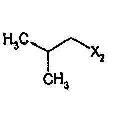 | 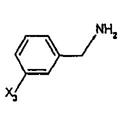 | 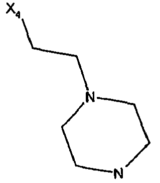 | 506,4 | 7,3 |
| 413 | 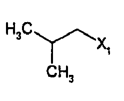 | 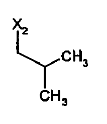 | 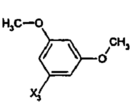 | 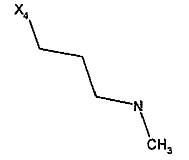 | 496,4 | 7,9 |
| 414 | 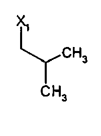 | 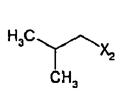 | 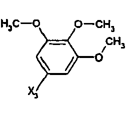 | 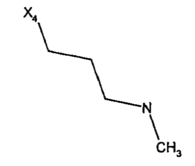 | 526,4 | 7,8 |
| 415 | 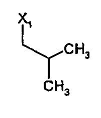 | 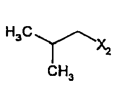 | 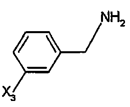 | 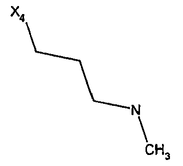 | 465,4 | 7,3 |
| 416 | 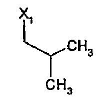 | 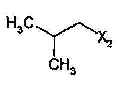 | 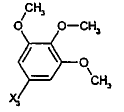 | 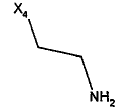 | 498,3 | 7,7 |
| 417 | 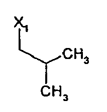 | 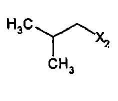 | 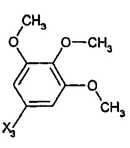 | 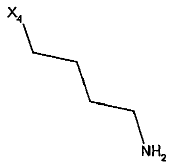 | 526,3 | 7,8 |
| 418 | 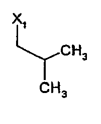 | 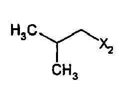 | 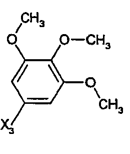 | 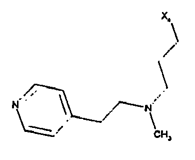 | 631,4 | 7,5 |
| 419 | 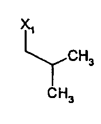 | 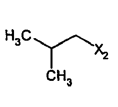 | 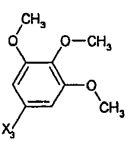 | 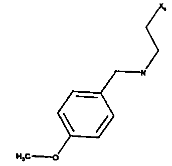 | 618,5 | 8,2 |
| 420 | 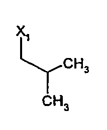 | 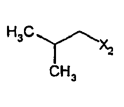 | 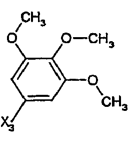 | 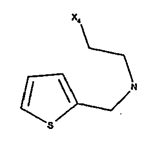 | 594,4 | 8,1 |
| 421 | 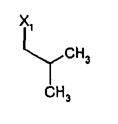 | 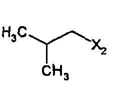 | 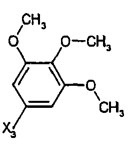 | 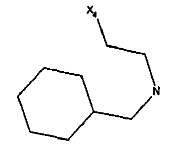 | 594,5 | 8,3 |
| 422 | 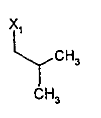 | 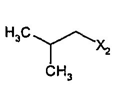 | 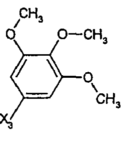 | 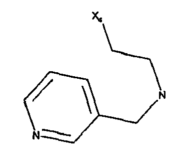 | 589,4 | 7,8 |
| 423 | 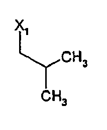 | 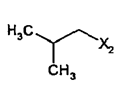 | 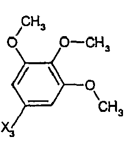 | 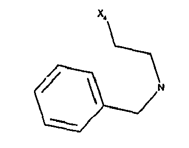 | 588,4 | 8,2 |
| 424 | 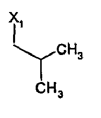 | 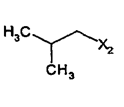 | 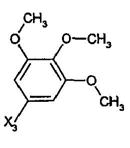 | 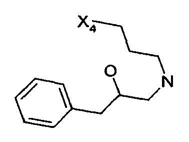 | 632,4 | 8,2 |
| 425 | 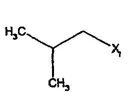 | 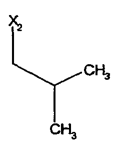 | 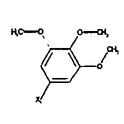 | 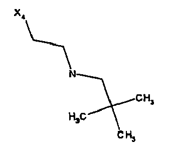 | 568,4 | 8,1 |
| 426 | 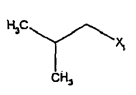 | 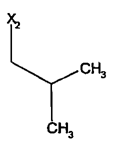 | 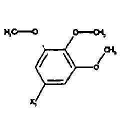 | 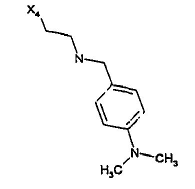 | 631,5 | 8,2 |
| 427 | 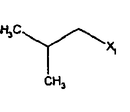 | 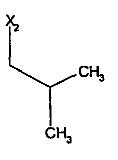 | 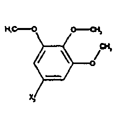 | 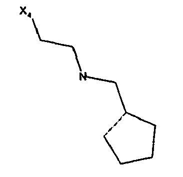 | 580,5 | 8,2 |
| 428 | 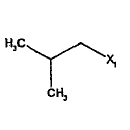 | 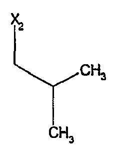 | 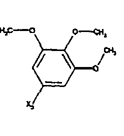 | 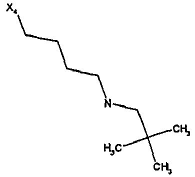 | 596,5 | 8,0 |
| 429 | 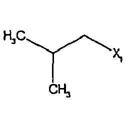 | 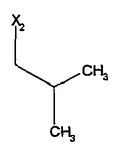 | 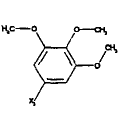 | 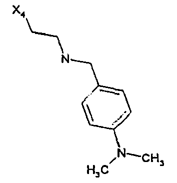 | 659,6 | 8,1 |
| 430 | 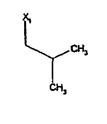 | 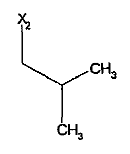 | 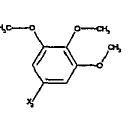 | 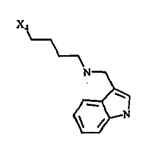 | 655,5 | 8,1 |
| 431 | 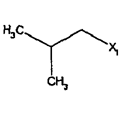 | 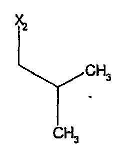 | 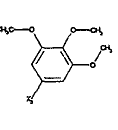 | 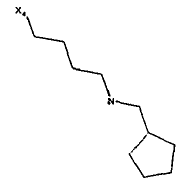 | 608,6 | 8,1 |
| 432 | 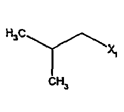 | 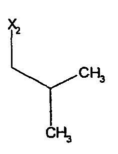 | 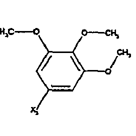 | 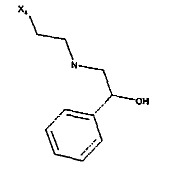 | 618,5 | 8,1 |
| 433 | 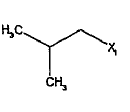 | 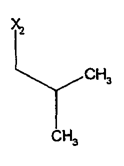 | 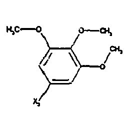 | 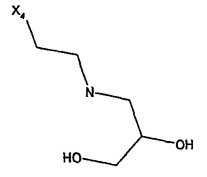 | 572,4 | 7,7 |
| 434 | 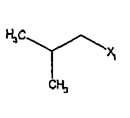 | 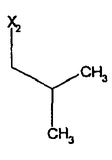 | 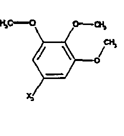 | 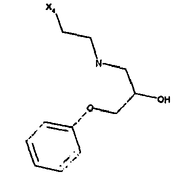 | 648,5 | 8,3 |
| 435 | 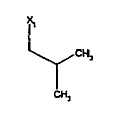 | 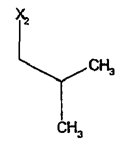 | 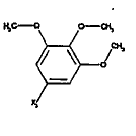 | 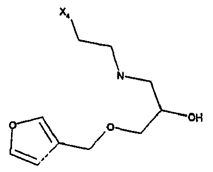 | 652,5 | 8,1 |
| 436 | 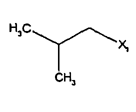 | 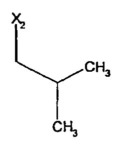 | 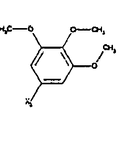 | 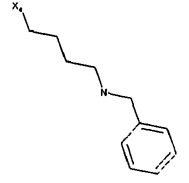 | 616,4 | 8,0 |
| 437 | 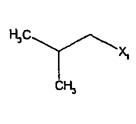 | 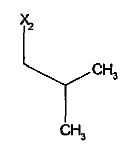 | 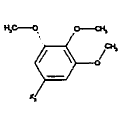 | 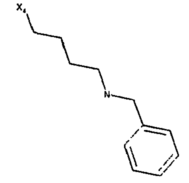 | 644,4 | 8,2 |
| 438 | 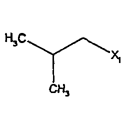 | 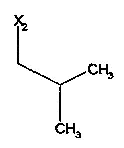 | 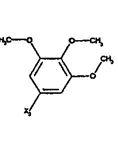 | 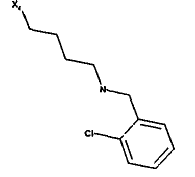 | 650,4 | 8,1 |
| 439 | 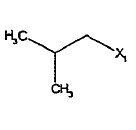 | 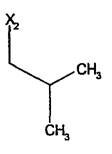 | 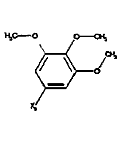 | 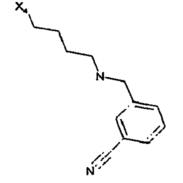 | 641,4 | 8,0 |
| 440 | 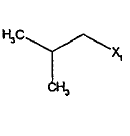 | 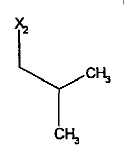 | 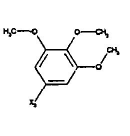 | 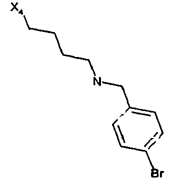 | 694,3 | 8,2 |
| 441 | 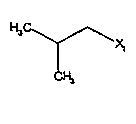 | 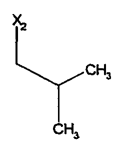 | 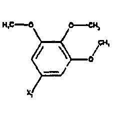 | 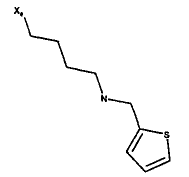 | 622,3 | 8,0 |
| 442 | 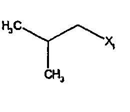 | 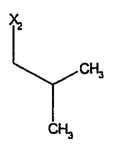 | 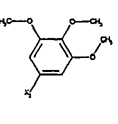 | 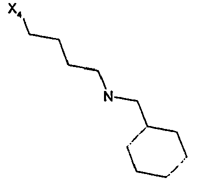 | 622,5 | 8,2 |
| 443 | 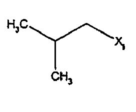 | 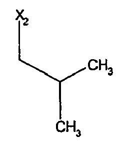 | 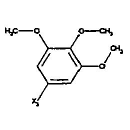 | 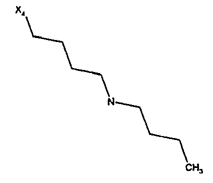 | 582,4 | 8,0 |
| 444 | 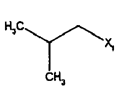 | 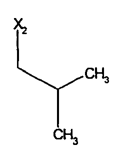 | 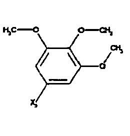 | 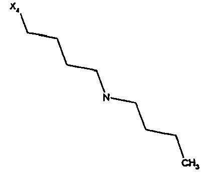 | 624,4 | 8,3 |
| 445 | 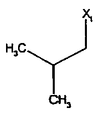 | 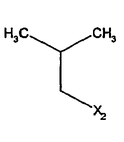 | 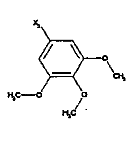 | 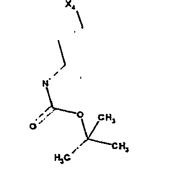 | 612,4 | 9,2 |
| 446 | 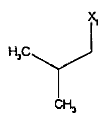 | 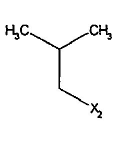 | 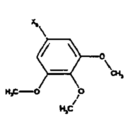 | 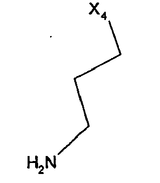 | 512,2 | 7,7 |
| 447 | 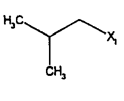 | 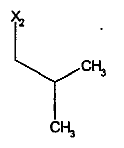 | 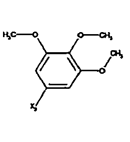 | 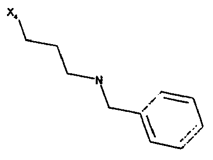 | 602,4 | 8,0 |
| 448 | 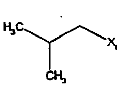 | 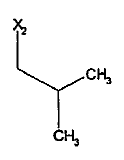 | 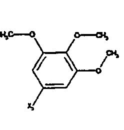 | 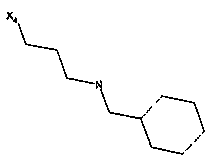 | 608,4 | 8,1 |
| 449 | 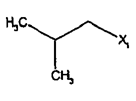 | 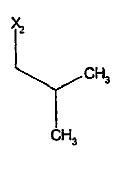 | 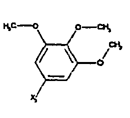 | 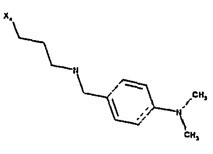 | 645,4 | 8,0 |
| 450 | 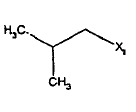 | 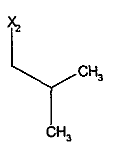 | 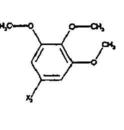 | 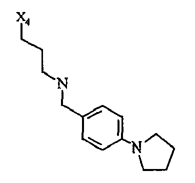 | 671,5 | 8,3 |
| 451 | 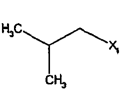 | 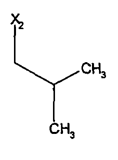 | 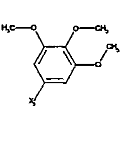 | 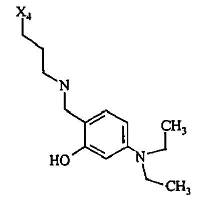 | 687,5 | 8,3 |
| 452 | 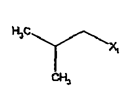 | 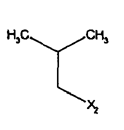 | 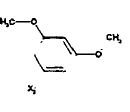 | 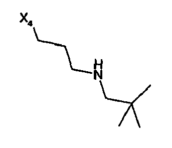 | 552,3 | 8,3 |
| 453 | 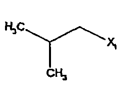 | 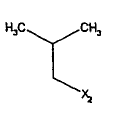 | 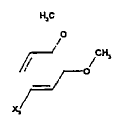 | 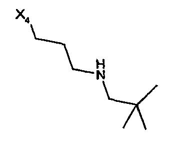 | 552,3 | 8,0 |
| 454 | 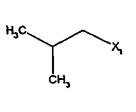 | 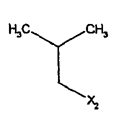 | 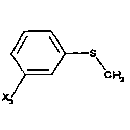 | 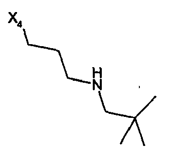 | 538,3 | 8,4 |
| 455 | 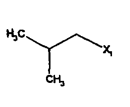 | 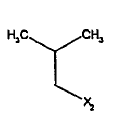 | 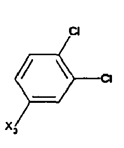 | 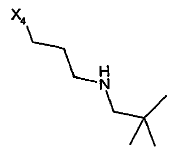 | 560,2 | 9,1 |
| 456 | 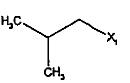 | 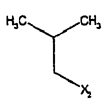 | 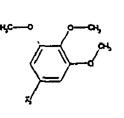 | 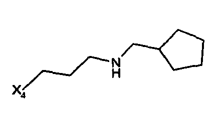 | 594,4 | 8,1 |
| 457 | 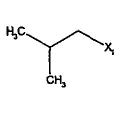 | 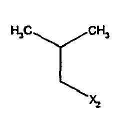 | 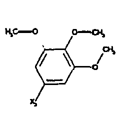 | 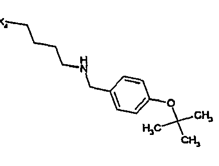 | 674,5 | 8,4 |
| 458 | 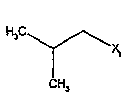 | 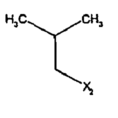 | 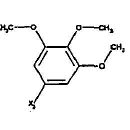 | 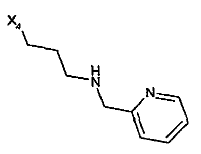 | 603,4 | 7,9 |
| 459 | 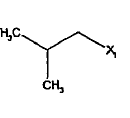 | 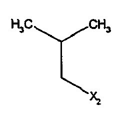 | 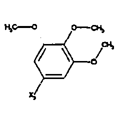 | 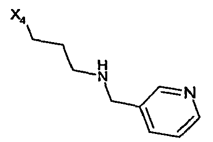 | 603,4 | 7,8 |
| 460 | 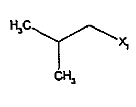 | 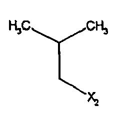 | 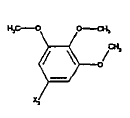 | 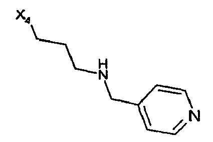 | 603,4 | 7,7 |
| 461 | 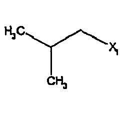 | 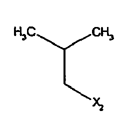 | 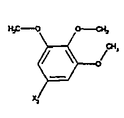 | 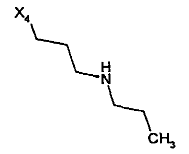 | 554,4 | 7,9 |
| 462 | 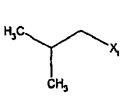 | 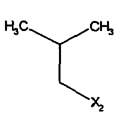 | 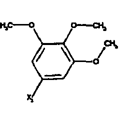 | 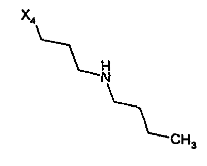 | 568,4 | 8,0 |
| 463 | 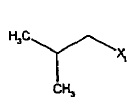 | 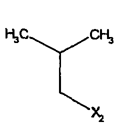 | 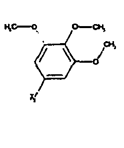 | 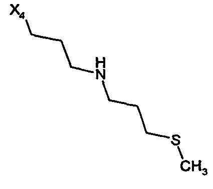 | 600,3 | 8,0 |
| 464 | 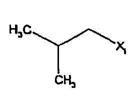 | 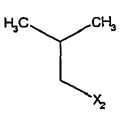 | 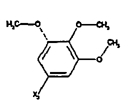 | 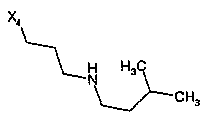 | 582,4 | 8,1 |
| 465 | 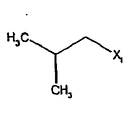 | 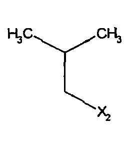 | 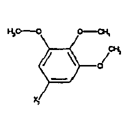 | 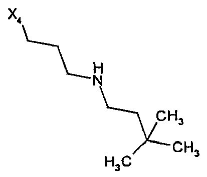 | 596,4 | 8,2 |
| 466 | 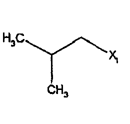 | 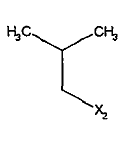 | 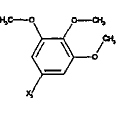 | 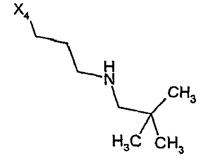 | 582,4 | 8,1 |
| 467 | 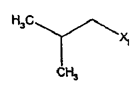 | 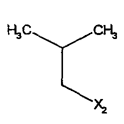 | 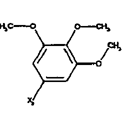 | 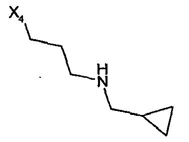 | 566,3 | 7,9 |
| 468 | 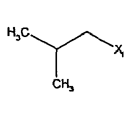 | 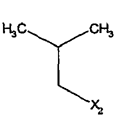 | 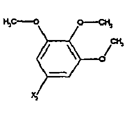 | 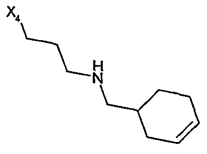 | 606,4 | 8,2 |
| 469 | 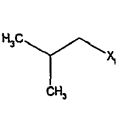 | 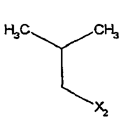 | 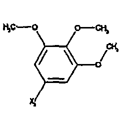 | 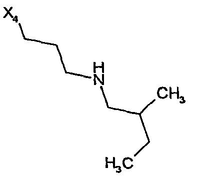 | 582,4 | 8,1 |
| 470 | 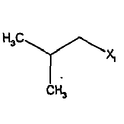 | 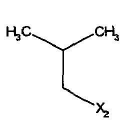 | 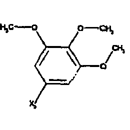 | 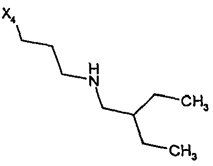 | 596,4 | 8,2 |
| 471 | 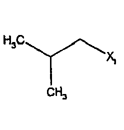 | 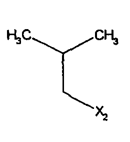 | 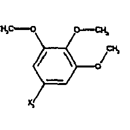 | 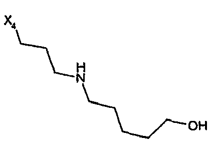 | 0,0 | 0,0 |
| 472 | 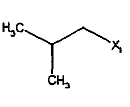 | 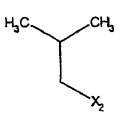 | 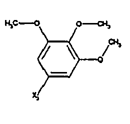 | 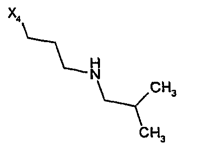 | 568,4 | 8,0 |
| 473 | 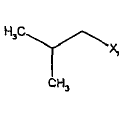 | 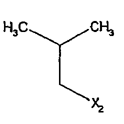 | 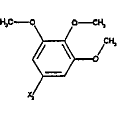 | 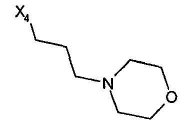 | 582,3 | 7,8 |
| 474 | 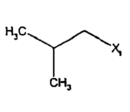 | 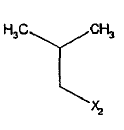 | 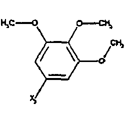 | 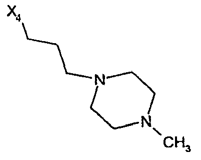 | 595,4 | 7,8 |
| 475 | 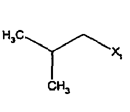 | 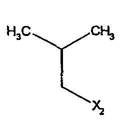 | 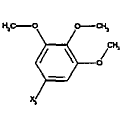 | 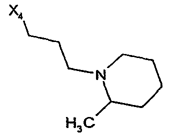 | 594,4 | 7,9 |
| 476 | 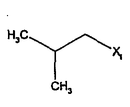 | 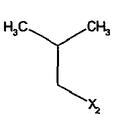 | 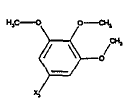 | 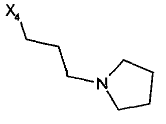 | 566,3 | 7,8 |
| 477 | 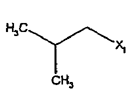 | 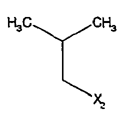 | 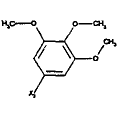 | 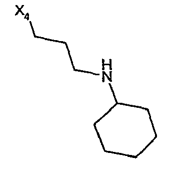 | 580,3 | 7,9 |
| 478 | 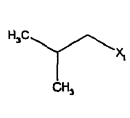 | 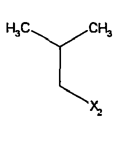 | 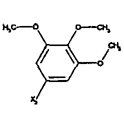 | 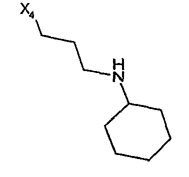 | 594,4 | 8,1 |
| 479 | 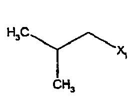 | 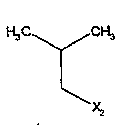 | 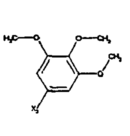 | 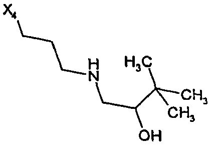 | 612,4 | 8,0 |
| 480 | 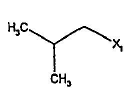 | 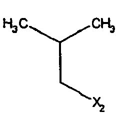 | 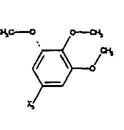 | 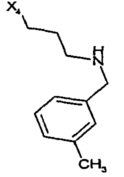 | 616,4 | 8,2 |
| 481 | 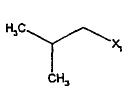 | 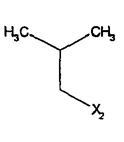 | 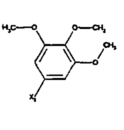 | 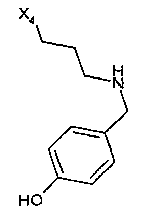 | 618,4 | 7,9 |
| 482 | 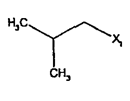 | 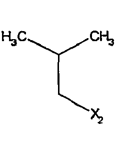 | 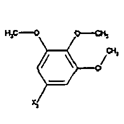 | 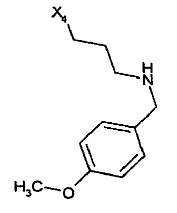 | 632,4 | 8,1 |
| 483 | 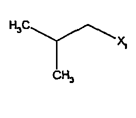 | 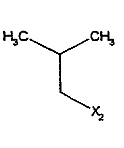 | 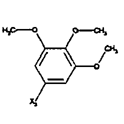 | 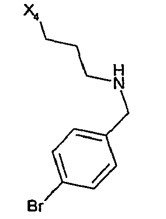 | 680,3 | 8,3 |
| 484 | 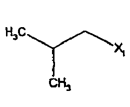 | 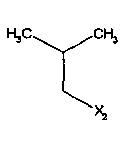 | 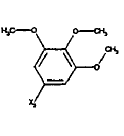 | 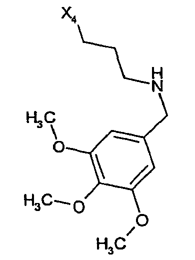 | 692,4 | 8,1 |
| 485 | 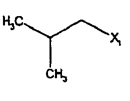 | 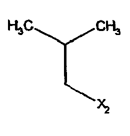 | 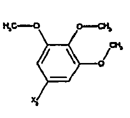 | 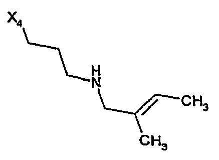 | 580,3 | 8,1 |
| 486 | 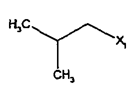 | 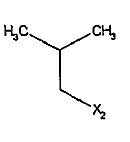 | 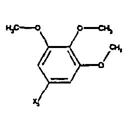 | 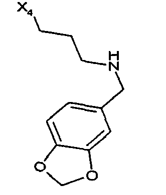 | 646,4 | 8,1 |
| 487 | 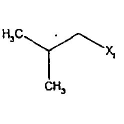 | 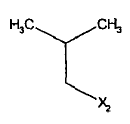 | 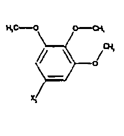 | 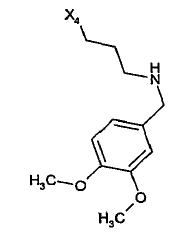 | 662,4 | 8,0 |
| 488 | 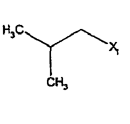 | 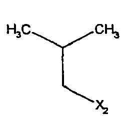 | 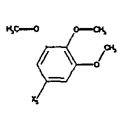 | 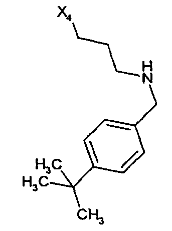 | 658,5 | 8,5 |
| 489 | 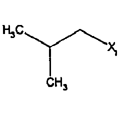 | 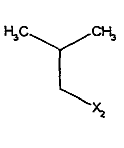 | 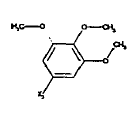 | 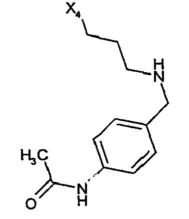 | 659,4 | 7,9 |
| 490 | 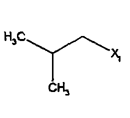 | 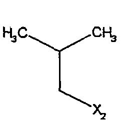 | 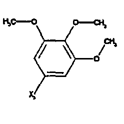 | 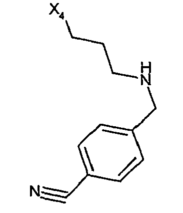 | 627,4 | 8,1 |
| 491 | 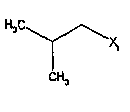 | 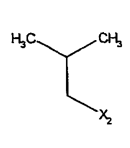 | 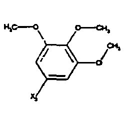 | 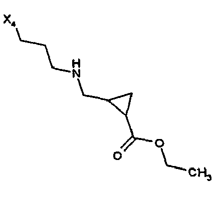 | 638,4 | 8,0 |
| 492 | 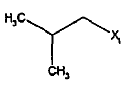 | 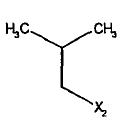 | 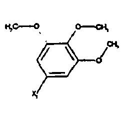 | 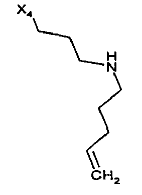 | 580,4 | 8,0 |
| 493 | 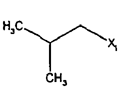 | 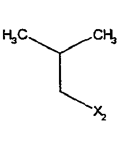 | 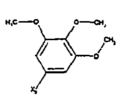 | 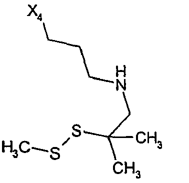 | 646,4 | 8,2 |
| 494 | 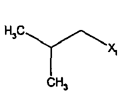 | 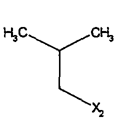 | 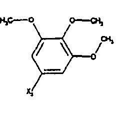 | 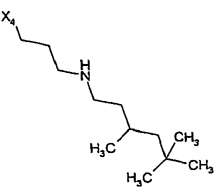 | 638,5 | 8,7 |
| 495 | 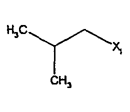 | 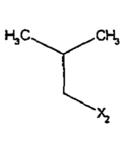 | 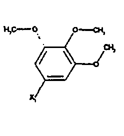 | 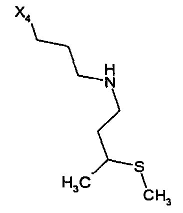 | 614,4 | 8,1 |
| 496 | 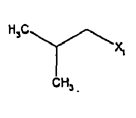 | 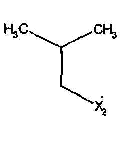 | 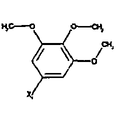 | 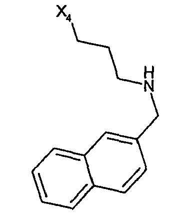 | 652,4 | 8,3 |
| 497 | 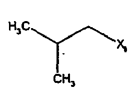 | 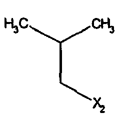 | 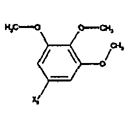 | 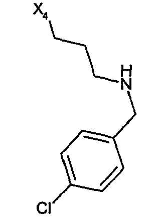 | 636,4 | 8,2 |
| 498 | 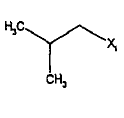 | 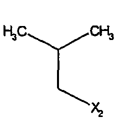 | 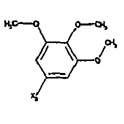 | 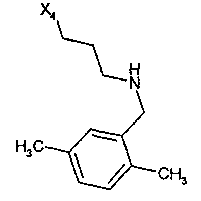 | 630,4 | 8,3 |
| 499 | 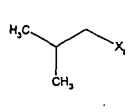 | 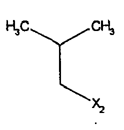 | 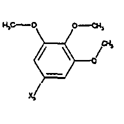 | 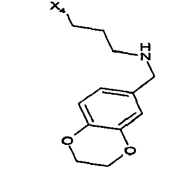 | 660,4 | 8,1 |
| 500 | 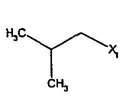 | 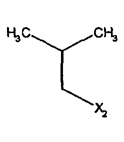 | 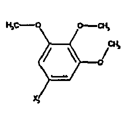 | 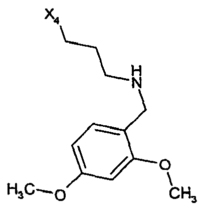 | 662,4 | 8,2 |
| 501 | 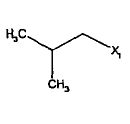 | 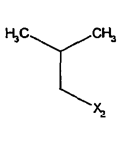 | 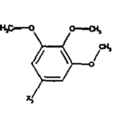 | 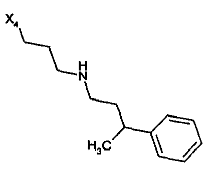 | 644,4 | 8,4 |
| 502 | 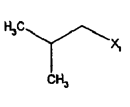 | 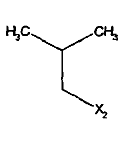 | 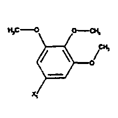 | 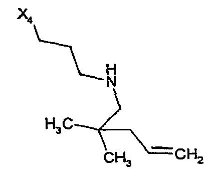 | 608,4 | 8,2 |
| 503 | 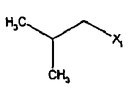 | 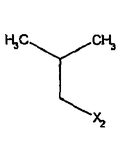 | 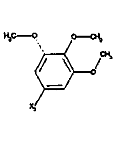 | 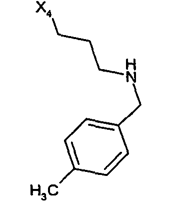 | 616,4 | 8,2 |
| 504 | 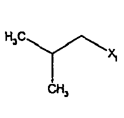 | 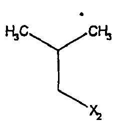 | 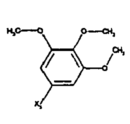 | 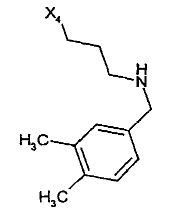 | 630,4 | 8,3 |
| 505 | 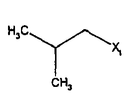 | 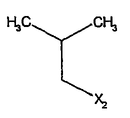 | 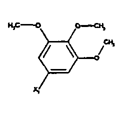 | 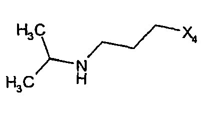 | 554,4 | 7,8 |
| 506 | 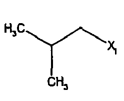 | 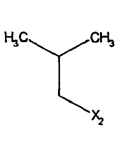 | 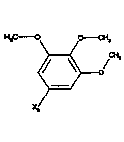 | 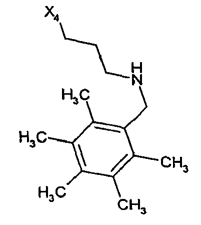 | 672,4 | 8,6 |
| 507 | 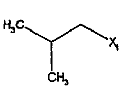 | 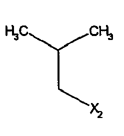 | 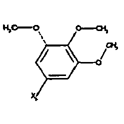 | 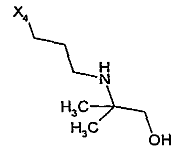 | 584,4 | 7,9 |
| 508 | 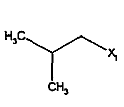 | 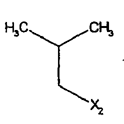 | 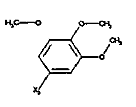 | 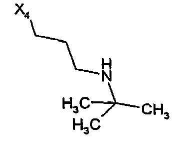 | 568,3 | 7,9 |
| 509 | 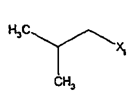 | 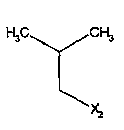 | 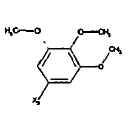 | 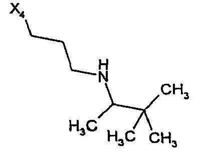 | 596,4 | 8,1 |
| 510 | 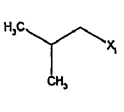 | 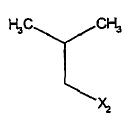 | 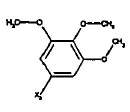 | 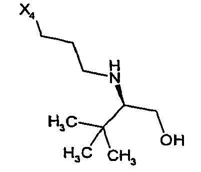 | 612,4 | 8,0 |
| 511 | 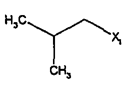 | 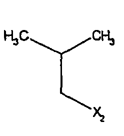 | 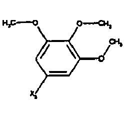 | 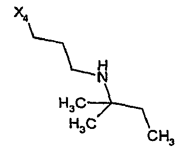 | 582,4 | 8,1 |
| 512 | 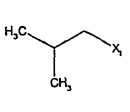 | 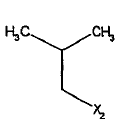 | 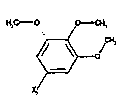 | 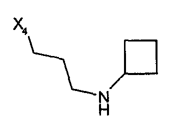 | 556,3 | 7,9 |
| 513 | 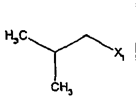 | 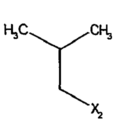 | 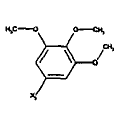 | 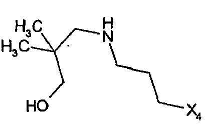 | 598,3 | 7,9 |
| 514 | 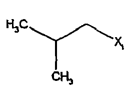 | 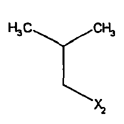 | 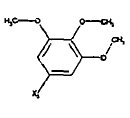 | 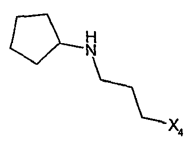 | 580,4 | 8,0 |
| 515 | 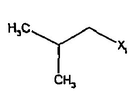 | 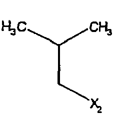 | 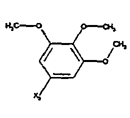 | 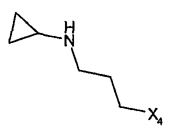 | 552,3 | 7,9 |
| 516 | 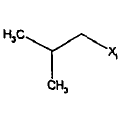 | 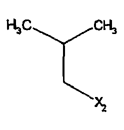 | 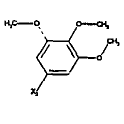 | 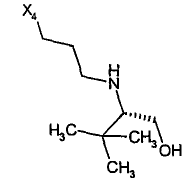 | 612,4 | 8,0 |
| 517 | 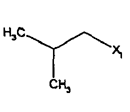 | 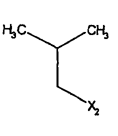 | 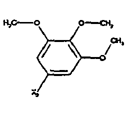 | 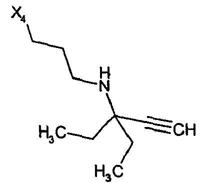 | 606,4 | 8,2 |
| 518 | 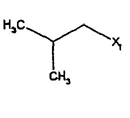 | 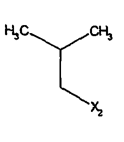 | 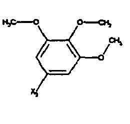 | 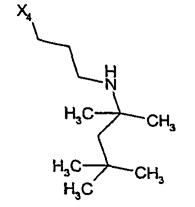 | 624,4 | 8,4 |
| 519 | 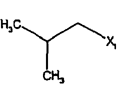 | 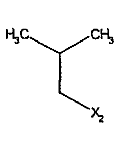 | 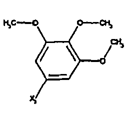 | 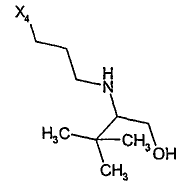 | 612,4 | 8,0 |
| 520 | 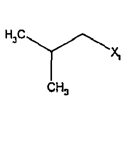 | 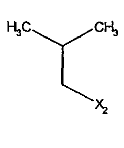 | 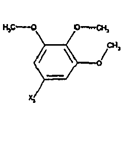 | 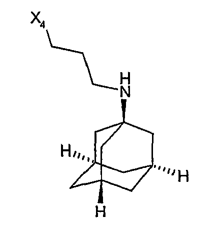 | 646,4 | 8,3 |
| 521 | 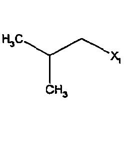 | 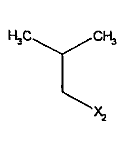 | 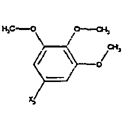 | 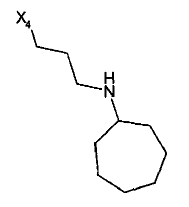 | 608,4 | 8,2 |
| 522 | 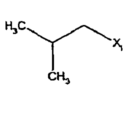 | 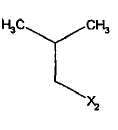 | 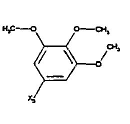 | 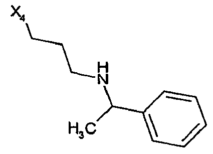 | 616,3 | 8,2 |
| 523 | 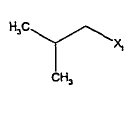 | 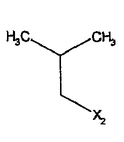 | 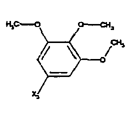 | 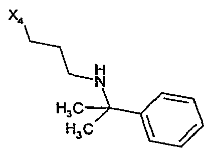 | 630,3 | 8,2 |
| 524 | 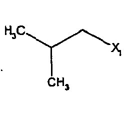 | 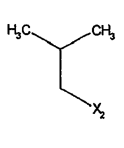 | 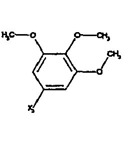 | 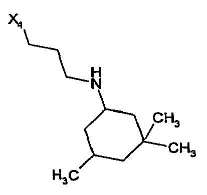 | 636,4 | 8,5 |
| 525 | 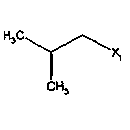 | 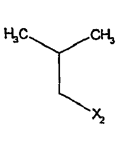 | 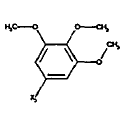 | 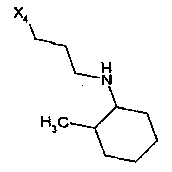 | 608,3 | 8,2 |
| 526 | 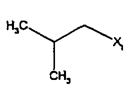 | 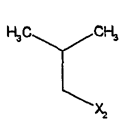 | 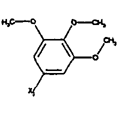 | 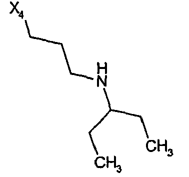 | 582,3 | 8,1 |
| 527 | 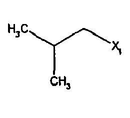 | 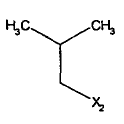 | 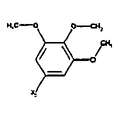 | 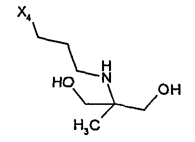 | 600,3 | 7,8 |
| 528 | 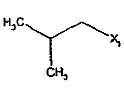 | 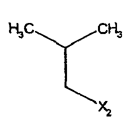 | 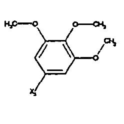 | 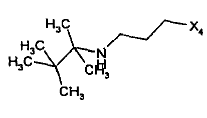 | 610,3 | 8,2 |
| 529 | 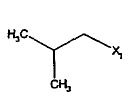 | 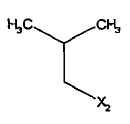 | 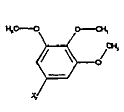 | 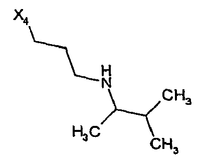 | 582,3 | 8,1 |
| 530 | 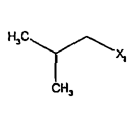 | 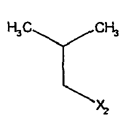 | 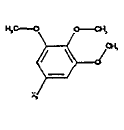 | 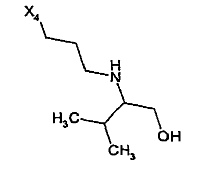 | 598,3 | 7,9 |
| 531 | 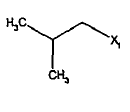 | 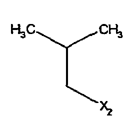 | 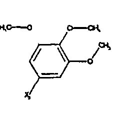 | 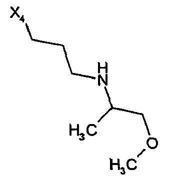 | 584,3 | 7,9 |
| 532 | 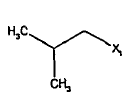 | 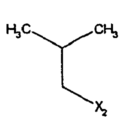 | 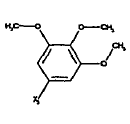 | 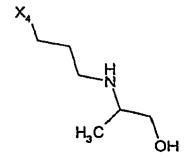 | 570,2 | 7,8 |
| 533 | 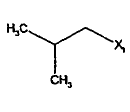 | 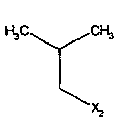 | 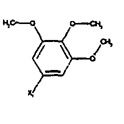 | 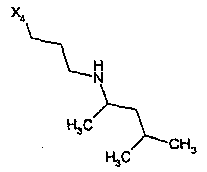 | 596,4 | 8,2 |
| 534 | 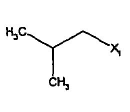 | 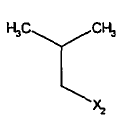 | 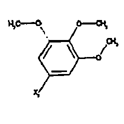 | 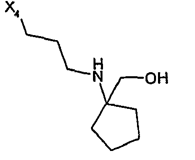 | 610,3 | 7,9 |
| 535 | 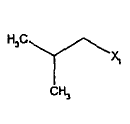 | 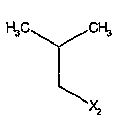 | 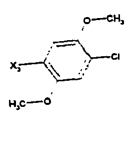 | 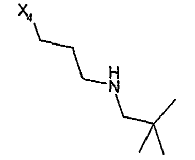 | 586,3 | 8,4 |
| 536 | 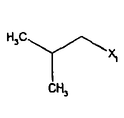 | 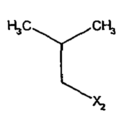 | 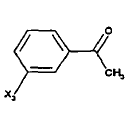 | 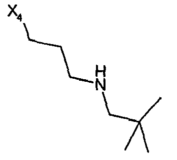 | 534,3 | 7,6 |
| 537 | 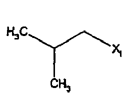 | 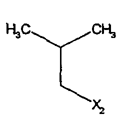 | 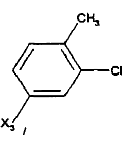 | 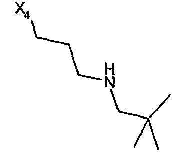 | 540,3 | 7,9 |
| 538 | 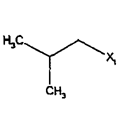 | 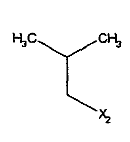 | 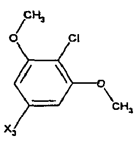 | 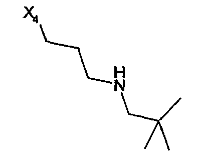 | 586,3 | 8,4 |

### Etude pharmacologique

L'activité antagoniste de la GnRH des composés selon l'invention est mesurée selon les protocoles suivants :

### Etablissement d'une lignée stable transfectée par le récepteur LHRH humain :

L'ADNc du récepteur LHRH humain est cloné dans le site EcoRI dans un vecteur d'expression de mammifère pCDNA3.1 (In Vitrogen Inc.). Cette construction plasmidique est transfectée grâce à l'Effectene selon les recommandations du fabricant (Qiagen) dans une lignée cellulaire dérivée de rein d'embryon humain, HEK-293 (ATCC) et la sélection est réalisée dans un milieu DMEM contenant 0,5 mg/ml de généticine. Les cellules contenant le vecteur d'expression pour le récepteur LHRH sont ensuite clonées par dilution limite puis multipliées en culture. Ces clones cellulaires sont ensuite testés pour l'expression du récepteur LHRH humain par des tests d'inhibition compétitive de liaison et de mesure d'inositol phosphates.

### Culture cellulaire et préparation membranaire :

Les cellules HEK-293 exprimant de manière stable le récepteur LHRH humain comme décrit ci-dessus sont cultivées dans un milieu DMEM en présence de 10 % de sérum de veau foetal et supplémenté par 0,4 mg/ml de généticine (G418, Sigma Chemical Company). Les cellules sont détachées du support de culture avec de l'EDTA 0,5 mM et centrifugées à 500g pendant 10 minutes à 4° C. Le culot cellulaire est lavé dans du Tris 50 mM, pH 7.4 et centrifugé deux fois à 500 g pendant 10 minutes. Les cellules sont enfin lysées par sonication puis centrifugées à 39000 g pendant 10 minutes à 4° C. Le culot est re-suspendu dans du Tris 50 mM, pH 7.4 et centrifugé à 50000 g pendant 10 minutes à 4° C afin d'obtenir un culot membranaire réparti en plusieurs aliquotes stockés à -80°C avant utilisation.

### Etude de l'affinité pour le récepteur LHRH humain :

L'affinité d'un composé de l'invention pour le récepteur LHRH humain est déterminée par la mesure de l'inhibition de la liaison de [¹²⁵I-Tyr5]-DTrp⁶-LHRH sur des cellules humaines transfectées par l'ADNc du récepteur LHRH humain.

Les tests d'inhibition compétitive de liaison de [¹²⁵I-Tyr5]-DTrp⁶-LHRH sont réalisés en duplicats dans des plaques 96 puits en polypropylène. Les membranes des cellules HEK-293 exprimant de manière stable le récepteur LHRH humain (20 µg protéines/puit) sont incubées en présence de [¹²⁵I-Tyr5]-DTrp⁶-LHRH (0,2 nM) pendant 60 minutes à 4° C dans un milieu contenant du Tris/HCl 50 mM pH 7,4, Bacitracine 0,1 mg/ml, BSA 0.1 % (1 mg/ml).

Le [¹²⁵I-Tyr⁵]-DTrp⁶-LHRH lié est séparé du [¹²⁵I-Tyr5]-DTrp⁶-LHRH libre par filtration au travers de plaques filtrantes constituées de fibre de verre GF/C (Unifilter, Packard) imprégnées de polyéthylènimine 0,1 %, en utilisant un Filtermate 96 (Packard). Les filtres sont ensuite lavés avec du tampon Tris/HCl 50 mM à 4° C pendant 4 secondes et la radioactivité est comptée à l'aide d'un compteur à scintillation (Packard, Topcount).

La liaison spécifique est calculée après soustraction de la liaison non spécifique (déterminée en présence de DTrp⁶-LHRH 0.1 µM) de la liaison totale. Les données relatives à la liaison proviennent d'une analyse par régression non-linéaire et les valeurs de constantes d'inhibition (Ki) sont déterminées.

La détermination du profil agoniste ou antagoniste d'un composé de la présente invention est effectuée par la méthode décrite ci-dessous.

### Test fonctionnel : Inhibition de la production d'Inositol Phosphates intracellulaire

Des cellules HEK-293 exprimant de manière stable le récepteur GnRH humain sont cultivées à raison de 200.000 cellules par puit en plaque 24 puits recouverts de poly-D-lysine (Falcon Biocoat) dans un milieu DMEM en présence de 10 % de sérum de veau foetal et 0,4 mg/ml de généticine pendant 24 heures.

Le milieu est ensuite remplacé par du DMEM ne contenant pas d'Inositol en présence de 10 % de sérum de veau foetal et de 1µCi/ml de [3H]myo-inositol (Amersham) pendant 16-18 heures à 37° C.

Les cellules sont lavées avec du DMEM ne contenant pas d'inositol en présence de 10mM de Chlorure de Lithium et incubées 30 minutes à 37° C.

La production d'Inositol Phosphates est stimulée par l'addition de DTrp⁶-LHRH 0.5 nM pendant 45 minutes à 37° C.

L'effet antagoniste d'un composé est mesuré par l'addition simultanée de DTrp⁶-LHRH 0,5 nM et du composé à tester à différentes concentrations croissantes (exemple : 10⁻¹⁰ M à 10⁻⁵M).

Le milieu réactionnel est éliminé et 1 ml de d'acide formique 0,1 M sont ajoutés et incubés 5 minutes à 4° C.

La plaque est ensuite congelée à -80° C puis décongelée à température ambiante.

Les inositols phosphates sont ensuite séparés de la totalité des inositols intracellulaires sur résine échangeuse d'ion (Biorad) par élution avec du formate d'ammonium 1M et de l'acide formique 0,1 M.

La quantité d'inositols phosphates en sortie de colonne est finalement mesurée en présence de liquide scintillant.

### Résultats :

Les tests effectués selon les protocoles décrits ci-dessus ont permis de montrer que les produits de formule générale (I) définie dans la présente demande ont une bonne affinité sur le récepteur LHRH, la constante d'inhibition Ki sur ce récepteur étant inférieure au micromolaire pour certains des composés exemplifiés.

## Revendications

1. Composé de formule générale 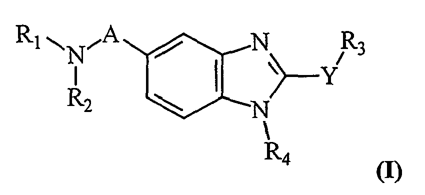 sous forme racémique, d'énantiomère ou toutes combinaisons de ces formes et dans laquelle :
A représente -CH₂- ou -C(O)- ;
Y représente -S- ou -NH- ;
R₁ et R₂ représentent, indépendamment, l'atome d'hydrogène, un radical (C₁-C₈)alkyle, un (C₅-C₉)bicycloalkyle éventuellement substitué par un ou plusieurs radicaux (C₁-C₆)akyle identiques ou différents, ou un radical de formule -(CH₂)ₙ-X dans laquelle
X représente, amino, (C₁-C₆)alkylamino, di((C₁-C₆)alkyl)amino, (C₃-C₇)cycloalkyle, adamantyle, hétérocycloalkyle, aryle, aryl-carbonyle ou hétéroaryle, ou bien un radical de formule 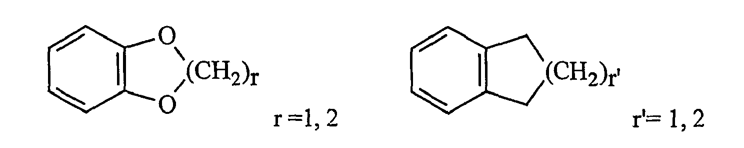 les radicaux (C₃-C₇)cycloalkyle, hétérocycloalkyle, aryle et hétéroaryle étant éventuellement substitués par un ou plusieurs substituants identiques ou différents choisis parmi : -(CH₂)_{n'}-X'-Y', halo, oxo, nitro, cyano, amino, (C₁-C₆)alkylamino et di((C₁-C₈)alkyl)amino, hydroxy, N₃ ;
X' représente -O-, -S-, -C(O)-, -C(O)-O-, -NH-C(O)-, -NH-SO₂- ou une liaison covalente ;
Y' représente un radical (C₁-C₆)alkyle éventuellement substitués par un ou plusieurs radicaux halo identiques ou différents ; hétéroaryle ou aryle ou hétérocycloalkyle éventuellement substitué par un ou plusieurs substituants identiques ou différents choisis parmi : (C₁-C₆)alkyle, (C₁-C₆)alkoxy, halo, nitro, cyano, amino, CF₃, OCF₃, hydroxy, N₃, (C₁-C₆)alkylamino et di((C₁-C₈)alkyl)amino ;
n représente un entier de 0 à 6 et n' un entier de 0 à 2 ;
ou bien R₁ et R₂ forment ensemble, avec l'atome d'azote auquel ils sont rattachés, un hétérocycloalkyle, un hétérobicycloalkyle ou un radical de formule : 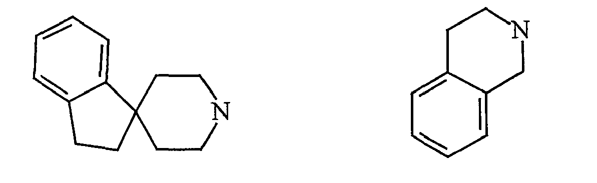 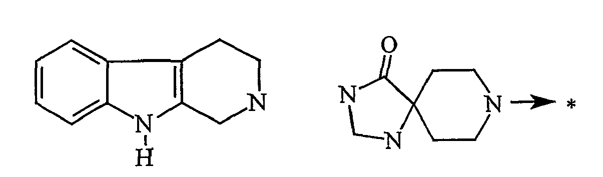 le radical que forment ensemble R₁ et R₂ étant éventuellement substitué par un ou plusieurs substituants identiques ou différents choisis parmi :
-(CH₂)_{n"}-X"-Y", oxo, hydroxy, halo, nitro, cyano;
X" représente -O-, -C(O)-, -C(O)-O- ou une liaison covalente ;
Y" représente un radical (C₁-C₆)alkyle, amino, (C₁-C₆)alkylamino, di((C₁-C₆)alkyl)amino, (C₃-C₇)cycloalkyle, hétérocycloalkyle, arylalkyle, ou aryle ou hétéroaryle éventuellement substitué par un ou plusieurs substituants identiques ou différents choisis parmi : (C₁-C₆)alkyle, (C₁-C₆)alkoxy, (C₁-C₆)alkyl-carbonyle, halo, hydroxy, nitro, cyano, CF₃, OCF₃, amino, (C₁-C₆)alkylamino et di((C₁-C₆)alkyl)amino) ; ou bien un radical de formule
n" représente un entier de 0 à 4 ;
R₃ représente -(CH₂)ₚ-W₃-(CH₂)_{p'}-Z₃
W₃ représente une liaison covalente, -CH(OH)- ou -C(O)- ;
Z₃ représente un radical (C₁-C₆)alkyle, adamantyle, aryle, un hétéroaryle, ou bien un radical de formule 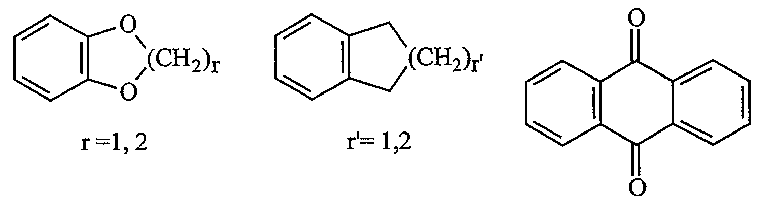 le radical aryle étant éventuellement susbtitué par un ou plusieurs substituants identiques ou différents choisis parmi : -(CH₂)_{p"}-V₃-Y₃, halo, nitro, cyano, N₃, hydroxy ;
V₃ représente -O-, -S-, -C(O)-, -C(O)-O-, -SO₂- ou une liaison covalente ;
Y₃ représente un radical (C₁-C₆)alkyle éventuellement substitué par un ou plusieurs radicaux halo identiques ou différents, amino, (C₁-C₆)alkylamino, di((C₁-C₆)alkyl)amino, phénylcarbonylméthyle, hétérocycloalkyle ou aryle ;
p, p' et p" représentent, indépendamment, un entier de 0 à 4 ;
R₄ représente un radical de formule-(CH₂)ₛ-R"₄
R"₄ représente un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)allcyle ; ou un radical de formule -NW₄W'₄
W₄ représente l'atome d'hydrogène, (C₁-C₈)alkyle ou (C₃-C₇)cycloalkyle ;
W'₄ représente un radical de formule -(CH₂)_{s'}-Q₄-Z₄ ;
Q₄ représente une liaison covalente, -CH₂-CH(OH)-[CH₂]ₜ-[O]_{t'}-[CH₂]_{t"}-ou -C(O)-O- ;
t, t' et t" représentent, indépendamment, 0 ou 1 ;
Z₄ représente l'atome d'hydrogène, (C₁-C₈)alkyle éventuellement substitué par un ou plusieurs substituents identiques ou différents choisis parmi : (C₁-C₆)alkoxy, (C₁-C₆)alkylthio, (C₁-C₆)alkyldithio et hydroxy ; (C₂-C₆)alkenyle ; (C₂-C₆)alkynyle ; (C₃-C₇)cycloalkyle éventuellement substitué par un ou plusieurs substituents identiques ou différents choisis parmi : (C₁-C₆)alkyle, (C₁-C₆)alkoxycarbonyle et (C₁-C₆)hydroxyalkyle ; cyclohexène ; adamantyle ; hétéroaryle ; aryle éventuellement substitué par un ou plusieurs radicaux identiques ou différents choisis de formule -(CH₂)_{q"}-V₄-Y₄, hydroxy, halo, nitro, cyano ;
V₄ représente -O-, -S-, -NH-C(O)- ou une liaison covalente ;
Y₄ représente un radical (C₁-C₆)alkyle éventuellement substitué par di((C₁-C₆)alkyl)amino ou un ou plusieurs radicaux halo identiques ou différents ; amino ; (C₁-C₆)alkylamino ; di((C₁-C₆)alkyl)amino ; aralkyle ; hétérocycloalkyle ;
q" représente un entier de 0 à 4 ;
ou bien Z₄ représente un radical de formule 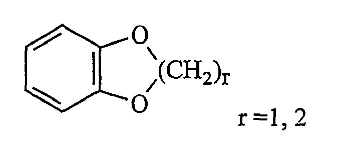 s et s' représentent, indépendamment, un entier de 0 à 6 ; ou un sel pharmaceutiquement acceptable de ce dernier.

2. Composé de formule I selon la revendication 1, **caractérisé en ce que** A représente -C(O)- ; ou un sel pharmaceutiquement acceptable de ce dernier.

3. Composé de formule I selon l'une des revendications 1 à 2, **caractérisé en ce que**
le cycloalkyle que représente X est le cyclohexyle ou le cycloheptyle ;
l'hétérocycloalkyle que représente X est choisi parmi : pipéridine, morpholine, pyrrolidine, thiazolidine et tétrahydrothiophène ;
l'aryle que représente X est le radical phényle, naphtyle ou fluorènyle ;
l'aryle du radical aryl-carbonyle que représente X, est le radical phényle ;
l'hétéroaryle que représente X est choisi parmi : pyridine, imidazole, thiophène, indole, carbazole et isoquinoléine ;
l'hétéroaryle que représente Y' est choisi parmi oxazole et imidazole ;
l'aryle que représente Y' est le radical phényle ;
l'hétérocycloalkyle que représente Y' est la pipérazine ;
l'hétérocycloalkyle que forment ensemble R₁ et R₂ avec l'atome d'azote auquel ils sont rattachés, est choisi parmi : pipéridine, pipérazine, diazépane, thiazolidine et morpholine ;
le cycloalkyle que représente Y" est le cyclopentyle ou le cyclohexyle ;
l'hétérocycloalkyle que représente Y" est choisi parmi : pipéridine, pyrrolidine et morpholine ;
l'arylalkyle et l'aryle que représente Y" sont respectivement le radical benzyle et le radical phényle ;
l'hétéroaryle que représente Y" est choisi parmi : pyridine, pyrazine, furane et thiophène. ; ou un sel pharmaceutiquement acceptable de ce dernier.

4. Composé de formule I selon l'une des revendications 1 à 3, **caractérisé en ce que**
l'aryle que représente Z₃ est le radical phényle ou naphtyle ;
l'hétéroaryle que représente Z₃ est choisi parmi benzo[b]thiophène et benzo[b]furanne ;
l'hétérocycloalkyle et l'aryle que représente Y₃ sont respectivement les radicaux pyrrolidine et phényle ; ou un sel pharmaceutiquement acceptable de ce dernier.

5. Composé de formule 1 selon l'une des revendications 1 à 4, **caractérisé en ce que**
l'hétérocycloalkyle que représente R"₄ est choisis parmi : pipérazine, pipéridine, morpholine et pyrrolidine ;
l'aralkyle qui substitue éventuellement l'hétérocycloalkyle que représente R"₄ est le radical benzyle ;
l'hétéroaryle que représente R"₄ est l'imidazole ;
le (C₃-C₇)cycloalkyle que représente Z₄ est le cyclopropyle, cyclobutyle, cyclopentyle, cyclohexyle ou cycloheptyle;
l'hétéroaryle que représente Z₄ est choisi parmi : pyridine, thiophène, indole et furane ;
l'aryle que représente Z₄ et est le phényle ou naphtyle ;
l'aralkyle que représente Y₄ est benzyle ;
l'hétérocycloalkyle que représente Y₄ est la pyrrolidine ;
l'aralkyle qui est substitué sur l'hétérocycloalkyle que forment ensemble W₄ et W'₄ est le radical benzyle ; ou un sel pharmaceutiquement acceptable de ce dernier.

6. Composé de formule I selon l'une des revendications 1 à 5, **caractérisé en ce que** A représente -C(O)- et R₁ et R₂ représentent, indépendamment, l'atome d'hydrogène, un radical ((C₁-C₈)alkyle ou un radical de formule -(CH₂)ₙ-X dans laquelle
X représente, amino, di(alkyl)amino, adamentyle, cyclohexyle, cycloheptyle, pipéridine, morpholine, pyrrolidine, phényle, pyridine, imidazole, thiophène, indole, carbazole étant éventuellement substitué (C₁-C₆)alkyle, ou bien un radical de formule 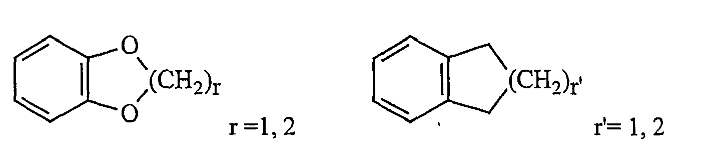 les radicaux pipéridine, pyrrolidine et phényle étant éventuellement substitués par un ou plusieurs substituants identiques ou différents choisis parmi :-(CH₂)ₙ'-X'-Y', halo, oxo, amino et di((C₁-C₈)alkyl)amino ;
X' représente -O-, -S-, -C(O)-O-, -NH-C(O)-, -NH-SO₂- ou une liaison covalente ;
Y' représente un radical (C₁-C₆)alkyle, oxazole, phényle éventuellement substitué par (C₁-C₄)alkyle ou pipérazine éventuellement substitué par (C₁-C₄)alkyle;
ou bien R₁ et R₂ forment ensemble, avec l'atome d'azote auquel ils sont rattachés, pipéridine, pipérazine et diazépane, thiazolidine, morpholine, ou un radical cyclique de formule : 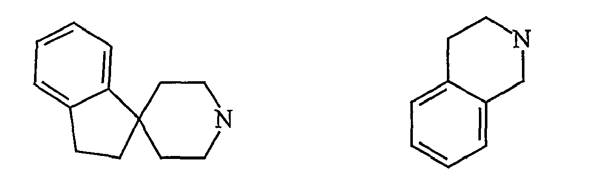 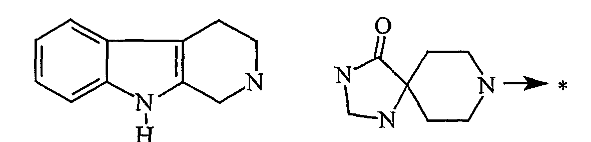 le radical que forment ensemble R₁ et R₂ étant éventuellement substitué par un ou plusieurs substituants identiques ou différents choisis parmi :
-(CH₂)_{n"}-X"-Y" ;
X" représente -C(O)-, -C(O)-O- ou une liaison covalente ;
Y" représente un radical (C₁-C₆)alkyle ; di(alkyl)amino, cyclopentyle, cyclohexyle, pipéridine, pyrrolidine, morpholine, benzyle, pyridine, pyrazine, furane, thiophène, ou phényle éventuellement substitué par un ou plusieurs substituants identiques ou différents choisis parmi (C₁-C₆)alkyle, (C₁-C₆)alkoxy, (C₁-C₆)alkyl-carbonyle et halo ; ou bien Y" représente un radical de formule 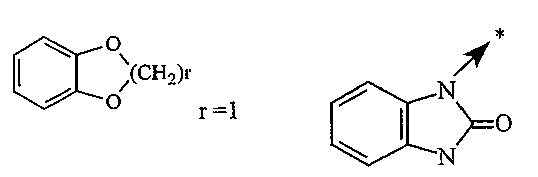
ou un sel pharmaceutiquement acceptable de ce dernier.

7. Composé de formule I selon l'une des revendications 1 à 6, **caractérisé en ce que** A représente -C(O)- et R₃ représente -(CH₂)ₚ-W₃-(CH₂)_{p'}-Z₃
W₃ représente une liaison covalente, -CH(OH)- ou -C(O)- ;
Z₃ représente un radical (C₁-C₆)alkyle, phényle, naphtyle, benzo[b]thiophène, benzo[b]furannyle, ou bien un radical de formule 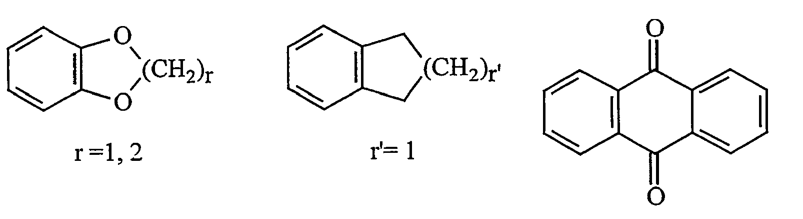 le radical phényle étant éventuellement substitué par un ou plusieurs substituants identiques ou différents choisis parmi : -(CH₂)_{p"}-V₃-Y₃, halo, nitro, cyano ;
V₃ représente -O-, -S-, -C(O)-, -C(O)-O-, -SO₂- ou une liaison covalente ;
Y₃ représente un radical (C₁-C₆)alkyle éventuellement substitué par un ou plusieurs radicaux halo identiques ou différents ; amino ; di((C₁-C₆)alkyl)amino ; phénylcarbonylméthyle ; pyrrolidine ou phényle ;
p, p' et p" représentent, indépendamment, un entier de 0 à 2 ; ou un sel pharmaceutiquement acceptable de ce dernier.

8. Composé de formule I selon l'une des revendications 1 à 7, **caractérisé en ce que** A représente -C(O)- et R₄ représente un radical de formule-(CH₂)ₛ-R"₄
R"₄ représente le cycle pipéridine éventuellement substituée par benzyle, pipérazine éventuellement substituée par benzyle, ou un radical de formule -NW₄W'₄
W₄ représente l'atome d'hydrogène ou (C₁-C₈)alkyle ;
W'₄ représente un radical de formule -(CH₂)_{s'}-Q₄-Z₄
Q₄ représente une liaison covalente, -CH₂-CH(OH)-, -CH₂-CH(OH)-CH₂-O-, -CH₂-CH(OH)-CH₂-, -CH₂-CH(OH)-CH₂-O-CH₂-ou -C(O)-O- ;
Z₄ représente l'atome d'hydrogène, (C₁-C₈)alkyle éventuellement substitué par (C₁-C₆)alkoxy, (C₁-C₆)alkylthio, (C₁-C₆)alkyldithio ou un ou deux radicaux hydroxy ; (C₂-C₆)alkenyle ; (C₂-C₆)alkynyle ; cyclopropyle éventuellement substitué par alkcoxycarbonyle; cyclobutyle ; cyclopentyle éventuellement substitué par hydroxyalkyl ; cyclohexyle éventuellement substitué par un ou plusieurs radicaux alkyle ; cycloheptyle ; cyclohexène ; adamantyle ; pyridine, thiophène, indole, furane, naphtyle ; phényle éventuellement substitué par un ou plusieurs radicaux identiques ou différents choisis parmi : -(CH₂)_{q"}-X₄-Y₄, hydroxy, halo et cyano ;
X₄ représente -O- ou une liaison covalente ;
Y₄ représente un radical (C₁-C₆)alkyle, di((C₁-C₆)alkyl)amino ou pyrrolidine ; ou un sel pharmaceutiquement acceptable de ce dernier.

9. Composé de formule I selon l'une des revendications 1 à 8, **caractérisé en ce que** A représente -C(O)-, Y représente -NH- et
R₁ et R₂ représentent, indépendamment, un radical (C₁-C₈)alkyle ;
R₃ représente -(CH₂)ₚ-W₃-(CH₂)_{p'}-Z₃
W₃ représente une liaison covalente ; Z₃ représente le radical phényle substitué par un ou plusieurs substituants identiques ou différents choisis parmi : -(CH₂)_{p"}-V₃-Y₃ et halo ; V₃ représente -O- ou -S- ; et Y₃ représente un radical (C₁-C₆)alkyle ; p, p' et p" représentent 0 ;
R₄ représente un radical de formule-(CH₂)ₛ-R"₄ ;
R"₄ représente un radical de formule -NW₄W'₄;
W₄ représente l'atome d'hydrogène ou (C₁-C₈)alkyle ;
W'₄ représente un radical de formule -(CH₂)_{s'}-Q₄-Z₄ ;
Q₄ représente une liaison covalente ;
Z₄ représente l'atome d'hydrogène, (C₁-C₈)alkyle éventuellement substitué par hydroxy, (C₃-C₇)cycloalkyle, hétéroaryle, aryle éventuellement substitué par un ou plusieurs radicaux identiques ou différents choisis de formule -(CH₂)_{q"}-V₄-Y₄ ;
V₄ représente -O- ou une liaison covalente ;
Y₄ représente un radical (C₁-C₆)alkyle ou di((C₁-C₆)alkyl)amino ;
q" représente 0 ; s représente un entier de 2 à 4, et s' un entier de 1 à 2 ;
ou un sel pharmaceutiquement acceptable de ce dernier.

10. Composé de formule I selon la revendication 9, **caractérisé en ce que** le (C₃-C₇)cycloalkyle est choisi parmi cyclopentyle et cyclohexyle, l'hétéroaryle représente la pyridine et l'aryle le phényle ; ou un sel pharmaceutiquement acceptable de ce dernier.

11. Composé de formule I selon l'une des revendications 1 à 8, **caractérisé en ce que** A représente -C(O)-, Y représente l'atome de soufre et
R₁ et R₂ représentent, indépendamment, un radical (C₁-C₈)alkyle ;
R₃ représente -(CH₂)ₚ-W₃-(CH₂)_{p'}-Z₃
W₃ représente une liaison covalente ou -C(O)- ; Z₃ représente le radical phényle substitué par un ou plusieurs substituants identiques ou différents choisis parmi : -(CH₂)_{p"}-V₃-Y₃ et halo ; V₃ représente -O- ou une liaison covalente ; et Y₃ représente un radical (C₁-C₆)alkyle ou di((C₁-C₆)alkyl)amino ; p représente 1, et p' et p" représentent 0 ;
R₄ représente un radical de formule-(CH₂)ₛ-R"₄;
R"₄ représente un radical de formule -NW₄W'₄;
W₄ représente l'atome d'hydrogène ou (C₁-C₈)alkyle;
W'₄ représente un radical de formule -(CH₂)_{s'}-Q₄-Z₄;
Q₄ représente une liaison covalente ;
Z₄ représente l'atome d'hydrogène, (C₁-C₈)alkyle, hétéroaryle, aryle
s représente un entier de 2 à 4, et s'un entier de 1 à 2, ou un sel pharmaceutiquement acceptable de ce dernier.

12. Composé de formule I selon la revendication 11, **caractérisé en ce que** l'hétéroaryle représente la pyridine et l'aryle le phényle ; ou un sel pharmaceutiquement acceptable de ce dernier.

13. Composé de formule I selon 1a revendication 1, **caractérisé en ce que** A représente -CH₂-, Y -NH- et R₁ et R₂ représentent, indépendamment, un radical ((C₁-C₆)alkyle ; R₃ représente un phényle substitué par un ou plusieurs substituants (C₁-C₆)alkoxy identiques ou différents ; R₄ représente un radical de formule-(CH₂)ₛ-R"₄ ; R"₄ représente un radical de formule -NW₄W'₄; W₄ représente (C₁-C₈)alkyle ; W'₄ représente un radical de formule -(CH₂)_{s'}-Q₄-Z₄ ; Q₄ représente une liaison covalente et Z₄ représente la pyridine ; ou un sel pharmaceutiquemene acceptable de ce dernier.

14. A titre de médicament, un composé de formule I telle que définie à l'une des revendications 1 à 13, ainsi que les sels d'addition avec les acides minéraux ou organiques pharmaceutiquement acceptables dudit composé de formule I.

15. Composition pharmaceutique contenant, à titre de principe actif, au moins un médicament tel que défini à la revendication 14, en association avec un support pharmaceutiquement acceptable.

16. Utilisation d'un composé selon l'une des revendications 1 à 13, pour la préparation d'un médicament pour le traitement, chez la femme, de l'endométriose, du fibrome, du syndrome des ovaires polykystiques, du cancer du sein, de l'ovaire et de l'endomètre, de la désensibilisation hypophysaire gonadotrope lors des protocoles de procréation médicalement assistée.

17. Utilisation d'un composé selon l'une des revendications 1 à 13, pour la préparation d'un médicament pour le traitement, chez l'homme, de l'hyperplasie bénigne de la prostate et le cancer de la prostate.

18. Utilisation d'un composé selon l'une des revendications 1 à 13, pour la préparation d'un médicament pour le traitement de la puberté précoce masculine ou féminine.

## Claims

1. Compound of general formula 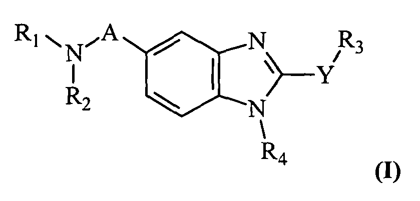 in racemic, enantiomeric form or any combination of these forms and in which:
A represents -CH₂- or -C(O)-;
Y represents -S- or -NH-;
R₁ and R₂ represent, independently, the hydrogen atom, a (C₁-C₈)alkyl, a (C₅-C₉)bicycloalkyl radical optionally substituted by one or more identical or different (C₁-C₆) alkyl radicals, or a radical of formula - (CH₂)ₙ-X in which
X represents amino, (C₁-C₆)alkylamino, di((C₁-C₆)alkyl)amino, (C₃-C₇)cycloalkyl, adamantyl, heterocycloalkyl, aryl, aryl-carbonyl or heteroaryl, or a radical of formula 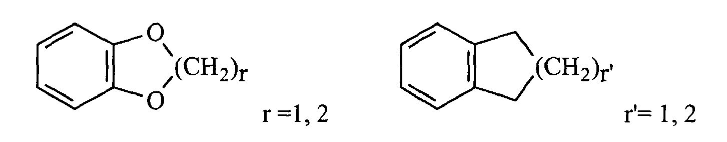 the (C₃-C₇)cycloalkyl, heterocycloalkyl, aryl and heteroaryl radicals being optionally substituted by one or more identical or different substituents chosen from: - (CH₂)_{n'}-X'-Y', halo, oxo, nitro, cyano, amino, (C₁-C₆)alkylamino and di((C₁-C₈)alkyl)amino, hydroxy, N₃;
X' represents -O-, -S-, -C(O)-, -C(O)-O-, -NH-C(O)-, -NH-SO₂- or a covalent bond;
Y' represents a (C₁-C₆)alkyl radical optionally substituted by one or more identical or different halo radicals; heteroaryl or aryl or heterocycloalkyl optionally substituted by one or more identical or different substituents chosen from: (C₁-C₆)alkyl, (C₁-C₆)alkoxy, halo, nitro, cyano, amino, CF₃, OCF₃, hydroxy, N₃, (C₁-C₆)alkylamino and di((C₁-C₈)alkyl)amino;
n represents an integer from 0 to 6 and n' an integer from 0 to 2;
or R₁ and R₂ form together with the nitrogen atom to which they are attached, a heterocycloalkyl, a heterobicycloalkyl or a radical of formula: 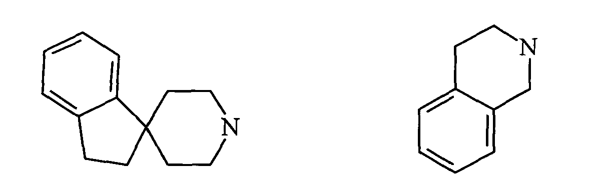 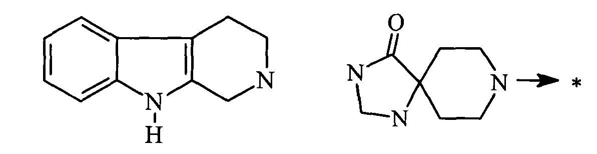 the radical which R₁ and R₂ together form being optionally substituted by one or more identical or different substituents chosen from:
-(CH₂)_{n"}-X"-Y", oxo , hydroxy, halo, nitro, cyano;
X" represents -O-, -C(O)-, -C(O)-O- or a covalent bond;
Y" represents a (C₁-C₆)alkyl, amino, (C₁-C₆)alkylamino, di((C₁-C₆)alkyl)amino, (C₃-C₇)cycloalkyl, heterocycloalkyl, arylalkyl, or aryl or heteroaryl radical optionally substituted by one or more identical or different substituents chosen from: (C₁-C₆)alkyl, (C₁-C₆)alkoxy, (C₁-C₆)alkyl-carbonyl, halo, hydroxy, nitro, cyano, CF₃, OCF₃, amino, (C₁-C₆)alkylamino and di((C₁-C₆)alkyl)amino); or a radical of formula 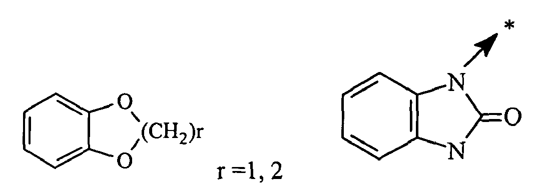
n" represents an integer from 0 to 4;
R₃ represents -(CH₂)ₚ-W₃-(CH₂)_{p'}-Z₃
W₃ represents a covalent bond, -CH(OH)- or -C(O)-;
Z₃ represents a (C₁-C₆)alkyl, adamantyl, aryl, a heteroaryl radical, or a radical of formula 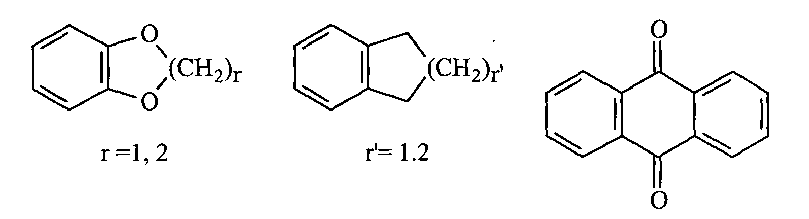 the aryl radical being optionally substituted by one or more identical or different substituents chosen from: -(CH₂)_{p"}-V₃-Y₃, halo, nitro, cyano, N₃, hydroxy;
V₃ represents -O-, -S-, -C(O)-, -C(O)-O-, -SO₂- or a covalent bond;
Y₃ represents a (C₁-C₆)alkyl radical optionally substituted by one or more identical or different halo, amino, (C₁-C₆)alkylamino, dl((C₁-C₆)alkyl)amino, phenylcarbonylmethyl, heterocycloalkyl or aryl radicals;
p, p' and p" represent, independently, an integer from 0 to 4;
R₄ represents a radical of formula-(CH₂)ₛ-R"₄
R"₄ represents a heterocycloalkyl containing at least one nitrogen atom and optionally substituted by (C₁-C₆)alkyl or aralkyl; a heteroaryl containing at least one nitrogen atom and optionally substituted by (C₁-C₆)alkyl; or a radical of formula -NW₄W'₄
W₄ represents the hydrogen atom, (C₁-C₈)alkyl or (C₃-C₇)cycloalkyl;
W'₄ represents a radical of formula -(CH₂)_{s'}-Q₄-Z₄;
Q₄ represents a covalent bond, -CH₂-CH(OH)-[CH₂]ₜ-[O]_{t'}-[CH₂]_{t"}-or -C(O)-O-;
t, t' and t" represent, independently, 0 or 1;
Z₄ represents the hydrogen atom, (C₁-C₈)alkyl optionally substituted by one or more identical or different substituents chosen from: (C₁-C₆)alkoxy, (C₁-C₆)alkylthio, (C₁-C₆)alkyldithio and hydroxy; (C₂-C₆)alkenyl; (C₂-C₆)alkynyl; (C₃-C₇)cycloalkyl optionally substituted by one or more identical or different substituents chosen from: (C₁-C₆)alkyl, (C₁-C₆)alkoxy-carbonyl and (C₁-C₆)hydroxyalkyl; cyclohexene; adamantyl; heteroaryl; aryl optionally substituted by one or more identical or different radicals chosen from formula -(CH₂)_{q"}-V₄-Y₄, hydroxy, halo, nitro, cyano;
V₄ represents -O-, -S-, -NH-C(O)- or a covalent bond;
Y₄ represents a (C₁-C₆)alkyl radical optionally substituted by di((C₁-C₆)alkyl)amino or one or more identical or different halo; amino; (C₁-C₆)alkylamino; di((C₁-C₆)alkyl)amino; aralkyl; heterocycloalkyl radicals;
q" represents an integer from 0 to 4;
or Z₄ represents a radical of formula 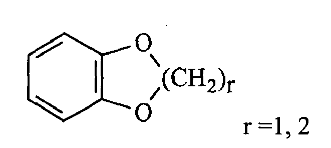 s and s' represent, independently, an integer from 0 to 6; or a pharmaceutically acceptable salt thereof.

2. Compound of formula I according to claim 1, **characterized in that** A represents -C(O)-; or a pharmaceutically acceptable salt thereof.

3. Compound of formula I according to one of claims 1 to 2, **characterized in that**
the cycloalkyl represented by X is cyclohexyl or cycloheptyl,
the heterocycloalkyl represented by X is chosen from: piperidine, morpholine, pyrrolidine, thiazolidine and tetrahydrothiophene;
the aryl represented by X is the phenyl, naphthyl or fluorenyl radical;
the aryl of the aryl-carbonyl radical represented by X, is the phenyl radical;
the heteroaryl represented by X is chosen from: pyridine, imidazole, thiophene, indole, carbazole and isoquinoline;
the heteroaryl represented by Y' is chosen from oxazole and imidazole;
the aryl represented by Y' is the phenyl radical;
the heterocycloalkyl represented by Y' is piperazine;
the heterocycloalkyl which R₁ and R₂ form together with the nitrogen atom to which they are attached, is chosen from: piperidine, piperazine, diazepane, thiazolidine and morpholine;
the cycloalkyl represented by Y" is cyclopentyl or cyclohexyl;
the heterocycloalkyl represented by Y" is chosen from: piperidine, pyrrolidine and morpholine;
the arylalkyl and the aryl represented by Y" are respectively the benzyl radical and the phenyl radical;
the heteroaryl represented by Y" is chosen from: pyridine, pyrazine, furan and thiophene; or a pharmaceutically acceptable salt thereof.

4. Compound of formula I according to one of claims 1 to 3, **characterized in that**
the aryl represented by Z₃ is the phenyl or naphthyl radical;
the heteroaryl represented by Z₃ is chosen from: benzo[b]thiophene and benzo[b]furan;
the heterocycloalkyl and the aryl represented by Y₃ are respectively the pyrrolidine and phenyl radicals; or a pharmaceutically acceptable salt thereof.

5. Compound of formula I according to one of claims 1 to 4, **characterized in that**
the heterocycloalkyl represented by R"₄ is chosen from: piperazine, piperidine, morpholine and pyrrolidine;
the aralkyl which optionally substitutes the heterocycloalkyl represented by R"₄ is the benzyl radical;
the heteroaryl represented by R"₄ is imidazole;
the (C₃-C₇)cycloalkyl represented by Z₄ is cyclopropyl, cyclobutyl, cyclopentyl, cyclohexyl or cycloheptyl;
the heteroaryl represented by Z₄ is chosen from: pyridine, thiophene, indole and furan;
the aryl represented by Z₄ and is phenyl or naphthyl;
the aralkyl represented by Y₄ is benzyl;
the heterocycloalkyl represented by Y₄ is pyrrolidine;
the aralkyl which is substituted on the heterocycloalkyl formed together by W₄ and W'₄ is the benzyl radical; or a pharmaceutically acceptable salt thereof.

6. Compound of formula I according to one of claims 1 to 5, **characterized in that** A represents -C(O)- and R₁ and R₂ represent, independently, the hydrogen atom, a ((C₁-C₈)alkyl radical or a radical of formula -(CH₂)ₙ-X in which
X represents, amino, di(alkyl)amino, adamentyl, cyclohexyl, cycloheptyl, piperidine, morpholine, pyrrolidine, phenyl, pyridine, imidazole, thiophene, indole, carbazole being optionally substituted (C₁-C₆)alkyl, or a radical of formula 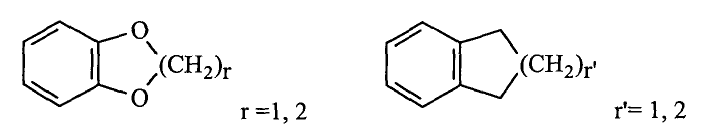 the piperidine, pyrrolidine and phenyl radicals being optionally substituted by one or more identical or different substituents chosen from:-(CH₂)_{n'}-X'-Y', halo, oxo, amino and di((C₁-C₈)alkyl)amino;
X' represents -O-, -S-, -C(O)-O-, -NH-C(O)-, -NH-SO₂- or a covalent bond;
Y' represents a (C₁-C₆)alkyl, oxazole, phenyl optionally substituted by (C₁-C₄)alkyl or piperazine optionally substituted by (C₁-C₄)alkyl radical;
or R₁ and R₂ form together with the nitrogen atom to which they are attached, piperidine, piperazine and diazepane, thiazolidine, morpholine, or a cyclic radical of formula: 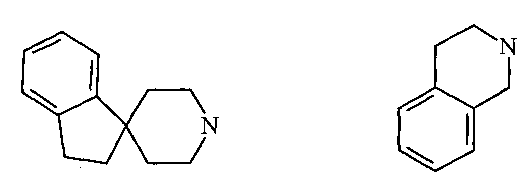 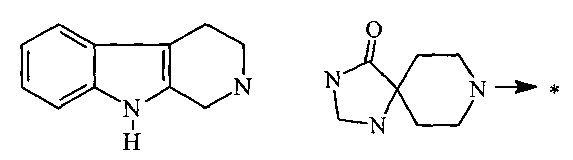 the radical which is formed by R₁ and R₂ together being optionally substituted by one or more identical or different substituents chosen from:
-(CH₂)_{n"}-X"-Y";
X" represents -C(O)-, -C(O)-O- or a covalent bond;
Y" represents a (C₁-C₆)alkyl; di(alkyl)amino, cyclopentyl, cyclohexyl, piperidine, pyrrolidine, morpholine, benzyl, pyridine, pyrazine, furan, thiophene, or phenyl radical optionally substituted by one or more identical or different substituents chosen from (C₁-C₆)alkyl, (C₁-C₆)alkoxy, (C₁-C₆)alkyl-carbonyl and halo; or Y" represents a radical of formula 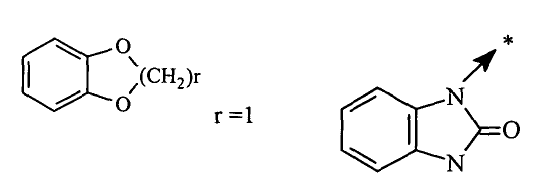
or a pharmaceutically acceptable salt thereof.

7. Compound of formula I according to one of claims 1 to 6, **characterized in that** A represents -C(O)- and R₃ represents -(CH₂)ₚ-W₃-(CH₂)_{p'}-Z₃
W₃ represents a covalent bond, -CH(OH)- or -C(O)-;
Z₃ represents a (C₁-C₆)alkyl, phenyl, naphthyl, benzo[b]thiophene, benzo[b]furannyl radical, or a radical of formula 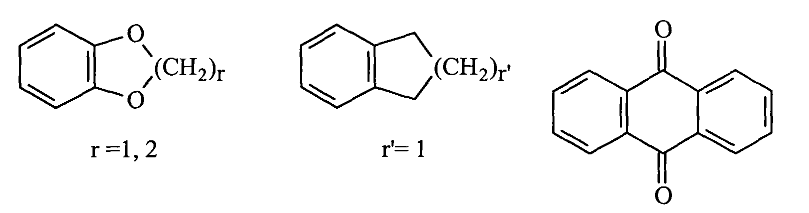 the phenyl radical being optionally substituted by one or more identical or different substituents chosen from: -(CH₂)_{p"}-V₃-Y₃, halo, nitro, cyano;
V₃ represents -O-, -S-, -C(O)-, -C(O)-O-, -SO₂- or a covalent bond;
Y₃ represents a (C₁-C₆)alkyl radical optionally substituted by one or more identical or different halo; amino; di((C₁-C₆)alkyl)amino; phenylcarbonylmethyl; pyrrolidine or phenyl radicals;
p, p' and p" represent, independently, an integer from 0 to 2; or a pharmaceutically acceptable salt thereof.

8. Compound of formula I according to one of claims 1 to 7, **characterized in that** A represents -C(O)- and R₄ represents a radical of formula-(CH₂)ₛ-R"₄
R"₄ represents the piperidine ring optionally substituted by benzyl, piperazine optionally substituted by benzyl, or a radical of formula -NW₄W'₄
W₄ represents the hydrogen atom or (C₁-C₈)alkyl;
W'₄ represents a radical of formula -(CH₂)_{s'}-Q₄-Z₄;
Q₄ represents a covalent bond, -CH₂-CH(OH)-, -CH₂-CH(OH)-CH₂-O-, -CH₂-CH(OH)-CH₂-, -CH₂-CH(OH)-CH₂-O-CH₂-or -C(O)-O-;
Z₄ represents the hydrogen atom, (C₁-C₈)alkyl optionally substituted by (C₁-C₆)alkoxy, (C₁-C₆)alkylthio, (C₁-C₆)alkyldithio or one or two hydroxy; (C₂-C₆)alkenyl; (C₂-C₆)alkynyl; cyclopropyl radical optionally substituted by alkoxycarbonyl; cyclobutyl, cyclopentyl optionally substituted by hydroxyalkyl; cyclohexyl optionally substituted by one or more alkyl; cycloheptyl; cyclohexene ; adamantyl ; pyridine ; thiophene ; indole ; furane ; naphthyl; phenyl radicals optionally substituted by one or more identical or different radicals chosen from: -(CH₂)_{q"}-X₄-Y₄, hydroxy, halo and cyano;
X₄ represents -O- or a covalent bond;
Y₄ represents a (C₁-C₆)alkyl, di((C₁-C₆)alkyl)amino or pyrrolidine radical; or a pharmaceutically acceptable salt thereof.

9. Compound of formula I according to one of claims 1 to 8, **characterized in that** A represents -C(O)-, Y represents -NH- and
R₁ and R₂ represent, independently, a (C₁-C₈)alkyl radical;
R₃ represents -(CH₂)ₚ-W₃-(CH₂)_{p'}-Z₃
W₃ represents a covalent bond; Z₃ represents the phenyl radical substituted by one or more identical or different substituents chosen from: -(CH₂)_{p"}-V₃-Y₃ and halo; V₃ represents -O- or -S-; and Y₃ represents a (C₁-C₆)alkyl radical; p, p' and p" represent 0;
R₄ represents a radical of formula-(CH₂)ₛ-R"₄
R"₄ represents a radical of formula -NW₄W'₄
W₄ represents the hydrogen atom or (C₁-C₈)alkyl;
W'₄ represents a radical of formula -(CH₂)_{s'}-Q₄-Z₄;
Q₄ represents a covalent bond;
Z₄ represents the hydrogen atom, (C₁-C₈)alkyl optionally substituted by hydroxy, (C₃-C₇)cycloalkyl, heteroaryl, aryl optionally substituted by one or more identical or different radicals chosen from formula -(CH₂)_{q"}-V₄-Y₄;
V₄ represents -O- or a covalent bond;
Y₄ represents a (C₁-C₆)alkyl or di((C₁-C₆)alkyl)amino radical;
q" represents 0; s represents an integer from 2 to 4, and s' an integer from 1 to 2;
or a pharmaceutically acceptable salt thereof.

10. Compound of formula I according to claim 9, **characterized in that** the (C₃-C₇)cycloalkyl is chosen from cyclopentyl and cyclohexyl, the heteroaryl represents pyridine and the aryl represents phenyl; or a pharmaceutically acceptable salt thereof.

11. Compound of formula I according to one of claims 1 to 8, **characterized in that** A represents -C(O)-, Y represents the sulphur atom and
R₁ and R₂ represent, independently, a (C₁-C₈)alkyl radical;
R₃ represents -(CH₂)ₚ-W₃-(CH₂)_{p'}-Z₃
W₃ represents a covalent bond or -C(O)-; Z₃ represents the phenyl radical substituted by one or more identical or different substituents chosen from: -(CH₂)_{p"}-V₃-Y₃ and halo; V₃ represents -O- or a covalent bond; and Y₃ represents a (C₁-C₆)alkyl or di((C₁-C₆)alkyl)amino radical; p represents 1, and p' and p" represent 0;
R₄ represents a radical of formula-(CH₂)ₛ-R"₄
R"₄ represents a radical of formula -NW₄W'₄
W₄ represents the hydrogen atom or (C₁-C₈)alkyl;
W'₄ represents a radical of formula -(CH₂)_{s'}-Q₄-Z₄;
Q₄ represents a covalent bond;
Z₄ represents the hydrogen atom, (C₁-C₈)alkyl, heteroaryl, aryl
s represents an integer from 2 to 4, and s' an integer from 1 to 2, or a pharmaceutically acceptable thereof.

12. Compound of formula I according to claim 11, **characterized in that** the heteroaryl represents pyridine and the aryl represents phenyl; or a pharmaceutically acceptable thereof.

13. Compound of formula I according to claim 1, **characterized in that** A represents -CH₂-, Y-NH- and R₁ and R₂ represent, independently, a ((C₁-C₆)alkyl radical; R₃ represents a phenyl substituted by one or more identical or different (C₁-C₆)alkoxy substituents; R₄ represents a radical of formula-(CH₂)ₛ-R"₄; R"₄ represents a radical of formula -NW₄W'₄; W₄ represents (C₁-C₈)alkyl; W'₄ represents a radical of formula -(CH₂)_{s'}-Q₄-Z₄; Q₄ represents a covalent bond and Z₄ represents pyridine; or a pharmaceutically acceptable thereof.

14. As a medicament, a compound of formula I as defined in one of claims 1 to 13, as well as the addition salts with pharmaceutically acceptable mineral or organic acids of said compound of formula I.

15. Pharmaceutical composition containing, as active ingredient, at least one medicament as defined in claim 14, in combination with a pharmaceutically acceptable support.

16. Use of a compound according to one of claims 1 to 13, for the preparation of a medicament for the treatment, in women, of endometriosis, fibroma, polycystic ovary syndrome, cancer of the breast, the ovary and the endometrium, gonadotropic hypophyseal desensitization during medically-assisted procreation protocols.

17. Use of a compound according to one of claims 1 to 13, for the preparation of a medicament for the treatment, in man, of benign prostatic hyperplasia and prostate cancer;

18. Use of a compound according to one of claims 1 to 13, for the preparation of a medicament for the treatment of precocious male or female puberty.

## Patentansprüche

1. Verbindung der allgemeinen Formel 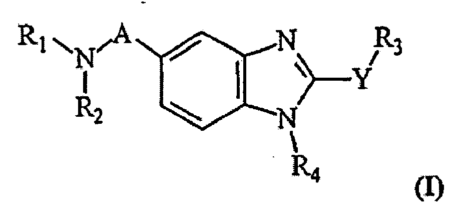 in racemischer Form, enantiomerer Form oder allen Kombinationen dieser Formen, in der:
A -CH₂- oder -C(O)- darstellt;
Y -S- oder -NH- darstellt;
R₁ und R₂ unabhängig voneinander ein Wasserstoffatom, einen Rest (C₁-C₈)-Alkyl, ein (C₅-C₉)-Bicycloalkyl, das gegebenenfalls mit einem oder mehreren gleichen oder verschiedenen (C₁-C₆)-Alkylresten substituiert ist, oder einen Rest der Formel -(CH₂)ₙ-X darstellen, in der
X Amino, (C₁-C₆)-Alkylamino, Di((C₁-C₆)-alkyl)amino, (C₃-C₇)-Cycloalkyl, Adamantyl, Heterocycloalkyl, Aryl, Arylcarbonyl oder Heteroaryl oder einen Rest der Formel 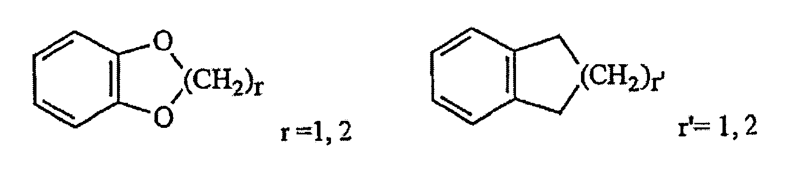 darstellt, wobei die Reste (C₃-C₇)-Cycloalkyl, Heterocycloalkyl, Aryl und Heteroaryl gegebenenfalls mit einem oder mehreren gleichen oder verschiedenen Substituenten substituiert sind, die ausgewählt sind aus: -(CH₂)_{n'}-X'-Y', Halogen, Oxo, Nitro, Cyan, Amino, (C₁-C₆)-Alkylamino und Di((C₁-C₈)-alkyl)amino, Hydroxy, N₃;
X' -O-, -S-, -C(O)-, -C(O)-O-, -NH-C(O)-, -NH-SO₂- oder eine kovalente Bindung darstellt;
Y' einen Rest (C₁-C₆)-Alkyl, der gegebenenfalls mit einem oder mehreren gleichen oder verschiedenen Halogenresten substituiert ist; Heteroaryl oder Aryl oder Heterocycloalkyl darstellt, gegebenenfalls mit einem oder mehreren gleichen öder verschiedenen Substituenten substituiert, die ausgewählt sind aus: (C₁-C₆)-Alkyl, (C₁-C₆)-Alkoxy, Halogen, Nitro, Cyan, Amino, CF₃, OCF₃, Hydroxy, N₃, (C₁-C₆)-Alkylamino und Di((C₁-C₈)-alkyl)amino;
n eine ganze Zahl von 0 bis 6 und n' eine ganze Zahl von 0 bis 2 darstellt;
oder R₁ und R₂ zusammen mit dem Stickstoffatom, an das sie gebunden sind, ein Heterocycloalkyl, ein Heterobicycloalkyl oder einen Rest der Formel bilden: 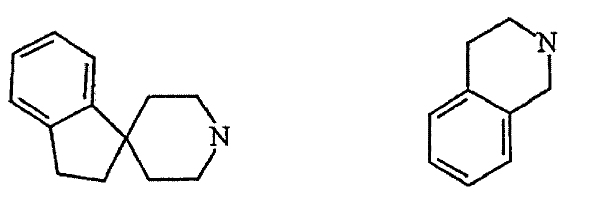 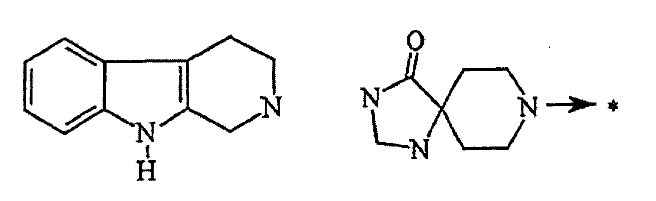 wobei der Rest, den R₁ und R₂ zusammen bilden, gegebenenfalls mit einem oder mehreren gleichen oder verschiedenen Substituenten substituiert ist, die ausgewählt sind aus:
-(CH₂)_{n"}-X"-Y", Oxo, Hydroxy, Halogen, Nitro, Cyan;
X" -O-, -C(O)-, -C(O)-O- oder eine kovalente Bindung darstellt;
Y" einen der Reste (C₁-C₆)-Alkyl, Amino, (C₁-C₆)-Alkylamino, Di((C₁-C₆)-alkyl)amino, (C₃-C₇)-Cycloalkyl, Heterocycloalkyl, Arylalkyl oder Aryl oder Heteroaryl, der gegebenenfalls mit einem oder mehreren gleichen oder verschiedenen Substituenten substituiert ist, die ausgewählt sind aus: (C₁-C₆)-Alkyl, (C₁-C₆)-Alkoxy, (C₁-C₆)-Alkylcarbonyl, Halogen, Hydroxy, Nitro, Cyan, CF₃, OCF₃, Amino, (C₁-C₆)-Alkylamino und Di((C₁-C₆)-alkyl)amino; oder einen Rest der Formel darstellt 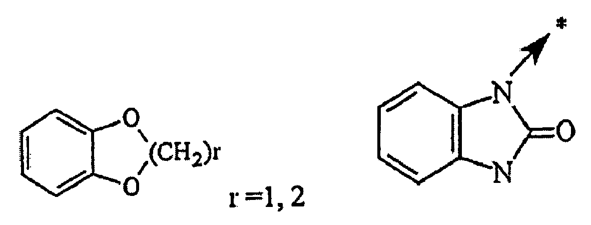
n" eine ganze Zahl von 0 bis 4 darstellt;
R₃ -(CH₂)ₚ-W₃-(CH₂)_{p'}-Z₃ darstellt
W₃ eine kovalente Bindung, -CH(OH)- oder -C(O)- darstellt;
Z₃ einen der Reste (C₁-C₆)-Alkyl, Adamantyl, Aryl, ein Heteroaryl oder einen Rest der Formel darstellt 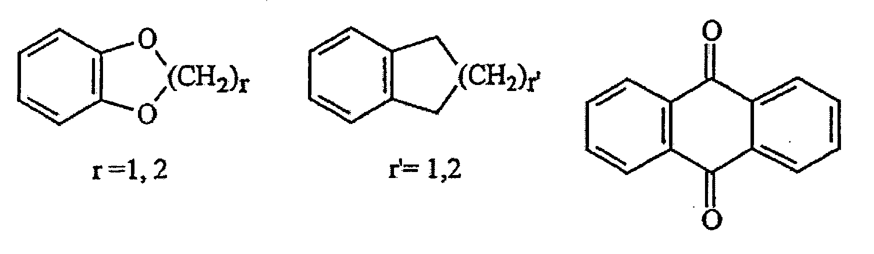 wobei der Arylrest gegebenenfalls mit einem oder mehreren gleichen oder verschiedenen Substituenten substituiert ist, die ausgewählt sind aus: -(CH₂)_{p"}-V₃-Y₃, Halogen, Nitro, Cyan, N₃, Hydroxy;
V₃ -O-, -S-, -C(O)-, -C(O)-O-, -SO₂- oder eine kovalente Bindung darstellt;
Y₃ einen (C₁-C₆)-Alkylrest darstellt, der gegebenenfalls mit einem oder mehreren gleichen oder verschiedenen Halogenresten, Amino, (C₁-C₆)-Alkylamino, Di((C₁-C₆)-alkyl)amino, Phenylcarbonylmethyl, Heterocycloalkyl oder Aryl substituiert ist;
p, p' und p" unabhängig voneinander eine ganze Zahl von 0 bis 4 darstellen;
R₄ einen Rest der Formel -(CH₂)ₛ-R"₄ darstellt
R"₄ darstellt: ein Heterocycloalkyl, das mindestens ein Stickstoffatom enthält und gegebenenfalls mit (C₁-C₆)-Alkyl oder Aralkyl substituiert ist; ein Heteroaryl, das mindestens ein Stickstoffatom enthält und gegebenenfalls mit (C₁-C₆)-Alkyl substituiert ist; oder einen Rest der Formel -NW₄W'₄
W₄ ein Wasserstoffatom, (C₁-C₈)-Alkyl oder (C₃-C₇)-Cycloalkyl darstellt;
W'₄ einen Rest der Formel -(CH₂)_{s'}-Q₄-Z₄ darstellt;
Q₄ eine kovalente Bindung, -CH₂-CH(OH)-[CH₂]ₜ-[O]_{t'}-[CH₂]_{t"}- oder -C(O)-O- darstellt;
t, t' und t" unabhängig voneinander 0 oder 1 darstellen;
Z₄ darstellt: ein Wasserstoffatom, (C₁-C₈)-Alkyl, das gegebenenfalls mit einem oder mehreren gleichen oder verschiedenen Substituenten substituiert ist, die ausgewählt sind aus: (C₁-C₆)-Alkoxy, (C₁-C₆)-Alkylthio, (C₁-C₆)-Alkyldithio und Hydroxy; (C₂-C₆)-Alkenyl; (C₂-C₆)-Alkinyl; (C₃-C₇)-Cycloalkyl, das gegebenenfalls mit einem oder mehreren gleichen oder verschiedenen Substituenten substituiert ist, die ausgewählt sind aus: (C₁-C₆)-Alkyl, (C₁-C₆)-Alkoxycarbonyl und (C₁-C₆)-Hydroxyalkyl; Cyclohexen; Adamantyl; Heteroaryl; Aryl, das gegebenenfalls mit einem oder mehreren gleichen oder verschiedenen Resten substituiert ist, die ausgewählt sind aus der Formel -(CH₂)_{q"}-V₄-Y₄, Hydroxy, Halogen, Nitro, Cyan;
V₄ -O-, -S-, -NH-C(O)- oder eine kovalente Bindung darstellt;
Y₄ einen der Reste (C₁-C₆)-Alkyl, der gegebenenfalls mit Di((C₁-C₆)-alkyl)amino oder einem oder mehreren gleichen oder verschiedenen Halogenresten substituiert ist; Amino; (C₁-C₆)-Alkylamino; Di((C₁-C₆)alkyl)amino; Aralkyl; Heterocycloalkyl darstellt;
q" eine ganze Zahl von 0 bis 4 darstellt;
oder Z₄ einen Rest der Formel darstellt 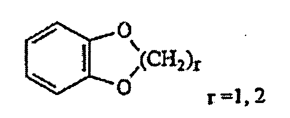 s und s' unabhängig voneinander eine ganze Zahl von 0 bis 6 darstellen; oder ein pharmazeutisch annehmbares Salz dieser Verbindung.

2. Verbindung der Formel I nach Anspruch 1, **dadurch gekennzeichnet, dass** A -C(O)- darstellt; oder ein pharmazeutisch annehmbares Salz dieser Verbindung.

3. Verbindung der Formel I nach einem der Ansprüche 1 bis 2, **dadurch gekennzeichnet, dass**
das Cycloalkyl, das X darstellt, Cyclohexyl oder Cycloheptyl ist;
das Heterocycloalkyl, das X darstellt, ausgewählt ist aus: Piperidin, Morpholin, Pyrrolidin, Thiazolidin und Tetrahydrothiophen;
das Aryl, das X darstellt, der Rest Phenyl, Naphtyl oder Fluorenyl ist;
das Aryl des Arylcarbonylrests, den X darstellt, der Phenylrest ist;
das Heteroaryl, das X darstellt, ausgewählt ist aus: Pyridin, Imidazol, Thiophen, Indol, Carbazol und Isochinolin;
das Heteroaryl, das Y' darstellt, aus Oxazol und Imidazol ausgewählt ist;
das Aryl, das Y' darstellt, der Phenylrest ist;
das Heterocycloalkyl, das Y' darstellt, Piperazin ist;
das Heterocycloalkyl, das R₁ und R₂ zusammen mit dem Stickstoffatom, an das sie gebunden sind, bilden, ausgewählt ist aus: Piperidin, Piperazin, Diazepan, Thiazolidin und Morpholin;
das Cycloalkyl, das Y" darstellt, Cyclopentyl oder Cyclohexyl ist;
das Heterocycloalkyl, das Y" darstellt, ausgewählt ist aus: Piperidin, Pyrrolidin und Morpholin;
das Arylalkyl und das Aryl, die Y" darstellt, der Benzylrest bzw. der Phenylrest sind;
das Heteroaryl, das Y" darstellt, ausgewählt ist aus: Pyridin, Pyrazin, Furan und Thiophen; oder ein pharmazeutisch annehmbares Salz dieser Verbindung.

4. Verbindung der Formel I nach einem der Ansprüche 1 bis 3, **dadurch gekennzeichnet, dass**,
das Aryl, das Z₃ darstellt, der Phenyl- oder Naphtylrest ist;
das Heteroaryl, das Z₃ darstellt, aus Benzo[b]thiophen und Benzo[b]furan ausgewählt ist;
das Heterocycloalkyl und das Aryl, die Y₃ darstellt, die Reste Pyrrolidin bzw. Phenyl sind; oder ein pharmazeutisch annehmbares Salz dieser Verbindung.

5. Verbindung der Formel I nach einem der Ansprüche 1 bis 4, **dadurch gekennzeichnet, dass**
das Heterocycloalkyl, das R"₄ darstellt, ausgewählt ist aus: Piperazin, Piperidin, Morpholin und Pyrrolidin;
das Aralkyl, das gegebenenfalls das Heterocycloalkyl substituiert, das R"₄ darstellt, der Benzylrest ist;
das Heteroaryl, das R"₄ darstellt, Imidazol ist;
das (C₃-C₇)-Cycloalkyl, das Z₄ darstellt, Cyclopropyl, Cyclobutyl, Cyclopentyl, Cyclohexyl oder Cycloheptyl ist;
das Heteroaryl, das Z₄ darstellt, ausgewählt ist aus: Pyridin, Thiophen, Indol und Furan;
das Aryl, das Z₄ darstellt, Phenyl oder Naphtyl ist;
das Aralkyl, das Y₄ darstellt, Benzyl ist;
das Heterocycloalkyl, das Y₄ darstellt, Pyrrolidin ist;
das Aralkyl, das an dem Heterocycloalkyl substituiert ist, das W₄ und W'₄ zusammen bilden, der Benzylrest ist; oder ein pharmazeutisch annehmbares Salz dieser Verbindung.

6. Verbindung der Formel I nach einem der Ansprüche 1 bis 5, **dadurch gekennzeichnet, dass** A -C(O)-darstellt und R₁ und R₂ unabhängig voneinander ein Wasserstoffatom, einen (C₁-C₈)-Alkylrest oder einen Rest der Formel -(CH₂)ₙ-X darstellen, in der
X Amino, Di(alkyl)amino, Adamantyl, Cyclohexyl, Cycloheptyl, Piperidin, Morpholin, Pyrrolidin, Phenyl, Pyridin, Imidazol, Thiophen, Indol, Carbazol, das gegebenenfalls mit (C₁-C₆)-Alkyl substituiert ist, oder einen Rest der Formel 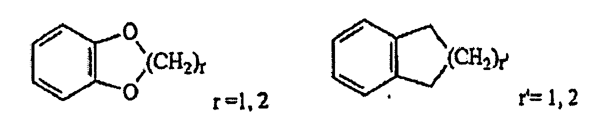 darstellt, wobei die Reste Piperidin, Pyrrolidin und Phenyl gegebenenfalls mit einem oder mehreren gleichen oder verschiedenen Substituenten substituiert sind, die ausgewählt sind aus: -(CH₂)_{n'}-X'-Y', Halogen, Oxo, Amino und Di((C₁-C₈)-alkyl)amino;
X' -O-, -S-, -C(O)-O-, -NH-C(O)-, -NH-SO₂- oder eine kovalente Bindung darstellt;
Y einen der Reste (C₁-C₆)-Alkyl, Oxazol, gegebenenfalls mit (C₁-C₄)-Alkyl substituiertes Phenyl oder gegebenenfalls mit (C₁-C₄)-Alkyl substituiertes Piperazin darstellt;
oder R₁ und R₂ zusammen mit dem Stickstoffatom, an das sie gebunden sind, Piperidin, Piperazin und Diazepan, Thiazolidin, Morpholin oder einen cyclischen Rest der Formel bilden: 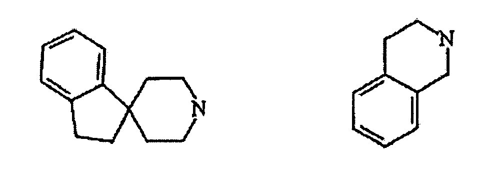 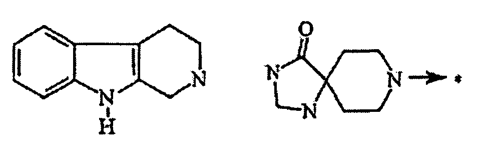 wobei der Rest, den R₁ und R₂ zusammen bilden, gegebenenfalls mit einem oder mehreren gleichen oder verschiedenen Substituenten substituiert sind, die ausgewählt sind aus:
-(CH₂)_{n"}-X"- Y";
X" -C(O)-, -C(O)-O- oder eine kovalente Bindung darstellt;
Y" einen Rest (C₁-C₆)-Alkyl; Di(alkyl)amino, Cyclopentyl, Cyclohexyl, Piperidin, Pyrrolidin, Morpholin, Benzyl, Pyridin, Pyrazin, Furan, Thiophen oder Phenyl darstellt, das gegebenenfalls mit einem oder mehreren gleichen oder verschiedenen Substituenten substituiert ist, die aus (C₁-C₆)-Alkyl, (C₁-C₆)-Alkoxy, (C₁-C₆)-Alkylcarbonyl und Halogen ausgewählt sind; oder Y" einen Rest der Formel darstellt: 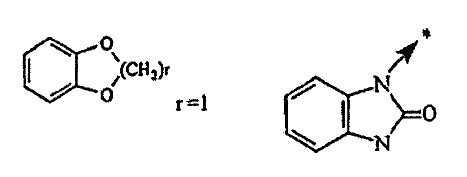
oder ein pharmazeugisch annehmbares Salz dieser Verbindung.

7. Verbindung der Formel I nach einem der Ansprüche 1 bis 6, **dadurch gekennzeichnet, dass** A -C(O)-darstellt und R₃ -(CH₂)ₚ-W₃-(CH₂)_{p'}-Z₃ darstellt,
W₃ eine kovalente Bindung, -CH(OH)- oder -C(O)- darstellt;
Z₃ einen der Reste (C₁-C₆)-Alkyl, Phenyl, Naphtyl, Benzo[b]thiophen, Benzo[b]furanyl oder einen Rest der Formel darstellt: 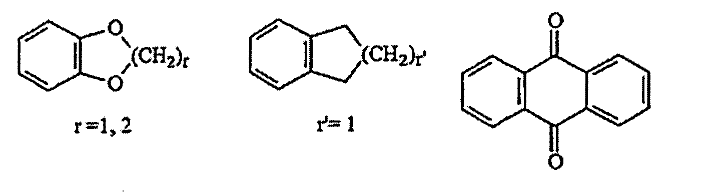 wobei der Phenylrest gegebenenfalls mit einem oder mehreren gleichen oder verschiedenen Substituenten substituiert ist, die ausgewählt sind aus: -(CH₂)_{p"}-V₃-Y₃, Halogen, Nitro, Cyan;
V₃ -O-, -S-, -C(O)-, -C(O)-O-, -SO₂- oder eine kovalente Bindung darstellt;
Y₃ einen gegebenenfalls mit einem oder mehreren gleichen oder verschiedenen Halogenresten substituierten Rest (C₁-C₆)-Alkyl; Amino; Di((C₁-C₆)-alkyl)amino; Phenylcarbonylmethyl; Pyrrolidin oder Phenyl darstellt;
p, p' und p" unabhängig voneinander eine ganze Zahl von 0 bis 2 darstellen; oder ein pharmazeutisch annehmbares Salz dieser Verbindung.

8. Verbindung der Formel I nach einem der Ansprüche 1 bis 7, **dadurch gekennzeichnet, dass** A -C(O)-darstellt und R₄ einen Rest der Formel -(CH₂)ₛ-R"₄ darstellt
R"₄ den gegebenenfalls mit Benzyl substituierten Piperidinring, gegebenenfalls mit Benzyl substituiertes Piperazin oder einen Rest der Formel -NW₄W'₄ darstellt
W₄ ein Wasserstoffatom oder (C₁-C₈)-Alkyl darstellt;
W'₄ einen Rest der Formel -(CH₂)_{s'}-Q₄-Z₄ darstellt;
Q₄ eine kovalente Bindung, -CH₂-CH(OH)-, -CH₂-CH(OH)-CH₂-O-, -CH₂-CH(OH)-CH₂-, -CH₂-CH(OH)-CH₂-O-CH₂- oder -C(O)-O- darstellt;
Z₄ darstellt: ein Wasserstoffatom, (C₁-C₈)-Alkyl, das gegebenenfalls mit (C₁-C₆)-Alkoxy, (C₁-C₆)-Alkylthio, (C₁-C₆)-Alkyldithio oder einem oder zwei Hydroxyresten substituiert ist; (C₂-C₆)-Alkenyl; (C₂-C₆)-Alkinyl; gegebenenfalls mit Alkoxycarbonyl substituiertes Cyclopropyl; Cyclobutyl; gegebenenfalls mit Hydroxyalkyl substituiertes Cyclopentyl; gegebenenfalls mit einem oder mehreren Alkylresten substituiertes Cyclohexyl; Cycloheptyl; Cyclohexen; Adamantyl; Pyridin, Thiophen, Indol, Furan, Naphtyl; Phenyl, das gegebenenfalls mit einem oder mehreren gleichen oder verschiedenen Resten substituiert ist, die ausgewählt sind aus: -(CH₂)_{q"}-X₄-Y₄, Hydroxy, Halogen und Cyan;
X₄ -O- oder eine kovalente Bindung darstellt;
Y₄ einen der Reste (C₁-C₆)-Alkyl, Di((C₁-C₆)-alkyl)amino oder Pyrrolidin darstellt; oder ein pharmazeutisch annehmbares Salz dieser Verbindung.

9. Verbindung der Formel I nach einem der Ansprüche 1 bis 8, **dadurch gekennzeichnet, dass** A -C(O)-darstellt; Y -NH- darstellt und
R₁ und R₂ unabhängig voneinander einen (C₁-C₈)-Alkylrest darstellen;
R₃ -(CH₂)ₚ-W₃-(CH₂)_{p'}-Z₃ darstellt
W₃ eine kovalente Bindung darstellt; Z₃ den Phenylrest darstellt, der mit einem oder mehreren gleichen oder verschiedenen Substituenten substituiert ist, die ausgewählt sind aus: -(CH₂)_{p"}-V₃-Y₃ und Halogen; V₃ -O- oder -S- darstellt; und Y₃ einen (C₁-C₆)-Alkylrest darstellt; p, p' und p" 0 darstellen;
R₄ einen Rest der Formel -(CH₂)ₛ-R"₄ darstellt;
R"₄ einen Rest der Formel -NW₄W'₄ darstellt;
W₄ ein Wasserstoffatom oder (C₁-C₈)-Alkyl darstellt;
W'₄ einen Rest der Formel -(CH₂)_{s'}-Q₄-Z₄ darstellt;
Q₄ eine kovalente Bindung darstellt;
Z₄ darstellt: ein Wasserstoffatom, (C₁-C₈)-Alkyl, das gegebenenfalls mit Hydroxy substituiert ist, (C₃-C₇)-Cycloalkyl, Heteroaryl, Aryl, das gegebenenfalls mit einem oder mehreren gleichen oder verschiedenen Resten substituiert ist, die aus der Formel -(CH₂)_{q"}-V₄-Y₄ ausgewählt sind;
V₄ -O- oder eine kovalente Bindung darstellt;
Y₄ einen Rest (C₁-C₆)-Alkyl oder Di((C₁-C₆)-alkyl)amino darstellt;
q" 0 darstellt; s eine ganze Zahl von 2 bis 4 und s' eine ganze Zahl von 1 bis 2 darstellt;
oder ein pharmazeutisch annehmbares Salz dieser Verbindung.

10. Verbindung der Formel I nach Anspruch 9, **dadurch gekennzeichnet, dass** das (C₃-C₇)-Cycloalkyl ausgewählt ist aus Cyclopentyl und Cyclohexyl, das Heteroaryl Pyridin und das Aryl Phenyl darstellt; oder ein pharmazeutisch annehmbares Salz dieser Verbindung.

11. Verbindung der Formel I nach einem der Ansprüche 1 bis 8, **dadurch gekennzeichnet, dass** A -C(O)-darstellt, Y ein Schwefelatom darstellt und
R₁ und R₂ unabhängig voneinander einen Rest (C₁-C₈)-Alkyl darstellen;
R₃ -(CH₂)ₚ-W₃-(CH₂)_{p'}-Z₃ darstellt
W₃ eine kovalente Bindung oder -C(O)- darstellt; Z₃ den Phenylrest darstellt, der mit einem oder mehreren gleichen oder verschiedenen Substituenten substituiert ist, die ausgewählt sind aus: -(CH₂)_{p"}-V₃-Y₃ und Halogen; V₃ -O- oder eine kovalente Bindung darstellt; und Y₃ einen Rest (C₁-C₆)-Alkyl oder Di((C₁-C₆)-alkyl)amino darstellt; p 1 darstellt und p' und p" 0 darstellen;
R₄ einen Rest der Formel -(CH₂)ₛ-R"₄ darstellt;
R"₄ einen Rest der Formel -NW₄W'₄ darstellt;
W₄ ein Wasserstoffatom oder (C₁-C₈)-Alkyl darstellt;
W'₄ einen Rest der Formel -(CH₂)_{s'}-Q₄-Z₄ darstellt;
Q₄ eine kovalente Bindung darstellt;
Z₄ ein Wasserstoffatom, (C₁-C₈)-Alkyl, Heteroaryl, Aryl darstellt
s eine ganze Zahl von 2 bis 4 darstellt und s' eine ganze Zahl von 1 bis 2 darstellt, oder ein pharmazeutisch annehmbares Salz dieser Verbindung.

12. Verbindung der Formel I nach Anspruch 11, **dadurch gekennzeichnet, dass** das Heteroaryl Pyridin und das Aryl Phenyl darstellt; oder ein pharmazeutisch annehmbares Salz dieser Verbindung.

13. Verbindung der Formel I nach Anspruch 1, **dadurch gekennzeichnet, dass** A -CH₂- darstellt, Y -NH- und R₁ und R₂ unabhängig voneinander einen Rest (C₁-C₆)-Alkyl darstellen; R₃ ein Phenyl darstellt, das mit einem oder mehreren gleichen oder verschiedenen Substituenten (C₁-C₆)-Alkoxy substituiert ist; R₄ einen Rest der Formel -(CH₂)ₛ-R"₄ darstellt; R"₄ einen Rest der Formel -NW₄W'₄ darstellt; W₄ (C₁-C₈)-Alkyl darstellt; W'₄ einen Rest der Formel -(CH₂)_{s'}-Q₄-Z₄ darstellt; Q₄ eine kovalente Bindung darstellt und Z₄ Pyridin darstellt; oder ein pharmazeutisch annehmbares Salz dieser Verbindung.

14. Eine Verbindung der Formel I, wie sie in einem der Ansprüche 1 bis 13 definiert ist, sowie die pharmazeutisch annehmbaren Additionssalze mit den mineralischen oder organischen Säuren dieser Verbindung der Formel I als Medikament.

15. Pharmazeugische Zusammensetzung, die als Wirkstoff mindestens ein Medikament, wie es in Anspruch 14 definiert ist, in Kombination mit einem pharmazeutisch annehmbaren Träger enthält.

16. Verwendung einer Verbindung nach einem der Ansprüche 1 bis 13 für die Herstellung eines Medikaments für die Behandlung der Endometriose, des Fibroms, des Syndroms der polyzystischen Eierstöcke, des Krebses der Brust, der Eierstöcke und der Gebärmutter, der gonadotropen hypophysären Desensibilisierung bei Protokollen der künstlichen Befruchtung bei Frauen.

17. Verwendung einer Verbindung nach einem der Ansprüche 1 bis 13 für die Herstellung eines Medikaments für die Behandlung der benignen Hyperplasie der Prostata und des Prostatakrebses bei Männern.

18. Verwendung einer Verbindung nach einem der Ansprüche 1 bis 13 für die Herstellung eines Medikaments für die Behandlung der vorzeitigen männlichen oder weiblichen Pubertät.
